# EUROPEAN PATENT SPECIFICATION

(11) **EP 2 270 208 B1**
(45) Date of publication and mention of the grant of the patent: **09.05.2012**
(21) Application number: 10187881.7
(22) Date of filing: 13.02.2007
(51) Int. Cl.: C12Q 1/68, C40B 40/06, C12N 15/10

(54) **Plant chimeric binding polypeptides for universal molecular recognition**
Chimäre bindende Polypeptide aus Pflanzen für universelle Molekülerkennung
Polypeptides se liant à des plantes chimériques pour une reconnaissance moléculaire universelle

(30) Priority: 13.02.2006 US 773086 P
(43) Date of publication of application: 05.01.2011
(62) Divisional of application: 07750756.4
(73) Proprietor: Divergence, Inc., St. Louis, MO 63132 (US)
(72) Inventor: Jones, Jennifer, Kirkwood, MO 63122 (US)
(74) Representative: Peterreins, Frank

(56) References cited:
- WO-A-02/20565
- HOSSE RALF J ET AL: "A new generation of protein display scaffolds for molecular recognition", PROTEIN SCIENCE, vol. 15, no. 1, January 2006 (2006-01), pages 14-27, XP002541836, ISSN: 0961-8368
- JAEGER DE G ET AL: "THE PLANTIBODY APPROACH: EXPRESSION OF ANTIBODY GENES IN PLANTS TO MODULATE PLANT METABOLISM OR TO OBTAIN PATHOGEN RESISTANCE", PLANT MOLECULAR BIOLOGY, SPRINGER, DORDRECHT, NL, vol. 43, no. 4, 1 July 2000 (2000-07-01), pages 419-428, XP000995953, ISSN: 0167-4412, DOI: DOI:10.1023/A:1006471528756

## Description

### BACKGROUND

The binding specificity and affinity of a protein for a target are determined primarily by the protein's amino acid sequence within one or more binding regions. Accordingly, varying the amino acid sequence of the relevant regions reconfigures the protein's binding properties.

In nature, combinatorial changes in protein binding are best illustrated by the vast array of immunoglobulins produced by the immune system. Each immunoglobulin includes a set of short, virtually unique, amino acid sequences known as hypervariable regions (i.e., protein binding domains), and another set of longer, invariant sequences known as constant regions. The constant regions form β sheets that stabilize the three dimensional structure of the protein in spite of the enormous sequence diversity among hypervariable regions in the population of immunoglobulins. Each set of hypervariable regions confers binding specificity and affinity. The assembly of two heavy chain and two light chain immunoglobulins into a large protein complex (i.e., an antibody) further increases the number of combinations with diverse binding activities.

The binding diversity of antibodies has been successfully exploited in many biomedical and industrial applications. For example, libraries have been constructed that express immunoglobulins bearing artificially diversified hypervariable regions. Immunoglobulin expression libraries are very useful for identifying high affinity antibodies to a target molecule (e.g., a receptor or receptor ligand). A nucleic acid encoding the identified immunoglobulin can then be isolated and expressed in host cells or organisms.

However, despite the usefulness of immunoglobulins and antibodies in general, their expression in transgenic plants can be problematic. Immunoglobulins may not fold properly in plant cytoplasm because they require the formation of multiple disulfide bonds. Further, the large size of immunoglobulins prevents their effective uptake by some plant pests. Thus, immunoglobulins are frequently not useful as protein pesticides or pesticide targeting molecules. Finally, expressing mammalian proteins such as immunoglobulins (e.g., as so called "plantibodies") in edible plants also raises potential issues of consumer acceptance and is thus an impediment to commercialization. This may effectively prevent use of plantibodies for many input and output traits in transgenic plants.

A review on plantibodies is provided in Jaeger De G. et al. (2000) PLANT MOLECULAR BIOLOGY, vol. 43, no. 4, pages 419-428.

The above-mentioned disadvantages of immunoglobulins can be circumvented by generating diverse libraries of binding proteins from other classes of structurally tolerant proteins, preferably plant-derived proteins. These libraries can be screened to identify individual proteins that bind with desired specificity and affinity to a target of interest. Afterwards, identified binding proteins can be efficiently expressed in transgenic plants.

### SUMMARY

Diverse libraries of nucleic acids encoding plant chimeric binding polypeptides, as well as methods for generating them are described herein. The chimeric binding polypeptides are conceptually analogous to immunoglobulins in that they feature highly varied binding domains in the framework of unvarying sequences that encode a structurally robust protein. However, the chimeric binding polypeptides described herein have the considerable advantage of being derived from plant protein sequences thereby avoiding many of the problems associated with immunoglobulin expression in plants. The amino acid sequences of the encoded plant chimeric binding proteins are derived from a scaffold polypeptide sequence that includes subsequences to be varied. The varied subsequences correspond to putative binding domains of the plant chimeric binding polypeptides, and are highly heterogeneous in the library of encoded plant chimeric binding proteins. In contrast the sequence of the encoded chimeric binding proteins outside of the varied subsequences is essentially the same as the parent scaffold polypeptide sequence and highly homogeneous throughout the library of encoded plant chimeric binding proteins. Such libraries can serve as a universal molecular recognition platform to select proteins with high selectivity and affinity binding for expression in transgenic plants.

Accordingly, one aspect described herein is a library of nucleic acid molecules encoding at least ten (e.g., at least 1,000, 10⁵, or 10⁶) different chimeric binding polypeptides. The amino acid sequence of each polypeptide includes C₁-X₁-C₂-X₂-C₃-X₃-C₄, where C₁-C₄ are backbone subsequences selected from purple acid phosphatase (i.e., SEQ ID NOs: 1-30, 31-60, 61-90, and 91-120, respectively) that can include up to 30 (e.g., 20, 10, or 5) single amino acid substitutions, deletions, insertion, or additions to the selected purple acid phosphatase sequences. The C₁-C₄ subsequences are homogeneous across many of the polypeptides encoded in the library. In contrast to the C₁-C₄ backbone subsequences, the X₁-X₃ subsequences are independent variable subsequences consisting of 2-20 amino acids, and these subsequences are heterogeneous across many of the polypeptides in the library. For example, the library of chimeric polypeptides can have the amino acid sequence of any one of SEQ ID NOs:124-126 including one to ten single amino acid substitutions, deletions, insertions, or additions to amino acid positions corresponding to 23-39, 51-49, and 79-84 of SEQ ID NOs:124-126.

Another aspect described herein is a method for generating the just-described library. The method includes providing a parental nucleic acid encoding a plant scaffold polypeptide sequence containing C₁-X₁-C₂-X₂-C₃-X₃-C₄ as defined above. The method further includes replicating the parental nucleic acid (e.g., at least one of the X₁-X₃ subsequences is selected from SEQ ID NOs: 121-123) under conditions that introduce up to 10 single amino acid substitutions, deletions, insertions, or additions to the parental X₁, X₂, or X₃ subsequences, whereby a heterogeneous population of randomly varied subsequences encoding X₁, X₂, or X₃ is generated. The population varied subsequences is then substituted into a population of parental nucleic acids at the positions corresponding to those encoding X₁, X₂, or X₃. The amino acid substitutions, deletions, insertions or additions can be introduced into the parental nucleic acid subsequences by replication *in vitro* (e.g., using a purified mutagenic polymerase or nucleotide analogs) or *in vivo* (e.g., in a mutagenic strain of *E*. *coli).* The just-described library can be introduced into a biological replication system (e.g., *E*. *coli* or bacteriophage) and amplified.

A related aspect described herein is another method for generating the above-described library of nucleic acids. The method includes selecting an amino acid sequence containing C₁-X₁-C₂-X₂-C₃-X₃-C₄ as defined above. The method further includes providing a first and second set of oligonucleotides having overlapping complementary sequences. Oligonucleotides of the first set encode the C₁-C₄ subsequences and multiple heterogeneous X₁-X₃ subsequences. Oligonucleotides of the second set are complementary to nucleotide sequences encoding the C1-C4 subsequences and multiple heterogeneous X₁-X₃ subsequences. The two sets of oligonucleotides are combined to form a first mixture and incubated under conditions that allow hybridization of the overlapping complementary sequences. The resulting hybridized sequences are then extended to form a second mixture containing the above-described library.

Yet another aspect of the invention is a library of nucleic acids encoding chimeric binding polypeptides each of which include an amino acid sequence at least 70% (i.e., any percentage between 70% and 100%) identical to any of SEQ ID NOs: 127-129. The amino acid sequence of each of the encoded polypeptides includes amino acids that differ from those of SEQ ID NOs: 127-129 in at least one of positions 13, 14, 33, 35-36, 38, 46-47, 66, 68-69, 71, 79, 80, 99, 101-102, 104, and 112-113 and the amino acid differences are heterogeneous across a plurality of the encoded polypeptides. The amino acid sequence of each of the encoded polypeptides outside of the above-listed positions is homogeneous across a plurality of the encoded chimeric polypeptides.

A related aspect described herein is a method for generating the just-described library. The method includes selecting an amino acid sequence corresponding to any of SEQ ID NOs: 127-129, in which the selected sequence differs from SEQ ID NOs:127-129 in at least one the above-mentioned positions. The method further includes providing a first and second set of oligonucleotides having overlapping complementary sequences. Oligonucleotides of the first set encode subsequences of the selected amino acid sequence, the subsequences being heterogeneous at the above-mentioned positions. Oligonucleotides of the second set are complementary to nucleotide sequences encoding subsequences of the selected amino acid sequence, the subsequences being heterogeneous at the above-mentioned positions. The two sets of oligonucleotides are combined to form a first mixture and incubated under conditions that allow hybridization of the overlapping complementary sequences. The resulting hybridized sequences are then extended to form a second mixture containing the above-described library.

Various implementations of the invention can include one or more of the following. For example, each nucleic acid in a library can include a vector sequence. Also featured is any nucleic acid isolated from one of the above-described libraries, as well as the chimeric binding polypeptide encoded by it, in pure form.

In one implementation, a population of cells (or individual cells selected from the population of cells) is provided which express chimeric binding polypeptides encoded by one of the libraries. Another implementation features a library of purified chimeric binding polypeptides encoded by one the nucleic acid libraries. Yet another implementation provides a population of filamentous phage displaying the chimeric binding polypeptides encoded by one of the nucleic acid libraries.

In various implementations of methods for generating the above described nucleic acid libraries by oligonucleotide assembly, one or more of the following can be included. For example, the method can further include, after the second mixture that contains the nucleic acid library is generated, performing a cycle of denaturing the population of nucleic acids followed by a hybridization and an elongation step. Optionally, this cycle can be repeated (e.g., up to 100 times). The nucleic acid libraries can be amplified by a polymerase chain reaction that includes a forward and a reverse primer that hybridize to the 5' and 3' end sequences, respectively, of all nucleic acids in the library. In one implementation, amino acids to be encoded in variable sequence positions are selected from a subset (e.g., only 4, 6, 8, 10, 12, 14 or 16) of alanine, arginine, asparagine, aspartate, glutamine, glutamate, glycine, histidine, isoleucine, leucine, lysine, methionine, phenylalanine, proline, serine, threonine, tryptophan, tyrosine, cysteine and valine (the 20 naturally occurring amino acids). In other cases 19 of the 20 are used (excludes cysteine). In other cases all 20 are used. In another implementation, the subset of amino acids includes at least one aliphatic, one acidic, one neutral, and one aromatic amino acid (e.g., alanine, aspartate, serine, and tyrosine).

Described herein is library of nucleic acids encoding at least ten different polypeptides, the amino acid sequence of each polypeptide comprising: C1-X1-C2-X2-C3-X3-C4, wherein: (i) subsequence C1 is selected from SEQ. ID NOs:1-30, subsequence C2 is selected from SEQ ID NOs:31-60, subsequence C3 is selected from SEQ. ID NOs:61-90; subsequence C4 is selected from SEQ. ID NOs:91-120, and each of C1-C4 comprise up to 10 single amino acid substitutions, deletions, insertions, or additions to the selected subsequence; (ii) C1-C4 are homogeneous across a plurality of the encoded polypeptides; (iii) each of X1-X3 is an independently variable subsequence consisting of 2-20 amino acids; and each of X1-X3 are heterogeneous across a plurality of the encoded polypeptides.

Also described is a library of nucleic acids encoding at least ten different polypeptides, the amino acid sequence of each polypeptide comprising:

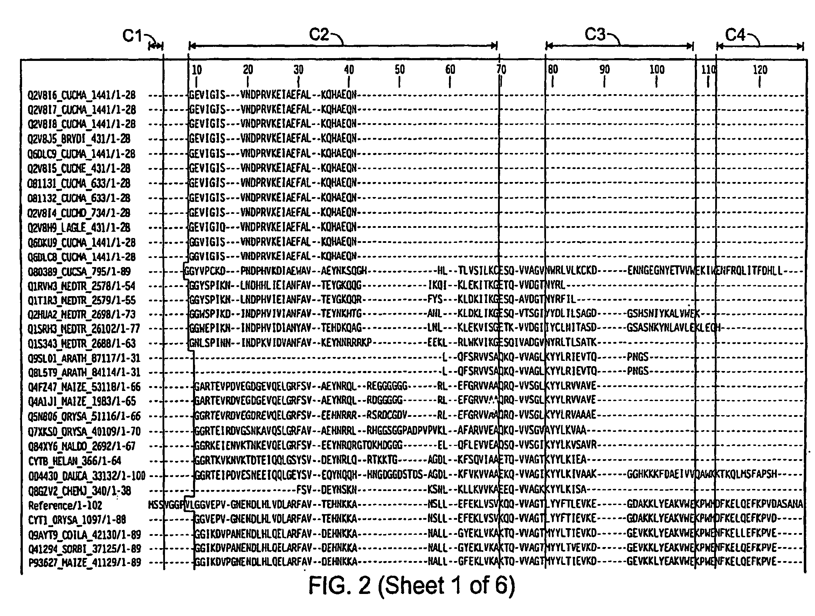

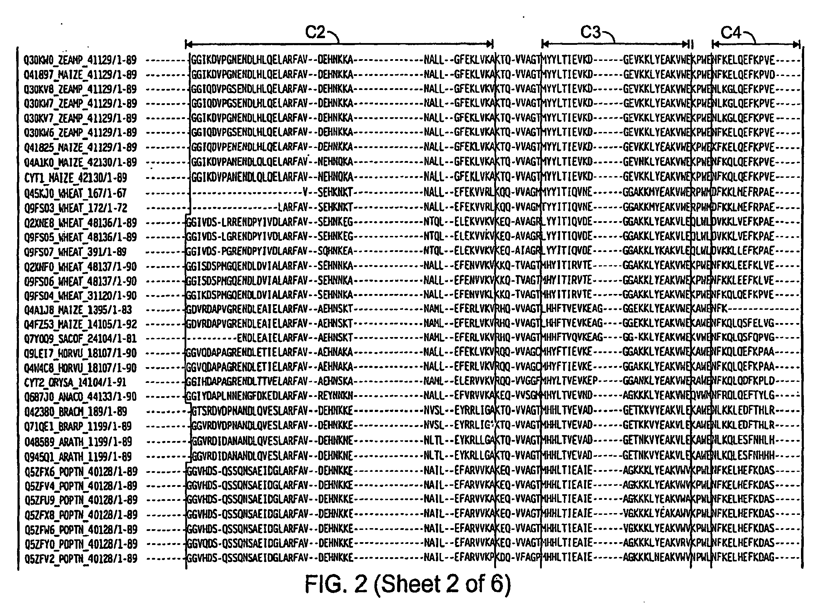

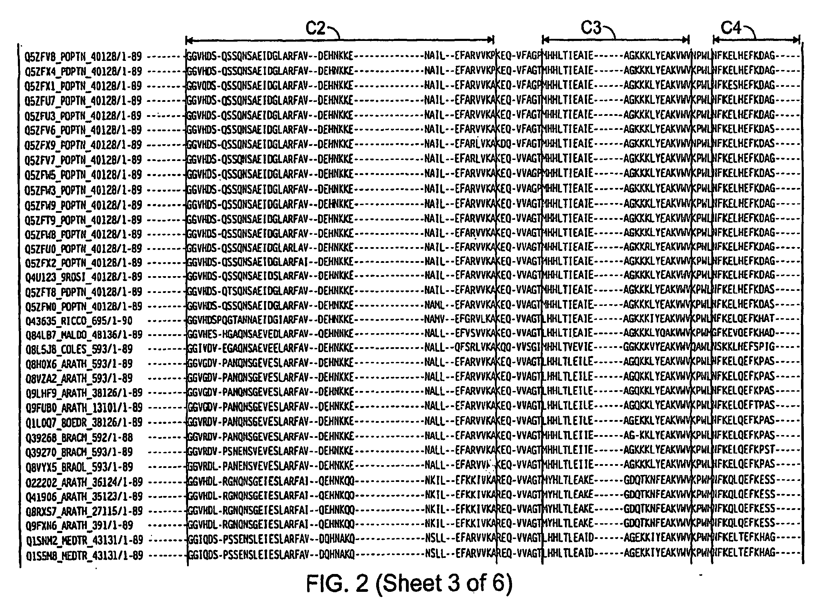

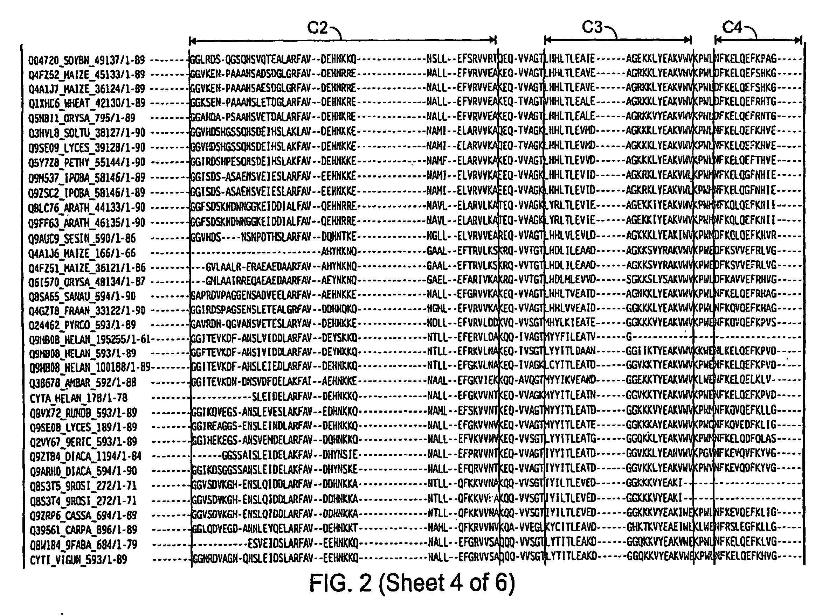

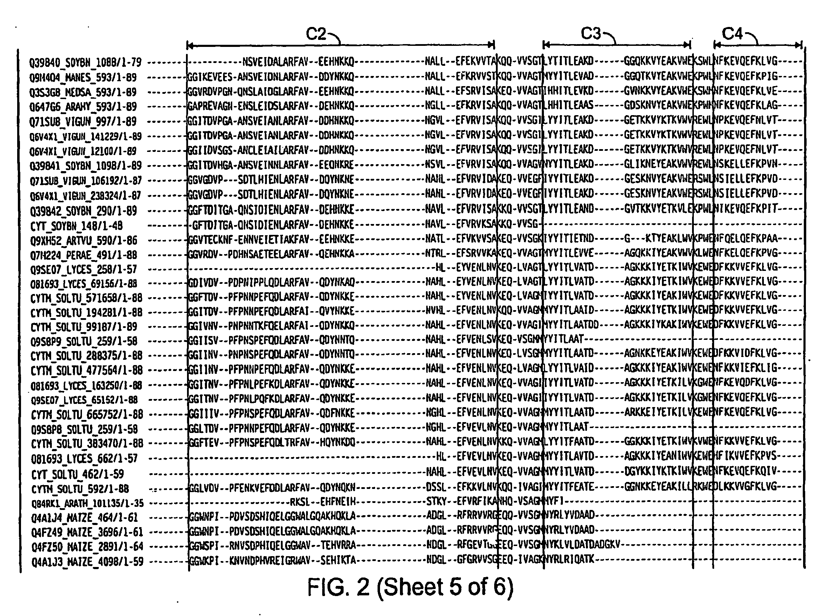

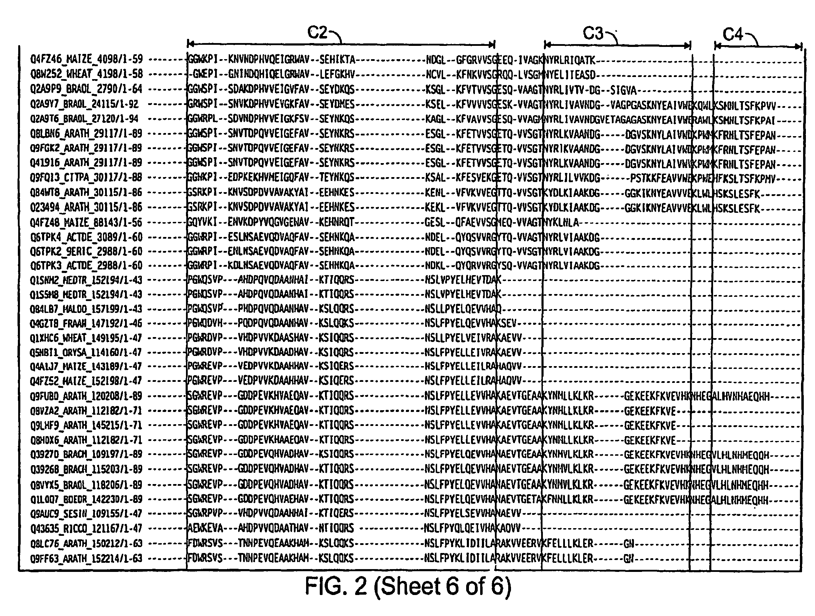

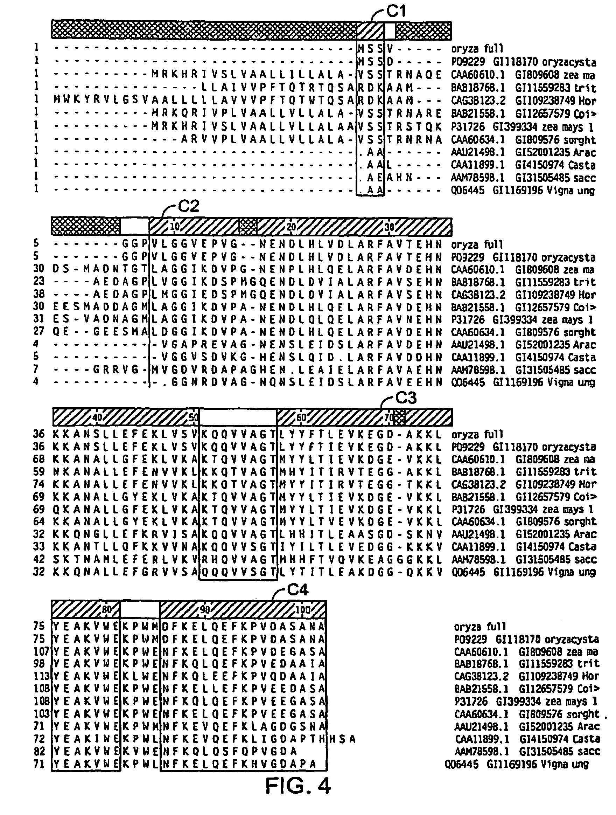

C1-X1-C2-X2-C3-X3-C4, wherein: (i) subsequence C1 is selected from FIG. 2 or FIG. 4, subsequence C2 is selected from FIG 2 or FIG 4, subsequence C3 is selected from FIG. 2 or FIG. 4; subsequence C4 is selected from FIG. 2 or FIG. 4, and each of C1-C4 comprise up to 10 single amino acid substitutions, deletions, insertions, or additions to the selected subsequence; (ii) C1-C4 are homogeneous across a plurality of the encoded polypeptides (iii) each of X1-X3 is an independently variable subsequence consisting of 2-20 amino acids; and each of X1-X3 are heterogeneous across a plurality of the encoded polypeptides.

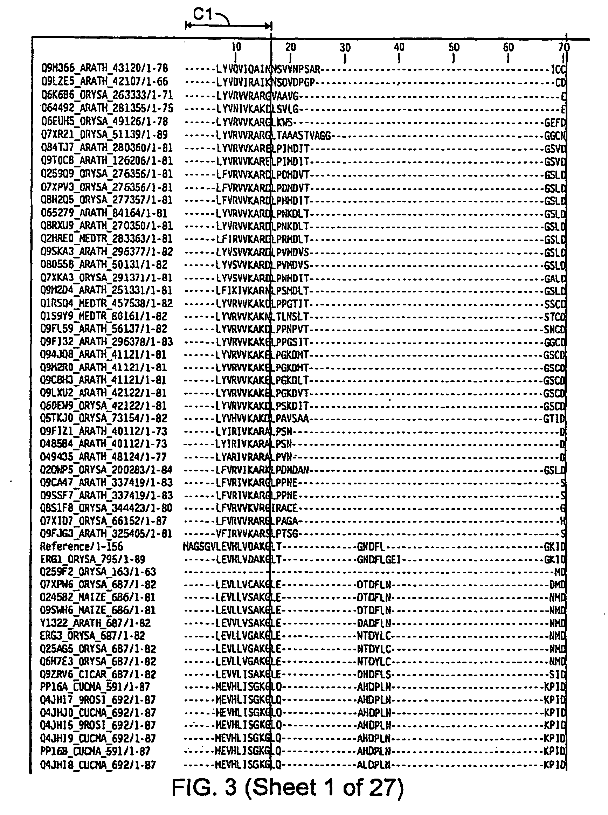

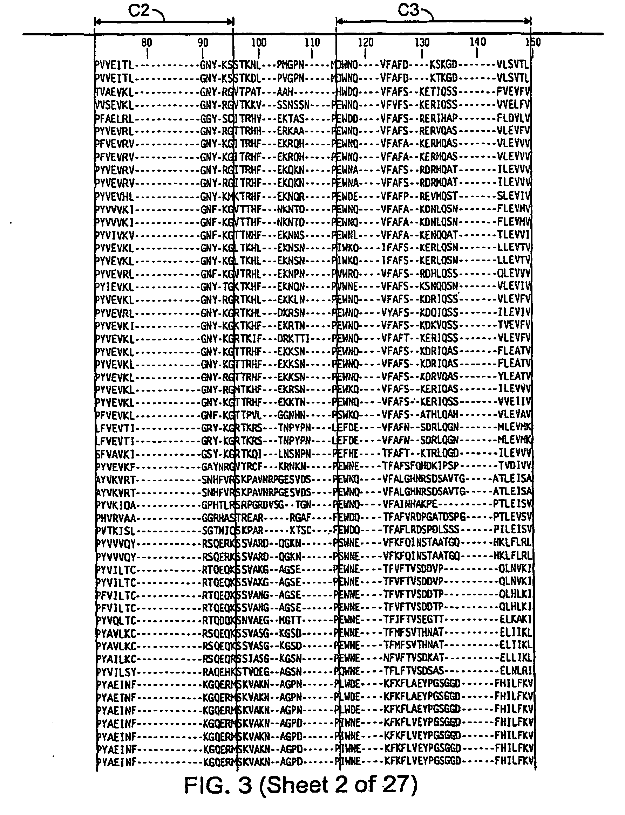

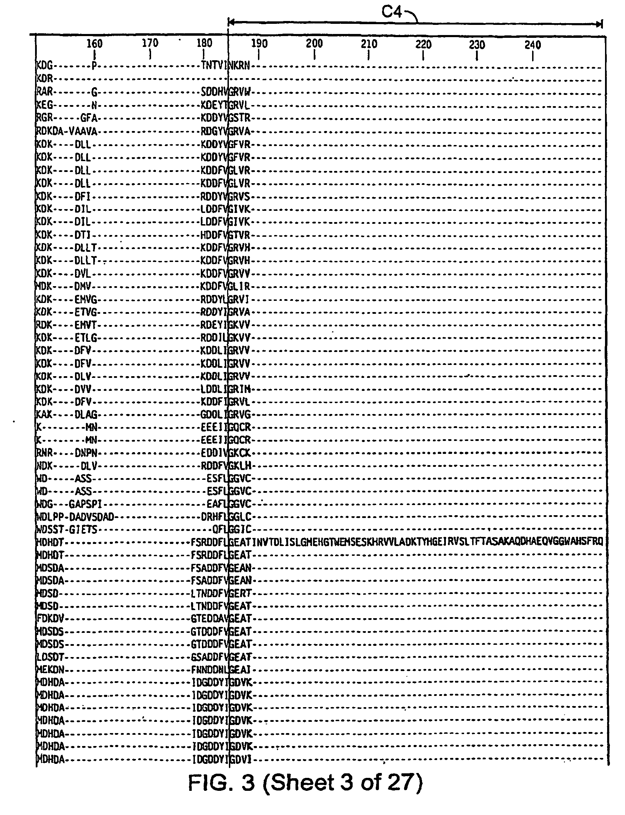

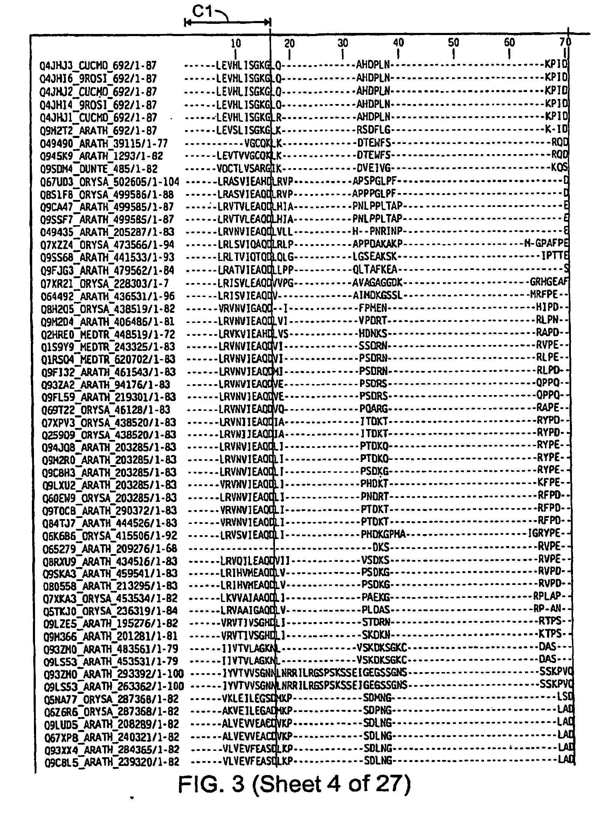

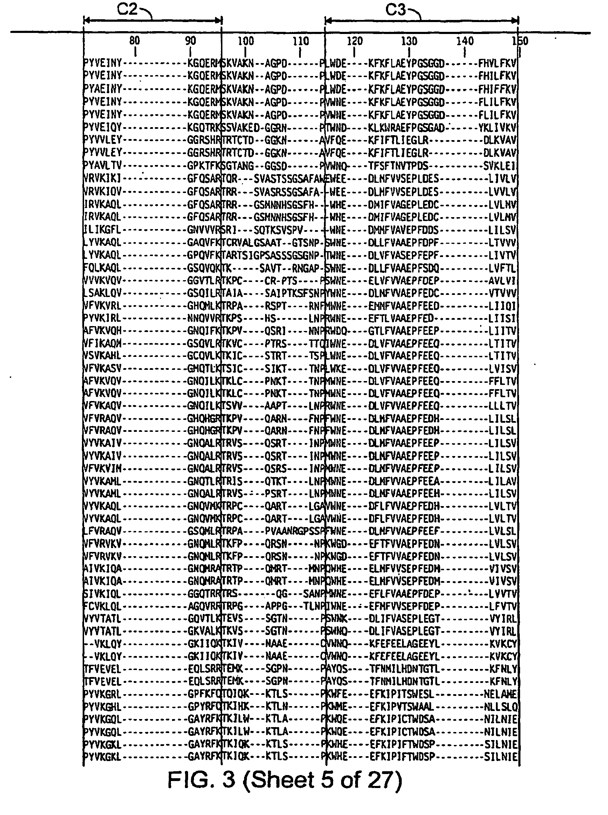

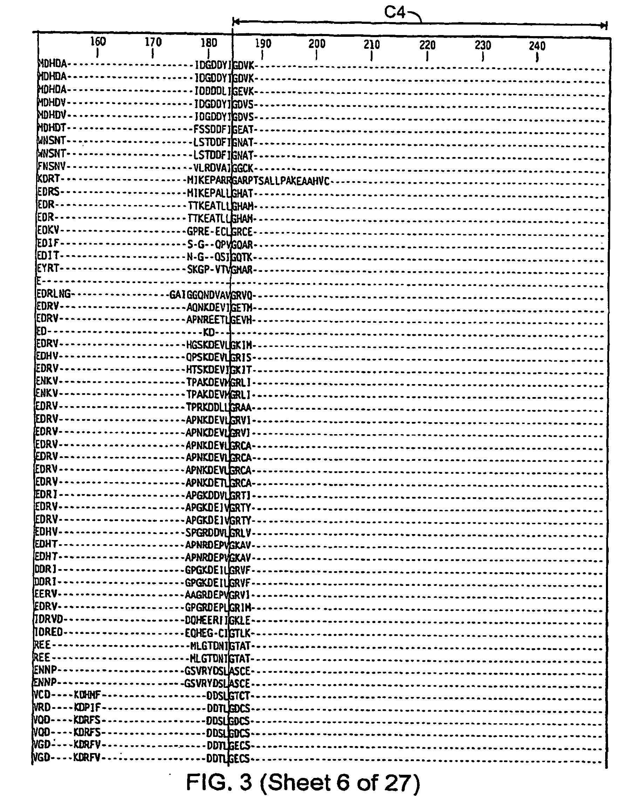

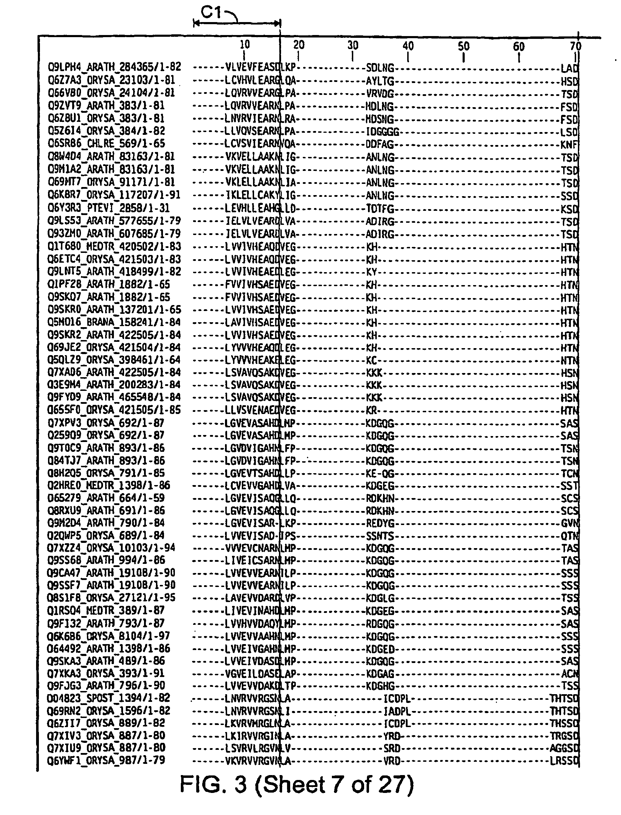

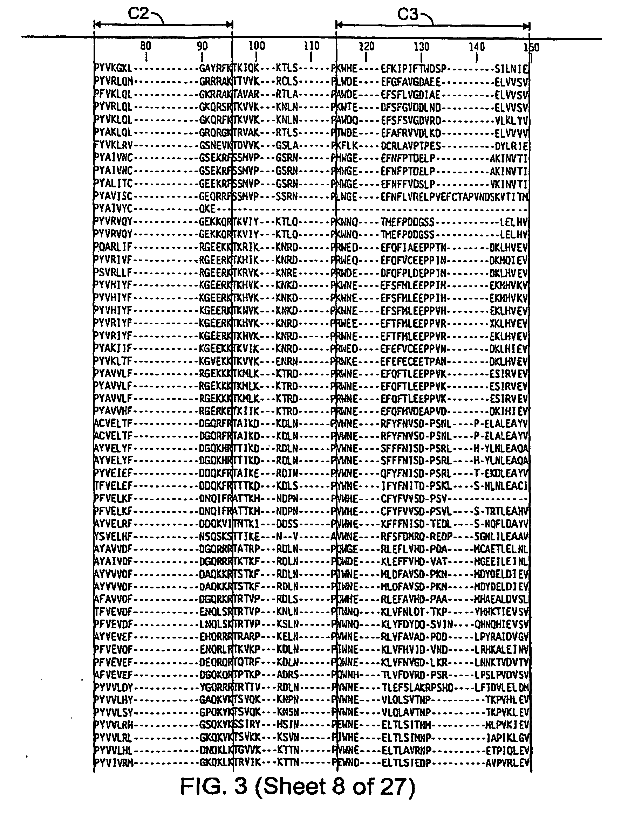

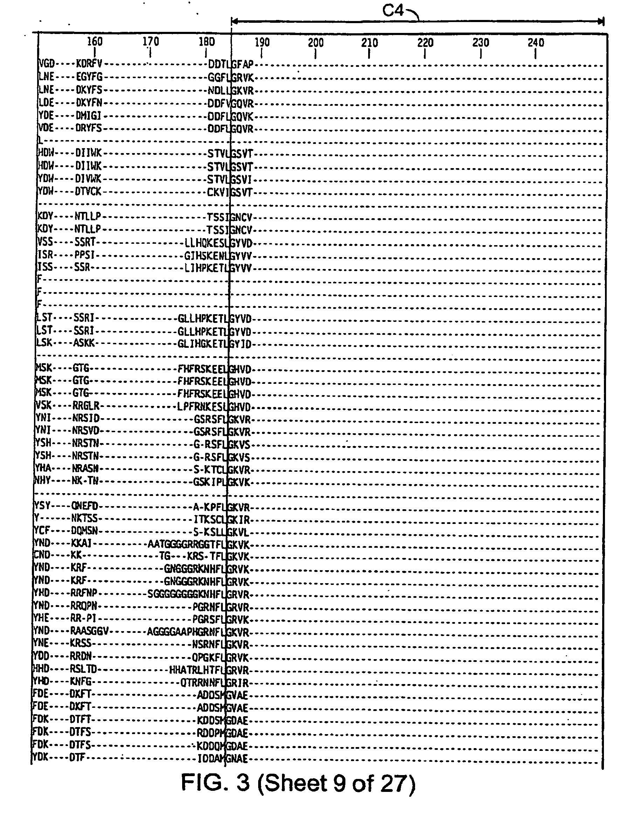

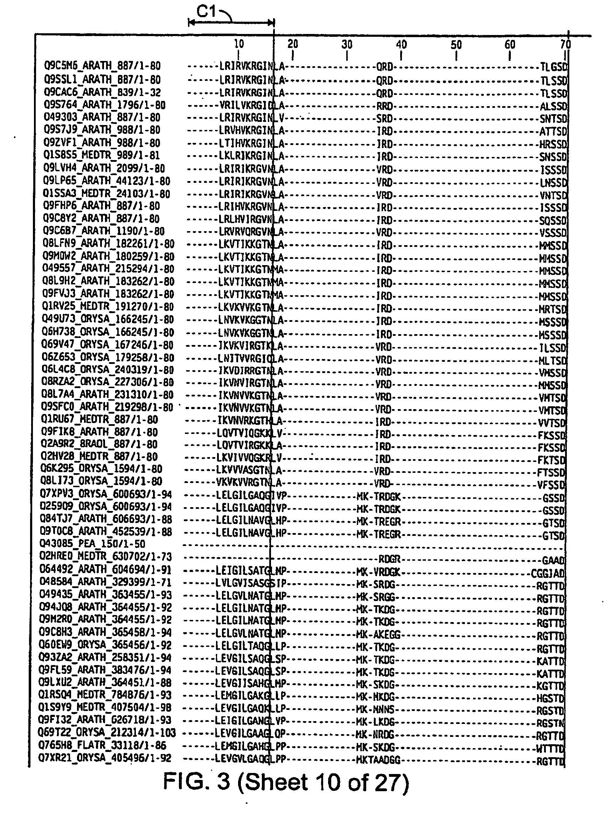

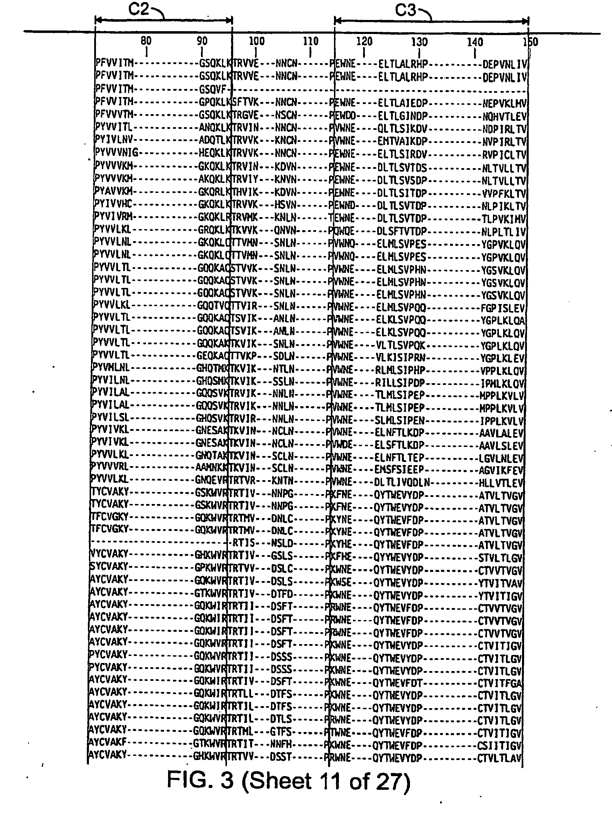

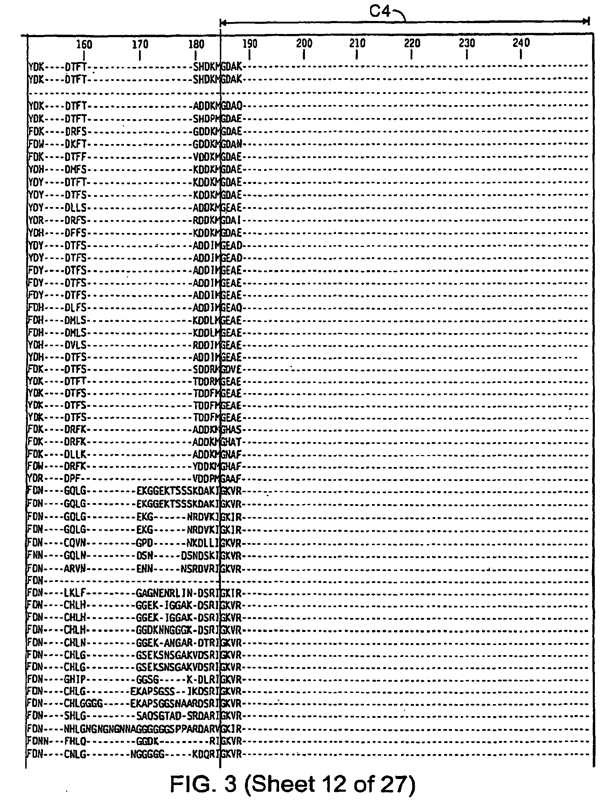

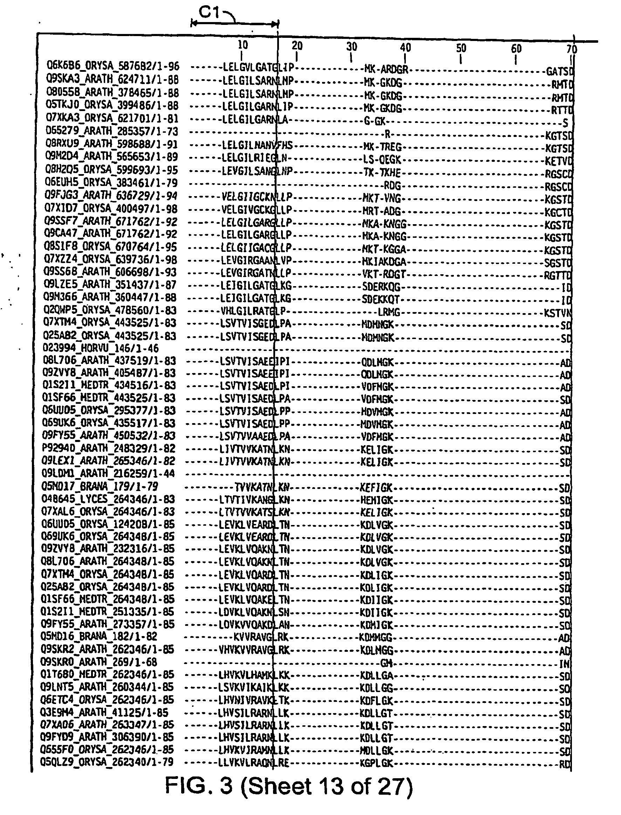

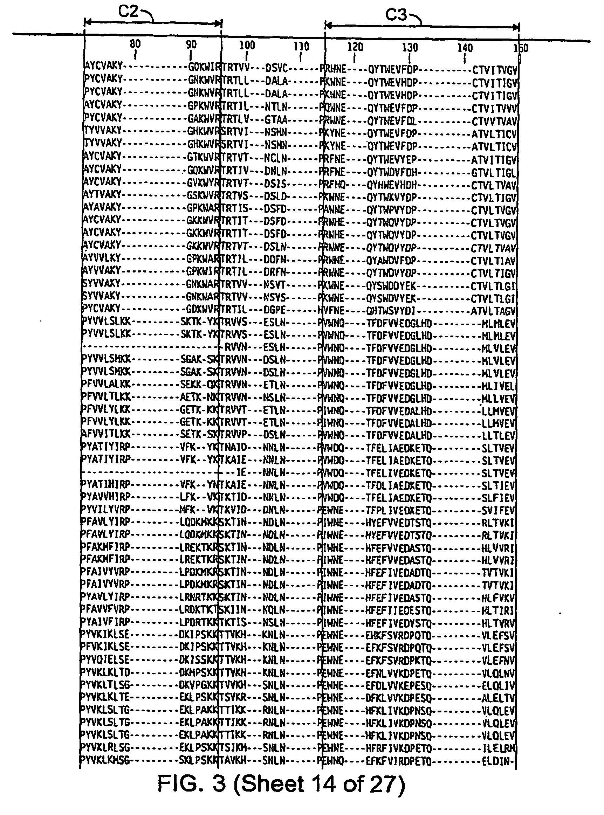

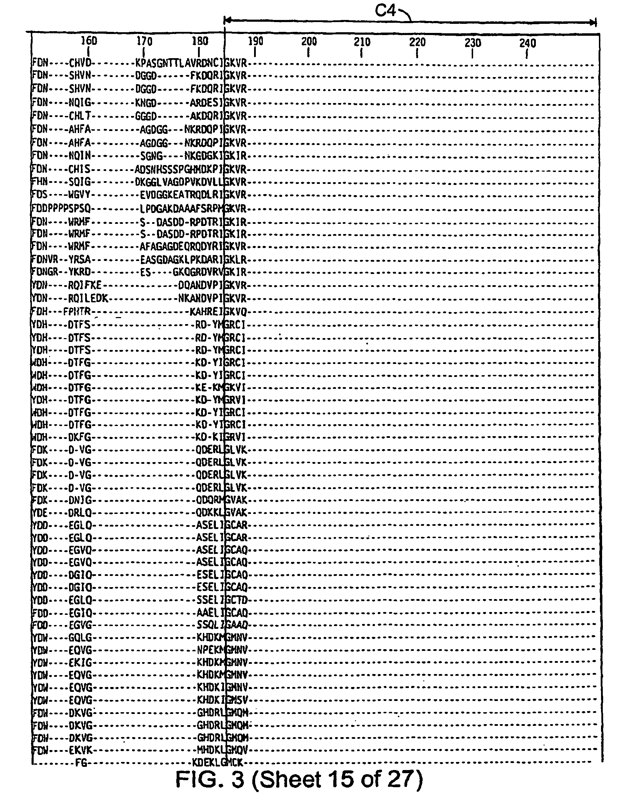

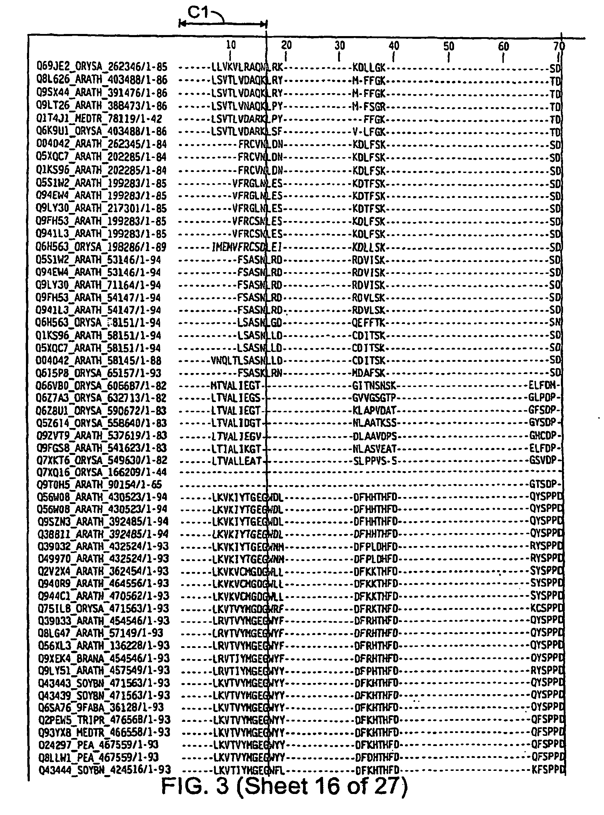

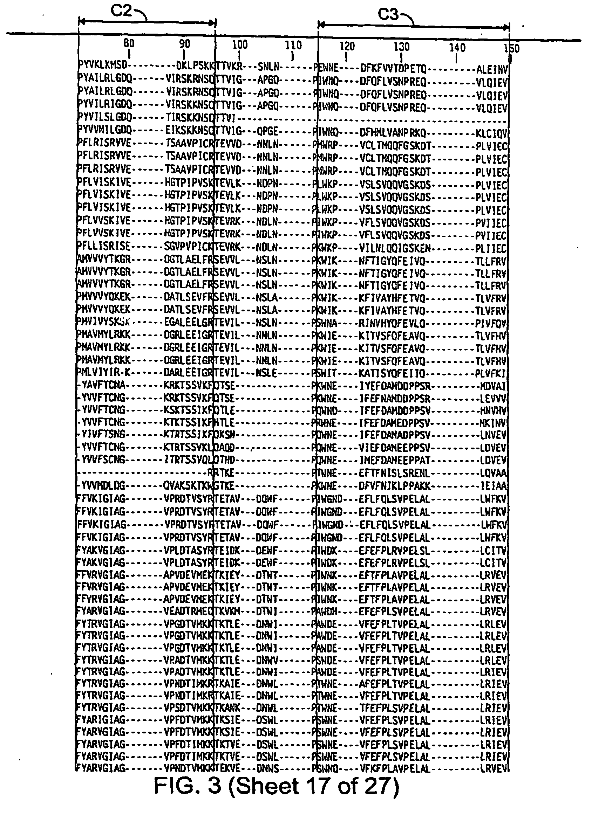

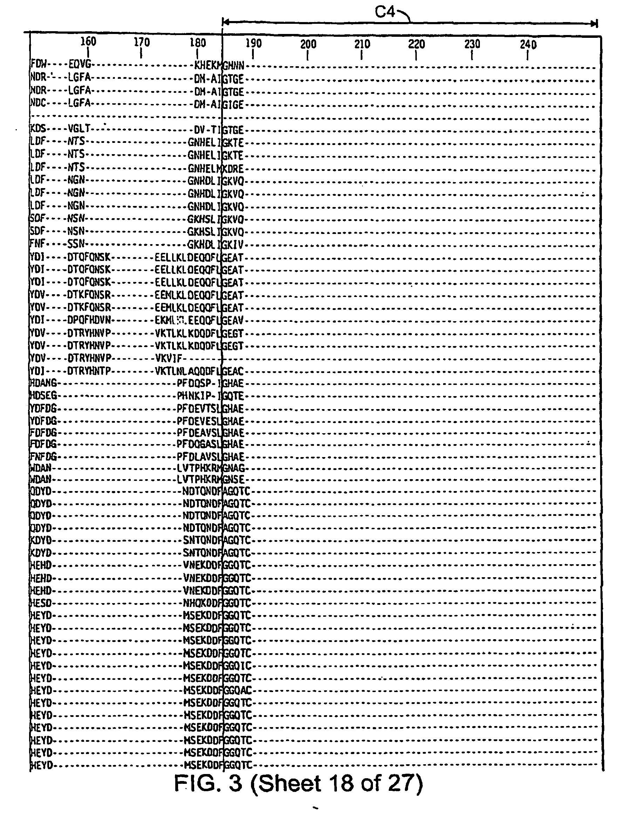

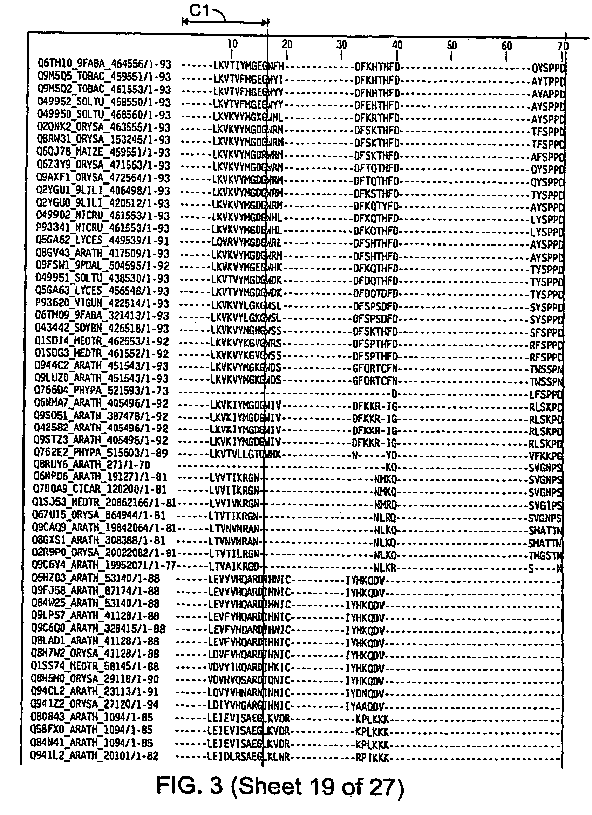

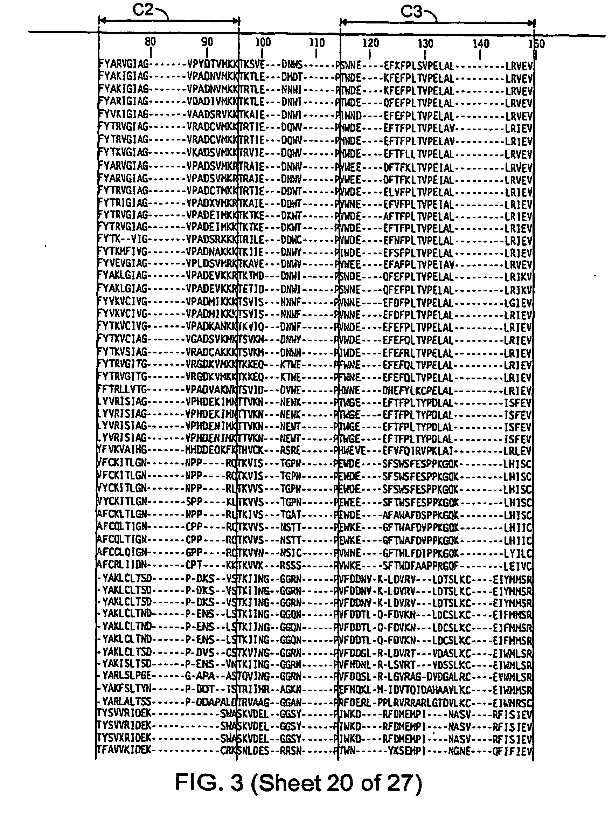

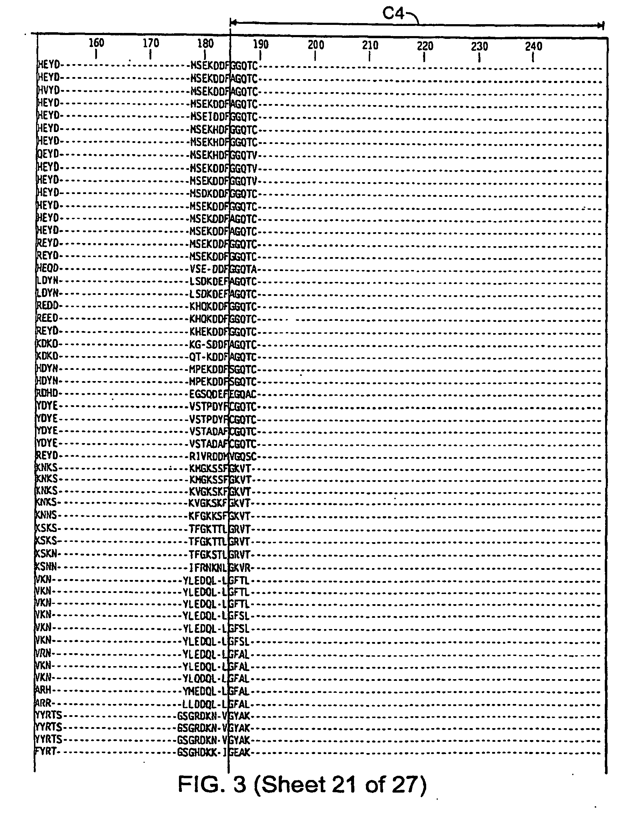

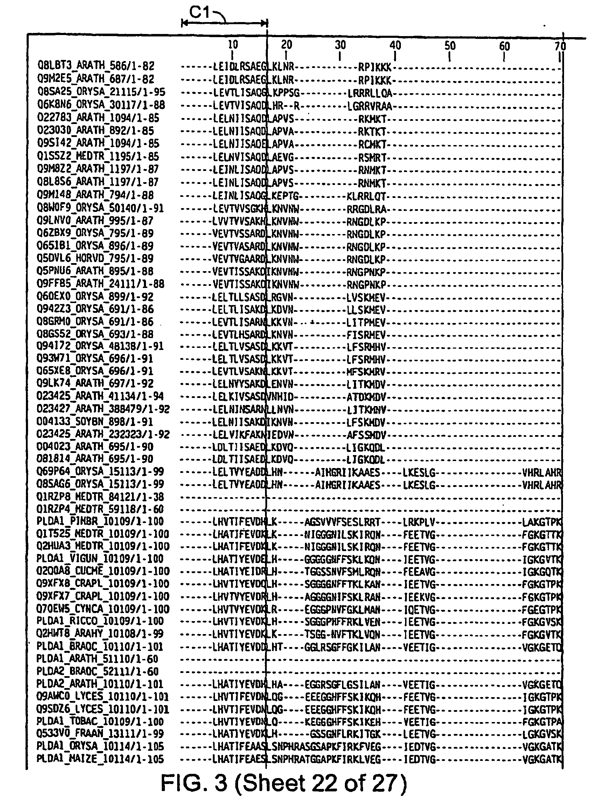

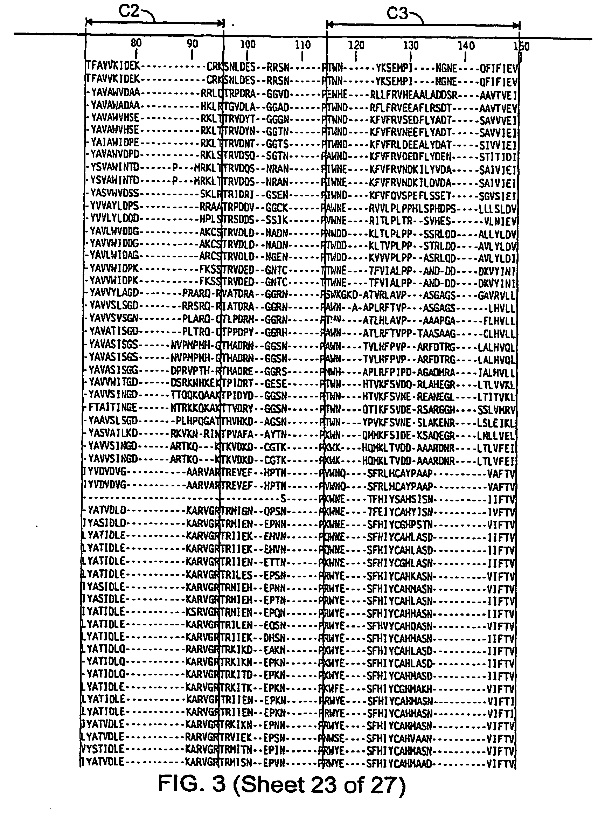

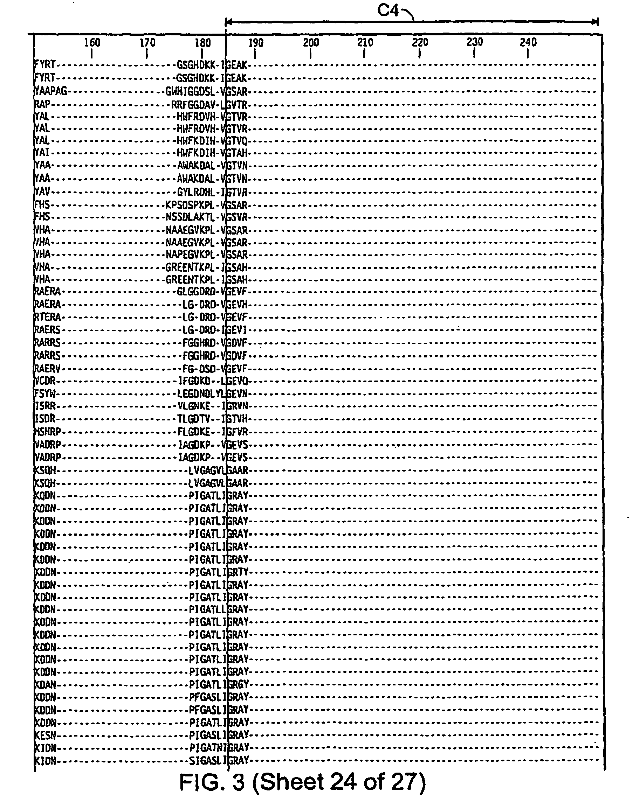

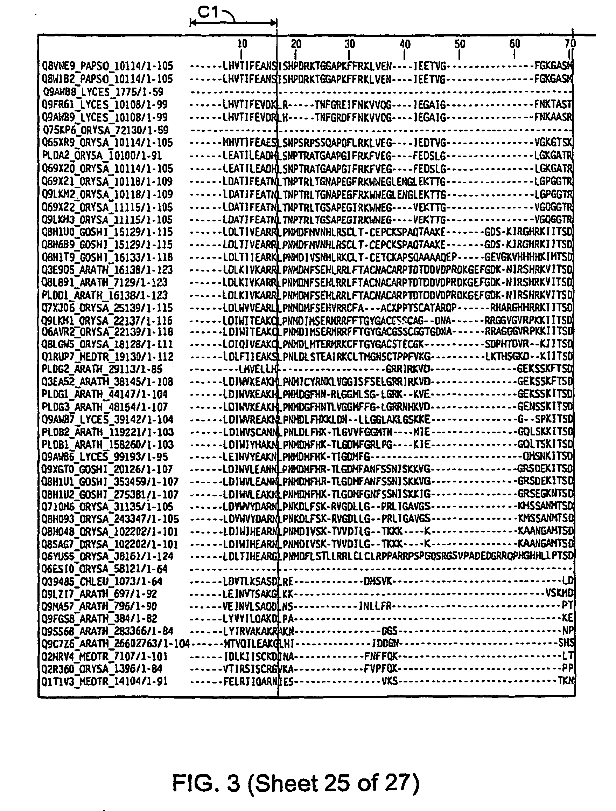

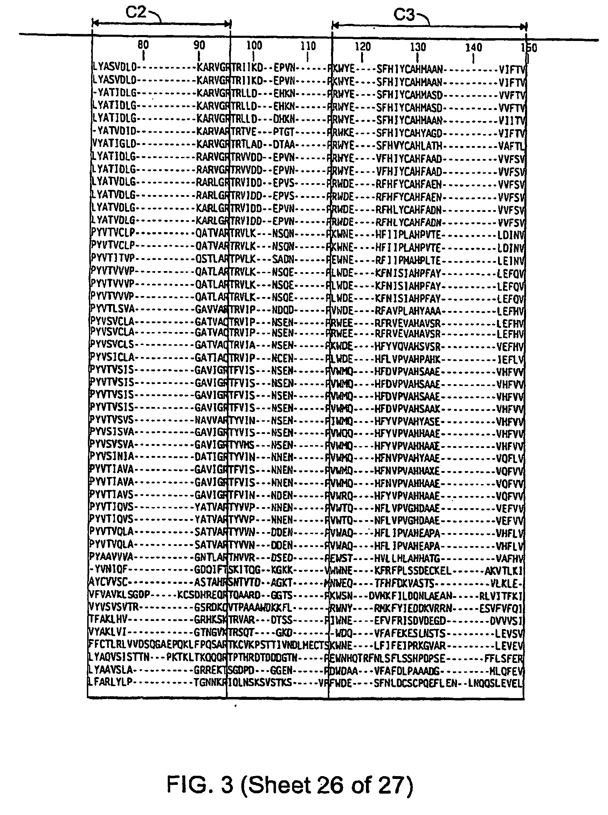

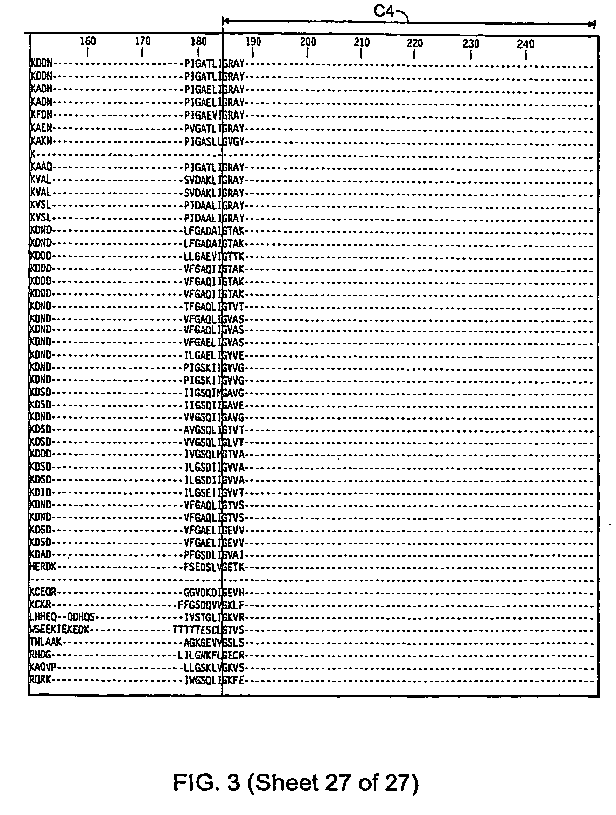

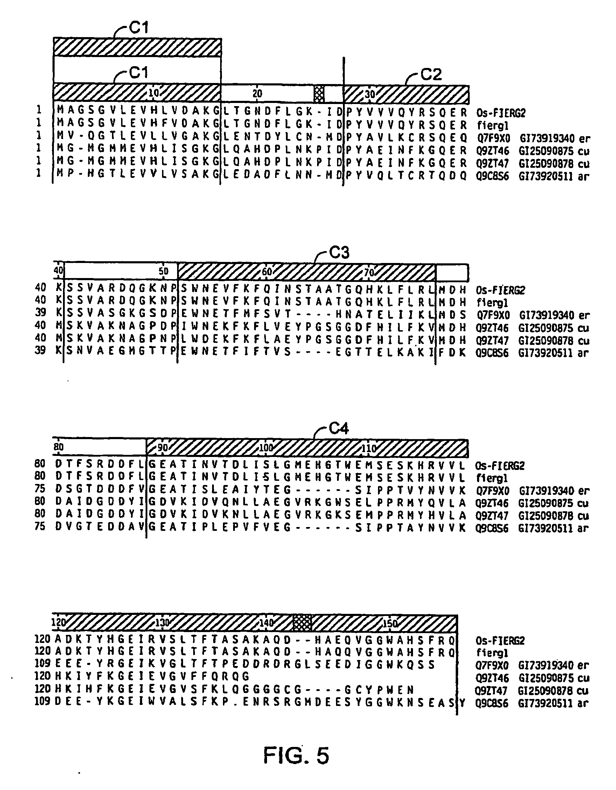

Also described is a library of nucleic acids encoding at least ten different polypeptides, the amino acid sequence of each polypeptide comprising:
C1-X1-C2-X2-C3-X3-C4, wherein (i) subsequence C1 is selected from FIG. 3 or FIG 5, subsequence C2 is selected from FIG 3 or FIG 5, subsequence C3 is selected from FIG 3 or FIG. 5; subsequence C4 is selected from FIG. 3 XX, and each of C1-C4 comprise up to 30 single amino acid substitutions, deletions, insertions, or additions to the selected subsequence; (ii) C1-C4 are homogeneous across a plurality of the encoded polypeptides (iii) each of X1-X3 is an independently variable subsequence consisting of 2-20 amino acids; and each of X1-X3 are heterogeneous across a plurality of the encoded polypeptides.

In various embodiments: at least 1,000 different polypeptides are encoded; at least 100,000 different polypeptides are encoded; at least 1,000,000 different polypeptides are encoded; each of C1-C4 independently comprises up to 20 single amino acid substitutions, deletions, insertions, or additions to the selected subsequence; each of C1-C4 independently comprises up to 10 single amino acid substitutions, deletions, insertions, or additions to the selected subsequence; each of C1-C4 independently comprises up to 5 single amino acid substitutions, deletions, insertions, or additions to the selected subsequence; none of C1-C4 comprise amino acid substitutions, deletions, insertions, or additions to the selected subsequence; amino acids of X1-X3 are selected from fewer than 20 amino acids genetically encoded in plants; amino acids of X1-X3 are selected from all 20 amino acids genetically encoded in plants; the fewer than 20 genetically encoded amino acids include at least one aliphatic amino acid, at least one acidic amino acid, at least one neutral amino acid, and at least one aromatic amino acid; fewer than 20 genetically encoded amino acids comprise alanine, aspartate, serine, and tyrosine.

In some cases: the amino acid sequence of each polypeptide is selected from:
(a). a polypeptide comprising C1-X1-C2-X2-C3-X3-C4 wherein C1= SEQ. ID NO:1, C2= SEQ. ID NO: 31, C3= SEQ. ID NO: 61, and C4= SEQ. ID NO: 91;
(b). a polypeptide comprising C1-X1-C2-X2-C3-X3-C4 wherein C1= SEQ. ID NO:2, C2= SEQ. ID NO: 32, C3= SEQ. ID NO: 62, and C4= SEQ. ID NO: 92; and
(c). a polypeptide comprising C1-X1-C2-X2-C3-X3-C4 wherein C1= SEQ. ID NO:3, C2= SEQ. ID NO: 33, C3= SEQ. ID NO: 63, and C4= SEQ. ID NO: 93.

In some cases: each encoded polypeptide comprises C1-X1-C2-X2-C3-X3-C4, wherein C1= SEQ. ID NO: X1, C2= SEQ. ID NO: X2, C3= SEQ. ID NO: X3, and C4= SEQ. ID NO: X4; designated SEQ. ID NO: 130.

In some cases: each encoded polypeptide comprises C1-X1-C2-X2-C3-X3-C4, wherein C1= SEQ. ID NO: X1, C2= SEQ. ID NO: X2, C3= SEQ. ID NO: X3, and C4= SEQ. ID NO: X4; designated SEQ. ID NO: 130.

In some embodiments: wherein each of the nucleic acids comprises a vector sequence.

Also described: are an isolated nucleic acid selected from the library and a isolated cell expressing the nucleic acid as well as a purified library of purified polypeptides encoded by the library; and a population of filamentous phage displaying the polypeptides encoded by the library.

Described herein is a method of generating a library, comprising: (i) providing a parental nucleic acid encoding a parental polypeptide comprising the amino acid sequence: C1-X1-C2-X2-C3-X3-C4, wherein subsequence C1 is selected from SEQ ID NOs:1-30, subsequence C2 is selected from SEQ ID NOs:31-60, subsequence C3 is selected from SEQ ID NOs:61-90; subsequence C4 is selected from SEQ ID NOs:91 120; each of C1-C4 comprises up to 10 singe amino acid substitutions, deletions, insertions, or additions to the selected subsequence; and each of X1-X3 is an independent subsequence consisting of 2-20 amino acids; (ii) replicating the parental nucleic acid under conditions that introduce up to 10 single amino acid substitutions, deletions, insertions, or additions to the X1, X2, or X3 subsequences, whereby a population of randomly varied subsequences encoding X1', X2', or X3' is generated; and (iii) the population of randomly varied subsequences X1', X2', or X3' is substituted, into a population of parental nucleic acids at the positions corresponding to those that encode X1, X2, or X3.

In various instances: at least one of the X1-X3 subsequences is selected from SEQ ID NOs:121-123; each of C1-C4 independently comprises up to 20 single amino acid substitutions, deletions, insertions, or additions to the selected subsequence; each of C1-C4 independently comprises up to 10 single amino acid substitutions, deletions, insertions, or additions to the selected subsequence; each of C1-C4 independently comprises up to 5 single amino acid substitutions, deletions, insertions, or additions to the selected subsequence; none of C1-C4 comprise amino acid substitutions, deletions, insertions, or additions to the selected subsequence; the replicating generates a heterogeneous population of randomly varied subsequences by introducing up to 5 amino acid substitutions in each of X1, X2, or X3; the method further comprises amplifying the library by introducing it into a biological replication system and proliferating the biological replication system; the biological replication system is a plurality ofE. coli cells; the biological replication system is a plurality of bacteriophage; the replicating occurs in vitro; the replicating is performed with a purified mutagenic polymerase; the replicating is performed in the presence of a nucleotide analog; the replicating occurs in vivo; the replicating in vivo occurs in a mutagenic species of E. coli.

Also described is a method of generating the library of embodiment 1, comprising:(i) selecting an amino acid sequence comprising the amino acid sequence C1-X1-C2-X2 C3 X3-C4 to be encoded, wherein: (a) subsequence C1 is selected from SEQ ID NOs:1-30, subsequence C2 is selected from SEQ ID NOs:31-60, subsequence C3 is selected from SEQ ID NOs:61 90, and subsequence C4 is selected from SEQ ID NOs:91-120; (b) each of C1-C4 comprises up to 10 single amino acid substitutions, deletions, insertions, or additions to the selected subsequence; (c) each of X1, X2, and X3 consists of an amino acid sequence 2-20 amino acids in length; (ii) providing a first plurality and a second plurality of oligonucleotides, wherein: (a) oligonucleotides of the first plurality encode the C1-C4 subsequences and multiple heterogeneous X1-X3 variant subsequences X1'-X3'; (b) oligonucleotides of the second plurality are complementary to nucleotide sequences encoding the C1-C4 subsequences and to nucleotide sequences encoding multiple heterogeneous X1' X3' subsequences; and (c) the oligonucleotides of the first and second pluralities have overlapping sequences complementary to one another; (iii) combining the population of oligonucleotides to form a first mixture; (iv) incubating the mixture under conditions effective for hybridizing the overlapping complementary sequences to form a plurality of hybridized complementary sequences; and (v) elongating the plurality of hybridized complementary sequences to form a second mixture containing the library.

In various instances: each of C1-C4 independently comprises up to 20 single amino acid substitutions, deletions, insertions, or additions to the selected subsequence; each ofCI-C4 independently comprises up to 10 single amino acid substitutions, deletions, insertions, or additions to the selected subsequence; each of C1-C4 independently comprises from zero and up to 5 single amino acid substitutions, deletions, insertions, or additions to the selected subsequence; the method further comprises performing a cycle of steps, the cycle of steps comprising denaturing the library by increasing the temperature of the second mixture to a temperature effective for denaturing double stranded DNA, followed by steps (iv) and (v); the method comprises repeating the cycle of steps up to 100 times; the method further comprises amplifying the library by a polymerase chain reaction consisting essentially of the library, a forward primer, and a reverse primer, wherein the forward and reverse primers can hybridize to the 5' and 3' end sequences, respectively, of all nucleic acids in the library; the amino acid to be encoded in each position of the X1, X2, or X3 subsequences, is selected from a subset of alanine, arginine, asparagine, aspartate, cysteine, glutamine, glutamate, glycine, histidine, isoleucine, leucine, lysine, methionine, phenylalanine, proline, serine, threonine, tryptophan, tyrosine, and valine; herein the amino acid selected for each single amino acid substitution is selected from a group of amino acids consisting of at least one aliphatic, at least one one acidic, at least one one neutral, and at least one one aromatic amino acid; and the group of amino acids consists of alanine, aspartate, serine, and tyrosine.

Also described herein is a method of generating a library, comprising: (i) providing a parental nucleic acid encoding a parental polypeptide comprising the amino acid sequence: C1-X1-C2-X2-C3-X3-C4, wherein subsequence C1 is selected from FIG 2 or FIG 4, subsequence C2 is selected from FIG 2 or FIG 4, subsequence C3 is selected from FIG.2 or FIG 4; subsequence C4 is selected from FIG.2 or FIG 4 each of C1-C4 comprises up to 10 single amino acid substitutions, deletions, insertions, or additions to the selected subsequence; and each of X1-X3 is an independent subsequence consisting of 2-20 amino acids; (ii) replicating the parental nucleic acid under conditions that introduce up to 10 single amino acid substitutions, deletions, insertions, or additions to the X1, X2, or X3 subsequences, whereby a population of randomly varied subsequences encoding X1', X2', or X3' is generated; and (iii) the population of randomly varied subsequences X1', X2', or X3' is substituted, into a population of parental nucleic acids at the positions corresponding to those that encode X1, X2, or X3.

In various embodiments: at least one of the X1-X3 subsequences is selected from SEQ ID NOs:121-123; each of C1-C4 independently comprises up to 20 single amino acid substitutions, deletions, insertions, or additions to the selected subsequence; each of C1-C4 independently comprises up to 10 single amino acid substitutions, deletions, insertions, or additions to the selected subsequence; each of C1-C4 independently comprises up to 5 single amino acid substitutions, deletions, insertions, or additions to the selected subsequence; none of C1-C4 comprise an amino acid substitutions, deletions, insertions, or additions to the selected subsequence; the replicating generates a heterogeneous population of randomly varied subsequences by introducing up to 5 amino acid substitutions in each of X1, X2, or X3; the method further comprises amplifying the library by introducing it into a biological replication system and proliferating the biological replication system; the biological replication system is a plurality of E. coli cells; the biological replication system is a plurality of bacteriophage; the replicating occurs in vitro; the replicating is performed with a purified mutagenic polymerase the replicating is performed in the presence of a nucleotide analog; the replicating occurs in vivo; and the replicating in vivo occurs in a mutagenic species of E. coli.

Also described is a method of generating the library, comprising: (i) selecting an amino acid sequence comprising C1-X1-C2-X2 C3 X3-C4 to be encoded, wherein (a) subsequence C1 is selected from FIG 2 or FIG 4, subsequence C2 is selected from FIG. 2 or FIG 4, subsequence C3 is selected from FIG 2 or FIG 4, and subsequence C4 is selected from FIG 2 or FIG 4; (b) each of C1-C4 comprises up to 10 single amino acid substitutions, deletions, insertions, or additions to the selected subsequence; (c) each of X1, X2, and X3 consists of an amino acid sequence 2-20 amino acids in length; (ii) providing a first plurality and a second plurality of oligonucleotides, wherein (a) oligonucleotides of the first plurality encode the C1-C4 subsequences and multiple heterogeneous X1-X3 variant subsequences X1'-X3'; (b) oligonucleotides of the second plurality are complementary to nucleotide sequences encoding the C1-C4 subsequences and to nucleotide sequences encoding multiple heterogeneous X1' X3' subsequences; and

(c) the oligonucleotides of the first and second pluralities have overlapping sequences complementary to one another; (iii) combining the population of oligonucleotides to form a first mixture; (iv) incubating the mixture under conditions effective for hybridizing the overlapping complementary sequences to form a plurality of hybridized complementary sequences; and (v) elongating the plurality of hybridized complementary sequences to form a second mixture containing the library.

In various cases: each of C1-C4 independently comprises up to 20 single amino acid substitutions, deletions, insertions, or additions to the selected subsequence; each of C1-C4 independently comprises up to 10 single amino acid substitutions, deletions, insertions, or additions to the selected subsequence; each of C1-C4 independently comprises from zero and up to 5 single amino acid substitutions, deletions, insertions, or additions to the selected subsequence; the method further comprises performing a cycle of steps, the cycle of stepscomprising denaturing the library by increasing the temperature of the second mixture to a temperature effective for denaturing double stranded DNA, followed by steps (iv) and (v); the method further comprises repeating the cycle of steps up to 100 times; the method further comprises amplifying the library by a polymerase chain reaction consisting essentially of the library, a forward primer, and a reverse primer, wherein the forward and reverse primers can hybridize to the 5' and 3' end sequences, respectively, of all nucleic acids in the library; the amino acid to be encoded in each position of the X1, X2, or X3 subsequences, is selected from a subset of alanine, arginine, asparagine, aspartate, cysteine, glutamine, glutamate, glycine, histidine, isoleucine, leucine, lysine, methionine, phenylalanine, proline, serine, threonine, tryptophan, tyrosine, and valine; the amino acid selected for each single amino acid substitution is selected from a group of amino acids consisting of at least one aliphatic, at least one acidic, one at least one neutral, and at least one aromatic amino acid; and the group of amino acids consists of alanine, aspartate, serine, and tyrosine.

Also disclosed is a method of generating the library, comprising: (i) providing a parental nucleic acid encoding a parental polypeptide comprising the amino acid sequence: C1-X1-C2-X2-C3-X3-C4, wherein subsequence C1 is selected from FIG 3 or FIG 5, subsequence C2 is selected from FIG 3 or FIG 5, subsequence C3 is selected from FIG 3 or FIG 5; subsequence C4 is selected from FIG 3 or FIG 5; each of C1-C4 comprises up to 10 single amino acid substitutions, deletions, insertions, or additions to the selected subsequence; and each of X1-X3 is an independent subsequence consisting of 2-20 amino acids; (ii) replicating the parental nucleic acid under conditions that introduce up to 10 single amino acid substitutions, deletions, insertions, or additions to the X1, X2, or X3 subsequences, whereby a population of randomly varied subsequences encoding X1', X2', or X3' is generated; and (iii) the population of randomly varied subsequences X1', X2', or X3' is substituted, into a population of parental nucleic acids at the positions corresponding to those that encode X1, X2, or X3.

In various instances: at least one of the X1-X3 subsequences is selected from SEQ ID NOs:121-123; each of C1-C4 independently comprises up to 20 single amino acid substitutions, deletions, insertions, or additions to the selected subsequence; each of C1-C4 independently comprises up to 10 single amino acid substitutions, deletions, insertions, or additions to the selected subsequence; each of C1-C4 independently comprises up to 5 single amino acid substitutions, deletions, insertions, or additions to the selected subsequence; none of C1-C4 comprise amino acid substitutions, deletions, insertions, or additions to the selected subsequence; the replicating generates a heterogeneous population of randomly varied subsequences by introducing up to 5 amino acid substitutions in each of X1, X2, or X3; the method further comprises amplifying the library by introducing it into a biological replication system and proliferating the biological replication system; the biological replication system is a plurality of E. coli cells; the biological replication system is a plurality of bacteriophage; the replicating occurs in vitro; the replicating is performed with a purified mutagenic polymerase; the replicating is performed in the presence of a nucleotide analog; the replicating occurs in vivo; and the replicating in vivo occurs in a mutagenic species of E. coli.

Also described is a method of generating the library, comprising: (i) selecting an amino acid sequence comprising: C1-X1-C2-X2 C3 X3-C4 to be encoded, wherein (a) subsequence C1 is selected from FIG 3 or FIG 5, subsequence C2 is selected from FIG 3 or FIG 5, subsequence C3 is selected from FIG 3 or FIG 5, and subsequence C4 is selected from FIG. 3 or FIG 5; (b) each of C1-C4 comprises up to 10 single amino acid substitutions, deletions, insertions, or additions to the selected subsequence; (c) each of X1, X2, and X3 consists of an amino acid sequence 2-20 amino acids in length; (ii) providing a first plurality and a second plurality of oligonucleotides, wherein (a) oligonucleotides of the first plurality encode the C1-C4 subsequences and multiple heterogeneous X1-X3 variant subsequences X1'-X3'; (b) oligonucleotides of the second plurality are complementary to nucleotide sequences encoding the C1-C4 subsequences and to nucleotide sequences encoding multiple heterogeneous X1' X3' subsequences; and (c) the oligonucleotides of the first and second pluralities have overlapping sequences complementary to one another; (iii) combining the population of oligonucleotides to form a first mixture; (iv) incubating the mixture under conditions effective for hybridizing the overlapping complementary sequences to form a plurality of hybridized complementary sequences; and (v) elongating the plurality of hybridized complementary sequences to form a second mixture containing the library.

In various embodiments: each ofCI-C4 comprises up to 20 single amino acid substitutions, deletions, insertions, or additions to the selected subsequence; each of C1-C4 independently comprises up to 10 single amino acid substitutions, deletions, insertions, or additions to the selected subsequence; each of C1-C4 independently comprises from zero and up to 5 single amino acid substitutions, deletions, insertions, or additions to the selected subsequence; the method further comprises performing a cycle of steps, the cycle comprising denaturing the library by increasing the temperature of the second mixture to a temperature effective for denaturing double stranded DNA, followed by steps (iv) and (v); the method further comprises repeating the cycle up to 100 times; the method further comprises amplifying the library by a polymerase chain reaction consisting essentially of the library, a forward primer, and a reverse primer, wherein the forward and reverse primers can hybridize to the 5' and 3' end sequences, respectively, of all nucleic acids in the library; the amino acid to be encoded in each position of the X1, X2, or X3 subsequences, is selected from a subset of alanine, arginine, asparagine, aspartate, cysteine, glutamine, glutamate, glycine, histidine, isoleucine, leucine, lysine, methionine, phenylalanine, proline, serine, threonine, tryptophan, tyrosine, and valine the amino acid selected for each single amino acid substitution is selected from a group of amino acids consisting of at least one aliphatic, one acidic, one neutral, and one aromatic amino acid; and the group of amino acids consists of alanine, aspartate, serine, and tyrosine.

Also described is a library of nucleic acids encoding at least ten different polypeptides, wherein: (i) the amino acid sequence of each of the encoded polypeptides comprises an amino acid sequence at least 70% identical to any of SEQ ID NOs: 127-129; (ii) the amino acid sequence of each of the encoded polypeptides includes amino acids that differ from those of SEQ ID NOs: 127-129 in at least one of positions 13-14, 33, 35-36, 38, 46-47, 66, 68-69, 71, 79-80, 99, 101-102, 104, and 112-113 and the amino acid differences are heterogeneous across a plurality of the encoded polypeptides; and (iii) the amino acid sequence of each of the encoded polypeptides outside of the residues corresponding to positions 14, 15, 33, 35-36, 38, 47-48, 66, 68-69, 71, 80, 81, 99, 101-102, and 104 of SEQ ID NOs: 127-129 is homogeneous across a plurality of the encoded polypeptides.

In various embodiments: the amino acid sequence of the polypeptides has at least 75% identity to any of SEQ ID NOs 127-129; the amino acid sequence of the polypeptides has at least 80% identity to any of SEQ ID NOs 127-129; and the amino acid sequence of the polypeptides has at least 85% identity to any of SEQ ID NOs 127-129 each of the nucleic acids comprises a vector sequence. Also disclosed: an isolated nucleic acid encoding a polypeptide, selected from the library; a purified polypeptide encoded by the nucleic acid; a population of cells expressing the polypeptides encoded by the library; .a cell selected from the population of cells; a purified library of polypeptides encoded by the library; a population of filamentous phage displaying the library of polypeptides encoded by the library.

Also disclosed is a method of generating the library, comprising: (i) selecting an amino acid sequence corresponding to any one of SEQ ID NOs: 127 129 to be encoded, wherein the selected sequence differs from those of SEQ ID NOs:127-129 in at least one of variable positions 13-14, 33, 35-36, 38, 46-47, 66, 68-69, 79-80, 81, 99, 101-102, 104, and 112-113 (ii) chemically providing a first and a second plurality of oligonucleotides, wherein (a) oligonucleotides of the first plurality encode amino acid subsequences of the selected amino acid sequence; the subsequences being heterogeneous at the encoded variable positions; (b) oligonucleotides of the second plurality are complementary to nucleotide sequences encoding subsequences of the selected amino acid sequence, the subsequences being heterogeneous at the encoded variable positions; and (c) the first and second pluralities comprise oligonucleotides have overlapping sequences complementary to one another; (iii) combining the population of oligonucleotides to form a first mixture; (iv) incubating the mixture under conditions effective for hybridizing the overlapping complementary sequences to form a plurality of hybridized complementary sequences; and (v) elongating the plurality of hybridized complementary sequences to form a second mixture containing the library.

In various instances: the method further comprises performing a cycle of denaturing the library by increasing the temperature of the second mixture to a temperature effective for denaturing double stranded DNA, followed by steps (iv) and (v); the method further comprises repeating the cycle up to 100 times; the method further comprises amplifying the library by a polymerase chain reaction consisting essentially of the library, a forward primer, and a reverse primer, wherein the forward and reverse primers can hybridize to the 5' and 3' end sequences, respectively, of all nucleic acids in the library; the amino acids to be encoded for the variable positions, are selected from a subset of alanine, arginine, asparagine, aspartate, cysteine, glutamine, glutamate, glycine, histidine, isoleucine, leucine, lysine, methionine, phenylalanine, proline, serine, threonine, tryptophan, tyrosine, and valine the amino acids selected for the variable positions are selected from a group consisting of an aliphatic, an acidic, a neutral, and an aromatic amino acid; the group of amino acids consists of alanine, aspartate, serine, and tyrosine.

The details of one or more embodiments of the invention are set forth in the description below. Other features, objects, and advantages of the invention will be apparent from the description and drawings, and from the embodiments.

### DESCRIPTION OF DRAWINGS

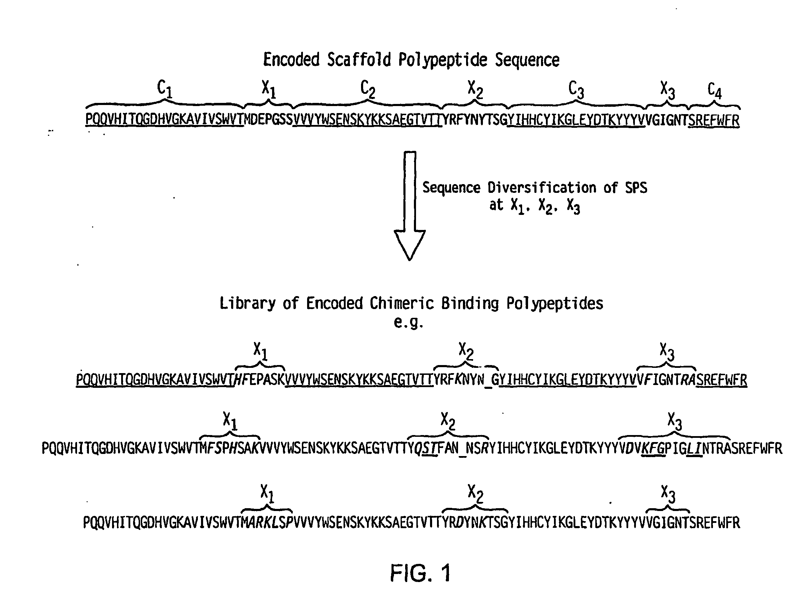

FIG. 1 is a schematic representation depicting the generation of a library of nucleic acids encoding chimeric binding polypeptides by diversifying subsequences within an encoded polypeptide scaffold sequence. Encoded scaffold polypeptide sequence is SEQ ID NO: 124. Library of encoded chimeric binding polypeptides are SEQ ID NOs: 838, 839, and 840, respectively (i.e., from top to bottom).
FIG. 2 is an alignment of the sequences of a number of proteins that have regions which can be used as a scaffold. These proteins are homologous to oryzacystatin. The C1, C2, C3 and C4 are boxed and labeled. The sequences shown are SEQ ID NO: 132 (i.e., homologous sequences Q2V8I6 CDCMA 144111-18 - Q2V8I4 CDCMO 73411-28); SEQ ID NO: 133 (i.e., Q2V8H9 LAGLE 43111-28); SEQ ID NO: 134 (i.e., Q6DKD9 CDCMA 144111-28 and Q6DLC8 CDCMA 144111-28); SEQ ID NO: 131 (i.e., 080389 CDCSA 79511-89); SEQ ID NOs: 136 - 150 (i.e., QIRVW3 MEDTR 257811-54 - Q8GZV2 CHEMJ 340/1-38); SEQ ID NO:130 (i.e., Referencell-102); and SEQ ID NOs: 151-330 (i.e., CYTI ORYSA 109711-88 to end).
FIG. 3 is an alignment of the sequences ofa number of proteins that have regions which can be used as a scaffold. These proteins are homologous to C2. The C1, C2, C3 and C4 are boxed and labeled. Sheets 1-3 show SEQ ID NOs: 331-367 (i.e., Q9M366 ARATH 4312011-78 - Q9FJG3 ARATH 32540511-81); SEQ ID NO: 130 (i.e., Referencel1-156); and SEQ ID NOs: 368-384 (i.e., ERG1 ORYSA 795/1-89 - Q4JHI8 CDCMA 692/1-87). Sheets 4-6,7-9, 10-12. 13-15, 16-18, 19-21, 22-24, and 25-27 show SEQ ID NOs: 385-821.
FIG. 4 is an alignment of the sequences of a number of proteins that have regions which can be used as a scaffold. The sequences shown are SEQ ID NO: 130 (i.e., oryza full) and SEQ ID NOs 822-832. These proteins are homologous to oryzacystatin. The C1, C2, C3 and C4 are boxed and labeled.
FIG. 5 is an alignment of the sequences of a number of proteins that have regions which can be used as a scaffold. The sequences shown are, from top to bottom, SEQ ID NO: 131 and SEQ ID NOs 833-837. These proteins are homologous to C2. The C1, C2, C3 and C4 are boxed and labeled.

### DETAILED DESCRIPTION

Diverse libraries of nucleic acids (e.g., cDNA libraries) encoding plant chimeric binding polypeptides, as well as methods for generating them are described below. The amino acid sequences of the library of encoded plant chimeric binding proteins are derived from a scaffold polypeptide sequence that includes subsequences to be varied. The varied subsequences correspond to putative binding domains of the plant chimeric binding proteins, and are highly heterogeneous in the library of plant chimeric binding proteins. In contrast, the sequence of the encoded chimeric binding proteins outside of the varied subsequences is essentially the same as the parent scaffold polypeptide sequence and highly homogeneous throughout the library of encoded plant chimeric binding proteins. Thus, libraries of plant chimeric binding proteins can serve as a universal molecular recognition library platform for selection of specialized binding proteins for expression in transgenic plants. Libraries of plant chimeric binding proteins can be expressed by transfected cells (i.e., as expression libraries) and tested for interaction with a molecular target of interest. For example, expression libraries can be screened to identify polypeptides that bind with high specificity and affinity to polypeptides expressed by plant pests, including nematodes. Ultimately, individual chimeric binding proteins with desired target binding properties can be expressed in a transgenic plant.

### I. Plant Scaffold Polypeptide Sequences

A plant scaffold polypeptide sequence is an amino acid sequence based on a plant protein that is structurally tolerant of extreme sequence variation within one or more regions. The regions to be varied within the scaffold polypeptide sequence are conceptually analogous to the hypervariable regions of immunoglobulins, and form putative binding domains in a chimeric binding polypeptide. Thus, a large library of nucleic acid sequences encoding diverse plant chimeric binding polypeptides is produced by diversifying specific sequences within a scaffold polypeptide sequence, as is described in detail below.

Plant scaffold polypeptide sequences are selected to have a number of properties, e.g., they: (i) are derived from sequences that are of plant origin; (ii) encode proteins that tolerate the introduction of sequence diversity structurally; (iii) only contain disulfide bonds that do not interfere with folding of the polypeptide when expressed in a plant; (iv) express at high levels in diverse plant tissues; and (v) can be targeted to different subcellular locations (e.g., cytoplasm, mitochondria, plastid) or secreted from the cell. Based on these properties, plant scaffold polypeptide sequences permit the generation of large libraries of chimeric binding polypeptides with highly diverse binding activities. Libraries of chimeric binding polypeptides can be screened for binding to a target molecule. Chimeric binding proteins having the desired binding activity can subsequently be expressed in plants to confer input traits (e.g., pest or pathogen resistance, drought tolerance) or output traits (e.g. modified lipid composition, heavy metal binding for phytoremediation, medicinal uses). Such binding proteins can also be used in various affinity-based applications, e.g., diagnostic detection of an antigen using a sandwich ELISA; histochemical detection of antigens; generation of protein biochips; and affinity purification of antigens.

It is helpful to select the scaffold polypeptide sequence based on the sequence of a plant protein or protein domain of known three dimensional structure (see, e.g., Nygren et al. (2004) "Binding Proteins from Alternative Scaffolds," J. of Immun. Methods 290:3-28). However, even without experimentally determined structural data for a potential scaffold polypeptide sequence, valuable inferences can be gleaned from computational structural analysis of a candidate amino acid sequence. Useful programs for structure prediction from an amino acid sequence include, e.g., the "SCRATCH Protein Predictor" suite of programs available to the public on the world wide web at ics.uci.edu/~baldig/scratch/index. It is important that introduction of sequence variation not destabilize the known or predicted secondary structure of the scaffold polypeptide sequence. Accordingly, the known or predicted secondary structure of the scaffold polypeptide sequence informs the selection of amino acid subsequences that can be varied within a scaffold polypeptide sequence to form putative binding domains. The structural adequacy of a particular scaffold polypeptide sequence can be readily tested, e.g., by phage display expression analysis methods that are commonly known in the art. For example, a scaffold polypeptide sequence containing 0, 1, 2, 3, or more disulfide bonds can be tested for its ability to fold into a stable protein. Since proteins that do not fold properly will not be incorporated into a phage coat, they will not be displayed. Thus, without undue effort, many candidate scaffold polypeptide sequences can be rapidly screened for their ability to fold into stable proteins once expressed.

The plant scaffold polypeptide sequences can be based on the accessory domain from purple acid phosphatases (PAPs). The crystal structure of the PAP accessory domain of kidney bean, *Phaseolus vulgaris,* has been determined (Strater et al. (1995), Science 268(5216):1489-1492). Three exposed loops within the protein are reminiscent of the hypervariable domains found in immunoglobulins. The loops are brought together by the rigid anti-parallel β-sheet framework of the protein. The subsequences that form each loop form the putative binding domains of a chimeric binding protein derived from a PAP. These subsequences are diversified by substituting, deleting, inserting, or adding up to 10 (e.g., up to 3, 4, 6, 8) amino acids. The loops that form the putative binding domains are particularly well suited to binding target molecules containing pockets or clefts.

PAP-based scaffold polypeptide sequences take the general form:

**C₁-X₁-C₂-X₂-C₃-X₃-C₄**

where C₁, C₂, C₃, and C₄ correspond to "backbone" subsequences which can include some introduced variation, but are not highly diversified. On the other hand, X₁, X₂, and X₃ correspond to highly varied subsequences that form the putative binding domains of each PAP-based chimeric binding protein. Table 1 shows a list of suitable C₁-C₄ backbone subequences derived from the amino acid sequences of 30 PAPs.

C₁, C₂, C₃, and C₄ correspond to SEQ ID NOs: 1-30, 31-60, 61-90, and 91-120, respectively, in Table 1.

X₁, X₂, and X₃ can be based on naturally occurring variants of corresponding PAP sequences, e.g., those shown in Table 2 as SEQ ID NOs: 127-123. Table 2 shows the range variation at each amino acid position in subsequences corresponding, respectively, to X₁, X₂, and X₃, within 30 naturally occurring PAP sequences. Alternatively, the parent variable subsequences, X₁,-X₃, can be arbitrary sequences 2-20 amino acids in length.

In some implementations, C₁, C₂, C₃, and C₄ of a scaffold polypeptide sequence can be selected from multiple PAP-based scaffold polypeptide sequence sequences listed in Table 1, in any combination, e.g., C_{1(SEQ ID NO:5)}, C_{2(SEQ ID NO:12)}, C_{3(SEQ ID NO:7)}, and C_{4(SEQ ID NO:19)}; C_{1(SEQ ID NO:5)}, C_{2(SEQ ID NO:12)}, C_{3(SEQ ID NO:5)}, and C_{4(SEQ ID NO:12)}; C_{4(SEQ ID NO:22)}; C_{1(SEQ ID NO:17)}, C_{2(SEQ ID NO:17)}, C_{3(SEQ ID NO:19)}, and C_{4(SEQ ID NO:1)}, and so forth.

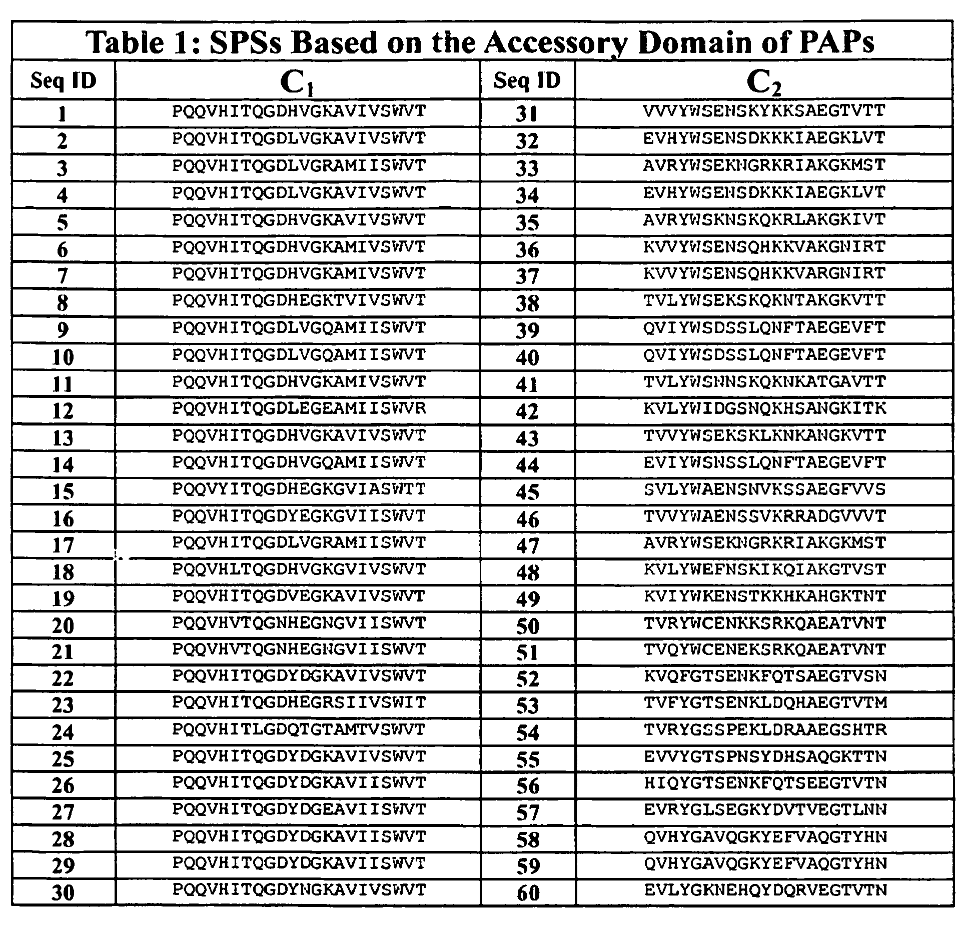

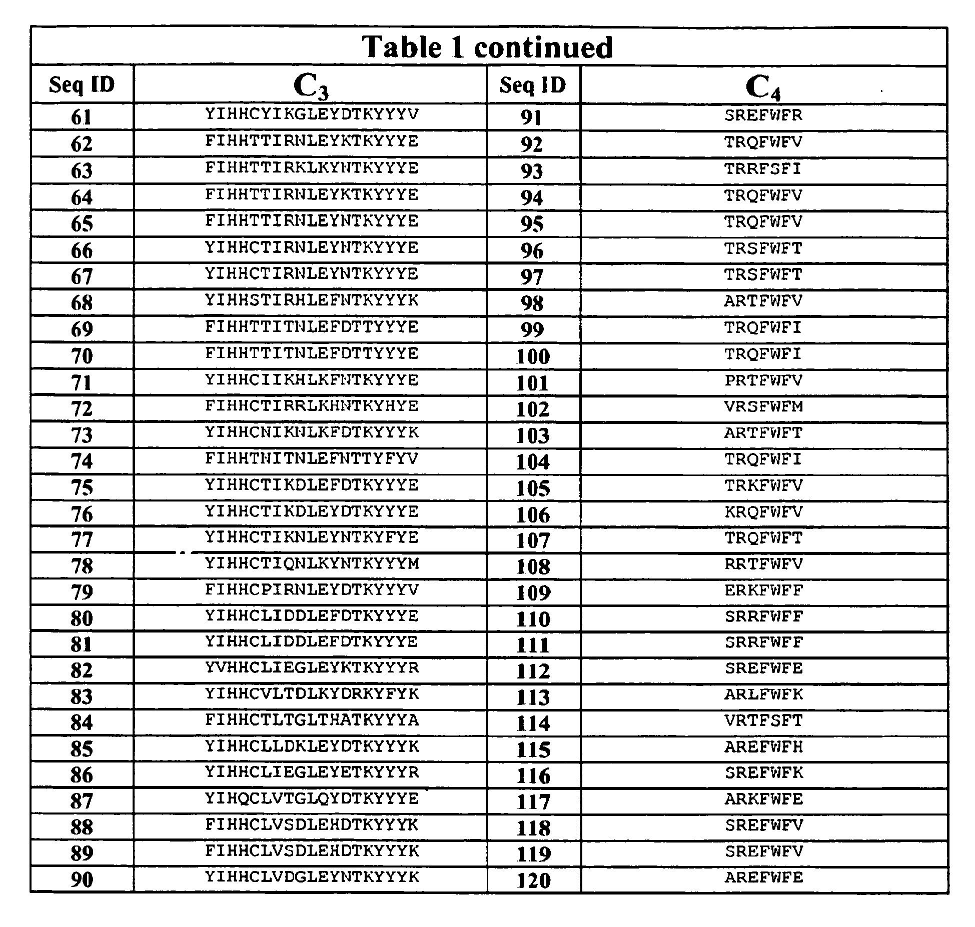

**Table 2: Naturally Occurring Residue Variation in PAP Subsequences Corresponding to X₁, X₂, and X₃ (SEQ ID NOs:121-123)**

| **X₁** | | | | | | | **X₂** | | | | | | | | | **X₃** | | | | | |
|---|---|---|---|---|---|---|---|---|---|---|---|---|---|---|---|---|---|---|---|---|---|
| **(SEQ ID NO: 121)** | | | | | | | **(SEQ ID NO: 122)** | | | | | | | | | **(SEQ ID NO: 123)** | | | | | |
| **Position** | | | | | | | **Position** | | | | | | | | | **Position** | | | | | |
| **a** | **b** | **c** | **d** | **e** | **f** | **g** | **a** | **b** | **c** | **d** | **e** | **f** | **g** | **h** | **i** | **a** | **b** | **c** | **d** | **e** | **f** |
| M | D | E | P | G | S | S | Y | K | Y | Y | N | Y | T | S | G | V | G | L | R | N | T |
| V | E | A | K | | P | N | | R | F | F | T | | S | | P | I | E | I | G | H | |
| E | N | K | L | | K | K | | T | | H | K | | N | | | L | | V | E | D | |
| P | V | D | | | | T | | F | | | D | | K | | | M | | E | D | Q | |
| Q | S | | | | H | | | | | | E | | E | | | | | T | | K | |
| T | I | | | | T | | | | | | | | | | | | | S | | S | |
| A | A | | | | | | | | | | | | | | | | | E | | E | |
| F | | | | | | | | | | | | | | | | | | F | | | |
| | | | | | | | | | | | | | | | | | | K | | | |

After diversification of the above-listed subsequences of the scaffold polypeptide sequence, the diversified X₁', X₂', and X₃' subsequences are highly heterogeneous within the library of encoded plant chimeric binding polypeptides, and can each contain up to 10 (e.g., 8, 6, 4, 3) single amino acid substitutions, deletions, insertions, or additions with respect to SEQ ID NOs: 121-123 listed in Tables 1, respectively (see, e.g., Fig. 1). For example, the length of the amino acid sequences corresponding to regions X₁, X₂, or X₃ can be unaltered, shortened, or lengthened relative to SEQ ID NOs: 121-123.

The regions outside of the putative binding domains are referred to as "backbone" regions (i.e., C₁, C₂, C₃, and C₄). Unlike the amino acid sequences for X₁, X₂, and X₃, the amino acid sequences of the backbone regions are generally not substantially diversified within the library of encoded chimeric binding proteins, although some sequence variation in these regions within the library is permissible. The backbone regions of a plant scaffold polypeptide sequence can be at least 70% (i.e., 80, 85, 90, 95, 98, or 100%) identical to any of SEQ ID NOs: 1-120. Alternatively, the backbone regions can contain up to 30 (i.e., 28, 26, 24, 22, 20, 18, 17, 16, 15, 14, 13, 12, 11, 10, 9, 8, 7, 6, 5, 4, 3, 2, or I) single amino acid substitutions, deletions, insertions or additions. For example, C₁, C₂, C₃, and C₄ can each include 0, 1, 2, 3, 4, or 5 or more single amino acid changes. If amino acid substitutions are to be introduced into the backbone regions, it is preferable to make conservative substitutions. A conservative substitution is one that preserves the substitutes an amino acid with one that has similar chemical properties (e.g., substitution of a polar amino acid such as serine with another polar amino acid such as threonine).

In one embodiment, the plant scaffold polypeptide sequence is one of SEQ ID NOs: 124-126 shown below. Sequences corresponding to X₁, X₂, and X₃ are in bold and underlined.

In other embodiments, a plant scaffold polypeptide sequence is based on the amino acid sequence of plant proteins that have ankyrin-like repeats. Ankryin-like repeats are small turn-helix-helix (THH) repeats consisting of approximately 33 amino acids. The number of THH repeats within a scaffold polypeptide sequence can vary from 2 to 20. The putative binding sites within the THH repeats are typically non-contiguous, but clustered on the same side of the protein of which they are a part.

WO02/20565 discloses libraries of ankyrin repeat protein scaffolds.

A plant THH repeat-containing scaffold polypeptide sequence can have an amino acid sequence that is based on any of SEQ ID NOs: 127-129 listed below. High levels of amino acid sequence variation are introduced at the bolded/underlined residues. The plant THH repeat-containing scaffold polypeptide sequences can contain substitutions of up to 3 amino acids or a deletion in the place of the amino acids corresponding to residues 13-14, 33, 35-36, 38, 46-47, 66, 68-69, 71, 79-80, 99, 101-102, 104, and 112-113 (residues in bold and underlined) of SEQ ID NOs:127-129.

The sequence of the scaffold polypeptide sequences can be at least 70% (i.e., 80, 85, 90, 95, 98, or 100%) identical to the sequence outside of the foregoing amino acid positions (in bold) of SEQ ID NOS: 127-129. Alternatively, the sequence of the scaffold polypeptide sequences outside of the foregoing amino acid positions (in bold) of SEQ ID NOS:127-129 can contain up to 30 (i.e., 28, 26, 24, 22, 20, 18, 17, 16, 15, 14, 13, 12, 11, 10, 9, 8, 7, 6, 5, 4, 3, 2, or 1) single amino acid substitutions, deletions, insertions or additions. In some cases it can be desirable to include additional repeating units. SEQ ID NOs: 127-129 have an amino-terminal cap, two internal repeats and a carboxy-terminal cap. It might be desirable to have 1-6 internal repeats. The amino-terminal cap sequence is aa 1-33. The first internal repeat is 34-66 and the second internal repeat is 67- 99. The carboxy-terminal cap sequence is aa 100-123. The first or the second internal repeats or both can be independently repeated 1, 2, 3, 4, 5 or 6 times.

The putative binding sites are formed by amino acid side chains protruding from the rigid secondary structure formed by the scaffold polypeptide sequence. These proteins may typically form a larger, flatter binding surface and are particularly useful for binding to targets that do not have deep clefts or pockets.

Another suitable scaffold can be based on oryzacystatin (J Biol Chem 262:16793 (1987); Biochemistry 39:14753 (2000)), a member of the cystatin/Papain Family (Pfam Identifier PF00031) that is identified as a cysteine proteinase inhibitor of rice. The sequence oforyzacystatin is depicted below. A scaffold having the amino acid sequence C1-X1-C2-X2-C3-X3-C4 where each of X1, X2, X3 and X4 is a variable region and C1, C2, C3 and C4 are the backbone regions can be created based on oryzacystatin. C1-MSS (aa 1-3 of SEQ ID NO: 130)
X1-VGGP (aa 4-7 of SEQ ID NO: 130)
C2-VLGGVEPVGNENDLHLVDLARFAVTEHNKKANSLLEFEKLVSV (aa-8-50 of SEQ ID NO: 130)
X2-KQQVVAGT (aa 51-58 of SEQ ID NO: 130)
C3-LYYFTLEVKEGDAKKLYEAKVWE (aa 59-81 of SEQ ID NO:130)
X3-KPWM (aa 82-85 of SEQ ID NO: 130)
C4-DFKELQEFKPVDASANA (aa 86-102 of SEQ ID NO: 130)

FIG. 2 depicts the sequences of a large number of plant proteins aligned with oryzacystatin. Examples of suitable C1-C4 regions are indicated. FIG. 4 depicts the sequences of a small number of plant proteins aligned with oryzacystatin. Examples of suitable C1-C4 regions are indicated. In general, X1 can be a sequence of 2-20 random amino acids (e.g., 3 amino acids). X2 can be a sequence of 2-20 random amino acids (e.g., 4 amino acids). X3 can be a sequence of 2-20 random amino acids (e.g., 4 amino acids).

Yet another suitable can be based on the C2 protein of rice *(*Biochemistry 42:11625 (2003)), a member of the C2 domain family (Pfam Identifier PF00168) that is thought to be be involved in plant defense signaling systems. The sequence of rice C2 is depicted below. A scaffold having the amino acid sequence C1-X1-C2-X2-C3-X3-C4 where each of X1, X2, X3 and X4 is a variable region and C1, C2, C3 and C4 are the backbone regions can be created based on rice C2. C1-MAGSGVLEVHLVDAKG (aa 1-16 of SEQ ID NO: 131)
X1-LTGNDFLGKID (aa 17-27 of SEQ ID NO: 131)
C2-PYVVVQYRSQERK (aa 28-40 of SEQ ID NO: 131)
X2-SSVARDQGKNP (aa 41-51 of SEQ ID NO: 131)
C3-SWNEVFKFQINSTAATGQHKLFLRL (aa 52-76 of SEQ ID NO:131)
X3- MDHDTFSRDDFL (aa 77-88 of SEQ ID NO: 131)

FIG. 3 depicts the sequences of a large number of plant proteins aligned with rice C2. Examples of suitable C1-C4 regions are indicated. FIG. 5 depicts the sequences of a small number of plant proteins aligned with oryzacystatin. Examples of suitable C1-C4 regions are indicated. In general, X1 can be a sequence of 2-20 random amino acids (e.g., 11 amino acids). X2 can be a sequence of 2-20 random amino acids (e.g., I I amino acids). X3 can be a sequence of 2-20 random amino acids (e.g., 12 amino acids).

The following sections disclose methods for generating libraries of nucleic acids encoding chimeric binding proteins based on plant scaffold polypeptide sequences.

### II. Generation of Nucleic Acid Libraries based on a Plant scaffold polypeptide sequence

A large library of nucleic acid sequence variants encoding the plant scaffold polypeptide sequence is created based on one or more plant scaffold polypeptide sequences. The library of nucleic acids encodes at least 5 (e.g., 1,000, 10⁵, 10⁶, 10⁷, 10⁹, 10¹², 10¹⁵ or more) different chimeric binding protein sequences. It is recognized that not every member of a library generated by the methods described herein will encode a unique amino acid sequence. Nevertheless, it is desirable that at least 10% (e.g., 25%, 30%, 40%, 50%, 60%, 70%, 75%, or 90%) of the encoded chimeric binding proteins represented in the library be unique.

Prior to diversifying a plant scaffold polypeptide sequence, it may be useful to estimate computationally the expected sequence diversity to be generated with a given set of sequence variation parameters. A method for estimating sequence diversity is described, e.g., in Volles *et al.* (2005), 33(11): 3667-3677. For example, the number of different sequences expected in a library of nucleic acids generated by PCR can be estimated based on the mutation frequency of the mutagenic polymerase used for the amplification. Useful algorithms for estimating sequence diversity in randomized protein-encoding libraries can also be found on the world wide web, e.g., at guinevere.otago.ac.nz/mlrgd/STATS/index.

Libraries of nucleic acids encoding plant chimeric binding proteins can be generated by a number of known methodologies. Sequence diversity is introduced into a plant scaffold polypeptide sequence by substitution, deletion, insertion, or addition of amino acids at the highly variable positions of a scaffold polypeptide sequence as described above. Since the set of 20 amino acids that are genetically encoded in plants have somewhat redundant chemical and structural properties, a subset of amino acids (e.g., a subset of 4 types of amino acids) that encompasses this structural diversity can be adopted for substitutions. For example, amino acids to be used for substitution or insertion can be selected to include an acidic amino acid, a neutral amino acid, an aliphatic amino acid, and an aromatic amino acid (see Table 3). For example, the amino acids used for substitution could be limited to aspartate, serine, alanine, and tyrosine. Limiting the redundancy of amino acid substitutions will increase the overall structural and binding diversity of the library of chimeric binding proteins.

| **Table 3 Chemical Properties of Amino Acids Genetically Encoded in Plants** | | | | |
|---|---|---|---|---|
| **Acidic** | **Neutral** | **Aliphatic** | **Aromatic** | **Basic** |
| Aspartate, Glutamate, | Asparagine, Cysteine Glutamine, Methionine, Proline, Serine, Threonine, | Alanine, Glycine, Isoleucine, Leucine, Valine | Histidine, Phenylalanine, Tryptophan, Tyrosine | Arginine, Lysine |

The library of nucleic acids can be generated *in vitro* by assembly of sets of oligonucleotides with overlapping complementary sequences. First, a scaffold polypeptide sequence sequence is selected that is to be encoded by sets of assembled oligonucleotides. The sequences to be encoded in the variable regions of a given scaffold polypeptide sequence will include a multitude of heterogeneous sequences containing substitutions, insertions, deletions in additions in accordance with the library of chimeric binding polypeptides to be generated as described above. The scaffold polypeptide sequences to be encoded can include the C₁-C₄ subequences corresponding to any of SEQ ID NOs:1-30, 31-60, 61-90, and 91-120, respectively.

One set of oligonucleotides encodes regions of the plant scaffold polypeptide sequence where diversity is to be introduced (e.g., at X₁, X₂, and X₃). In contrast, regions of the scaffold polypeptide sequence in which little or no variation is to be introduced (e.g., in backbone domains of PAP scaffold polypeptide sequences) are encoded by a set of oligonucleotides encoding amino acid sequences with no less than 70% (i.e., 75%, 80%, 85%, 90%, 95%, or 100%) identity to any one of the above-mentioned scaffold polypeptide sequences. The details of this method are described, e.g., in U.S. patent No. 6,521,453, hereby incorporated by reference.

Sequence-varied oligonucleotides used to generate libraries of nucleic acids are typically synthesized chemically according to the solid phase phosphoramidite triester method described by Beaucage and Caruthers (1981), Tetrahedron Letts., 22(20):1859-1862, e.g., using an automated synthesizer, as described in Needham-VanDevanter et al. (1984) Nucleic Acids Res., 12:6159-6168. A wide variety of equipment is commercially available for automated oligonucleotide synthesis. Multi-nucleotide synthesis approaches (e.g., tri-nucleotide synthesis), as discussed, supra, are also useful.

Nucleic acids can be custom ordered from a variety of commercial sources, such as Sigma-Genosys (at sigma-genosys.com/oligo.asp); The Midland Certified Reagent Company (mcrc@oligos.com), The Great American Gene Company (at genco.com), ExpressGen Inc. (at expressgen.com), Operon Technologies Inc. (Alameda, Calif.) and many others.

The oligonucleotides can have a codon use optimized for expression in a particular cell type (e.g., in a plant cell, a mammalian cell, a yeast cell, or a bacterial cell). Codon usage frequency tables are publicly available, e.g., on the world wide web at kazusa.or.jp/codon. Codon biasing can be used to optimize expression in a cell or on the surface of a cell in which binding of a plant chimeric binding protein is to be assessed, and can also be used to optimize expression of the chimeric binding protein in a transgenic organism of commercial interest (e.g., a transgenic plant). In general, codons with a usage frequency of less than 10% are not used. Before synthesis oligonucleotide sequences are checked for potentially problematic sequences, e.g, restriction sites useful for subcloning, potential plant splice acceptor or donor sites (see, e.g., cbs.dtu.dk/services/FeatureExtract/), potential mRNA destabilization sequences (e.g., "ATTTA"), and stretches of more than four occurrences of the same nucleotide. Potentially problematic sequences are changed accordingly.

Populations of oligonucleotides are synthesized that encode amino acid variations in the putative binding regions of the selected scaffold polypeptide sequence (e.g., in regions X₁, X₂, and X₃ of a PAP scaffold polypeptide sequence).

Preferably, all of the oligonucleotides of a selected length (e.g., about 10, 12, 15, 20, 30, 40, 50, 60, 70, 80, 90, or 100 or more nucleotides) that correspond to regions where sequence diversity is to be introduced in the scaffold polypeptide sequence encode all possible amino acid variations from a diverse set of amino acids as described above. This includes N oligonucleotides per N sequence variations, where N is the number of different sequences at a locus. The N oligonucleotides are identical in sequence, except for the nucleotide(s) encoding the variant amino acid(s). In generating the sequence-varied oligonucleotides, it can be advantageous to utilize parallel or pooled synthesis strategies in which a single synthesis reaction or set of reagents is used to make common portions of each oligonucleotide. This can be performed e.g., by well-known solid-phase nucleic acid synthesis techniques, or, e.g., utilizing array-based oligonucleotide synthetic methods (see e.g., Fodor et al. (1991) Science, 251: 767-777; Fodor (1997) "Genes, Chips and the Human Genome" FASEB Journal. 11:121-121; Fodor (1997) "Massively Parallel Genomics" Science. 277:393-395; and Chee et al. (1996) "Accessing Genetic Information with High-Density DNA Arrays" Science 274:610-614).

In typical synthesis strategies the oligonucleotides have at least about 10 bases of sequence identity to either side of a region of variance to ensure reasonably efficient recombination. However, flanking regions with identical bases can have fewer identical bases (e.g., 4, 5, 6, 7, 8, or 9) and can, of course, have larger regions of identity (e.g., 11, 12, 13, 14, 15, 16, 17, 18, 19, 20, 25, 30, 50, or more).

The oligonucleotides to be assembled together are incubated to allow hybridization between oligonucleotides containing overlapping complementary sequences. Each set of hybridizing overlapping oligonucleotides thereby forms a contiguous nucleic acid interrupted by small gaps. These small gaps can be filled to form full length sequences using any of a variety of polymerase-mediated reassembly methods, e.g., as described herein and as known to one of skill. The greatest sequence diversity is introduced in oligonucleotides encoding the plant scaffold polypeptide sequence putative binding regions and residues. However, oligonucleotides encoding specific sequence variations can be "spiked" in the recombination mixture at any selected concentration, thus causing preferential incorporation of desirable modifications into the encoded plant chimeric binding proteins in regions outside of the putative binding domains.

For example, during oligonucleotide elongation, hybridized oligonucleotides are incubated in the presence of a nucleic acid polymerase, e.g., Taq, Klenow, or the like, and dNTP's (i.e., dATP, dCTP, dGTP and dTTP). If regions of sequence identity are large, Taq or other high-temperature polymerase can be used with a hybridization temperature of between about room temperature (i.e., about 25 °C) and, e.g., about 65 °C. If the areas of identity are small, Klenow, Taq or polymerases can be used with a hybridization temperature of below room temperature. The polymerase can be added to the assembly reaction prior to, simultaneously with, or after hybridization of the oligonucleotides. Afterwards, the resulting elongated double-stranded nucleic acid sequences are denatured, hybridized, and elongated again. This cycle can be repeated for any desired number of times. The cycle is repeated e.g., from about 2 to about 100 times.

Optionally, after multiple cycles of combinatorial nucleic acid assembly, the resulting products can be amplified, e.g., by standard polymerase chain reaction (PCR). A portion of the volume of the above-described assembly reaction is incubated with unique forward and reverse primers that hybridize universally to the ends of the nucleic acids, as well as dNTPs and a suitable polymerase (e.g., *pfu* polymerase). The PCR reaction is then carried out for about 10 to 40 cycles.

To determine the extent of oligonucleotide incorporation any approach which distinguishes similar nucleic acids can be used. For example, the nucleic acids can be cloned and sequenced, or amplified (*in vitro* or by cloning, e.g., into a standard cloning or expression vector) and cleaved with a restriction enzyme which specifically recognizes a particular oligonucleotide sequence variant.

It is useful to include rare restriction sites (e.g., Not I) in the 5' ends of the 5' and 3' most primers used either in the assembly or PCR reactions. Inclusion of restriction sites in these primers facilitates subcloning of the nucleic acids into a vector by restriction digestion and subsequent ligation. Alternatively, the assembly reaction or PCR products can also be subcloned, without being restriction digested, using standard methods, e.g., "TA" cloning.

Other methods for introducing diversity into a plant scaffold polypeptide sequence can also be used. For example, a scaffold polypeptide sequence can be encoded in a nucleic acid template, e.g., a plasmid contruct. Alternatively, a PCR product, mRNA or genomic DNA from an appropriate plant species such as soybean may also serve as a template encoding a plant scaffold polypeptide sequence. One or more scaffold polypeptide sequence subsequences to be diversified (e.g., the X₂ region of a PAP scaffold polypeptide sequence) can be diversified during or after amplification from the scaffold polypeptide sequence nucleic acid template by any of a number of error-prone PCR methods. Error-prone PCR methods can be divided into (a) methods that reduce the fidelity of the polymerase by unbalancing nucleotides concentrations and/or adding of chemical compounds such as manganese chloride (see, e.g., Lin-Goerke et al. (1997) Biotechniques, 23, 409-412), (b) methods that employ nucleotide analogs (see, e.g., U.S. Patent No. 6,153,745), (c) methods that utilize 'mutagenic' polymerases (see, e.g., Cline, J. and Hogrefe,H.H. (2000) Strategies (Stratagene Newsletter), 13, 157-161 and (d) combined methods (see, e.g., Xu, H., Petersen, E.I., Petersen, S.B. and el-Gewely, M.R. (1999) Biotechniques, 27, 1102-1108. Other PCR-based mutagenesis methods include those, e.g., described by Osuna J, Yanez J, Soberon X, and Gaytan P. (2004), Nucleic Acids Res. 2004, 32(17):e136 and Wong TS, Tee KL, Hauer B, and Schwaneberg, Nucleic Acids Res. 2004 Feb 10;32(3):e26), and others known in the art.

After generating a population of sequence variants, these can be substituted into the appropriate region of a chosen plant scaffold polypeptide sequence nucleic acid (e.g., a plasmid containing a scaffold polypeptide sequence) by subcloning which thereby effectively acts as a vector for the library of diversified sequences.

Yet another approach to mutagenizing specific plant scaffold polypeptide sequence regions is the use of a mutagenic F*E*. *coli* strain (see, e.g., Wu et al. (1999), Plant Mol. Biol., 39(2):381-386). A nucleic acid vector containing a target sequence to be mutated is introduced into the mutator strain, which is then propagated. Error-prone DNA replication in the mutator *E*. *coli* strain introduces mutations into the introduced target sequence. The population of altered target sequences is then recovered and subcloned into the appropriate position of a nucleic acid encoding the selected plant scaffold polypeptide sequence to generate a diverse library of nucleic acids encoding plant chimeric binding proteins.

### III. Expression and Screening of Plant chimeric binding proteins

The library of nucleic acids based on a plant scaffold polypeptide sequence and encoding plant chimeric binding polypeptides are subcloned into an expression vector and introduced into a biological replication system to generate an expression library. The expression library can be propagated and screened to identify plant chimeric binding proteins that bind a target molecule (TM) of interest (e.g., a nematode, insect, fungal, viral or plant protein).

The biological replication system on which screening of plant chimeric binding proteins will be practiced should be capable of growth in a suitable environment, after selection for binding to a target. Alternatively, the nucleic acid encoding the selected plant chimeric binding protein can be isolated by in vitro amplification. During at least part of the growth of the biological replication system, the increase in number is preferably approximately exponential with respect to time. The frequency of library members that exhibits the desired binding properties may be quite low, for example, one in 10⁶ or less.

Biological replication systems can be bacterial DNA viruses, vegetative bacterial cells, bacterial spores. Eukaryotic cells (e.g., yeast cells) can also be used as a biological replication system.

In a particularly useful embodiment, a chimeric binding protein-phage coat protein fusion is encoded in a phagemid construct. The phagemid constructs are transformed into host bacteria, which are subsequently infected with a helper phage that expresses wild type coat proteins. The resulting phage progeny have protein coats that include both fusion protein and wild-type coat proteins. This approach has the advantage that phage viability is greater compared to viability of phage that have exclusively chimeric binding protein-coat fusion proteins. Phagemid-based display library construction and screening kits are commercially available, e.g., the EZnet™ Phage Display cDNA Library Construction Kit and Screening Kit (Maxim Biotech, Inc., San Francisco, CA).

Nonetheless, a strain of any living cell or virus is potentially useful if the strain can be: 1) genetically altered with reasonable facility to encode a plant chimeric binding protein, 2) maintained and amplified in culture, 3) manipulated to display the potential binding protein domain where it can interact with the target material, and 4) selected while retaining the genetic information encoding the expressed plant chimeric binding protein in recoverable form. Preferably, the biological replication system remains viable after affinity-based selection.

When the biological replication system is a bacterial cell or a phage which is assembled in the periplasm, the expression vector for display of the plant chimeric binding protein encodes the chimeric binding protein itself fused to two additional components. The first component is a secretion signal which directs the initial expression product to the inner membrane of the cell (a host cell when the package is a phage). This secretion signal is cleaved off by a signal peptidase to yield a processed, mature, plant chimeric binding protein. The second component is an outer surface transport signal which directs the biological replication system to assemble the processed protein into its outer surface. This outer surface transport signal can be derived from a surface protein native to the biological replication system (e.g., the M13 phage coat protein gIII).

For example, the expression vector comprises a DNA encoding a plant chimeric binding protein operably linked to a signal sequence (e.g., the signal sequences of the bacterial phoA or bla genes or the signal sequence of M 13 phage qene III) and to DNA encoding a coat protein (e.g., the M 13 gene III or gene VIII proteins) of a filamentous phage (e.g., M 13). The expression product is transported to the inner membrane (lipid bilayer) of the host cell, whereupon the signal peptide is cleaved off to leave a processed hybrid protein. The C-terminus of the coat protein-like component of this hybrid protein is trapped in the lipid bilayer, so that the hybrid protein does not escape into the periplasmic space. As the single-stranded DNA of the nascent phage particle passes into the periplasmic space, it collects both wild-type coat protein and the hybrid protein from the lipid bilayer. The hybrid protein is thus packaged into the surface sheath of the filamentous phage, leaving the plant chimeric binding protein exposed on its outer surface. Thus, the filamentous phage, not the host bacterial cell, is the biological replication system in this embodiment. If a secretion signal is necessary for the display of the plant chimeric binding protein, a "secretion-permissive" bacterial strain can be used for growth of the filamentous phage biological replication system.

It is unnecessary to use an inner membrane secretion signal when the biological replication system is a bacterial spore, or a phage whose coat is assembled intracellularly. In these cases, the display means is merely the outer surface transport signal, typically a derivative of a spore or phage coat protein.

Filamentous phage in general are attractive as biological replication systems for display of plant chimeric binding proteins, and M13 in particular, is especially attractive because: 1) the 3D structure of the virion is known; 2) the processing of the coat protein is well understood; 3) the genome is expandable; 4) the genome is small; 5) the sequence of the genome is known; 6) the virion is physically resistant to shear, heat, cold, urea, guanidinium Cl, low pH, and high salt; 7) the phage is a sequencing vector so that sequencing is especially easy; 8) antibiotic-resistance genes have been cloned into the genome; 9) It is easily cultured and stored, with no unusual or expensive media requirements for the infected cells, 10) it has a high burst size, each infected cell yielding 100 to 1000 M13 progeny after infection; and 11) it is easily harvested and concentrated by standard methods.

For example, when the biological replication system is M 13 the gene III or the gene VIII proteins can be used as an outer surface targeting signal. Alternatively, the proteins from genes VI, VII, and IX may also be used.

The encoded plant chimeric binding protein can be fused to the surface targeting signal (e.g., the M13 gene III coat protein) at its carboxy or amino terminal. The fusion boundary between the plant chimeric binding protein and the targeting signal can also include a short linker sequence (e.g., up to 20 amino acids long) to avoid undesirable interactions between the chimeric binding protein and the fused targeting signal. In some embodiments it is advantageous to include within the linker sequence a specific proteolytic cleavage site. In addition, the amino terminal or carboxy terminal of the fused protein can include a short epitope tag (e.g., a hemaglutinin tag). Inclusion of a proteolytic cleavage site or a short epitope tag is particularly useful for purification of a library of chimeric binding proteins from a population of cells expressing the library. Epitope-tagged chimeric binding proteins can be conveniently purified by proteolytic cleavage of linker sequence followed by affinity chromatography utilizing an antibody or other binding agent that recognizes the epitope tag.

Many methods exist for screening phage display libraries (see, e.g., Willats (2002), Plant Mol. Biol., 50:837-854). As commonly practiced, the target molecule of interest is adsorbed to a support and then exposed to solutions of phage displaying plant chimeric binding proteins. The target molecule can be immobilized by passive adsorption on a support medium, e.g, tubes, plates, columns, or magnetic beads. Generally, the adsorptive support medium is pre-blocked, e.g., with bovine serum albumin, milk, or gelatin, to reduce non-specific binding of the phage during screening. Alternatively, the target molecule can be biotinylated, so interaction between chimeric binding protein-bearing phage and the target molecule can be carried out in solution. Phage that bind to the target can then be selected using avidin or streptavidin bound to a solid substrate (e.g., beads or a column).

After phage are allowed to interact with the target molecule, non-interacting phage are removed by washing. The remaining, specifically binding phage are then eluted by one of any number of treatments including, e.g., lowering or increasing pH, application of reducing agents, or use of detergents. In one embodiment, a specific proteolytic cleavage site is introduced between the plant chimeric binding protein sequence and the phage coat protein sequence. Thus, phage elution can be accomplished simply by addition of the appropriate protease.

Eluted phage are then amplified by infection of host cells and can subsequently be re-screened by the method just outlined to reduce the number of false positive binders. During each round of phage screening, care should be taken to include growth of the phage on a solid medium rather than exclusively in a liquid medium as this minimizes loss of phage clones that grow sub-optimally.

Plant chimeric binding proteins can also be expressed and screened for binding solely *in vitro* using ribosomal display. An exclusively *in vitro* approach circumvents the requirement to introduce the library of nucleic acids encoding plant chimeric binding proteins into a biological replication system. Methods for screening polypeptides *in vitro* by ribosomal protein display are described in detail, e.g., in U.S. Patent No. 6,589,741. The nucleic acids described in the section above are modified by adding a phage promoter sequence (e.g., a T7 promoter) enabling *in vitro* transcription, a ribosome binding sequence upstream to the start of translation of the encoded plant chimeric binding protein, and a transcription termination sequence (e.g., from phage T3). The modified library of nucleic acids is then transcribed *in vitro* to generate a corresponding mRNA population encoding plant chimeric binding proteins. Plant chimeric binding proteins are then expressed *in vitro* by translating the population of mRNA molecules devoid of stop codons in the correct reading frame in an in vitro translation system, under conditions that allow the formation of polysomes. The polysomes so formed are then brought into contact with a target molecule under conditions that allow the interaction of plant chimeric binding proteins with the target molecule. Polysomes displaying chimeric binding proteins that interact with the target molecule are then separated from non-interacting polysomes displaying no such (poly)peptides; and the mRNA associated with the interacting polysome is then amplified (e.g., by PCR) and sequenced.

Interaction of a plant chimeric binding protein with a target protein can also be detected in a genetic screen. In the screen, the target protein functions as a "bait protein" and each plant chimeric binding protein functions as a potential "prey" protein in a binding assay that utilizes a two-hybrid assay or three-hybrid assay (see, e.g., U.S. Patent No. 5,283,317; Zervos et al. (1993) Cell 72:223-232; Madura et al. (1993) J. Biol. Chem. 268:12046-12054; Bartel et al. (1993) Biotechniques 14:920-924; Iwabuchi et al. (1993) Oncogene 8:1693-1696; Hubsman et al. (2001) Nuc. Acids Res. Feb 15;29(4):E18; and Brent WO94/10300).

A two-hybrid assay can be carried out using a target polypeptide as the bait protein. In sum, the target polypeptide is fused to the LexA DNA binding domain and used as bait. The prey is plant chimeric binding protein library cloned into the active site loop ofTrxA as a fusion protein with an N-terminal nuclear localization signal, a LexA activation domain, and an epitope tag (Colas et al. 1996 Nature 380:548; and Gyuris et al. Cell 1993 75:791). Yeast cells are transformed with bait and prey genes. When the target fusion protein binds to a plant chimeric binding protein fusion protein, the LexA activation domain is brought into proximity with the LexA DNA binding domain and expression of reporter genes or selectable marker genes having an appropriately positioned LexA binding site increases. Suitable reporter genes include fluorescent proteins (e.g., EGFP), enzymes (e.g., luciferase, β-galactosidase, alkaline phosphatase, etc.) Suitable selectable marker genes include, for example, the yeast LEU2 gene.

After identification of one or more target-binding chimeric binding proteins, the isolated nucleic acids encoding the chimeric binding proteins can be mutagenized by the methods described herein, to generate small expression libraries expressing variant chimeric binding proteins. The chimeric binding protein-variant expression libraries can be screened to identify chimeric binding protein variants with improved target binding properties (e.g., increased affinity or specificity).

The following specific examples are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### EXAMPLES

### Example 1 Design and Expression of Plant Scaffold Polypeptide Sequences

Several protein domain families were analyzed for their potential use as scaffolds. A search of PFAM domains (pfam.wustl.edu; see Bateman *et al.* (2004)), restricting the output to *Viridiplantae,* was conducted to limit domains only to those present in green plants. Four protein domain families were selected to develop plant universal molecular recognition libraries; the accessory domain of purple acid phosphatase (PAP), plant cystatins, plant C2 domains and the turn-helix-helix (THH) motif found in ankyrin repeat proteins.

Three purple acid phosphatase scaffolds were designed having the sequence of SEQ ID NOs:34-36. The amino acid sequence of the accessory domain from kidney bean PAP was used as a query sequence to BLAST the NCBI database. When the output was restricted to proteins found in Viridiplantae, 62 unique sequences were identified. From an alignment of these sequences, a consensus plant PAP sequence was generated (SEQ ID NO:34) by selecting the most frequent amino acid at each position in the alignment. The kidney bean (*Phaseolus vulgaris*) PAP was selected as a parental scaffold (SEQ ID NO:35), because of its known structure. A PAP from soybean, *Glycine max,* was also chosen (SEQ ID NO:36), as this species represents a common crop species in which transgenic products are generated.

A set of scaffold polypeptide sequences which contain plant ankyrin-like repeats was also designed. Ankyrin-like repeats are small turn-helix-helix (THH) motifs consisting of approximately 33 amino acids. They are common elements of proteins from all organisms and are often found in tandem arrays of 2 to 20 repeats within a protein.

Three THH scaffolds were generated. These proteins are similar in structure to GA binding protein (GABP-β). This protein consists of THH like amino and carboxy terminal caps with 3 THH internal repeats. In this protein, it is thought that the caps help stabilize the protein by shielding hydrophobic residues found in the internal repeats.

Three hundred and twelve Viridiplantae ankyrin repeats proteins found in PFAM were aligned to aid in designing plant-specific THH scaffolds. A plant consensus THH sequence was generated by selecting the most frequently occurring amino acid at each position. This sequence was termed the plant consensus internal repeat sequence. This sequence was used to search the NCBI databases by BLAST alignment to find the closest natural THH sequence found in plants. A sequence from wheat *(Triticum aestivum)* was found. The designed repeat based on *T. aestivum* contains a substitution of valine for the single cysteine occurring in the *T. aestivum* sequence. Two sets of N and C terminal caps were generated. One set consists of sequences derived from GABP-β and the second set was derived from the plant THH consensus sequence and optimized to resemble the structure of GABP-β. In particular, the N terminal cap has an extended alpha-helical structure, while the C terminal cap has a truncated helix compared to the typical THH repeat.

Three THH scaffolds were designed, one consists of plant consensus N and C caps and two plant consensus internal THH repeats (SEQ ID NO:37). Another consists of plant consensus N and C caps and two wheat internal repeats (SEQ ID NO:38) and the third consists of ankyrin like N and C caps with two wheat internal repeats (SEQ ID NO:39).

The genes encoding the plant scaffold polypeptide sequences were designed for expression testing in plants, bacteria, and on the surface of phage. Codons were selected for plant expression using a publicly available *Glycine max* codon usage table (at kazusa.or.jp/codon, codon usage tabulated from the international DNA sequence databases: status for the year 2000. Nakamura, Y, Gojobori, T and Ikemura, T (2000) *Nucl. Acids Res.* 28:292.). Codon selection was done manually with the aim for the final codon frequency to roughly reflect the natural frequency for *Glycine max.* Rarely used codons (<10% frequency) were not used. Final sequences were checked for potential problematic sequences, including removal of restriction sites needed for cloning, potential plant splice acceptor or donor sites (see website at cbs.dtu.dk/services/NetPgene/), potential mRNA destabilization sequences (ATTTA) and stretches of more than 4 occurrences of the same nucleotide. Any potential problematic sequences were altered in the genes by modifying codon usage. Since the THH sequences have 4 similar repeat sequences within each protein, steps were taken to reduce nucleotide similarity within repeats; the average repeat identity was reduced 10-15% by these means.

Seven constructs were produced using synthetic gene assembly, (three based on THH scaffold polypeptide sequences, two based on PAP scaffold polypeptide sequences, one plant cystatin and one plant C2 domain protein). The three THH scaffold polypeptide sequences were placed into a phagemid vector as fusion sequences with the gene III coat protein (gIII) at its carboxy terminus (Phage 3.2, Maxim Biotech, Inc., South San Francisco, CA). A 6-His tag was included at the 5' end of the gene as well as a c-Myc tag between the scaffold gene and the encoded amino terminus of gIII. The phagemid constructs were then packaged into phage particles and the phage were tested for expression and surface display of the THH scaffold. A phage ELISA using either anti-His and anti-Myc indicated that the THH scaffold proteins were expressed on the surface of phage in phage ELISAs, suggesting that all 3 THH scaffold polypeptide sequence constructs are folding and expressing well on the phage surface. The selected scaffold polypeptide sequences were then used to generate expression vectors to evaluate their expression in transgenic plants by immunoblotting.

Tobacco leaves were injected with *agrobacterium,* LB4404 transformed with THH containing plant expression vectors. Two days later, sections of leaves injected with agrobacterium were harvested, frozen on dry ice, then ground into a fine powder with a pestle. PBS containing 0.2% Tween-20 was added to the fine powder at a 1:1 weight to volume ratio and additional grinding was done. Insoluble material was removed by centrifugation and 10 ul of the remaining supernatant was loaded onto a 4-12% acrylamide SDS page gel (NuPage, Intvitrogen). Proteins were transferred to PVDF membranes. Proteins were detected using a rat anti-HA antibody (Roche) and an anti-rat HRP conjugated secondary antibody (Chemicon). HRP was detected using Amerham Lumigen reagents.

All three THH scaffold were found to be expressed, with the relative level of expression of the three scaffolds being TA-THH > CC-THH >. TC-THH.

## Claims

1. A library of nucleic acids encoding at least ten different polypeptides, wherein
(i) the amino acid sequence of each of the encoded polypeptides comprises an amino acid sequence at least 70% identical to any of SEQ ID NOs: 127-129;
(ii) the amino acid sequence of each of the encoded polypeptides includes amino acids that differ from those of SEQ ID NOs: 127-129 in at least one of positions 13, 14, 33, 35-36, 38, 46-47, 66, 68-69, 71, 79, 80, 99, 101-102, 104, and 112-113 and the amino acid differences are heterogeneous across a plurality of the encoded polypeptides; and
(iii) the amino acid sequence of each of the encoded polypeptides outside of the residues corresponding to positions 13, 14, 33, 35-36, 38, 46-47, 66, 68-69, 71, 79, 80, 99, 101-102, 104, and 112-113 of SEQ ID NOs: 127-129 is homogeneous across a plurality of the encoded polypeptides.

2. The library of claim 1, wherein the amino acid sequence of the polypeptides has at least 75% identity to any of SEQ ID NOs: 127-129.

3. A method of generating the library of claim 1, comprising:
(i) selecting an amino acid sequence corresponding to anyone of SEQ ID NOs: 127-129 to be encoded, wherein the selected sequence differs from those of SEQ ID NOs: 127-129 in at least one of variable positions 13, 14, 33, 35-36, 38, 46-47, 66, 68-69, 71, 79, 80, 99, 101-102, 104, and 112-113;
(ii) chemically providing a first and a second plurality of oligonucleotides, wherein
(a) oligonucleotides of the first plurality encode amino acid subsequences of the selected amino acid sequence; the subsequences being heterogeneous at the encoded variable positions;
(b) oligonucleotides of the second plurality are complementary to nucleotide sequences encoding subsequences of the selected amino acid sequence, the subsequences being heterogeneous at the encoded variable positions; and
(c) the first and second pluralities comprise oligonucleotides have overlapping sequences complementary to one another;
(iii) combining the population of oligonucleotides to form a first mixture;
(iv) incubating the mixture under conditions effective for hybridizing the overlapping complementary sequences to form a plurality of hybridized complementary sequences; and
(v) elongating the plurality of hybridized complementary sequences to form a second mixture containing the library.

4. The method of claim 3, further comprising performing a cycle of denaturing the library by increasing the temperature of the second mixture to a temperature effective for denaturing double stranded DNA, followed by steps (iv) and (v).

5. The method of claim 4, comprising repeating the cycle up to 100 times.

6. The method of claim 5, further comprising amplifying the library by a polymerase chain reaction consisting essentially of the library, a forward primer, and a reverse primer, wherein the forward and reverse primers can hybridize to the 5' and 3' end sequences, respectively, of all nucleic acids in the library.

7. The method of claim 3, wherein amino acids to be encoded for the variable positions, are selected from a subset of alanine, arginine, asparagine, aspartate, cysteine, glutamine, glutamate, glycine, histidine, isoleucine, leucine, lysine, methionine, phenylalanine, proline, serine, threonine, tryptophan, tyrosine, and valine.

8. The method of claim 7, wherein the amino acids selected for the variable positions are selected from a group consisting of an aliphatic, an acidic, a neutral, and an aromatic amino acid.

9. The method of claim 8, wherein the group of amino acids consists of alanine, aspartate, serine, and tyrosine.

## Patentansprüche

1. Bibliothek aus für wenigstens zehn verschiedene Polypeptide codierenden Nukleinsäuren, wobei
(i) die Aminosäuresequenzen der codierten Polypeptide jeweils eine Aminosäuresequenz mit wenigstens 70% Identität zu einer der SEQ ID NO: 127-129 umfassen;
(ii) die Aminosäuresequenzen der codierten Polypeptide jeweils Aminosäuren enthalten, die sich von denen der SEQ ID NO: 127-129 in zumindest einer der Positionen 13, 14, 33, 35-36, 38, 46-47, 66, 68-69, 71, 79, 80, 99, 101-102, 104 und 112-113 unterscheiden, wobei die Aminosäureunterschiede über mehrere der codierten Polypeptide heterogen sind; und
(iii) die Aminosäuresequenzen der codierten Polypeptide jeweils außerhalb der Position 13, 14, 33, 35-36, 38, 46-47, 66, 68-69, 71, 79, 80, 99, 101-102, 104 und 112-113 der SEQ ID NO: 127-129 entsprechenden Reste über mehrere der codierten Polypeptide homogen sind.

2. Bibliothek nach Anspruch 1, wobei die Aminosäuresequenz der Polypeptide eine Identität von wenigstens 75% zu einer der SEQ ID NO: 127-129 aufweist.

3. Verfahren zur Erzeugung der Bibliothek nach Anspruch 1, bei dem man
(i) eine einer der SEQ ID NO: 127-129 entsprechende Aminosäuresequenz zur Codierung auswählt, wobei sich die ausgewählte Sequenz von denen der SEQ ID NO: 127-129 in wenigstens einer der variablen Positionen 13, 14, 33, 35-36, 38, 46-47, 66, 68-69, 71, 79, 80, 99, 101-102, 104 und 112-113 unterscheidet,
(ii) eine erste und eine zweite Gruppe aus jeweils mehreren Oligonukleotiden chemisch bereitstellt, wobei
(a) die mehreren Oligonukleotide der ersten Gruppe für Aminosäureteilsequenzen der ausgewählten Aminosäuresequenz codieren, wobei die Teilsequenzen an den codierten variablen Positionen heterogen sind;
(b) die mehreren Oligonukleotide der zweiten Gruppe zu für Teilsequenzen der ausgewählten Aminosäuresequenz codierenden Nukleotidsequenzen komplementär sind, wobei die Teilsequenzen an den codierten variablen Positionen heterogen sind; und
(c) die jeweils mehreren Oligonukleotide der ersten und der zweiten Gruppe zueinander komplementäre überlappende Sequenzen aufweisen,
(iii) die Population von Oligonukleotiden unter Bildung eines ersten Gemischs vereinigt,
(iv) das Gemisch unter für die Hybridisierung der überlappenden komplementären Sequenzen effektiven Bedingungen inkubiert, so dass mehrere hybridisierte komplementäre Sequenzen gebildet werden, und
(v) die mehreren hybridisierten komplementären Sequenzen unter Bildung eines zweiten, die Bibliothek enthaltenden Gemischs verlängert.

4. Verfahren nach Anspruch 3, bei dem man ferner die Bibliothek einem Denaturierungszyklus unterzieht, indem man die Temperatur des zweiten Gemischs auf eine für die Denaturierung von Doppelstrang-DNA effektive Temperatur anhebt, wonach die Schritte (iv) und (v) erfolgen.

5. Verfahren nach Anspruch 4, bei dem man den Zyklus bis zu 100-mal wiederholt.

6. Verfahren nach Anspruch 5, bei dem man ferner die Bibliothek mittels einer Polymerasekettenreaktion amplifiziert, die im Wesentlichen aus der Bibliothek, einem Vorwärtsprimer und einem Rückwärtsprimer besteht, wobei der Vorwärts- bzw. der Rückwärtsprimer an die 5'- bzw. 3 '-terminalen Sequenzen aller Nukleinsäuren in der Bibliothek hybridisieren kann.

7. Verfahren nach Anspruch 3, wobei für die variablen Positionen zu codierende Aminosäuren aus einer Teilmenge von Alanin, Arginin, Asparagin, Aspartat, Cystein, Glutamin, Glutamat, Glycin, Histidin, Isoleucin, Leucin, Lysin, Methionin, Phenylalanin, Prolin, Serin, Threonin, Tryptophan, Tyrosin und Valin ausgewählt werden.

8. Verfahren nach Anspruch 7, wobei die für die variablen Positionen ausgewählten Aminosäuren aus einer aus einer aliphatischen, einer sauren, einer neutralen und einer aromatischen Aminosäure bestehenden Gruppe ausgewählt werden.

9. Verfahren nach Anspruch 8, wobei die Gruppe von Aminosäuren aus Alanin, Aspartat, Serin und Tyrosin besteht.

## Revendications

1. Banque d'acides nucléiques codant pour au moins dix polypeptides différents, dans laquelle
(i) la séquence d'acides aminés de chacun des polypeptides codés comprend une séquence d'acides aminés qui est identique au moins à 70% à l'une quelconque des identifications de séquences n° : 127 à 129 ;
(ii) la séquence d'acides aminés de chacun des polypeptides codés comprend des acides aminés qui diffèrent de ceux des identifications de séquences n° : 127 à 129 aux une ou plusieurs des positions 13, 14, 33, 35-36, 38, 46-47, 66, 68-69, 71, 79, 80, 99, 101-102, 104, et 112-113 les différences d'acides aminés sont hétérogènes pour plusieurs des polypeptides codés ; et
(iii) la séquence d'acides aminés de chacun des polypeptides codés, en dehors des résidus correspondant aux positions 13, 14, 33, 35-36, 38, 46-47, 66, 68-69, 71, 79, 80, 99, 101-102, 104, et 112-113 des identifications de séquences n° : 127 à 129, est homogène pour plusieurs des polypeptides codés.

2. Banque selon la revendication 1, dans laquelle la séquence d'acides aminés des polypeptides a une identité d'au moins 75% avec l'une quelconque des identifications de séquences n° : 127 à 129.

3. Procédé de génération de la banque selon la revendication 1, comprenant les étapes consistant à :
(i) choisir une séquence d'acides aminés correspondant à l'une quelconque des identifications de séquences n° : 127 à 129 devant être codées, dans laquelle la séquence choisie diffère de celles des identifications de séquences n° : 127 à 129 au niveau d'une au moins des positions variables 13, 14, 33, 35-36, 38, 46-47, 66, 68-69, 71, 79, 80, 99, 101-102, 104, et 112-113 ;
(ii) fournir par voie chimique une première et une deuxième pluralité d'oligonucléotides, dans lesquelles
(a) les oligonucléotides de la première pluralité codent pour des sous-séquences d'acides aminés de la séquence d'acides aminés choisie ; les sous-séquences étant hétérogènes au niveau des positions variables codées ;
(b) les oligonucléotides de la deuxième pluralité sont complémentaires à des séquences nucléotidiques qui codent pour des sous-séquences de la séquence d'acides aminés choisie, les sous-séquences étant hétérogènes au niveau des positions variables codées ; et
(c) les première et deuxième pluralités comprennent des oligonucléotides ayant des séquences chevauchantes qui sont complémentaires les unes des autres ;
(iii) combiner la population d'oligonucléotides pour former un premier mélange ;
(iv) incuber le mélange dans des conditions étant efficaces pour hybrider les séquences chevauchantes complémentaires, pour former une pluralité de séquences complémentaires hybridées ; et
(v) allonger la pluralité de séquences complémentaires hybridées pour former un deuxième mélange contenant la banque.

4. Procédé selon la revendication 3, comprenant en outre l'étape consistant à effectuer un cycle de dénaturation de la banque, en augmentant la température du deuxième mélange jusqu'à une température efficace pour dénaturer un ADN en double brin, suivie des étapes (iv) et (v).

5. Procédé selon la revendication 4, comprenant la répétition du cycle jusqu'à 100 fois.

6. Procédé selon la revendication 5, comprenant en outre l'étape consistant à amplifier la banque par une réaction en chaîne par polymérase, constituée essentiellement par la banque, une amorce avant et une amorce arrière, dans laquelle les amorces avant et arrière peuvent s'hybrider respectivement aux séquences terminales en 5' et 3' de tout acide nucléique dans la banque.

7. Procédé selon la revendication 3, dans lequel les acides aminés devant être codés pour les positions variables sont choisis parmi un sous-ensemble d'alanine, d'arginine, d'asparagine, d'aspartate, de cystéine, de glutamine, de glutamate, de glycine, d'histidine, d'isoleucine, de leucine, de lysine, de méthionine, de phénylalanine, de proline, de sérine, de thréonine, de tryptophane, de tyrosine et de valine.

8. Procédé selon la revendication 7, dans lequel les acides aminés choisis pour les positions variables sont choisis dans un groupe constitué par un acide aminé du type aliphatique, acide, neutre et aromatique.

9. Procédé selon la revendication 8, dans lequel le groupe d'acides aminés est constitué par l'alanine, l'aspartate, la sérine, et la tyrosine.


### SEQUENCE LISTING

<110> Divergence, Inc.
<120> PLANT CHIMERIC BINDING POLYPEPTIDES FOR UNIVERSAL MOLECULAR RECOGNITION
<130> 12557-0027EP2
<140> PCT/US2007/003937
   <141> 2007-02-13
<150> US 60/773,086
   <151> 2006-02-13
<160> 837
<170> FastSEQ for windows Version 4.0
<210> 1
   <211> 22
   <212> PRT
   <213> Phaseolus vulgaris
<400> 1
   Pro Gln Gln Val His Ile Thr Gln Gly Asp His Val Gly Lys Ala Val
    1               5                  10                  15
   Ile Val Ser Trp Val Thr
               20
<210> 2
   <211> 22
   <212> PRT
   <213> Phaseolus vulgaris
<400> 2
   Pro Gln Gln Val His Ile Thr Gln Gly Asp Leu Val Gly Lys Ala Val
   1 5 10 15
   Ile Val Ser Trp Val Thr
   20
<210> 3
   <211> 22
   <212> PRT
   <213> Phaseolus vulgaris
<400> 3
   Pro Gln Gln Val His Ile Thr Gln Gly Asp Leu Val Gly Arg Ala Met
   1 5 10 15
   Ile Ile Ser Trp Val Thr
   20
<210> 4
   <211> 22
   <212> PRT
   <213> Phaseolus vulgaris
<400> 4
   Pro Gln Gln Val His Ile Thr Gln Gly Asp Leu Val Gly Lys Ala Val
   1 5 10 15
   Ile Val Ser Trp Val Thr
   20
<210> 5
   <211> 22
   <212> PRT
   <213> Phaseolus vulgaris
<400> 5
   Pro Gln Gln Val His Ile Thr Gln Gly Asp His Val Gly Lys Ala Val
   1 5 10 15
   Ile Val Ser Trp Val Thr
   20
<210> 6
   <211> 22
   <212> PRT
   <213> Phaseolus vulgaris
<400> 6
   Pro Gln Gln Val His Ile Thr Gln Gly Asp His Val Gly Lys Ala Met
   1 5 10 15
   Ile Val Ser Trp Val Thr
   20
<210> 7
   <211> 22
   <212> PRT
   <213> Phaseolus vulgaris
<400> 7
   Pro Gln Gln Val His Ile Thr Gln Gly Asp His Val Gly Lys Ala Met
    1               5                  10                  15
   Ile Val Ser Trp Val Thr
               20
<210> 8
   <211> 22
   <212> PRT
   <213> Phaseolus vulgaris
<400> 8
   Pro Gln Gln Val His Ile Thr Gln Gly Asp His Glu Gly Lys Thr Val
    1               5                  10                  15
   Ile Val Ser Trp Val Thr
               20
<210> 9
   <211> 22
   <212> PRT
   <213> Phaseolus vulgaris
<400> 9
   Pro Gln Gln Val His Ile Thr Gln Gly Asp Leu Val Gly Gln Ala Met
    1               5                  10                  15
   Ile Ile Ser Trp Val Thr
               20
<210> 10
   <211> 22
   <212> PRT
   <213> Phaseolus vulgaris
<400> 10
   Pro Gln Gln Val His Ile Thr Gln Gly Asp Leu Val Gly Gln Ala Met
    1               5                  10                  15
   Ile Ile Ser Trp Val Thr
               20
<210> 11
   <211> 22
   <212> PRT
   <213> Phaseolus vulgaris
<400> 11
   Pro Gln Gln Val His Ile Thr Gln Gly Asp His Val Gly Lys Ala Met
   1               5                   10                  15
   Ile Val Ser Trp Val Thr
               20
<210> 12
   <211> 22
   <212> PRT
   <213> Phaseolus vulgaris
<400> 12
   Pro Gln Gln Val His Ile Thr Gln Gly Asp Leu Glu Gly Glu Ala Met
   1               5                   10                  15
   Ile Ile Ser Trp Val Arg
               20
<210> 13
   <211> 22
   <212> PRT
   <213> Phaseolus vulgaris
<400> 13
   Pro Gln Gln Val His Ile Thr Gln Gly Asp His Val Gly Lys Ala Val
   1               5                   10                  15
   Ile Val Ser Trp Val Thr
               20
<210> 14
   <211> 22
   <212> PRT
   <213> Phaseolus vulgaris
<400> 14
   Pro Gln Gln Val His Ile Thr Gln Gly Asp His Val Gly Gln Ala Met
   1               5                   10                  15
   Ile Ile Ser Trp Val Thr
               20
<210> 15
   <211> 22
   <212> PRT
   <213> Phaseolus vulgaris
<400> 15
   Pro Gln Gln Val Tyr Ile Thr Gln Gly Asp His Glu Gly Lys Gly Val
   1               5                   10                  15
   Ile Ala Ser Trp Thr Thr
               20
<210> 16
   <211> 22
   <212> PRT
   <213> Phaseolus vulgaris
<400> 16
   Pro Gln Gln Val His Ile Thr Gln Gly Asp Tyr Glu Gly Lys Gly Val
   1               5                   10                  15
   Ile Ile Ser Trp Val Thr
               20
<210> 17
   <211> 22
   <212> PRT
   <213> Phaseolus vulgaris
<400> 17
   Pro Gln Gln Val His Ile Thr Gln Gly Asp Leu Val Gly Arg Ala Met
   1               5                   10                  15
   Ile Ile Ser Trp Val Thr
               20
<210> 18
   <211> 22
   <212> PRT
   <213> Phaseolus vulgaris
<400> 18
   Pro Gln Gln Val His Leu Thr Gln Gly Asp His Val Gly Lys Gly Val
   1               5                   10                  15
   Ile Val Ser Trp Val Thr
               20
<210> 19
   <211> 22
   <212> PRT
   <213> Phaseolus vulgaris
<400> 19
   Pro Gln Gln Val His Ile Thr Gln Gly Asp Val Glu Gly Lys Ala Val
   1               5                   10                  15
   Ile Val Ser Trp Val Thr
               20
<210> 20
   <211> 22
   <212> PRT
   <213> Phaseolus vulgaris
<400> 20
   Pro Gln Gln Val His Val Thr Gln Gly Asn His Glu Gly Asn Gly Val
   1               5                   10                  15
   Ile Ile Ser Trp Val Thr
               20
<210> 21
   <211> 22
   <212> PRT
   <213> Phaseolus vulgaris
<400> 21
   Pro Gln Gln Val His Val Thr Gln Gly Asn His Glu Gly Asn Gly Val
    1              5                   10                  15
   Ile Ile Ser Trp Val Thr
               20
<210> 22
   <211> 22
   <212> PRT
   <213> Phaseolus vulgaris
<400> 22
   Pro Gln Gln Val His Ile Thr Gln Gly Asp Tyr Asp Gly Lys Ala Val
    1              5                   10                  15
   Ile Val Ser Trp Val Thr
               20
<210> 23
   <211> 22
   <212> PRT
   <213> Phaseolus vulgaris
<400> 23
   Pro Gln Gln Val His Ile Thr Gln Gly Asp His Glu Gly Arg Ser Ile
    1              5                   10                  15
   Ile Val Ser Trp Ile Thr
               20
<210> 24
   <211> 22
   <212> PRT
   <213> Phaseolus vulgaris
<400> 24
   Pro Gln Gln Val His Ile Thr Leu Gly Asp Gln Thr Gly Thr Ala Met
    1              5                   10                  15
   Thr Val Ser Trp Val Thr
               20
<210> 25
   <211> 22
   <212> PRT
   <213> Phaseolus vulgaris
<400> 25
   Pro Gln Gln Val His Ile Thr Gln Gly Asp Tyr Asp Gly Lys Ala Val
   1               5                   10                  15
   Ile Val Ser Trp Val Thr
               20
<210> 26
   <211> 22
   <212> PRT
   <213> Phaseolus vulgaris
<400> 26
   Pro Gln Gln Val His Ile Thr Gln Gly Asp Tyr Asp Gly Lys Ala Val
   1               5                   10                  15
   Ile Ile Ser Trp Val Thr
               20
<210> 27
   <211> 22
   <212> PRT
   <213> Phaseolus vulgaris
<400> 27
   Pro Gln Gln Val His Ile Thr Gln Gly Asp Tyr Asp Gly Glu Ala Val
   1               5                   10                  15
   Ile Ile Ser Trp Val Thr
               20
<210> 28
   <211> 22
   <212> PRT
   <213> Phaseolus vulgaris
<400> 28
   Pro Gln Gln Val His Ile Thr Gln Gly Asp Tyr Asp Gly Lys Ala Val
   1               5                   10                  15
   Ile Ile Ser Trp Val Thr
               20
<210> 29
   <211> 22
   <212> PRT
   <213> Phaseolus vulgaris
<400> 29
   Pro Gln Gln Val His Ile Thr Gln Gly Asp Tyr Asp Gly Lys Ala Val
   1               5                   10                  15
   Ile Ile Ser Trp Val Thr
               20
<210> 30
   <211> 22
   <212> PRT
   <213> Phaseolus vulgaris
<400> 30
   Pro Gln Gln Val His Ile Thr Gln Gly Asp Tyr Asn Gly Lys Ala Val
   1               5                   10                  15
   Ile Val Ser Trp Val Thr
               20
<210> 31
   <211> 21
   <212> PRT
   <213> Phaseolus vulgaris
<400> 31
   Val Val Val Tyr Trp Ser Glu Asn Ser Lys Tyr Lys Lys Ser Ala Glu
   1               5                   10                  15
   Gly Thr Val Thr Thr
               20
<210> 32
   <211> 21
   <212> PRT
   <213> Phaseolus vulgaris
<400> 32
   Glu Val His Tyr Trp Ser Glu Asn Ser Asp Lys Lys Lys Ile Ala Glu
   1               5                   10                  15
   Gly Lys Leu Val Thr
               20
<210> 33
   <211> 21
   <212> PRT
   <213> Phaseolus vulgaris
<400> 33
   Ala Val Arg Tyr Trp Ser Glu Lys Asn Gly Arg Lys Arg Ile Ala Lys
   1               5                   10                  15
   Gly Lys Met Ser Thr
               20
<210> 34
   <211> 21
   <212> PRT
   <213> Phaseolus vulgaris
<400> 34
   Glu Val His Tyr Trp Ser Glu Asn Ser Asp Lys Lys Lys Ile Ala Glu
   1 5 10 15
   Gly Lys Leu Val Thr
   20
<210> 35
   <211> 21
   <212> PRT
   <213> Phaseolus vulgaris
<400> 35
   Ala Val Arg Tyr Trp Ser Lys Asn Ser Lys Gln Lys Arg Leu Ala Lys
   1 5 10 15
   Gly Lys Ile Val Thr
   20
<210> 36
   <211> 21
   <212> PRT
   <213> Phaseolus vulgaris
<400> 36
   Lys Val Val Tyr Trp Ser Glu Asn Ser Gln His Lys Lys Val Ala Lys
   1 5 10 15
   Gly Asn Ile Arg Thr
   20
<210> 37
   <211> 19
   <212> PRT
   <213> Phaseolus vulgaris
<400> 37
   Lys Val Val Tyr Trp Ser Glu Asn Ser Gln His Lys Lys Val Arg Asn
   1 5 10 15
   Ile Arg Thr
<210> 38
   <211> 21
   <212> PRT
   <213> Phaseolus vulgaris
<400> 38
   Thr Val Leu Tyr Trp Ser Glu Lys Ser Lys Gln Lys Asn Thr Ala Lys
   1 5 10 15
   Gly Lys Val Thr Thr
   20
<210> 39
   <211> 21
   <212> PRT
   <213> Phaseolus vulgaris
<400> 39
   Gln Val Ile Tyr Trp Ser Asp Ser Ser Leu Gln Asn Phe Thr Ala Glu
   1               5                   10                  15
   Gly Glu Val Phe Thr
               20
<210> 40
   <211> 21
   <212> PRT
   <213> Phaseolus vulgaris
<400> 40
   Gln Val Ile Tyr Trp Ser Asp Ser Ser Leu Gln Asn Phe Thr Ala Glu
   1               5                   10                  15
   Gly Glu Val Phe Thr
               20
<210> 41
   <211> 21
   <212> PRT
   <213> Phaseolus vulgaris
<400> 41
   Thr Val Leu Tyr Trp Ser Asn Asn Ser Lys Gln Lys Asn Lys Ala Thr
   1               5                   10                  15
   Gly Ala Val Thr Thr
               20
<210> 42
   <211> 21
   <212> PRT
   <213> Phaseolus vulgaris
<400> 42
   Lys Val Leu Tyr Trp Ile Asp Gly Ser Asn Gln Lys His Ser Ala Asn
   1               5                   10                  15
   Gly Lys Ile Thr Lys
               20
<210> 43
   <211> 21
   <212> PRT
   <213> Phaseolus vulgaris
<400> 43
   Thr Val Val Tyr Trp Ser Glu Lys Ser Lys Leu Lys Asn Lys Ala Asn
   1 5 10 15
   Gly Lys Val Thr Thr
   20
<210> 44
   <211> 21
   <212> PRT
   <213> Phaseolus vulgaris
<400> 44
   Glu Val Ile Tyr Trp Ser Asn Ser Ser Leu Gln Asn Phe Thr Ala Glu
   1 5 10 15
   Gly Glu Val Phe Thr
   20
<210> 45
   <211> 21
   <212> PRT
   <213> Phaseolus vulgaris
<400> 45
   Ser Val Leu Tyr Trp Ala Glu Asn Ser Asn Val Lys Ser Ser Ala Glu
   1 5 10 15
   Gly Phe Val Val Ser
   20
<210> 46
   <211> 21
   <212> PRT
   <213> Phaseolus vulgaris
<400> 46
   Thr Val Val Tyr Trp Ala Glu Asn Ser Ser Val Lys Arg Arg Ala Asp
   1 5 10 15
   Gly Val Val Val Thr
   20
<210> 47
   <211> 21
   <212> PRT
   <213> Phaseolus vulgaris
<210> 48
   <211> 21
   <212> PRT
   <213> Phaseolus vulgaris
<400> 48
   Lys Val Leu Tyr Trp Glu Phe Asn Ser Lys Ile Lys Gln Ile Ala Lys
   1               5                   10                  15
   Gly Thr Val Ser Thr
               20
<210> 49
   <211> 21
   <212> PRT
   <213> Phaseolus vulgaris
<400> 49
   Lys Val Ile Tyr Trp Lys Glu Asn Ser Thr Lys Lys His Lys Ala His
   1               5                   10                  15
   Gly Lys Thr Asn Thr
               20
<210> 50
   <211> 21
   <212> PRT
   <213> Phaseolus vulgaris
<400> 50
   Thr Val Arg Tyr Trp Cys Glu Asn Lys Lys Ser Arg Lys Gln Ala Glu
   1               5                   10                  15
   Ala Thr Val Asn Thr
               20
<210> 51
   <211> 21
   <212> PRT
   <213> Phaseolus vulgaris
<400> 51
   Thr Val Gln Tyr Trp Cys Glu Asn Glu Lys Ser Arg Lys Gln Ala Glu
   1               5                   10                  15
   Ala Thr Val Asn Thr
               20
<210> 52
   <211> 21
   <212> PRT
   <213> Phaseolus vulgaris
<400> 52
   Lys Val Gln Phe Gly Thr Ser Glu Asn Lys Phe Gln Thr Ser Ala Glu
   1               5                   10                  15
   Gly Thr Val Ser Asn
               20
<210> 53
   <211> 21
   <212> PRT
   <213> Phaseolus vulgaris
<400> 53
   Thr Val Phe Tyr Gly Thr Ser Glu Asn Lys Leu Asp Gln His Ala Glu
   1               5                   10                  15
   Gly Thr Val Thr Met
               20
<210> 54
   <211> 21
   <212> PRT
   <213> Phaseolus vulgaris
<400> 54
   Thr Val Arg Tyr Gly Ser Ser Pro Glu Lys Leu Asp Arg Ala Ala Glu
   1               5                   10                  15
   Gly Ser His Thr Arg
               20
<210> 55
   <211> 21
   <212> PRT
   <213> Phaseolus vulgaris
<400> 55
   Glu Val Val Tyr Gly Thr Ser Pro Asn Ser Tyr Asp His Ser Ala Gln
   1               5                   10                  15
   Gly Lys Thr Thr Asn
               20
<210> 56
   <211> 21
   <212> PRT
   <213> Phaseolus vulgaris
<400> 56
   His Ile Gln Tyr Gly Thr Ser Glu Asn Lys Phe Gln Thr Ser Glu Glu
   1               5                   10                  15
   Gly Thr Val Thr Asn
               20
<210> 57
   <211> 21
   <212> PRT
   <213> Phaseolus vulgaris
<400> 57
   Glu Val Arg Tyr Gly Leu Ser Glu Gly Lys Tyr Asp Val Thr Val Glu
   1               5                   10                  15
   Gly Thr Leu Asn Asn
               20
<210> 58
   <211> 21
   <212> PRT
   <213> Phaseolus vulgaris
<400> 58
   Gln Val His Tyr Gly Ala Val Gln Gly Lys Tyr Glu Phe Val Ala Gln
   1               5                   10                  15
   Gly Thr Tyr His Asn
               20
<210> 59
   <211> 21
   <212> PRT
   <213> Phaseolus vulgaris
<400> 59
   Gln Val His Tyr Gly Ala Val Gln Gly Lys Tyr Glu Phe Val Ala Gln
   1               5                   10                  15
   Gly Thr Tyr His Asn
               20
<210> 60
   <211> 21
   <212> PRT
   <213> Phaseolus vulgaris
<400> 60
   Glu Val Leu Tyr Gly Lys Asn Glu His Gln Tyr Asp Gln Arg Val Glu
   1               5                   10                  15
   Gly Thr Val Thr Asn
               20
<210> 61
   <211> 19
   <212> PRT
   <213> Phaseolus vulgaris
<400> 61
   Tyr Ile His His Cys Tyr Ile Lys Gly Leu Glu Tyr Asp Thr Lys Tyr
   1 5 10 15
   Tyr Tyr Val
<210> 62
   <211> 19
   <212> PRT
   <213> Phaseolus vulgaris
<400> 62
   Phe Ile His His Thr Thr Ile Arg Asn Leu Glu Tyr Lys Thr Lys Tyr
   1 5 10 15
   Tyr Tyr Glu
<210> 63
   <211> 19
   <212> PRT
   <213> Phaseolus vulgaris
<400> 63
   Phe Ile His His Thr Thr Ile Arg Lys Leu Lys Tyr Asn Thr Lys Tyr
   1 5 10 15
   Tyr Tyr Glu
<210> 64
   <211> 19
   <212> PRT
   <213> Phaseolus vulgaris
<400> 64
   Phe Ile His His Thr Thr Ile Arg Asn Leu Glu Tyr Lys Thr Lys Tyr
   1 5 10 15
   Tyr Tyr Glu
<210> 65
   <211> 19
   <212> PRT
   <213> Phaseolus vulgaris
<400> 65
   Phe Ile His His Thr Thr Ile Arg Asn Leu Glu Tyr Asn Thr Lys Tyr
   1 5 10 15
   Tyr Tyr Glu
<210> 66
   <211> 19
   <212> PRT
   <213> Phaseolus vulgaris
<400> 66
   Tyr Ile His His Cys Thr Ile Arg Asn Leu Glu Tyr Asn Thr Lys Tyr
   1               5                   10                  15
   Tyr Tyr Glu
<210> 67
   <211> 19
   <212> PRT
   <213> Phaseolus vulgaris
<400> 67
   Tyr Ile His His Cys Thr Ile Arg Asn Leu Glu Tyr Asn Thr Lys Tyr
   1               5                   10                  15
   Tyr Tyr Glu
<210> 68
   <211> 19
   <212> PRT
   <213> Phaseolus vulgaris
<400> 68
   Tyr Ile His His Ser Thr Ile Arg His Leu Glu Phe Asn Thr Lys Tyr
   1               5                   10                  15
   Tyr Tyr Lys
<210> 69
   <211> 19
   <212> PRT
   <213> Phaseolus vulgaris
<400> 69
   Phe Ile His His Thr Thr Ile Thr Asn Leu Glu Phe Asp Thr Thr Tyr
   1               5                   10                  15
   Tyr Tyr Glu
<210> 70
   <211> 19
   <212> PRT
   <213> Phaseolus vulgaris
<400> 70
   Phe Ile His His Thr Thr Ile Thr Asn Leu Glu Phe Asp Thr Thr Tyr
   1               5                   10                  15
   Tyr Tyr Glu
<210> 71
   <211> 19
   <212> PRT
   <213> Phaseolus vulgaris
<400> 71
   Tyr Ile His His Cys Ile Ile Lys His Leu Lys Phe Asn Thr Lys Tyr
   1               5                   10                  15
   Tyr Tyr Glu
<210> 72
   <211> 19
   <212> PRT
   <213> Phaseolus vulgaris
<400> 72
   Phe Ile His His Cys Thr Ile Arg Arg Leu Lys His Asn Thr Lys Tyr
   1               5                   10                  15
   His Tyr Glu
<210> 73
   <211> 19
   <212> PRT
   <213> Phaseolus vulgaris
<400> 73
   Tyr Ile His His Cys Asn Ile Lys Asn Leu Lys Phe Asp Thr Lys Tyr
   1               5                   10                  15
   Tyr Tyr Lys
<210> 74
   <211> 19
   <212> PRT
   <213> Phaseolus vulgaris
<400> 74
   Phe Ile His His Thr Asn Ile Thr Asn Leu Glu Phe Asn Thr Thr Tyr
   1               5                   10                  15
   Phe Tyr Val
<210> 75
   <211> 19
   <212> PRT
   <213> Phaseolus vulgaris
<400> 75
   Tyr Ile His His Cys Thr Ile Lys Asp Leu Glu Phe Asp Thr Lys Tyr
   1               5                   10                  15
   Tyr Tyr Glu
<210> 76
   <211> 19
   <212> PRT
   <213> Phaseolus vulgaris
<400> 76
   Tyr Ile His His Cys Thr Ile Lys Asp Leu Glu Tyr Asp Thr Lys Tyr
   1 5 10 15
   Tyr Tyr Glu
<210> 77
   <211> 19
   <212> PRT
   <213> Phaseolus vulgaris
<400> 77
   Tyr Ile His His Cys Thr Ile Lys Asn Leu Glu Tyr Asn Thr Lys Tyr
   1 5 10 15
   Phe Tyr Glu
<210> 78
   <211> 19
   <212> PRT
   <213> Phaseolus vulgaris
<400> 78
   Tyr Ile His His Cys Thr Ile Gln Asn Leu Lys Tyr Asn Thr Lys Tyr
   1 5 10 15
   Tyr Tyr Met
<210> 79
   <211> 19
   <212> PRT
   <213> Phaseolus vulgaris
<400> 79
   Phe Ile His His Cys Pro Ile Arg Asn Leu Glu Tyr Asp Thr Lys Tyr
   1 5 10 15
   Tyr Tyr Val
<210> 80
   <211> 19
   <212> PRT
   <213> Phaseolus vulgaris
<400> 80
   Tyr Ile His His Cys Leu Ile Asp Asp Leu Glu Phe Asp Thr Lys Tyr
   1 5 10 15
   Tyr Tyr Glu
<210> 81
   <211> 19
   <212> PRT
   <213> Phaseolus vulgaris
<400> 81
   Tyr Ile His His Cys Leu Ile Asp Asp Leu Glu Phe Asp Thr Lys Tyr
   1 5 10 15
   Tyr Tyr Glu
<210> 82
   <211> 19
   <212> PRT
   <213> Phaseolus vulgaris
<400> 82
   Tyr Val His His Cys Leu Ile Glu Gly Leu Glu Tyr Lys Thr Lys Tyr
   1 5 10 15
   Tyr Tyr Arg
<210> 83
   <211> 19
   <212> PRT
   <213> Phaseolus vulgaris
<400> 83
   Tyr Ile His His Cys Val Leu Thr Asp Leu Lys Tyr Asp Arg Lys Tyr
   1 5 10 15
   Phe Tyr Lys
<210> 84
   <211> 19
   <212> PRT
   <213> Phaseolus vulgaris
<400> 84
   Phe Ile His His Cys Thr Leu Thr Gly Leu Thr His Ala Thr Lys Tyr
   1 5 10 15
   Tyr Tyr Ala
<210> 85
   <211> 19
   <212> PRT
   <213> Phaseolus vulgaris
<400> 85
   Tyr Ile His His Cys Leu Leu Asp Lys Leu Glu Tyr Asp Thr Lys Tyr
   1               5                   10                  15
   Tyr Tyr Lys
<210> 86
   <211> 19
   <212> PRT
   <213> Phaseolus vulgaris
<400> 86
   Tyr Ile His His Cys Leu Ile Glu Gly Leu Glu Tyr Glu Thr Lys Tyr
   1               5                   10                  15
   Tyr Tyr Arg
<210> 87
   <211> 19
   <212> PRT
   <213> Phaseolus vulgaris
<400> 87
   Tyr Ile His Gln Cys Leu Val Thr Gly Leu Gln Tyr Asp Thr Lys Tyr
   1               5                   10                  15
   Tyr Tyr Glu
<210> 88
   <211> 19
   <212> PRT
   <213> Phaseolus vulgaris
<400> 88
   Phe Ile His His Cys Leu Val Ser Asp Leu Glu His Asp Thr Lys Tyr
   1               5                   10                  15
   Tyr Tyr Lys
<210> 89
   <211> 19
   <212> PRT
   <213> Phaseolus vulgaris
<400> 89
   Phe Ile His His Cys Leu Val Ser Asp Leu Glu His Asp Thr Lys Tyr
   1               5                   10                  15
   Tyr Tyr Lys
<210> 90
   <211> 19
   <212> PRT
   <213> Phaseolus vulgaris
<400> 90
   Tyr Ile His His Cys Leu Val Asp Gly Leu Glu Tyr Asn Thr Lys Tyr
   1 5 10 15
   Tyr Tyr Lys
<210> 91
   <211> 7
   <212> PRT
   <213> Phaseolus vulgaris
<400> 91
   Ser Arg Glu Phe Trp Phe Arg
   1 5
<210> 92
   <211> 7
   <212> PRT
   <213> Phaseolus vulgaris
<400> 92
   Thr Arg Gln Phe Trp Phe Val
   1 5
<210> 93
   <211> 7
   <212> PRT
   <213> Phaseolus vulgaris
<400> 93
   Thr Arg Arg Phe Ser Phe Ile
   1 5
<210> 94
   <211> 7
   <212> PRT
   <213> Phaseolus vulgaris
<400> 94
   Thr Arg Gln Phe Trp Phe Val
   1 5
<210> 95
   <211> 7
   <212> PRT
   <213> Phaseolus vulgaris
<400> 95
   Thr Arg Gln Phe Trp Phe Val
   1 5
<210> 96
   <211> 7
   <212> PRT
   <213> Phaseolus vulgaris
<400> 96
   Thr Arg Ser Phe Trp Phe Thr
   1 5
<210> 97
   <211> 7
   <212> PRT
   <213> Phaseolus vulgaris
<400> 97
   Thr Arg Ser Phe Trp Phe Thr
   1 5
<210> 98
   <211> 7
   <212> PRT
   <213> Phaseolus vulgaris
<400> 98
   Ala Arg Thr Phe Trp Phe Val
   1 5
<210> 99
   <211> 7
   <212> PRT
   <213> Phaseolus vulgaris
<400> 99
   Thr Arg Gln Phe Trp Phe Ile
   1 5
<210> 100
   <211> 7
   <212> PRT
   <213> Phaseolus vulgaris
<400> 100
   Thr Arg Gln Phe Trp Phe Ile
   1 5
<210> 101
   <211> 7
   <212> PRT
   <213> Phaseolus vulgaris
<400> 101
   Pro Arg Thr Phe Trp Phe Val
   1 5
<210> 102
   <211> 7
   <212> PRT
   <213> Phaseolus vulgaris
<400> 102
   Val Arg Ser Phe Trp Phe Met
   1 5
<210> 103
   <211> 7
   <212> PRT
   <213> Phaseolus vulgaris
<400> 103
   Ala Arg Thr Phe Trp Phe Thr
   1 5
<210> 104
   <211> 7
   <212> PRT
   <213> Phaseolus vulgaris
<400> 104
   Thr Arg Gln Phe Trp Phe Ile
   1 5
<210> 105
   <211> 7
   <212> PRT
   <213> Phaseolus vulgaris
<400> 105
   Thr Arg Lys Phe Trp Phe Val
   1 5
<210> 106
   <211> 7
   <212> PRT
   <213> Phaseolus vulgaris
<400> 106
   Lys Arg Gln Phe Trp Phe Val
   1 5
<210> 107
   <211> 7
   <212> PRT
   <213> Phaseolus vulgaris
<400> 107
   Thr Arg Gln Phe Trp Phe Thr
   1 5
<210> 108
   <211> 7
   <212> PRT
   <213> Phaseolus vulgaris
<400> 108
   Arg Arg Thr Phe Trp Phe Val
   1 5
<210> 109
   <211> 7
   <212> PRT
   <213> Phaseolus vulgaris
<400> 109
   Glu Arg Lys Phe Trp Phe Phe
   1 5
<210> 110
   <211> 7
   <212> PRT
   <213> Phaseolus vulgaris
<400> 110
   Ser Arg Arg Phe Trp Phe Phe
   1 5
<210> 111
   <211> 7
   <212> PRT
   <213> Phaseolus vulgaris
<400> 111
   Ser Arg Arg Phe Trp Phe Phe
   1 5
<210> 112
   <211> 7
   <212> PRT
   <213> Phaseolus vulgaris
<400> 112
   Ser Arg Glu Phe Trp Phe Glu
   1 5
<210> 113
   <211> 7
   <212> PRT
   <213> Phaseolus vulgaris
<400> 113
   Ala Arg Leu Phe Trp Phe Lys
   1 5
<210> 114
   <211> 7
   <212> PRT
   <213> Phaseolus vulgaris
<400> 114
   Val Arg Thr Phe Ser Phe Thr
   1 5
<210> 115
   <211> 7
   <212> PRT
   <213> Phaseolus vulgaris
<400> 115
   Ala Arg Glu Phe Trp Phe His
   1 5
<210> 116
   <211> 7
   <212> PRT
   <213> Phaseolus vulgaris
<400> 116
   Ser Arg Glu Phe Trp Phe Lys
   1 5

<210> 117
   <211> 7
   <212> PRT
   <213> Phaseolus vulgaris
<400> 117
   Ala Arg Lys Phe Trp Phe Glu
   1 5
<210> 118
   <211> 7
   <212> PRT
   <213> Phaseolus vulgaris
<400> 118
   Ser Arg Glu Phe Trp Phe Val
   1 5
<210> 119
   <211> 7
   <212> PRT
   <213> Phaseolus vulgaris
<400> 119
   Ser Arg Glu Phe Trp Phe Val
   1 5
<210> 120
   <211> 7
   <212> PRT
   <213> Phaseolus vulgaris
<400> 120
   Ala Arg Glu Phe Trp Phe Glu
   1 5
<210> 121
   <211> 7
   <212> PRT
   <213> Artificial sequence
<220>
   <223> Synthetically generated peptide
<220>
   <221> VARIANT
   <222> 1
   <223> xaa = Met, val, Glu, Pro, Gln, Thr, Ala or Phe
<220>
   <221> VARIANT
   <222> 2
   <223> xaa = Asp, Glu, Asn, val, ser, Ile or Ala
<220>
   <221> VARIANT
   <222> 3
   <223> xaa = Glu, Ala, lys or Asp
<220>
   <221> VARIANT
<222> 4
   <223> Xaa = Pro, Lys or Leu
<220>
   <221> VARIANT
   <222> 6
   <223> xaa = Ser, Pro, Lys, His or Thr
<220>
   <221> VARIANT
   <222> 7
   <223> xaa = Ser, Asn, Lys or Thr
<400> 121
   Xaa Xaa Xaa Xaa Gly Xaa Xaa
   1               5
<210> 122
   <211> 9
   <212> PRT
   <213> Artificial sequence
<220>
   <223> synthetically generated peptide
<220>
   <221> VARIANT
   <222> 2
   <223> xaa = Lys, Arg, Thr or Phe
<220>
   <221> VARIANT
   <222> 3
   <223> xaa = Tyr or Phe
<220>
   <221> VARIANT
   <222> 4
   <223> Xaa = Tyr, Phe or His
<220>
   <221> VARIANT
   <222> 5
   <223> Xaa = Asn, Thr, Lys, Asp or Glu
<220>
   <221> VARIANT
   <222> 7
   <223> Xaa = Thr, Ser, Asn, Lys or Glu
<220>
   <221> VARIANT
   <222> 9
   <223> Xaa = Gly or Pro
<400> 122
   Tyr Xaa Xaa Xaa Xaa Tyr Xaa Ser Xaa
   1               5
<210> 123
   <211> 6
   <212> PRT
   <213> Artificial sequence
<220>
   <223> synthetically generated peptide
<220>
   <221> VARIANT
   <222> 1
   <223> Xaa = val, Ile, Leu or Met
<220>
   <221> VARIANT
   <222> 2
   <223> Xaa = Gly or Glu
<220>
   <221> VARIANT
   <222> 3
   <223> Xaa = Leu, Ile, val, Glu, Thr, Ser, Glu, Phe or Lys
<220>
   <221> VARIANT
   <222> 4
   <223> Xaa = Arg, Gly, Glu or Asp
<220>
   <221> VARIANT
   <222> 5
   <223> Xaa = Asn, His, Asp, Gln, Lys, Ser or Glu
<400> 123
   Xaa Xaa Xaa Xaa Xaa Thr
   1 5
<210> 124
   <211> 91
   <212> PRT
   <213> Artificial Sequence
<220>
   <223> synthetically generated peptide
<400> 124
   Pro Gln Gln Val His Ile Thr Gln Gly Asp His Val Gly Lys Ala Val
   1 5 10 15
   Ile Val Ser Trp Val Thr Met Asp Glu Pro Gly Ser Ser Val Val Val
   20 25 30
   Tyr Trp Ser Glu Asn Ser Lys Tyr Lys Lys Ser Ala Glu Gly Thr Val
   35 40 45
   Thr Thr Tyr Arg Phe Tyr Asn Tyr Thr Ser Gly Tyr Ile His His Cys
   50 55 60
   Tyr Ile Lys Gly Leu Glu Tyr Asp Thr Lys Tyr Tyr Tyr Val Val Gly
   65 70 75 80
   Ile Gly Asn Thr Ser Arg Glu Phe Trp Phe Arg
   85 90
<210> 125
   <211> 91
   <212> PRT
   <213> Artificial sequence
<220>
   <223> Synthetically generated peptide
<400> 125
   Pro Gln Gln Val His Ile Thr Gln Gly Asp Leu Val Gly Lys Ala Val
   1 5 10 15
   Ile Val Ser Trp Val Thr Val Asp Glu Pro Gly Ser Ser Glu Val His
   20 25 30
   Tyr Trp Ser Glu Asn Ser Asp Lys Lys Lys Ile Ala Glu Gly Lys Leu
   35 40 45
   Val Thr Tyr Arg Phe Phe Asn Tyr Ser Ser Gly Phe Ile His His Thr
   50 55 60
   Thr Ile Arg Asn Leu Glu Tyr Lys Thr Lys Tyr Tyr Tyr Glu Val Gly
   65 70 75 80
   Leu Gly Asn Thr Thr Arg Gln Phe Trp Phe Val
   85 90
<210> 126
   <211> 91
   <212> PRT
   <213> Artificial sequence
<220>
   <223> synthetically generated peptide
<400> 126
   Pro Gln Gln Val His Ile Thr Gln Gly Asp Leu Val Gly Arg Ala Met
   1               5                   10                  15
   Ile Ile Ser Trp Val Thr Met Asp Glu Pro Gly Ser Ser Ala Val Arg
               20                  25                  30
   Tyr Trp Ser Glu Lys Asn Gly Arg Lys Arg Ile Ala Lys Gly Lys Met
           35                  40                  45
   Ser Thr Tyr Arg Phe Phe Asn Tyr Ser Ser Gly Phe Ile His His Thr
       50                  55                  60
   Thr Ile Arg Lys Leu Lys Tyr Asn Thr Lys Tyr Tyr Tyr Glu Val Gly
   65                  70                  75                  80
   Leu Arg Asn Thr Thr Arg Arg Phe Ser Phe Ile
                   85                  90
<210> 127
   <211> 123
   <212> PRT
   <213> Artificial Sequence
<220>
   <223> synthetically generated peptide
<400> 127
   Gly Asp Asp Leu Gly Lys Lys Leu His Leu Ala Ala Ser Arg Gly His
   1               5                   10                  15
   Leu Glu Ile Val Arg Val Leu Val Glu Ala Gly Ala Asp Val Asn Ala
               20                  25                  30
   Leu Asp Lys Phe Gly Arg Thr Ala Leu His Ile Ala Ala Ser Arg Gly
           35                  40                  45
   His Leu Glu Val Val Lys Leu Leu Leu Glu Ala Gly Ala Asp Val Asn
       50                  55                  60
   Ala Leu Asp Lys Phe Gly Arg Thr Ala Leu His Leu Ala Ala Ser Arg
   65                  70                  75                  80
   Gly His Leu Glu Val Val Lys Leu Leu Leu Glu Ala Gly Ala Asp Val
                   85                  90                  95
   Asn Ala Leu Asp Lys Phe Gly Asp Thr Ala Leu His Val Ser Ile Asp
               100                 105                 110
   Asn Gly Asn Glu Asp Ile Ala Glu Ile Leu Gln
           115                 120
<210> 128
   <211> 123
   <212> PRT
   <213> Artificial Sequence
<220>
   <223> synthetically generated peptide
<400> 128
   Gly Asp Asp Leu Gly Lys Lys Leu His Leu Ala Ala Ser Arg Gly His
   1 5 10 15
   Leu Glu Ile Val Arg Val Leu Val Glu Ala Gly Ala Asp Val Asn Ala
   20 25 30
   Leu Asp Lys Phe Gly Arg Thr Pro Leu His Ile Ala Ala Ser Lys Gly
   35 40 45
   Asn Glu Gln Val Val Lys Leu Leu Leu Glu Ala Gly Ala Asp Pro Asn
   50 55 60
   Ala Leu Asp Lys Phe Gly Arg Thr Pro Leu His Ile Ala Ala Ser Lys
   65 70 75 80
   Gly Asn Glu Gln Val Val Lys Leu Leu Leu Glu Ala Gly Ala Asp Pro
   85 90 95
   Asn Ala Gln Asp Lys Phe Gly Asp Thr Ala Leu His Val Ser Ile Asp
   100 105 110
   Asn Gly Asn Glu Asp Ile Ala Glu Ile Leu Gln
   115 120
<210> 129
   <211> 123
   <212> PRT
   <213> Artificial sequence
<220>
   <223> Synthetically generated peptide
<400> 129
   Gly Ser Asp Leu Gly Lys Lys Leu Leu Glu Ala Ala Arg Ala Gly Gln
   1 5 10 15
   Asp Asp Glu Val Arg Ile Leu Met Ala Asn Gly Ala Asp Val Asn Ala
   20 25 30
   Leu Asp Lys Phe Gly Arg Thr Pro Leu His Ile Ala Ala Ser Lys Gly
   35 40 45
   Asn Glu Gln Val Val Lys Leu Leu Leu Glu Ala Gly Ala Asp Pro Asn
   50 55 60
   Ala Leu Asp Lys Phe Gly Arg Thr Pro Leu His Ile Ala Ala Ser Lys
   65 70 75 80
   Gly Asn Glu Gln Val Val Lys Leu Leu Leu Glu Ala Gly Ala Asp Pro
   85 90 95
   Asn Ala Gln Asp Lys Phe Gly Lys Thr Ala Phe Asp Ile Ser Ile Asp
   100 105 110
   Asn Gly Asn Glu Asp Leu Ala Glu Ile Leu Gln
   115 120
<210> 130
   <211> 102
   <212> PRT
   <213> oryza sativa
<400> 130
   Met Ser Ser Val Gly Gly Pro Val Leu Gly Gly Val Glu Pro Val Gly
   1 5 10 15
   Asn Glu Asn Asp Leu His Leu Val Asp Leu Ala Arg Phe Ala Val Thr
   20 25 30
   Glu His Asn Lys Lys Ala Asn Ser Leu Leu Glu Phe Glu Lys Leu Val
   35 40 45
   Ser Val Lys Gln Gln Val Val Ala Gly Thr Leu Tyr Tyr Phe Thr Leu
   50 55 60
   Glu Val Lys Glu Gly Asp Ala Lys Lys Leu Tyr Glu Ala Lys Val Trp
   65 70 75 80
   Glu Lys Pro Trp Met Asp Phe Lys Glu Leu Gln Glu Phe Lys Pro Val
   85 90 95
   Asp Ala Ser Ala Asn Ala
   100
<210> 131
   <211> 156
   <212> PRT
   <213> Oryza sativa
<400> 131
   Met Ala Gly Ser Gly Val Leu Glu Val His Leu Val Asp Ala Lys Gly
   1 5 10 15
   Leu Thr Gly Asn Asp Phe Leu Gly Lys Ile Asp Pro Tyr Val Val Val
   20 25 30
   Gln Tyr Arg Ser Gln Glu Arg Lys Ser Ser Val Ala Arg Asp Gln Gly
   35 40 45
   Lys Asn Pro Ser Trp Asn Glu Val Phe Lys Phe Gln Ile Asn Ser Thr
   50 55 60
   Ala Ala Thr Gly Gln His Lys Leu Phe Leu Arg Leu Met Asp His Asp
   65 70 75 80
   Thr Phe Ser Arg Asp Asp Phe Leu Gly Glu Ala Thr Ile Asn Val Thr
   85 90 95
   Asp Leu Ile Ser Leu Gly Met Glu His Gly Thr Trp Glu Met Ser Glu
   100 105 110
   Ser Lys His Arg Val Val Leu Ala Asp Lys Thr Tyr His Gly Glu Ile
   115 120 125
   Arg Val Ser Leu Thr Phe Thr Ala Ser Ala Lys Ala Gln Asp His Ala
   130 135 140
   Glu Gln Val Gly Gly Trp Ala His Ser Phe Arg Gln
   145 150 155
<210> 132
   <211> 28
   <212> PRT
   <213> Artificial sequence
<220>
   <223> synthetically generated peptide
<400> 132
   Gly Glu Val Ile Gly Ile Ser Val Asn Asp Pro Arg Val Lys Glu Ile
   1 5 10 15
   Ala Glu Phe Ala Leu Lys Gln His Ala Glu Gln Asn
   20 25
<210> 133
   <211> 28
   <212> PRT
   <213> Artificial sequence
<220>
   <223> synthetically generated peptide
<400> 133
   Gly Glu Val Ile Gly Ile Gln Val Asn Asp Pro Arg Val Lys Glu Ile
    1               5                   10                  15
   Ala Glu Phe Ala Leu Lys Gln His Ala Glu Gln Asn
               20                  25
<210> 134
   <211> 28
   <212> PRT
   <213> Artificial Sequence
<220>
   <223> synthetically generated peptide
<400> 134
   Gly Gly Val Ile Gly Ile Ser Val Asn Asp Pro Arg Val Lys Glu Ile
    1               5                   10                  15
   Ala Glu Phe Ala Leu Lys Gln His Ala Glu Gln Asn
               20                  25
<210> 135
   <211> 89
   <212> PRT
   <213> Artificial sequence
<220>
   <223> synthetically generated peptide
<400> 135
   Gly Gly Tyr Val Pro Cys Lys Asp Pro Asn Asp Pro His Val Lys Asp
    1               5                   10                  15
   Ile Ala Glu Trp Ala Val Ala Glu Tyr Asn Lys Ser Gln Gly His His
               20                  25                  30
   Leu Thr Leu Val Ser Ile Leu Lys Cys Glu Ser Gln Val Val Ala Gly
           35                  40                  45
   Val Asn Trp Arg Leu Val Leu Lys Cys Lys Asp Glu Asn Asn Gly Glu
       50                  55                  60
   Gly Asn Tyr Glu Thr Val Val Trp Glu Lys Ile Trp Glu Asn Phe Arg
   65                  70                  75                  80
   Gln Leu Ile Thr Phe Asp His Leu Leu
                   85
<210> 136
   <211> 54
   <212> PRT
   <213> Artificial sequence
<220>
   <223> synthetically generated peptide
<400> 136
   Gly Gly Tyr Ser Pro Ile Lys Asn Leu Asn Asp His His Leu Ile Glu
    1               5                  10                  15
   Ile Ala Asn Phe Ala Val Thr Glu Tyr Gly Lys Gln Gln Gly Ile Lys
               20                  25                  30
   Gln Ile Lys Leu Glu Lys Ile Thr Lys Gly Glu Thr Gln Val Val Asp
           35                  40                  45
   Gly Thr Asn Tyr Arg Leu
       50
<210> 137
   <211> 55
   <212> PRT
   <213> Artificial Sequence
<220>
   <223> synthetically generated peptide
<400> 137
   Gly Gly Tyr Ser Pro Ile Lys Asn Leu Asn Asp Pro His Val Ile Glu
    1               5                  10                  15
   Ile Ala Asn Phe Ala Val Thr Glu Tyr Gly Lys Gln Gln Arg Phe Tyr
               20                  25                  30
   Ser Lys Leu Asp Lys Ile Ile Lys Gly Glu Ser Gln Ala Val Asp Gly
           35                  40                  45
   Thr Asn Tyr Arg Phe Ile Leu
       50                  55
<210> 138
   <211> 73
   <212> PRT
   <213> Artificial sequence
<220>
   <223> synthetically generated peptide
<400> 138
   Gly Gly Trp Ser Pro Ile Lys Asp Ile Asn Asp Pro His Val Ile Val
    1               5                  10                  15
   Ile Ala Asn Phe Ala Val Thr Glu Tyr Asn Lys His Thr Gly Ala Asn
               20                  25                  30
   Leu Lys Leu Asp Lys Leu Ile Lys Gly Glu Ser Gln Val Thr Ser Gly
           35                  40                  45
   Ile Tyr Tyr Asp Leu Ile Leu Ser Ala Gly Asp Gly Ser His Ser Asn
       50                  55                  60
   Ile Tyr Lys Ala Leu Val Trp Glu Lys
   65                  70
<210> 139
   <211> 73
   <212> PRT
   <213> Artificial sequence
<220>
   <223> synthetically generated peptide
<400> 139
   Gly Gly Trp Glu Pro Ile Lys Asn Ile Asn Asp Pro His Val Ile Asp
    1               5                  10                  15
   Ile Ala Asn Tyr Ala Val Thr Glu His Asp Lys Gln Ala Gln Leu Lys
               20                  25                  30
   Leu Glu Lys Val Ile Ser Gly Glu Thr Lys Val Val Asp Gly Ile Ile
           35                  40                  45
   Tyr Cys Leu Asn Ile Thr Ala Ser Asp Gly Ser Asn Lys Tyr Asn Leu
       50                  55                  60
   Ala Val Leu Glu Lys Leu Glu Gln His
   65                  70
<210> 140
   <211> 63
   <212> PRT
   <213> Artificial sequence
<220>
   <223> synthetically generated peptide
<400> 140
   Gly Asn Leu Ser Pro Ile Asn Asn Ile Asn Asp Pro Lys Val Ile Asp
    1               5                  10                  15
   Val Ala Asn Phe Ala Val Lys Glu Tyr Asn Asn Arg Arg Arg Lys Pro
               20                  25                  30
   Glu Glu Lys Leu Arg Leu Trp Lys Val Ile Lys Gly Glu Ser Gln Ile
           35                  40                  45
   Val Ala Asp Gly Val Asn Tyr Arg Leu Thr Leu Ser Ala Thr Lys
       50                  55                  60
<210> 141
   <211> 31
   <212> -PRT
   <213> Artificial sequence
<220>
   <223> synthetically generated peptide
<400> 141
   Leu Gln Phe Ser Arg Val Val Ser Ala Gln Lys Gln Val Val Ala Gly
    1               5                  10                  15
   Leu Lys Tyr Tyr Leu Arg Ile Glu Val Thr Gln Pro Asn Gly Ser
               20                  25                  30
<210> 142
   <211> 31
   <212> PRT
   <213> Artificial Sequence
<220>
   <223> synthetically generated peptide
<400> 142
   Leu Gln Phe Ser Arg Val Val Ser Ala Gln Lys Gln Val Val Ala Gly
   1               5                   10                  15
   Leu Lys Tyr Tyr Leu Arg Ile Glu Val Thr Gln Pro Asn Gly Ser
               20                  25                  30
<210> 143
   <211> 66
   <212> PRT
   <213> Artificial sequence
<220>
   <223> synthetically generated peptide
<400> 143
   Gly Ala Arg Thr Glu Val Pro Asp Val Glu Gly Asp Gly Glu Val Gln
   1               5                   10                  15
   Glu Leu Gly Arg Phe Ser Val Ala Glu Tyr Asn Arg Gln Leu Arg Glu
               20                  25                  30
   Gly Gly Gly Gly Gly Gly Arg Leu Glu Phe Gly Arg Val Val Ala Ala
           35                  40                  45
   Gln Arg Gln Val Val Ser Gly Leu Lys Tyr Tyr Leu Arg Val Val Ala
       50                  55                  60
   Val Glu
   65
<210> 144
   <211> 65
   <212> PRT
   <213> Artificial Sequence
<220>
   <223> synthetically generated peptide
<400> 144
   Gly Ala Arg Thr Glu Val Arg Asp Val Glu Gly Asp Gly Glu Val Gln
   1               5                   10                  15
   Glu Leu Gly Arg Phe Ser Val Ala Glu Tyr Asn Arg Gln Leu Arg Asp
               20                  25                  30
   Gly Gly Gly Gly Gly Arg Leu Glu Phe Gly Arg Val Val Ala Ala Gln
           35                  40                  45
   Arg Gln Val Val Ser Gly Leu Lys Tyr Tyr Leu Arg Val Val Ala Val
       50                  55                  60
   Glu
   65
<210> 145
   <211> 66
   <212> PRT
   <213> Artificial Sequence
<220>
   <223> synthetically generated peptide
<400> 145
   Gly Gly Arg Thr Glu Val Arg Asp Val Glu Gly Asp Arg Glu Val Gln
    1               5                  10                  15
   Glu Leu Gly Arg Phe Ser Val Glu Glu His Asn Arg Arg Arg Arg Ser
               20                  25                  30
   Arg Asp Cys Gly Asp Val Arg Leu Glu Phe Gly Arg Val Val Ala Ala
           35                  40                  45
   Gln Arg Gln Val Val Ser Gly Leu Lys Tyr Tyr Leu Arg Val Ala Ala
       50                  55                  60
   Ala Glu
   65
<210> 146
   <211> 70
   <212> PRT
   <213> Artificial sequence
<220>
   <223> synthetically generated peptide
<400> 146
   Gly Gly Arg Thr Glu Ile Arg Asp Val Gly Ser Asn Lys Ala Val Gln
    1               5                  10                  15
   Ser Leu Gly Arg Phe Ala Val Ala Glu His Asn Arg Arg Leu Arg His
               20                  25                  30
   Gly Gly Ser Gly Gly Pro Ala Asp Pro Val Pro Val Lys Leu Ala Phe
           35                  40                  45
   Ala Arg Val Val Glu Ala Gln Lys Gln Val Val Ser Gly Val Ala Tyr
       50                  55                  60
   Tyr Leu Lys Val Ala Ala
   65                  70
<210> 147
   <211> 67
   <212> PRT
   <213> Artificial Sequence
<220>
   <223> synthetically generated peptide
<400> 147
   Gly Gly Arg Lys Glu Ile Glu Asn Val Lys Thr Asn Lys Glu Val Gln
    1               5                  10                  15
   Glu Leu Gly Arg Phe Ser Val Glu Glu Tyr Asn Arg Gln Arg Gly Thr
               20                  25                  30
   Gln Lys Met Asp Gly Gly Gly Glu Leu Gln Phe Leu Glu Val Val Glu
           35                  40                  45
   Ala Gln Ser Gln Val Val Ser Gly Ile Lys Tyr Tyr Leu Lys Val Ser
       50                  55                  60
   Ala Val Arg
   65
<210> 148
   <211> 64
   <212> PRT
   <213> Artificial Sequence
<220>
   <223> synthetically generated peptide
<400> 148
   Gly Gly Arg Thr Lys Val Lys Asn Val Lys Thr Asp Thr Glu Ile Gln
   1 5 10 15
   Gln Leu Gly Ser Tyr Ser Val Asp Glu Tyr Asn Arg Leu Gln Arg Thr
   20 25 30
   Lys Lys Thr Gly Ala Gly Asp Leu Lys Phe Ser Gln Val Ile Ala Ala
   35 40 45
   Glu Thr Gln Val Val Ala Gly Thr Lys Tyr Tyr Leu Lys Ile Glu Ala
   50 55 60
<210> 149
   <211> 100
   <212> PRT
   <213> Artificial sequence
<220>
   <223> Synthetically generated peptide
<400> 149
   Gly Gly Arg Thr Glu Ile Pro Asp Val Glu Ser Asn Glu Glu Ile Gln
   1 5 10 15
   Gln Leu Gly Glu Tyr Ser Val Glu Gln Tyr Asn Gln Gln His His Asn
   20 25 30
   Gly Asp Gly Gly Asp Ser Thr Asp Ser Ala Gly Asp Leu Lys Phe Val
   35 40 45
   Lys Val Val Ala Ala Glu Lys Gln Val Val Ala Gly Ile Lys Tyr Tyr
   50 55 60
   Leu Lys Ile Val Ala Ala Lys Gly Gly His Lys Lys Lys Phe Asp Ala
   65 70 75 80
   Glu Ile Val Val Gln Ala Trp Lys Lys Thr Lys Gln Leu Met Ser Phe
   85 90 95
   Ala Pro Ser His
   100
<210> 150
   <211> 38
   <212> PRT
   <213> Artificial sequence
<220>
   <223> synthetically generated peptide
<400> 150
   Phe Ser Val Asp Glu Tyr Asn Ser Lys Asn Lys Ser Asn Leu Lys Leu
   1 5 10 15
   Leu Lys Val Val Lys Ala Glu Glu Gln Val Val Ala Gly Lys Lys Tyr
   20 25 30
   Tyr Leu Lys Ile Ser Ala
   35
<210> 151
   <211> 88
   <212> PRT
   <213> Artificial Sequence
<220>
   <223> Synthetically generated peptide
<400> 151
   Gly Gly Val Glu Pro Val Gly Asn Glu Asn Asp Leu His Leu Val Asp
   1 5 10 15
   Leu Ala Arg Phe Ala Val Thr Glu His Asn Lys Lys Ala Asn Ser Leu
   20 25 30
   Leu Glu Phe Glu Lys Leu Val Ser Val Lys Gln Gln Val Val Ala Gly
   35 40 45
   Thr Leu Tyr Tyr Phe Thr Ile Glu Val Lys Glu Gly Asp Ala Lys Lys
   50 55 60
   Leu Tyr Glu Ala Lys Val Trp Glu Lys Pro Trp Met Asp Phe Lys Glu
   65 70 75 80
   Leu Gln Glu Phe Lys Pro Val Asp
   85
<210> 152
   <211> 89
   <212> PRT
   <213> Artificial Sequence
<220>
   <223> Synthetically generated peptide
<400> 152
   Gly Gly Ile Lys Asp Val Pro Ala Asn Glu Asn Asp Leu His Leu Gln
   1 5 10 15
   Glu Leu Ala Arg Phe Ala Val Asp Glu His Asn Lys Lys Ala Asn Ala
   20 25 30
   Leu Leu Gly Tyr Glu Lys Leu Val Lys Ala Lys Thr Gln Val Val Ala
   35 40 45
   Gly Thr Met Tyr Tyr Leu Thr Ile Glu Val Lys Asp Gly Glu Val Lys
   50 55 60
   Lys Leu Tyr Glu Ala Lys Val Trp Glu Lys Pro Trp Glu Asn Phe Lys
   65 70 75 80
   Glu Leu Leu Glu Phe Lys Pro Val Glu
   85
<210> 153
   <211> 89
   <212> PRT
   <213> Artificial Sequence
<220>
   <223> Synthetically generated peptide
<400> 153
   Gly Gly Ile Lys Asp Val Pro Ala Asn Glu Asn Asp Leu His Leu Gln
   1               5                   10                  15
   Glu Leu Ala Arg Phe Ala Val Asp Glu His Asn Lys Lys Ala Asn Ala
               20                  25                  30
   Leu Leu Gly Tyr Glu Lys Leu Val Lys Ala Lys Thr Gln Val Val Ala
           35                  40                  45
   Gly Thr Met Tyr Tyr Leu Thr Val Glu Val Lys Asp Gly Glu Val Lys
       50                  55                  60
   Lys Leu Tyr Glu Ala Lys Val Trp Glu Lys Pro Trp Glu Asn Phe Lys
   65                  70                  75                  80
   Glu Leu Gln Glu Phe Lys Pro Val Glu
                   85
<210> 154
   <211> 89
   <212> PRT
   <213> Artificial sequence
<220>
   <223> Synthetically generated peptide
<400> 154
   Gly Gly Ile Lys Asp Val Pro Gly Asn Glu Asn Asp Leu His Leu Gln
   1               5                   10                  15
   Glu Leu Ala Arg Phe Ala Val Asp Glu His Asn Lys Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Phe Lys
   65                  70                  75                  80
   Glu Leu Gln Glu Phe Lys Pro Val Glu
                   85
<210> 155
   <211> 89
   <212> PRT
   <213> Artificial Sequence
<220>
   <223> Synthetically generated peptide
<400> 155
   Gly Gly Ile Lys Asp Val Pro Gly Asn Glu Asn Asp Leu His Leu Gln
    1               5                  10                  15
   Glu Leu Ala Arg Phe Ala Val Asp Glu His Asn Lys Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Phe Lys
   65                  70                  75                  80
   Glu Leu Gln Glu Phe Lys Pro Val Glu
                   85
<210> 156
   <211> 89
   <212> PRT
   <213> Artificial Sequence
<220>
   <223> Synthetically generated peptide
<400> 156
   Gly Gly Ile Lys Asp Val Pro Gly Asn Glu Asn Asp Leu His Leu Gln
    1               5                  10                  15
   Glu Leu Ala Arg Phe Ala Val Asp Glu His Asn Lys Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Phe Lys
   65                  70                  75                  80
   Glu Leu Gln Glu Phe Lys Pro Val Asp
                   85
<210> 157
   <211> 89
   <212> PRT
   <213> Artificial Sequence
<220>
   <223> Synthetically generated peptide
<400> 157
   Gly Gly Ile Gln Asp Val Pro Gly Ser Glu Asn Asp Leu His Leu Gln
    1               5                  10                  15
   Glu Leu Ala Arg Phe Ala Val Asp Glu His Asn Lys Lys Ala Asn Ala
               20                  25                  30
   Leu Leu Gly Phe Glu Lys Leu Val Lys Val Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Leu Lys
   65                  70                  75                  80
   Gly Leu Gln Glu Phe Lys Pro Val Glu
                   85
<210> 158
   <211> 89
   <212> PRT
   <213> Artificial sequence
<220>
   <223> Synthetically generated.peptide
<400> 158
   Gly Gly Ile Gln Asp Val Pro Gly Ser Glu Asn Asp Leu His Leu Gln
   1               5                   10                  15
   Glu Leu Ala Arg Phe Ala Val Asp Glu His Asn Lys Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Leu Lys
   65                  70                  75                  80
   Gly Leu Gln Glu Phe Lys Pro Val Glu
                   85
<210> 159
   <211> 89
   <212> PRT
   <213> Artificial Sequence
<220>
   <223> Synthetically generated peptide
<400> 159
   Gly Gly Ile Lys Asp Val Pro Gly Asn Glu Asn Asp Leu His Leu Gln
   1               5                   10                  15
   Glu Leu Ala Arg Phe Ala Val Asp Glu His Asn Lys Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Leu Lys
   65                  70                  75                  80
   Gly Leu Gln Glu Phe Lys Pro Val Glu
                   85
<210> 160
   <211> 89
   <212> PRT
   <213> Artificial Sequence
<220>
   <223> Synthetically generated peptide
<400> 160
   Gly Gly Ile Gln Asp Val Pro Gly Ser Glu Asn Asp Leu His Leu Gln
   1               5                   10                  15
   Glu Leu Ala Arg Phe Ala Val Asp Glu His Asn Lys Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Phe Lys
   65                  70                  75                  80
   Glu Leu Gln Glu Phe Lys Pro Val Glu
                   85
<210> 161
   <211> 89
   <212> PRT
   <213> Artificial Sequence
<220>
   <223> Synthetically generated peptide
<400> 161
   Gly Gly Ile Gln Asp Val Pro Glu Asn Glu Asn Asp Leu His Leu Gln
   1               5                   10                  15
   Glu Leu Ala Arg Phe Ala Val Asp Glu His Asn Lys Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Lys Phe Lys
   65                  70                  75                  80
   Glu Leu Gln Glu Phe Lys Pro Val Glu
                   85
<210> 162
   <211> 89
   <212> PRT
   <213> Artificial Sequence
<220>
   <223> Synthetically generated peptide
<400> 162
   Gly Gly Ile Lys Asp Val Pro Ala Asn Glu Asn Asp Leu Gln Leu Gln
   1               5                   10                  15
   Glu Leu Ala Arg Phe Ala Val Asn Glu His Asn Gln Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Asn
       50                  55                  60
   Lys Leu Tyr Glu Ala Lys Val Trp Glu Lys Pro Trp Glu Asn Phe Lys
   65                  70                  75                  80
   Gln Leu Gln Glu Phe Lys Pro Val Glu
                   85
<210> 163
   <211> 89
   <212> PRT
   <213> Artificial Sequence
<220>
   <223> synthetically generated peptide
<400> 163
   Gly Gly Ile Lys Asp Val Pro Ala Asn Glu Asn Asp Leu Gln Leu Gln
   1               5                   10                  15
   Glu Leu Ala Arg Phe Ala Val Asn Glu His Asn Gln Lys Ala Asn Ala
               20                  25                  30
   Leu Leu Gly Phe Glu Lys Leu Val Lys Ala Lys Thr Gln Val Val Ala
           35                  40                  45
   Gly Thr Met Tyr Tyr Leu Thr Ile Glu Val Lys Asp Gly Glu Val Lys
       50                  55                  60
   Lys Leu Tyr Glu Ala Lys Val Trp Glu Lys Pro Trp Glu Asn Phe Lys
   65                  70                  75                  80
   Gln Leu Gln Glu Phe Lys Pro Val Glu
                   85
<210> 164
   <211> 67
   <212> PRT
   <213> Artificial Sequence
<220>
   <223> Synthetically generated peptide
<400> 164
   Val Ser Glu His Lys Asn Lys Thr Asn Ala Leu Leu Glu Phe Glu Lys
   1               5                   10                  15
   Val Val Arg Leu Lys Gln Gln Val Val Ala Gly Met Met Tyr Tyr Ile
               20                  25                  30
   Thr Ile Gln Val Asn Glu Gly Gly Ala Lys Lys Met Tyr Glu Ala Lys
           35                  40                  45
   Val Trp Glu Arg Pro Trp Met Asp Phe Lys Lys Leu Met Glu Phe Arg
       50                  55                  60
   Pro Ala Glu
   65
<210> 165
   <211> 72
   <212> PRT
   <213> Artificial Sequence
<220>
   <223> Synthetically generated peptide
<400> 165
   Leu Ala Arg Phe Ala Val Ser Glu His Lys Asn Lys Thr Asn Ala Leu
   1               5                   10                  15
   Leu Glu Phe Glu Lys Val Val Arg Leu Lys Gln Gln Val Val Ala Gly
               20                  25                  30
   Met Thr Tyr Tyr Ile Thr Ile Gln Val Asn Glu Gly Gly Ala Lys Lys
           35                  40                  45
   Met Tyr Glu Ala Lys Val Trp Glu Arg Pro Trp Met Asp Phe Lys Lys
       50                  55                  60
   Leu Met Glu Phe Arg Pro Ala Glu
   65                  70
<210> 166
   <211> 89
   <212> PRT
   <213> Artificial Sequence
<220>
   <223> Synthetically generated peptide
<400> 166
   Gly Gly Ile Val Asp Ser Leu Arg Arg Glu Asn Asp Pro Tyr Ile Val
   1               5                   10                  15
   Asp Leu Ala Arg Phe Ala Val Ser Glu His Asn Lys Glu Gly Asn Thr
               20                  25                  30
   Gln Leu Glu Leu Glu Lys Val Val Lys Val Lys Glu Gln Ala Val Ala
           35                  40                  45
   Gly Arg Leu Tyr Tyr Ile Thr Ile Gln Val Asp Glu Gly Gly Ala Lys
       50                  55                  60
   Lys Leu Tyr Glu Ala Lys Val Leu Glu Gln Leu Trp Leu Asp Val Lys
   65                  70                  75                  80
   Lys Leu Val Glu Phe Lys Pro Ala Glu
                   85
<210> 167
   <211> 83
   <212> PRT
   <213> Artificial Sequence
<220>
   <223> Synthetically generated peptide
<400> 167
   Leu Gly Arg Glu Asn Asp Pro Tyr Ile Val Asp Leu Ala Arg Phe Ala
   1 5 10 15
   Val Ser Glu His Asn Lys Glu Gly Asn Thr Gln Leu Glu Leu Glu Lys
   20 25 30
   Val Val Lys Val Lys Glu Gln Ala Val Ala Gly Arg Leu Tyr Tyr Ile
   35 40 45
   Thr Ile Gln Val Asp Glu Gly Gly Ala Lys Lys Leu Tyr Glu Ala Lys
   50 55 60
   Val Leu Glu Gln Leu Trp Leu Asp Val Lys Lys Leu Val Glu Phe Lys
   65 70 75 80
   Pro Ala Glu
<210> 168
   <211> 83
   <212> PRT
   <213> Artificial Sequence
<220>
   <223> Synthetically generated peptide
<400> 168
   Pro Gly Arg Glu Asn Asp Pro Tyr Ile Val Asp Leu Ala Arg Phe Ala
   1 5 10 15
   Val Ser Gln His Asn Lys Glu Ala Asn Thr Gln Leu Glu Leu Glu Lys
   20 25 30
   Val Val Lys Val Lys Glu Gln Ala Ile Ala Gly Arg Leu Tyr Tyr Ile
   35 40 45
   Thr Ile Gln Val Asp Glu Gly Gly Ala Lys Lys Leu Tyr Lys Ala Lys
   50 55 60
   Val Leu Glu Gln Leu Trp Leu Asp Val Lys Lys Leu Leu Glu Phe Lys
   65 70 75 80
   Pro Ala Glu
<210> 169
   <211> 88
   <212> PRT
   <213> Artificial Sequence
<220>
   <223> Synthetically generated peptide
<400> 169
   Gly Gly Ile Ser Asp Ser Pro Met Gly Gln Glu Asn Asp Leu Asp Val
   1 5 10 15
   Ile Ala Arg Phe Ala Val Ser Glu His Asn Asn Lys Ala Asn Ala Leu
   20 25 30
   Leu Glu Phe Glu Asn Val Val Lys Val Lys Lys Gln Thr Val Ala Gly
   35 40 45
   Thr Met His Tyr Ile Thr Ile Arg Val Thr Glu Gly Gly Ala Lys Lys
   50 55 60
   Leu Tyr Glu Ala Lys Val Trp Glu Lys Pro Trp Glu Asn Phe Lys Lys
   65 70 75 80
   Leu Glu Glu Phe Lys Leu Val Glu
   85
<210> 170
   <211> 88
   <212> PRT
   <213> Artificial sequence
<220>
   <223> Synthetically generated peptide
<400> 170
   Gly Gly Ile Ser Asp Ser Pro Met Gly Gln Glu Asn Asp Leu Asp Val
    1               5                  10                  15
   Ile Ala Arg Phe Ala Val Ser Glu His Asn Asn Lys Ala Asn Ala Leu
               20                  25                  30
   Leu Glu Phe Glu Asn Val Val Lys Val Lys Lys Gln Thr Val Ala Gly
           35                  40                  45
   Thr Met His Tyr Ile Thr Ile Arg Val Thr Glu Gly Gly Ala Lys Lys
       50                  55                  60
   Leu Tyr Glu Ala Lys Val Trp Glu Lys Pro Trp Glu Asn Phe Lys Lys
   65                  70                  75                  80
   Leu Glu Glu Phe Lys Leu Val Glu
                   85
<210> 171
   <211> 88
   <212> PRT
   <213> Artificial sequence
<220>
   <223> Synthetically generated peptide
<400> 171
   Gly Gly Ile Lys Asp Ser Pro Met Gly Gln Glu Asn Asp Leu Asp Val
    1               5                  10                  15
   Ile Ala Arg Phe Ala Val Ser Glu His Asn Asn Lys Ala Asn Ala Leu
               20                  25                  30
   Leu Glu Phe Glu Asn Val Val Lys Leu Lys Lys Gln Thr Val Ala Gly
           35                  40                  45
   Thr Met His Tyr Ile Thr Ile Arg Val Thr Glu Gly Gly Ala Lys Lys
       50                  55                  60
   Leu Tyr Glu Ala Lys Val Trp Glu Lys Pro Trp Glu Asn Phe Lys Gln
   65                  70                  75                  80
   Leu Gln Glu Phe Lys Pro Val Glu
                   85
<210> 172
   <211> 83
   <212> PRT
   <213> Artificial Sequence
<220>
   <223> Synthetically generated peptide
<400> 172
   Gly Asp Val Arg Asp Ala Pro Val Gly Arg Glu Asn Asp Leu Glu Ala
   1 5 10 15
   Ile Glu Leu Ala Arg Phe Ala Val Ala Glu His Asn Ser Lys Thr Asn
   20 25 30
   Ala Met Leu Glu Phe Glu Arg Leu Val Lys Val Arg His Gln Val Val
   35 40 45
   Ala Gly Thr Leu His His Phe Thr Val Glu Val Lys Glu Ala Gly Gly
   50 55 60
   Gly Glu Lys Lys Leu Tyr Glu Ala Lys Val Trp Glu Lys Ala Trp Glu
   65 70 75 80
   Asn Phe Lys
<210> 173
   <211> 92
   <212> PRT
   <213> Artificial Sequence
<220>
   <223> Synthetically generated peptide
<400> 173
   Gly Asp Val Arg Asp Ala Pro Val Gly Arg Glu Asn Asp Leu Glu Ala
   1 5 10 15
   Ile Glu Leu Ala Arg Phe Ala Val Ala Glu His Asn Ser Lys Thr Asn
   20 25 30
   Ala Met Leu Glu Phe Glu Arg Leu Val Lys Val Arg His Gln Val Val
   35 40 45
   Ala Gly Thr Leu His His Phe Thr Val Glu Val Lys Glu Ala Gly Gly
   50 55 60
   Gly Glu Lys Lys Leu Tyr Glu Ala Lys Val Trp Glu Lys Ala Trp Glu
   65 70 75 80
   Asn Phe Lys Gln Leu Gln Ser Phe Glu Leu Val Gly
   85 90
<210> 174
   <211> 81
   <212> PRT
   <213> Artificial sequence
<220>
   <223> Synthetically generated peptide
<400> 174
   Glu Asn Asp Leu Glu Ala Ile Glu Leu Ala Arg Phe Ala Val Ala Glu
    1               5                  10                  15
   His Asn Ser Lys Thr Asn Ala Met Leu Glu Phe Glu Arg Leu Val Lys
               20                  25                  30
   Val Arg His Gln Val Val Ala Gly Thr Met His His Phe Thr Val Gln
           35                  40                  45
   Val Lys Glu Ala Gly Gly Gly Lys Lys Leu Tyr Glu Ala Lys Val Trp
       50                  55                  60
   Glu Lys Val Trp Glu Asn Phe Lys Gln Leu Gln Ser Phe Gln Pro Val
   65                  70                  75                  80
   Gly
<210> 175
   <211> 90
   <212> PRT
   <213> Artificial Sequence
<220>
   <223> synthetically generated peptide
<400> 175
   Gly Gly Val Gln Asp Ala Pro Ala Gly Arg Glu Asn Asp Leu Glu Thr
    1               5                  10                  15
   Ile Glu Leu Ala Arg Phe Ala Val Ala Glu His Asn Ala Lys Ala Asn
               20                  25                  30
   Ala Leu Leu Glu Phe Glu Lys Leu Val Lys Val Arg Gln Gln Val Val
           35                  40                  45
   Ala Gly Cys Met His Tyr Phe Thr Ile Glu Val Lys Glu Gly Gly Ala
       50                  55                  60
   Lys Lys Leu Tyr Glu Ala Lys Val Trp Glu Lys Ala Trp Glu Asn Phe
   65                  70                  75                  80
   Lys Gln Leu Gln Glu Phe Lys Pro Ala Ala
                   85                  90
<210> 176
   <211> 90
   <212> PRT
   <213> Artificial Sequence
<220>
   <223> Synthetically generated peptide
<400> 176
   Gly Gly Val Gln Asp Ala Pro Ala Gly Arg Glu Asn Asp Leu Glu Thr
    1               5                  10                  15
   Ile Glu Leu Ala Arg Phe Ala Val Ala Glu His Asn Ala Lys Ala Asn
               20                  25                  30
   Ala Leu Leu Glu Phe Glu Arg Leu Val Lys Val Arg Gln Gln Val Val
           35                  40                  45
   Ala Gly Cys Met His Tyr Phe Thr Ile Glu Val Lys Glu Gly Gly Ala
       50                  55                  60
   Lys Lys Leu Tyr Glu Ala Lys Val Trp Glu Lys Ala Trp Glu Asn Phe
   65                  70                  75                  80
   Lys Gln Leu Gln Glu Phe Lys Pro Ala Ala
                   85                  90
<210> 177
   <211> 91
   <212> PRT
   <213> Artificial Sequence
<220>
   <223> synthetically generated peptide
<400> 177
   Gly Gly Ile His Asp Ala Pro Ala Gly Arg Glu Asn Asp Leu Thr Thr
    1               5                  10                  15
   Val Glu Leu Ala Arg Phe Ala Val Ala Glu His Asn Ser Lys Ala Asn
               20                  25                  30
   Ala Met Leu Glu Leu Glu Arg Val Val Lys Val Arg Gln Gln Val Val
           35                  40                  45
   Gly Gly Phe Met His Tyr Leu Thr Val Glu Val Lys Glu Pro Gly Gly
       50                  55                  60
   Ala Asn Lys Leu Tyr Glu Ala Lys Val Trp Glu Arg Ala Trp Glu Asn
   65                  70                  75                  80
   Phe Lys Gln Leu Gln Asp Phe Lys Pro Leu Asp
                   85                  90
<210> 178
   <211> 90
   <212> PRT
   <213> Artificial sequence
<220>
   <223> synthetically generated peptide
<400> 178
   Gly Gly Ile Tyr Asp Ala Pro Leu Asn Asn Glu Asn Gly Phe Asp Lys
    1               5                  10                  15
   Glu Asp Leu Ala Arg Phe Ala Val Arg Glu Tyr Asn Asn Lys Asn Asn
               20                  25                  30
   Ala Leu Leu Glu Phe Val Arg Val Val Lys Ala Lys Glu Gln Val Val
           35                  40                  45
   Ser Gly Met Met His Tyr Leu Thr Val Glu Val Asn Asp Ala Gly Lys
       50                  55                  60
   Lys Lys Leu Tyr Glu Ala Lys Val Trp Glu Gln Val Trp Met Asn Phe
   65                  70                  75                  80
   Arg Gln Leu Gln Glu Phe Thr Tyr Leu Gly
                   85                  90

<210> 179
   <211> 89
   <212> PRT
   <213> Artificial Sequence
<220>
   <223> Synthetically generated peptide
<400> 179
   Gly Thr Ser Arg Asp Val Asp Pro Asn Ala Asn Asp Leu Gln Val Glu
   1 5 10 15
   Ser Leu Ala Arg Phe Ala Val Asp Glu His Asn Lys Lys Glu Asn Val
   20 25 30
   Ser Leu Glu Tyr Arg Arg Leu Ile Gly Ala Lys Thr Gln Val Val Ala
   35 40 45
   Gly Thr Met His His Leu Thr Val Glu Val Ala Asp Gly Glu Thr Lys
   50 55 60
   Lys Val Tyr Glu Ala Lys Val Leu Glu Lys Ala Trp Glu Asn Leu Lys
   65 70 75 80
   Lys Leu Glu Asp Phe Thr His Leu Arg
   85
<210> 180
   <211> 89
   <212> PRT
   <213> Artificial Sequence
<220>
   <223> Synthetically generated peptide
<400> 180
   Gly Gly Val Arg Asp Val Asp Pro Asn Ala Asn Asp Leu Gln Val Glu
   1 5 10 15
   Ser Leu Ala Arg Phe Ala Val Asp Glu His Asn Lys Lys Glu Asn Val
   20 25 30
   Ser Leu Glu Tyr Arg Arg Leu Ile Gly Ala Lys Thr Gln Val Val Ala
   35 40 45
   Gly Thr Met His His Leu Thr Val Glu Val Ala Asp Gly Glu Thr Lys
   50 55 60
   Lys Val Tyr Glu Ala Lys Val Leu Glu Lys Ala Trp Glu Asn Leu Lys
   65 70 75 80
   Lys Leu Glu Asp Phe Thr His Leu Arg
   85
<210> 181
   <211> 89
   <212> PRT
   <213> Artificial sequence
<220>
   <223> Synthetically generated peptide
<400> 181
   Gly Gly Val Arg Asp Ile Asp Ala Asn Ala Asn Asp Leu Gln Val Glu
   1 5 10 15
   Ser Leu Ala Arg Phe Ala Val Asp Glu His Asn Lys Asn Glu Asn Leu
   20 25 30
   Thr Leu Glu Tyr Lys Arg Leu Leu Gly Ala Lys Thr Gln Val Val Ala
   35 40 45
   Gly Thr Met His His Leu Thr Val Glu Val Ala Asp Gly Glu Thr Asn
   50 55 60
   Lys Val Tyr Glu Ala Lys Val Leu Glu Lys Ala Trp Glu Asn Leu Lys
   65 70 75 80
   Gln Leu Glu Ser Phe Asn His Leu His
   85
<210> 182
   <211> 89
   <212> PRT
   <213> Artificial Sequence
<220>
   <223> Synthetically generated peptide
<400> 182
   Gly Gly Val Arg Asp Ile Asp Ala Asn Ala Asn Asp Leu Gln Val Glu
   1 5 10 15
   Ser Leu Ala Arg Phe Ala Val Asp Glu His Asn Lys Asn Glu Asn Leu
   20 25 30
   Thr Leu Glu Tyr Lys Arg Leu Leu Gly Ala Lys Thr Gln Val Val Ala
   35 40 45
   Gly Thr Met His His Leu Thr Val Glu Val Ala Asp Gly Glu Thr Asn
   50 55 60
   Lys Val Tyr Glu Ala Lys Val Leu Glu Lys Ala Trp Glu Asn Leu Lys
   65 70 75 80
   Gln Leu Glu Ser Phe Asn His His His
   85
<210> 183
   <211> 87
   <212> PRT
   <213> Artificial Sequence
<220>
   <223> Synthetically generated peptide
<400> 183
   Gly Gly Val His Asp Ser Gln Ser Ser Gln Asn Ser Ala Glu Ile Asp
   1 5 10 15
   Gly Leu Ala Arg Phe Ala Val Asp Glu His Asn Lys Lys Glu Asn Ala
   20 25 30
   Ile Phe Ala Arg Val Val Lys Ala Lys Glu Gln Val Val Ala Gly Thr
   35 40 45
   Met His His Leu Thr Ile Glu Ala Ile Glu Ala Gly Lys Lys Lys Leu
   50 55 60
   Tyr Glu Ala Lys Val Trp Val Lys Pro Trp Leu Asn Phe Lys Glu Leu
   65 70 75 80
   His Glu Phe Lys Asp Ala Ser
   85
<210> 184
   <211> 87
   <212> PRT
   <213> Artificial Sequence
<220>
   <223> synthetically generated peptide
<400> 184
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Ser
                   85
<210> 185
   <211> 87
   <212> PRT
   <213> Artificial Sequence
<220>
   <223> Synthetically generated peptide
<400> 185
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Ala Lys Pro Trp Leu Asn Phe Lys Glu Leu
   65                  70                  75                  80
   His Glu Phe Lys Asp Ala Ser
                   85
<210> 186
   <211> 87
   <212> PRT
   <213> Artificial Sequence
<220>
   <223> Synthetically generated peptide
<400> 186
   Gly Gly Val His Asp Ser Gln Ser Ser Gln Asn Ser Ala Glu Ile Asp
   1               5                   10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Val Gly Lys Lys Lys Leu
       50                  55                  60
   Tyr Glu Ala Lys Ala Trp Val Lys Pro Trp Leu Asn Phe Lys Glu Leu
   65                  70                  75                  80
   His Glu Phe Lys Asp Ala Ser
                   85
<210> 187
   <211> 87
   <212> PRT
   <213> Artificial Sequence
<220>
   <223> Synthetically generated peptide
<400> 187
   Gly Gly Val His Asp Ser Gln Ser Ser Gln Asn Ser Ala Glu Ile Asp
   1               5                   10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Val Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Ser
                   85
<210> 188
   <211> 87
   <212> PRT
   <213> Artificial Sequence
<220>
   <223> Synthetically generated peptide
<400> 188
   Gly Gly Val Gln Asp Ser Gln Ser Ser Gln Asn Ser Ala Glu Ile Asp
   1               5                   10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Arg Val Lys Pro Trp Leu Asn Phe Lys Glu Leu
   65                  70                  75                  80
   His Glu Phe Lys Asp Ala Ser
                   85
<210> 189
   <211> 87
   <212> PRT
   <213> Artificial Sequence
<220>
   <223> Synthetically generated peptide
<400> 189
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Pro Lys Asp Gln Val Phe Ala Gly Pro
           35                  40                  45
   Met His His Leu Thr Ile Glu Ala Ile Glu Ala Gly Lys Lys Lys Leu
       50                  55                  60
   Asn Glu Ala Lys Val Trp Val Asn Pro Trp Leu Asn Phe Lys Glu Leu
   65                  70                  75                  80
   His Glu Phe Lys Asp Ala Gly
                   85
<210> 190
   <211> 87
   <212> PRT
   <213> Artificial Sequence
<220>
   <223> Synthetically generated peptide
<400> 190
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Pro Lys Glu Gln Val Phe Ala Gly Pro
           35                  40                  45
   Met His His Leu Thr Ile Glu Ala Ile Glu Ala Gly Lys Lys Lys Leu
       50                  55                  60
   Tyr Glu Ala Lys Val Trp Val Asn Pro Trp Leu Asn Phe Lys Glu Leu
   65                  70                  75                  80
   His Glu Phe Lys Asp Ala Gly
                   85
<210> 191
   <211> 87
   <212> PRT
   <213> Artificial Sequence
<220>
   <223> Synthetically generated peptide
<400> 191
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Pro Lys Glu Gln Val Phe Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 192
   <211> 87
   <212> PRT
   <213> Artificial Sequence
<220>
   <223> synthetically generated peptide
<400> 192
   Gly Gly Val Gln Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Phe Ala Gly Pro
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Ser
   65                  70                  75                  80
   His Glu Phe Lys Asp Ala Gly
                   85
<210> 193
   <211> 87
   <212> PRT
   <213> Artificial Sequence
<220>
   <223> Synthetically generated peptide
<400> 193
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Phe Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 194
   <211> 87
   <212> PRT
   <213> Artificial Sequence
<220>
   <223> Synthetically generated peptide
<400> 194
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Phe Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 195
   <211> 87
   <212> PRT
   <213> Artificial Sequence
<220>
   <223> Synthetically generated peptide
<400> 195
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Phe Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Ser
                   85
<210> 196
   <211> 87
   <212> PRT
   <213> Artificial Sequence
<220>
   <223> Synthetically generated peptide
<400> 196
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Leu Val Lys Ala Lys Asp Gln Val Phe Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Asn Pro Trp Leu Asn Phe Lys Glu Leu
   65                  70                  75                  80
   His Glu Phe Lys Asp Ala Ser
                   85
<210> 197
   <211> 87
   <212> PRT
   <213> Artificial Sequence
<220>
   <223> Synthetically generated peptide
<400> 197
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Leu Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 198
   <211> 87
   <212> PRT
   <213> Artificial Sequence
<220>
   <223> Synthetically generated peptide
<400> 198
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Leu Val Lys Ala Lys Glu Gln Val Val Ala Gly Thr
           35                  40                  45
   Met His His Leu Thr Val Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Ser
                   85
<210> 199
   <211> 87
   <212> PRT
   <213> Artificial Sequence
<220>
   <223> Synthetically generated peptide
<400> 199
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Pro
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Ser
                   85
<210> 200
   <211> 87
   <212> PRT
   <213> Artificial sequence
<220>
   <223> Synthetically generated peptide
<400> 200
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Pro
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 201
   <211> 87
   <212> PRT
   <213> Artificial Sequence
<220>
   <223> synthetically generated peptide
<400> 201
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 202
   <211> 87
   <212> PRT
   <213> Artificial Sequence
<220>
   <223> Synthetically generated peptide
<400> 202
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 203
   <211> 87
   <212> PRT
   <213> Artificial Sequence
<220>
   <223> Synthetically generated peptide
<400> 203
   Gly Gly Val His Asp Ser Gln Ser Ser Gln Asn Ser Ala Glu Ile Asp
    1               5                  10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 204
   <211> 87
   <212> PRT
   <213> Artificial Sequence
<220>
   <223> Synthetically generated peptide
<400> 204
   Gly Gly Val His Asp Ser Gln Ser Ser Gln Asn Ser Ala Glu Ile Asp
    1               5                  10                  15
   Gly Leu Ala Arg Leu Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Arg Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 205
   <211> 87
   <212> PRT
   <213> Artificial Sequence
<220>
   <223> Synthetically generated peptide
<400> 205
   Gly Gly Val His Asp Ser Gln Ser Ser Gln Asn Ser Ala Glu Ile Asp
    1               5                  10                  15
   Gly Leu Ala Arg Phe Ala Ile Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 206
   <211> 87
   <212> PRT
   <213> Artificial Sequence
<220>
   <223> Synthetically generated peptide
<400> 206
   Gly Gly Val His Asp Ser Gln Ser Ser Gln Asn Ser Ala Glu Ile Asp
   1               5                   10                  15
   Ser Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Gly
                   85
<210> 207
   <211> 87
   <212> PRT
   <213> Artificial Sequence
<220>
   <223> Synthetically generated peptide
<400> 207
   Gly Gly Val His Asp Ser Gln Thr Ser Gln Asn Ser Ala Glu Ile Asp
   1               5                   10                  15
   Gly Leu Ala Arg Phe Ala Val Asp Glu His Asn Lys Lys Glu Asn Ala
               20                  25                  30
   Ile Phe Ala Arg Val Val Lys Ala Lys Glu Gln Val Val Ala Gly Thr
           35                  40                  45
   Met His His Leu Thr Ile Glu Ala Ile Glu Ala Gly Lys Lys Lys Leu
       50                  55                  60
   Tyr Glu Ala Lys Val Trp Val Lys Pro Trp Leu Asn Phe Lys Glu Leu
   65                  70                  75                  80
   His Glu Phe Lys Asp Ala Ser
                   85
<210> 208
   <211> 89
   <212> PRT
   <213> Artificial sequence
<220>
   <223> synthetically generated peptide
<400> 208
   Gly Gly Val His Asp Ser Gln Ser Ser Gln Asn Ser Ala Glu Ile Asp
   1               5                   10                  15
   Gly Leu Ala Arg Phe Ala Val Asp Glu His Asn Lys Lys Glu Asn Ala
               20                  25                  30
   Met Leu Glu Phe Ala Arg Val Val Lys Ala Lys Glu Gln Val Val Ala
           35                  40                  45
   Gly Thr Met His His Leu Thr Ile Glu Ala Ile Glu Ala Gly Lys Lys
       50                  55                  60
   Lys Leu Tyr Glu Ala Lys Val Trp Val Lys Pro Trp Leu Asn Phe Lys
   65                  70                  75                  80
   Glu Leu His Glu Phe Lys Asp Ala Ser
                   85
<210> 209
   <211> 90
   <212> PRT
   <213> Artificial Sequence
<220>
   <223> synthetically generated peptide
<400> 209
   Gly Gly Val His Asp Ser Pro Gln Gly Thr Ala Asn Asn Ala Glu Ile
    1               5                  10                  15
   Asp Gly Ile Ala Arg Phe Ala Val Asp Glu His Asn Lys Lys Glu Asn
               20                  25                  30
   Ala Met Val Glu Phe Gly Arg Val Leu Lys Ala Lys Glu Gln Val Val
           35                  40                  45
   Ala Gly Thr Leu His His Leu Thr Ile Glu Ala Ile Glu Ala Gly Lys
       50                  55                  60
   Lys Lys Ile Tyr Glu Ala Lys Val Trp Val Lys Pro Trp Leu Asn Phe
   65                  70                  75                  80
   Lys Glu Leu Gln Glu Phe Lys His Ala Thr
                   85                  90
<210> 210
   <211> 89
   <212> PRT
   <213> Artificial Sequence
<220>
   <223> Synthetically generated peptide
<400> 210
   Gly Gly Val His Glu Ser His Gly Ala Gln Asn Ser Ala Glu Val Glu
    1               5                  10                  15
   Asp Leu Ala Arg Phe Ala Val Gln Glu His Asn Asn Lys Glu Asn Ala
               20                  25                  30
   Leu Leu Glu Phe Val Ser Val Val Lys Ala Lys Glu Gln Val Val Ala
           35                  40                  45
   Gly Thr Leu His His Leu Thr Ile Glu Ala Ile Glu Ala Gly Lys Lys
       50                  55                  60
   Lys Leu Tyr Gln Ala Lys Val Trp Val Lys Pro Trp Met Gly Phe Lys
   65                  70                  75                  80
   Glu Val Gln Glu Phe Lys His Ala Asp
                   85
<210> 211
   <211> 89
   <212> PRT
   <213> Artificial sequence
<220>
   <223> Synthetically generated peptide
<400> 211
   Gly Gly Ile Val Asp Val Glu Gly Ala Gln Asn Ser Ala Glu Val Glu
   1               5                   10                  15
   Glu Leu Ala Arg Phe Ala Val Asp Glu His Asn Lys Lys Glu Asn Ala
               20                  25                  30
   Leu Leu Gln Phe Ser Arg Leu Val Lys Ala Lys Gln Gln Val Val Ser
           35                  40                  45
   Gly Ile Met His His Leu Thr Val Glu Val Ile Glu Gly Gly Lys Lys
       50                  55                  60
   Lys Val Tyr Glu Ala Lys Val Trp Val Gln Ala Trp Leu Asn Ser Lys
   65                  70                  75                  80
   Lys Leu His Glu Phe Ser Pro Ile Gly
                   85
<210> 212
   <211> 87
   <212> PRT
   <213> Artificial Sequence
<220>
   <223> Synthetically generated peptide
<400> 212
   Gly Gly Val Gly Asp Val Pro Ala Asn Gln Asn Ser Gly Glu Val Glu
   1               5                   10                  15
   Ser Leu Ala Arg Phe Ala Val Asp Glu His Asn Lys Lys Glu Asn Ala
               20                  25                  30
   Leu Leu Glu Phe Ala Arg Val Val Lys Ala Lys Glu Gln Val Val Ala
           35                  40                  45
   Gly Thr Leu His His Leu Thr Leu Glu Ile Ala Gly Gln Lys Lys Leu
       50                  55                  60
   Tyr Glu Ala Lys Val Trp Val Lys Pro Trp Leu Asn Phe Lys Glu Leu
   65                  70                  75                  80
   Gln Glu Phe Lys Pro Ala Ser
                   85
<210> 213
   <211> 87
   <212> PRT
   <213> Artificial sequence
<220>
   <223> Synthetically generated peptide
<400> 213
   Gly Gly Val Gly Asp Val Pro Ala Asn Gln Asn Ser Gly Glu Val Glu
   1               5                   10                  15
   Ser Leu Ala Arg Phe Ala Val Asp Glu His Asn Lys Lys Glu Asn Ala
               20                  25                  30
   Leu Leu Glu Phe Ala Arg Val Val Lys Ala Lys Glu Gln Val Val Ala
           35                  40                  45
   Gly Thr Leu His His Leu Thr Leu Glu Ile Ala Gly Gln Lys Lys Leu
       50                  55                  60
   Tyr Glu Ala Lys Val Trp Val Lys Pro Trp Leu Asn Phe Lys Glu Leu
   65                  70                  75                  80
   Gln Glu Phe Lys Pro Ala Ser
                   85
<210> 214
   <211> 87
   <212> PRT
   <213> Artificial sequence
<220>
   <223> Synthetically generated peptide
<400> 214
   Gly Gly Val Gly Asp Val Pro Ala Asn Gln Asn Ser Gly Glu Val Glu
   1               5                   10                  15
   Ser Leu Ala Arg Phe Ala Val Asp Glu His Asn Lys Lys Glu Asn Ala
               20                  25                  30
   Leu Leu Glu Phe Ala Arg Val Val Lys Ala Lys Glu Gln Val Val Ala
           35                  40                  45
   Gly Thr Leu His His Leu Thr Leu Glu Ile Ala Gly Gln Lys Lys Leu
       50                  55                  60
   Tyr Glu Ala Lys Val Trp Val Lys Pro Trp Leu Asn Phe Lys Glu Leu
   65                  70                  75                  80
   Gln Glu Phe Lys Pro Ala Ser
                   85
<210> 215
   <211> 87
   <212> PRT
   <213> Artificial Sequence
<220>
   <223> Synthetically generated peptide
<400> 215
   Gly Gly Val Gly Asp Val Pro Ala Asn Gln Asn Ser Gly Glu Val Glu
   1               5                   10                  15
   Ser Leu Ala Arg Phe Ala Val Asp Glu His Asn Lys Lys Glu Asn Ala
               20                  25                  30
   Leu Leu Glu Phe Ala Arg Val Val Lys Ala Lys Glu Gln Val Val Ala
           35                  40                  45
   Gly Thr Leu His His Leu Thr Leu Glu Ile Ala Gly Gln Lys Lys Leu
       50                  55                  60
   Tyr Glu Ala Lys Val Trp Val Lys Pro Trp Leu Asn Phe Lys Glu Leu
   65                  70                  75                  80
   Gln Glu Phe Thr Pro Ala Ser
                   85
<210> 216
   <211> 87
   <212> PRT
   <213> Artificial Sequence
<220>
   <223> Synthetically generated peptide
<400> 216
   Gly Gly Val Arg Asp Val Pro Ala Asn Gln Asn Ser Gly Glu Val Glu
   1 5 10 15
   Ser Leu Ala Arg Phe Ala Val Asp Glu His Asn Lys Lys Glu Asn Ala
   20 25 30
   Leu Leu Glu Phe Ala Arg Val Val Lys Ala Lys Glu Gln Val Val Ala
   35 40 45
   Gly Thr Leu His His Leu Thr Leu Glu Ile Ala Gly Glu Lys Lys Leu
   50 55 60
   Tyr Glu Ala Lys Val Trp Val Lys Pro Trp Leu Asn Phe Lys Glu Leu
   65 70 75 80
   Gln Glu Phe Lys Pro Ala Ser
   85
<210> 217
   <211> 88
   <212> PRT
   <213> Artificial sequence
<220>
   <223> synthetically generated peptide
<400> 217
   Gly Gly Val Arg Asp Val Pro Ala Asn Gln Asn Ser Gly Glu Val Glu
   1 5 10 15
   Ser Leu Ala Arg Phe Ala Val Asp Glu His Asn Lys Lys Glu Asn Ala
   20 25 30
   Leu Leu Glu Phe Ala Arg Val Val Lys Ala Lys Glu Gln Val Val Ala
   35 40 45
   Gly Thr Met His His Leu Thr Leu Glu Ile Ile Glu Ala Gly Lys Lys
   50 55 60
   Leu Tyr Glu Ala Lys Val Trp Val Lys Pro Trp Leu Asn Phe Lys Glu
   65 70 75 80
   Leu Gln Glu Phe Lys Pro Ala Ser
   85
<210> 218
   <211> 89
   <212> PRT
   <213> Artificial Sequence
<220>
   <223> Synthetically generated peptide
<400> 218
   Gly Gly Val Arg Asp Val Pro Ser Asn Glu Asn Ser Val Glu Val Glu
   1 5 10 15
   Ser Leu Ala Arg Phe Ala Val Asp Glu His Asn Lys Lys Glu Asn Ala
   20 25 30
   Leu Leu Glu Phe Ala Arg Val Val Lys Ala Lys Glu Gln Val Val Ala
   35 40 45
   Gly Thr Met His His Leu Thr Leu Glu Ile Ile Glu Ala Gly Lys Lys
   50 55 60
   Lys Leu Tyr Glu Ala Lys Val Trp Val Lys Pro Trp Leu Asn Phe Lys
   65 70 75 80
   Glu Leu Gln Glu Phe Lys Pro Ser Thr
   85
<210> 219
   <211> 89
   <212> PRT
   <213> Artificial Sequence
<220>
   <223> Synthetically generated peptide
<400> 219
   Gly Gly Val Arg Asp Leu Pro Ala Asn Glu Asn Ser Val Glu Val Glu
    1               5                  10                  15
   Ser Leu Ala Arg Phe Ala Val Asp Glu His Asn Lys Lys Glu Asn Ala
               20                  25                  30
   Leu Leu Glu Phe Ala Arg Val Val Lys Ala Lys Glu Gln Val Val Ala
           35                  40                  45
   Gly Thr Met His His Leu Thr Leu Glu Ile Ile Glu Ala Gly Lys Lys
       50                  55                  60
   Lys Leu Tyr Glu Ala Lys Val Trp Val Lys Pro Trp Leu Asn Phe Lys
   65                  70                  75                      80
   Glu Leu Gln Glu Phe Lys Pro Ala Ser
                   85
<210> 220
   <211> 87
   <212> PRT
   <213> Artificial Sequence
<220>
   <223> Synthetically generated peptide
<400> 220
   Gly Gly Val His Asp Leu Arg Gly Asn Gln Asn Ser Gly Glu Ile Glu
    1               5                  10                  15
   Ser Leu Ala Arg Phe Ala Ile Gln Glu His Asn Lys Gln Gln Asn Lys
               20                  25                  30
   Ile Phe Lys Lys Ile Val Lys Ala Arg Glu Gln Val Val Ala Gly Thr
           35                  40                  45
   Met Tyr His Leu Thr Leu Glu Ala Lys Glu Gly Asp Gln Thr Lys Asn
       50                  55                  60
   Phe Glu Ala Lys Val Trp Val Lys Pro Trp Met Asn Phe Lys Gln Leu
   65                  70                  75                      80
   Gln Glu Phe Lys Glu Ser Ser
                   85
<210> 221
   <211> 87
   <212> PRT
   <213> Artificial Sequence
<220>
   <223> Synthetically generated peptide
<400> 221
   Gly Gly Val His Asp Leu Arg Gly Asn Gln Asn Ser Gly Glu Ile Glu
   1 5 10 15
   Ser Leu Ala Arg Phe Ala Ile Gln Glu His Asn Lys Gln Gln Asn Lys
   20 25 30
   Ile Phe Lys Lys Ile Val Lys Ala Arg Glu Gln Val Val Ala Gly Thr
   35 40 45
   Met Tyr His Leu Thr Leu Glu Ala Lys Glu Gly Asp Gln Thr Lys Asn
   50 55 60
   Phe Glu Ala Lys Val Trp Val Lys Pro Trp Met Asn Phe Lys Gln Leu
   65 70 75 80
   Gln Glu Phe Lys Glu Ser Ser
   85
<210> 222
   <211> 87
   <212> PRT
   <213> Artificial Sequence
<220>
   <223> Synthetically generated peptide
<400> 222
   Gly Gly Val His Asp Leu Arg Gly Asn Gln Asn Ser Gly Glu Ile Glu
   1 5 10 15
   Ser Leu Ala Arg Phe Ala Ile Gln Glu His Asn Lys Gln Gln Asn Lys
   20 25 30
   Ile Phe Lys Lys Ile Val Lys Ala Arg Glu Gln Val Val Ala Gly Thr
   35 40 45
   Met Tyr His Leu Thr Leu Glu Ala Lys Glu Gly Asp Gln Thr Lys Asn
   50 55 60
   Phe Glu Ala Lys Val Trp Val Lys Pro Trp Met Asn Phe Lys Gln Leu
   65 70 75 80
   Gln Glu Phe Lys Glu Ser Ser
   85
<210> 223
   <211> 87
   <212> PRT
   <213> Artificial sequence
<220>
   <223> Synthetically generated peptide
<400> 223
   Gly Gly Val His Asp Leu Arg Gly Asn Gln Asn Ser Gly Glu Ile Glu
   1 5 10 15
   Ser Leu Ala Arg Phe Ala Ile Gln Glu His Asn Lys Gln Gln Asn Lys
   20 25 30
   Ile Phe Lys Lys Ile Val Lys Ala Arg Glu Gln Val Val Ala Gly Thr
   35 40 45
   Met Tyr His Leu Thr Leu Glu Ala Lys Glu Gly Asp Gln Thr Lys Asn
   50 55 60
   Phe Glu Ala Lys Val Trp Val Lys Pro Trp Met Asn Phe Lys Gln Leu
   65 70 75 80
   Gln Glu Phe Lys Glu Ser Ser
   85
<210> 224
   <211> 89
   <212> PRT
   <213> Artificial sequence
<220>
   <223> Synthetically generated peptide
<400> 224
   Gly Gly Ile Gln Asp Ser Pro Ser Ser Glu Asn Ser Leu Glu Ile Glu
    1               5                  10                  15
   Ser Leu Ala Arg Phe Ala Val Asp Gln His Asn Ala Lys Gln Asn Ser
               20                  25                  30
   Leu Leu Glu Phe Ala Arg Val Val Lys Ala Arg Glu Gln Val Val Ala
           35                  40                  45
   Gly Thr Leu His His Leu Thr Leu Glu Ala Ile Asp Ala Gly Glu Lys
       50                  55                  60
   Lys Ile Tyr Glu Ala Lys Val Trp Val Lys Pro Trp Met Asn Phe Lys
   65                  70                  75                  80
   Glu Leu Thr Glu Phe Lys His Ala Gly
                   85
<210> 225
   <211> 89
   <212> PRT
   <213> Artificial Sequence
<220>
   <223> Synthetically generated peptide
<400> 225
   Gly Gly Ile Gln Asp Ser Pro Ser Ser Glu Asn Ser Leu Glu Ile Glu
    1               5                  10                  15
   Ser Leu Ala Arg Phe Ala Val Asp Gln His Asn Ala Lys Gln Asn Ser
               20                  25                  30
   Leu Leu Glu Phe Ala Arg Val Val Lys Ala Arg Glu Gln Val Val Ala
           35                  40                  45
   Gly Thr Leu His His Leu Thr Leu Glu Ala Ile Asp Ala Gly Glu Lys
       50                  55                  60
   Lys Ile Tyr Glu Ala Lys Val Trp Val Lys Pro Trp Met Asn Phe Lys
   65                  70                  75                  80
   Glu Leu Thr Glu Phe Lys His Ala Gly
                   85
<210> 226
   <211> 87
   <212> PRT
   <213> Artificial Sequence
<220>
   <223> Synthetically generated peptide
<400> 226
   Gly Gly Leu Arg Asp Ser Gln Gly Ser Gln Asn Ser Val Gln Thr Glu
   1 5 10 15
   Ala Arg Phe Ala Val Asp Glu His Asn Lys Lys Gln Asn Ser Leu Leu
   20 25 30
   Glu Phe Ser Arg Val Val Arg Thr Gln Glu Gln Val Val Ala Gly Thr
   35 40 45
   Leu His His Leu Thr Leu Glu Ala Ile Glu Ala Gly Glu Lys Lys Leu
   50 55 60
   Tyr Glu Ala Lys Val Trp Val Lys Pro Trp Leu Asn Phe Lys Glu Leu
   65 70 75 80
   Gln Glu Phe Lys Pro Ala Gly
   85
<210> 227
   <211> 89
   <212> PRT
   <213> Artificial Sequence
<220>
   <223> Synthetically generated peptide
<400> 227
   Gly Gly Val Lys Glu Asn Pro Ala Ala Ala Asn Ser Ala Asp Ser Asp
   1 5 10 15
   Gly Leu Gly Arg Phe Ala Val Asp Glu His Asn Arg Arg Glu Asn Ala
   20 25 30
   Leu Leu Glu Phe Val Arg Val Val Glu Ala Lys Glu Gln Val Val Ala
   35 40 45
   Gly Thr Leu His His Leu Thr Leu Glu Ala Val Glu Ala Gly Arg Lys
   50 55 60
   Lys Leu Tyr Glu Ala Lys Val Trp Val Lys Pro Trp Leu Asp Phe Lys
   65 70 75 80
   Glu Leu Gln Glu Phe Ser His Lys Gly
   85
<210> 228
   <211> 89
   <212> PRT
   <213> Artificial Sequence
<220>
   <223> Synthetically generated peptide
<400> 228
   Gly Gly Val Lys Glu Asn Pro Ala Ala Ala Asn Ser Ala Glu Ser Asp
   1 5 10 15
   Gly Leu Gly Arg Phe Ala Val Asp Glu His Asn Arg Arg Glu Asn Ala
   20 25 30
   Leu Leu Glu Phe Val Arg Val Val Glu Ala Lys Glu Gln Val Val Ala
   35 40 45
   Gly Thr Leu His His Leu Thr Leu Glu Ala Val Glu Ala Gly Arg Lys
   50 55 60
   Lys Leu Tyr Glu Ala Lys Val Trp Val Lys Pro Trp Leu Asp Phe Lys
   65 70 75 80
   Glu Leu Gln Glu Phe Ser His Lys Gly
   85
<210> 229
   <211> 89
   <212> PRT
   <213> Artificial Sequence
<220>
   <223> Synthetically generated peptide
<400> 229
   Gly Gly Lys Ser Glu Asn Pro Ala Ala Ala Asn Ser Leu Glu Thr Asp
    1               5                  10                  15
   Gly Leu Ala Arg Phe Ala Val Asp Glu His Asn Lys Arg Glu Asn Ala
               20                  25                  30
   Leu Leu Glu Phe Val Arg Val Val Glu Ala Lys Glu Gln Thr Val Ala
           35                  40                  45
   Gly Thr Val His His Leu Thr Leu Glu Ala Leu Glu Ala Gly Arg Lys
       50                  55                  60
   Lys Leu Tyr Glu Ala Lys Val Trp Val Lys Pro Trp Leu Asp Phe Lys
   65                  70                  75                  80
   Glu Leu Gln Glu Phe Arg His Thr Gly
                   85
<210> 230
   <211> 87
   <212> PRT
   <213> Artificial Sequence
<220>
   <223> Synthetically generated peptide
<400> 230
   Gly Gly Ala His Asp Ala Pro Ser Ala Ala Asn Ser Val Glu Thr Asp
    1               5                  10                  15
   Ala Arg Phe Ala Val Asp Glu His Asn Lys Arg Glu Asn Ala Leu Leu
               20                  25                  30
   Glu Phe Val Arg Val Val Glu Ala Lys Glu Gln Val Val Ala Gly Thr
           35                  40                  45
   Leu His His Leu Thr Leu Glu Ala Leu Glu Ala Gly Arg Lys Lys Val
       50                  55                  60
   Tyr Glu Ala Lys Val Trp Val Lys Pro Trp Leu Asp Phe Lys Glu Leu
   65                  70                  75                  80
   Gln Glu Phe Arg Asn Thr Gly
                   85
<210> 231
   <211> 90
   <212> PRT
   <213> Artificial sequence
<220>
   <223> Synthetically generated peptide
<400> 231
   Gly Gly Val His Asp Ser His Gly Ser Ser Gln Asn Ser Asp Glu Ile
    1               5                  10                  15
   His Ser Leu Ala Lys Leu Ala Val Asp Glu His Asn Lys Lys Glu Asn
               20                  25                  30
   Ala Met Ile Glu Leu Ala Arg Val Val Lys Ala Gln Glu Gln Thr Val
           35                  40                  45
   Ala Gly Lys Leu His His Leu Thr Leu Glu Val Met Asp Ala Gly Lys
       50                  55                  60
   Lys Lys Leu Tyr Glu Ala Lys Val Trp Val Lys Pro Trp Leu Asn Phe
   65                  70                  75                  80
   Lys Glu Leu Gln Glu Phe Lys His Val Glu
                   85                  90
<210> 232
   <211> 90
   <212> PRT
   <213> Artificial Sequence
<220>
   <223> Synthetically generated peptide
<400> 232
   Gly Gly Val His Asp Ser His Gly Ser Ser Gln Asn Ser Asp Glu Ile
    1               5                  10                  15
   His Ser Leu Ala Lys Phe Ala Val Asp Glu His Asn Lys Lys Glu Asn
               20                  25                  30
   Ala Met Ile Glu Leu Ala Arg Val Val Lys Ala Gln Glu Gln Thr Val
           35                  40                  45
   Ala Gly Lys Leu His His Leu Thr Leu Glu Val Met Asp Ala Gly Lys
       50                  55                  60
   Lys Lys Leu Tyr Glu Ala Lys Val Trp Val Lys Pro Trp Leu Asn Phe
   65                  70                  75                  80
   Lys Glu Leu Gln Glu Phe Lys His Val Glu
                   85                  90
<210> 233
   <211> 90
   <212> PRT
   <213> Artificial Sequence
<220>
   <223> Synthetically generated peptide
<400> 233
   Gly Gly Ile Arg Asp Ser His Pro Glu Ser Gln Asn Ser Asp Glu Ile
   1 5 10 15
   His Ser Leu Ala Lys Phe Ala Val Asp Glu His Asn Lys Lys Glu Asn
   20 25 30
   Ala Met Phe Glu Leu Ala Arg Val Val Lys Ala Lys Glu Gln Val Val
   35 40 45
   Ala Gly Thr Leu His His Leu Thr Leu Glu Val Val Asp Ala Gly Lys
   50 55 60
   Lys Lys Leu Tyr Glu Ala Lys Val Trp Val Lys Pro Trp Leu Asn Phe
   65 70 75 80
   Lys Glu Leu Gln Glu Phe Thr His Val Glu
   85 90
<210> 234
   <211> 89
   <212> PRT
   <213> Artificial Sequence
<220>
   <223> Synthetically generated peptide
<400> 234
   Gly Gly Ile Ser Asp Ser Ala Ser Ala Glu Asn Ser Val Glu Ile Glu
   1 5 10 15
   Ser Leu Ala Arg Phe Ala Val Glu Glu His Asn Lys Lys Glu Asn Ala
   20 25 30
   Met Ile Glu Leu Val Arg Val Val Lys Ala Glu Glu Gln Val Val Ala
   35 40 45
   Gly Lys Leu His His Leu Thr Leu Glu Val Ile Asp Ala Gly Lys Arg
   50 55 60
   Lys Leu Tyr Glu Ala Lys Val Trp Leu Lys Pro Trp Met Asn Phe Lys
   65 70 75 80
   Glu Leu Gln Gly Phe Asn His Ile Glu
   85
<210> 235
   <211> 89
   <212> PRT
   <213> Artificial Sequence
<220>
   <223> Synthetically generated peptide
<400> 235
   Gly Gly Ile Ser Asp Ser Ala Ser Ala Glu Asn Ser Val Glu Ile Glu
   1 5 10 15
   Ser Leu Ala Arg Phe Ala Val Glu Glu His Asn Lys Lys Glu Asn Ala
   20 25 30
   Met Ile Glu Leu Val Arg Val Val Lys Ala Glu Glu Gln Val Val Ala
   35 40 45
   Gly Lys Leu His His Leu Thr Leu Glu Val Ile Asp Ala Gly Lys Arg
   50 55 60
   Lys Leu Tyr Glu Ala Lys Val Trp Leu Lys Pro Trp Met Asn Phe Lys
   65 70 75 80
   Glu Leu Gln Gly Phe Asn His Ile Glu
   85
<210> 236
   <211> 90
   <212> PRT
   <213> Artificial Sequence
<220>
   <223> Synthetically generated peptide
<400> 236
   Gly Gly Phe Ser Asp Ser Lys Asn Asp Trp Asn Gly Gly Lys Glu Ile
   1 5 10 15
   Asp Asp Ile Ala Leu Phe Ala Val Gln Glu His Asn Arg Arg Glu Asn
   20 25 30
   Ala Val Leu Glu Leu Ala Arg Val Leu Lys Ala Thr Glu Gln Val Val
   35 40 45
   Ala Gly Lys Leu Tyr Arg Leu Thr Leu Glu Val Ile Glu Ala Gly Glu
   50 55 60
   Lys Lys Ile Tyr Glu Ala Lys Val Trp Val Lys Pro Trp Met Asn Phe
   65 70 75 80
   Lys Gln Leu Gln Glu Phe Lys Asn Ile Ile
   85 90
<210> 237
   <211> 90
   <212> PRT
   <213> Artificial Sequence
<220>
   <223> Synthetically generated peptide
<400> 237
   Gly Gly Phe Ser Asp Ser Lys Asn Asp Trp Asn Gly Gly Lys Glu Ile
   1 5 10 15
   Asp Asp Ile Ala Leu Phe Ala Val Gln Glu His Asn Arg Arg Glu Asn
   20 25 30
   Ala Val Leu Glu Leu Ala Arg Val Leu Lys Ala Thr Glu Gln Val Val
   35 40 45
   Ala Gly Lys Leu Tyr Arg Leu Thr Leu Glu Val Ile Glu Ala Gly Glu
   50 55 60
   Lys Lys Ile Tyr Glu Ala Lys Val Trp Val Lys Pro Trp Met Asn Phe
   65 70 75 80
   Lys Gln Leu Gln Glu Phe Lys Asn Ile Ile
   85 90

<210> 238
   <211> 86
   <212> PRT
   <213> Artificial Sequence
<220>
   <223> synthetically generated peptide
<400> 238
   Gly Gly Val His Asp Ser Asn Ser Asn Pro Asp Thr His Ser Leu Ala
   1 5 10 15
   Arg Phe Ala Val Asp Gln His Asn Thr Lys Glu Asn Gly Leu Leu Glu
   20 25 30
   Leu Val Arg Val Val Glu Ala Arg Glu Gln Val Val Ala Gly Thr Leu
   35 40 45
   His His Leu Val Leu Glu Val Leu Asp Ala Gly Lys Lys Lys Leu Tyr
   50 55 60
   Glu Ala Lys Ile Trp Val Lys Pro Trp Met Asp Phe Lys Gln Leu Gln
   65 70 75 80
   Glu Phe Lys His Val Arg
   85
<210> 239
   <211> 64
   <212> PRT
   <213> Artificial sequence
<220>
   <223> Synthetically generated peptide
<400> 239
   Ala His Tyr Asn Lys Asn Gln Gly Ala Ala Leu Glu Phe Thr Arg Val
   1 5 10 15
   Leu Lys Ser Lys Arg Gln Val Val Thr Gly Thr Leu His Asp Leu Ile
   20 25 30
   Ala Ala Asp Ala Gly Lys Lys Ser Val Tyr Arg Ala Lys Val Trp Val
   35 40 45
   Lys Pro Trp Glu Asp Phe Lys Ser Val Val Glu Phe Arg Leu Val Gly
   50 55 60
<210> 240
   <211> 84
   <212> PRT
   <213> Artificial Sequence
<220>
   <223> synthetically generated peptide
<400> 240
   Gly Val Leu Ala Ala Leu Arg Glu Arg Ala Glu Ala Glu Asp Ala Ala
   1 5 10 15
   Arg Phe Ala Val Ala His Tyr Asn Lys Asn Gln Gly Ala Ala Leu Glu
   20 25 30
   Phe Thr Arg Val Leu Lys Ser Lys Arg Gln Val Val Thr Gly Thr Leu
   35 40 45
   His Asp Leu Ile Ala Ala Asp Ala Gly Lys Lys Ser Val Tyr Arg Ala
   50 55 60
   Lys Val Trp Val Lys Pro Trp Glu Asp Phe Lys Ser Val Val Glu Phe
   65 70 75 80
   Arg Leu Val Gly
<210> 241
   <211> 85
   <212> PRT
   <213> Artificial sequence
<220>
   <223> Synthetically generated peptide
<400> 241
   Gly Met Leu Ala Ala Ile Arg Arg Glu Gln Ala Glu Ala Glu Asp Ala
   1 5 10 15
   Ala Arg Phe Ala Val Ala Glu Tyr Asn Lys Asn Gln Gly Ala Glu Leu
   20 25 30
   Glu Phe Ala Arg Ile Val Lys Ala Lys Arg Gln Val Val Thr Gly Thr
   35 40 45
   Leu His Asp Leu Met Leu Glu Val Val Asp Ser Gly Lys Lys Ser Lys
   50 55 60
   Ala Lys Val Trp Val Lys Pro Trp Leu Asp Phe Lys Ala Val Val Glu
   65 70 75 80
   Phe Arg His Val Gly
   85
<210> 242
   <211> 90
   <212> PRT
   <213> Artificial Sequence
<220>
   <223> Synthetically generated peptide
<400> 242
   Gly Ala Pro Arg Asp Val Pro Ala Gly Gly Glu Asn Ser Ala Asp Val
   1 5 10 15
   Glu Glu Leu Ala Arg Phe Ala Val Ala Glu His Asn Lys Lys Glu Asn
   20 25 30
   Ala Leu Leu Glu Phe Gly Arg Val Val Lys Ala Lys Glu Gln Val Val
   35 40 45
   Ala Gly Thr Leu His His Leu Thr Val Glu Ala Ile Asp Ala Gly Asn
   50 55 60
   Lys Lys Leu Tyr Glu Ala Lys Val Trp Val Lys Pro Trp Leu Asn Phe
   65 70 75 80
   Lys Glu Leu Gln Glu Phe Arg His Ala Gly
   85 90
<210> 243
   <211> 90
   <212> PRT
   <213> Artificial sequence
<220>
   <223> Synthetically generated peptide
<400> 243
   Gly Gly Ile Arg Asp Ser Pro Ala Gly Ser Glu Asn Ser Leu Glu Thr
   1 5 10 15
   Glu Ala Leu Gly Arg Phe Ala Val Asp Asp His Asn Gln Lys Gln Asn
   20 25 30
   Gly Met Leu Glu Phe Val Arg Val Val Lys Ala Lys Glu Gln Val Val
   35 40 45
   Ala Gly Thr Leu His His Leu Val Val Glu Ala Ile Asp Gly Gly Lys
   50 55 60
   Lys Lys Leu Tyr Glu Ala Lys Val Trp Val Lys Pro Trp Leu Asn Phe
   65 70 75 80
   Lys Gln Val Gln Glu Phe Lys His Ala Gly
   85 90
<210> 244
   <211> 89
   <212> PRT
   <213> Artificial Sequence
<220>
   <223> Synthetically generated peptide
<400> 244
   Gly Ala Val Arg Asp Asn Gln Gly Val Ala Asn Ser Val Glu Thr Glu
   1 5 10 15
   Ser Leu Ala Arg Tyr Ala Val Asp Glu His Asn Lys Lys Glu Asn Asp
   20 25 30
   Leu Leu Glu Phe Val Arg Val Leu Asp Asp Lys Val Gln Val Val Ser
   35 40 45
   Gly Thr Met His Tyr Leu Lys Ile Glu Ala Thr Glu Gly Gly Lys Lys
   50 55 60
   Lys Val Tyr Glu Ala Lys Val Trp Val Lys Pro Trp Glu Asn Phe Lys
   65 70 75 80
   Gln Val Gln Glu Phe Lys Pro Val Ser
   85
<210> 245
   <211> 59
   <212> PRT
   <213> Artificial sequence
<220>
   <223> Synthetically generated peptide
<400> 245
   Gly Gly Ile Thr Glu Val Lys Asp Phe Ala Asn Ser Leu Val Ile Asp
   1 5 10 15
   Asp Leu Ala Arg Phe Ala Val Asp Glu Tyr Ser Lys Lys Gln Asn Thr
   20 25 30
   Leu Leu Glu Phe Glu Arg Val Leu Asp Ala Lys Gln Gln Ile Val Ala
   35 40 45
   Gly Thr Met Tyr Tyr Phe Ile Ala Thr Val Gly
   50 55
<210> 246
   <211> 89
   <212> PRT
   <213> Artificial Sequence
<220>
   <223> Synthetically generated peptide
<400> 246
   Gly Gly Phe Thr Glu Val Lys Asp Phe Ala Asn Ser Ile Val Ile Asp
   1 5 10 15
   Asp Leu Ala Arg Phe Ala Val Asp Glu Tyr Asn Lys Lys Gln Asn Thr
   20 25 30
   Leu Leu Glu Phe Arg Lys Val Leu Asn Ala Lys Glu Gln Ile Val Ser
   35 40 45
   Gly Thr Leu Tyr Tyr Ile Thr Leu Asp Ala Ala Asn Gly Gly Ile Ile
   50 55 60
   Lys Thr Tyr Glu Ala Lys Val Trp Val Lys Lys Trp Glu Asn Leu Lys
   65 70 75 80
   Glu Leu Gln Glu Phe Lys Pro Val Asp
   85
<210> 247
   <211> 89
   <212> PRT
   <213> Artificial Sequence
<220>
   <223> synthetically generated peptide
<400> 247
   Gly Gly Ile Thr Glu Val Lys Asp Phe Ala Asn Ser Leu Glu Ile Glu
    1               5                  10                  15
   Asp Leu Ala Arg Phe Ala Val Asp Glu His Asn Lys Lys Gln Asn Thr
               20                  25                  30
   Leu Leu Glu Phe Gly Lys Val Leu Asn Ala Lys Glu Gln Ile Val Ala
           35                  40                  45
   Gly Lys Leu Cys Tyr Ile Thr Leu Glu Ala Thr Asp Gly Gly Val Lys
       50                  55                  60
   Lys Thr Tyr Glu Ala Lys Val Trp Val Lys Pro Trp Glu Asn Phe Lys
   65                  70                  75                  80
   Glu Leu Gln Glu Phe Lys Pro Val Asp
                   85
<210> 248
   <211> 88
   <212> PRT
   <213> Artificial Sequence
<220>
   <223> Synthetically generated peptide
<400> 248
   Gly Gly Ile Thr Glu Val Lys Asp Asn Asp Asn Ser Val Asp Phe Asp
    1               5                  10                  15
   Glu Leu Ala Lys Phe Ala Ile Ala Glu His Asn Lys Lys Glu Asn Ala
               20                  25                  30
   Ala Leu Glu Phe Gly Lys Val Ile Glu Lys Lys Gln Gln Ala Val Gln
           35                  40                  45
   Gly Thr Met Tyr Tyr Ile Lys Val Glu Ala Asn Asp Gly Gly Glu Lys
       50                  55                  60
   Lys Thr Tyr Glu Ala Lys Val Trp Val Lys Leu Trp Glu Asn Phe Lys
   65                  70                  75                  80
   Glu Leu Gln Glu Leu Lys Leu Val
                   85
<210> 249
   <211> 78
   <212> PRT
   <213> Artificial Sequence
<220>
   <223> Synthetically generated peptide
<400> 249
   Ser Leu Glu Ile Asp Glu Leu Ala Arg Phe Ala Val Asp Glu His Asn
    1               5                  10                  15
   Lys Lys Gln Asn Ala Leu Leu Glu Phe Gly Lys Val Val Asn Thr Lys
               20                  25                  30
   Glu Gln Val Val Ala Gly Lys Met Tyr Tyr Ile Thr Leu Glu Ala Thr
           35                  40                  45
   Asn Gly Gly Val Lys Lys Thr Tyr Glu Ala Lys Val Trp Val Lys Pro
       50                  55                  60
   Trp Glu Asn Phe Lys Glu Leu Gln Glu Phe Lys Pro Val Asp
   65                  70                  75
<210> 250
   <211> 89
   <212> PRT
   <213> Artificial Sequence
<220>
   <223> Synthetically generated peptide
<400> 250
   Gly Gly Ile Lys Gln Val Glu Gly Ser Ala Asn Ser Leu Glu Val Glu
    1               5                  10                  15
   Ser Leu Ala Lys Phe Ala Val Glu Asp His Asn Lys Lys Gln Asn Ala
               20                  25                  30
   Met Leu Glu Phe Ser Lys Val Val Asn Thr Lys Glu Gln Val Val Ala
           35                  40                  45
   Gly Thr Met Tyr Tyr Ile Thr Leu Glu Ala Thr Asp Gly Gly Lys Lys
       50                  55                  60
   Lys Val Tyr Glu Ala Lys Val Trp Val Lys Pro Trp Met Asn Phe Lys
   65                  70                  75                  80
   Gln Val Gln Glu Phe Lys Leu Leu Gly
                   85
<210> 251
   <211> 89
   <212> PRT
   <213> Artificial Sequence
<220>
   <223> Synthetically generated peptide
<400> 251
   Gly Gly Ile Arg Glu Ala Gly Gly Ser Glu Asn Ser Leu Glu Ile Asn
    1               5                  10                  15
   Asp Leu Ala Arg Phe Ala Val Asp Glu His Asn Lys Lys Gln Asn Ala
               20                  25                  30
   Leu Leu Glu Phe Gly Lys Val Val Asn Val Lys Glu Gln Val Val Ala
           35                  40                  45
   Gly Thr Met Tyr Tyr Ile Thr Leu Glu Ala Thr Glu Gly Gly Lys Lys
       50                  55                  60
   Lys Ala Tyr Glu Ala Lys Val Trp Val Lys Pro Trp Gln Asn Phe Lys
   65                  70                  75                  80
   Gln Val Glu Asp Phe Lys Leu Ile Gly
                   85
<210> 252
   <211> 89
   <212> PRT
   <213> Artificial Sequence
<220>
   <223> Synthetically generated peptide
<400> 252
   Gly Gly Ile His Glu Lys Glu Gly Ser Ala Asn Ser Val Glu Met Asp
    1               5                  10                  15
   Glu Leu Ala Arg Phe Ala Val Asp Gln His Asn Lys Lys Gln Asn Ala
               20                  25                  30
   Leu Leu Glu Phe Val Lys Val Val Asn Val Lys Glu Gln Val Val Ser
           35                  40                  45
   Gly Thr Leu Tyr Tyr Ile Thr Leu Glu Ala Thr Gly Gly Gly Gln Lys
       50                  55                  60
   Lys Leu Tyr Glu Ala Lys Val Trp Val Lys Pro Trp Met Asn Phe Lys
   65                  70                  75                  80
   Glu Leu Gln Asp Phe Gln Leu Ala Ser
                   85
<210> 253
   <211> 84
   <212> PRT
   <213> Artificial sequence
<220>
   <223> Synthetically generated peptide
<400> 253
   Gly Gly Ser Ser Ala Ile Ser Leu Glu Ile Asp Glu Leu Ala Lys Phe
    1               5                  10                  15
   Ala Val Asp His Tyr Asn Ser Ile Glu Asn Ala Leu Leu Glu Phe Pro
               20                  25                  30
   Arg Val Val Asn Thr Lys Glu Gln Val Val Ala Gly Thr Ile Tyr Tyr
           35                  40                  45
   Ile Thr Leu Glu Ala Thr Asp Gly Gly Val Lys Lys Leu Tyr Glu Ala
       50                  55                  60
   Asn Val Trp Val Lys Pro Gly Val Asn Phe Lys Glu Val Gln Val Phe
   65                  70                  75                  80
   Lys Tyr Val Gly
<210> 254
   <211> 90
   <212> PRT
   <213> Artificial Sequence
<220>
   <223> Synthetically generated peptide
<400> 254
   Gly Gly Ile Lys Asp Ser Gly Gly Ser Ser Ala Asn Ser Leu Glu Ile
   1 5 10 15
   Asp Glu Leu Ala Lys Phe Ala Val Asp His Tyr Asn Ser Lys Glu Asn
   20 25 30
   Ala Leu Leu Glu Phe Gln Arg Val Val Asn Thr Lys Glu Gln Val Val
   35 40 45
   Ala Gly Thr Ile Tyr Tyr Ile Thr Leu Glu Ala Thr Asp Gly Gly Val
   50 55 60
   Lys Lys Leu Tyr Glu Ala Lys Val Trp Val Lys Pro Trp Val Asn Phe
   65 70 75 80
   Lys Glu Val Gln Asp Phe Lys Tyr Val Gly
   85 90
<210> 255
   <211> 71
   <212> PRT
   <213> Artificial Sequence
<220>
   <223> Synthetically generated peptide
<400> 255
   Gly Gly Val Ser Asp Val Lys Gly His Glu Asn Ser Leu Gln Ile Asp
   1 5 10 15
   Asp Leu Ala Arg Phe Ala Val Asp Asp His Asn Lys Lys Ala Asn Thr
   20 25 30
   Leu Leu Gln Phe Lys Lys Val Val Asn Ala Lys Gln Gln Val Val Ser
   35 40 45
   Gly Thr Ile Tyr Ile Leu Thr Leu Glu Val Glu Asp Gly Gly Lys Lys
   50 55 60
   Lys Val Tyr Glu Ala Lys Ile
   65 70
<210> 256
   <211> 71
   <212> PRT
   <213> Artificial sequence
<220>
   <223> Synthetically generated peptide
<400> 256
   Gly Gly Val Ser Asp Val Lys Gly His Glu Asn Ser Leu Gln Ile Asp
   1 5 10 15
   Asp Leu Ala Arg Phe Ala Val Asp Asp His Asn Lys Lys Ala Asn Thr
   20 25 30
   Leu Leu Gln Phe Lys Lys Val Val Asn Ala Lys Gln Gln Val Val Ser
   35 40 45
   Gly Thr Ile Tyr Ile Leu Thr Leu Glu Val Glu Asp Gly Gly Lys Lys
   50 55 60
   Lys Val Tyr Glu Ala Lys Ile
   65 70
<210> 257
   <211> 89
   <212> PRT
   <213> Artificial sequence
<220>
   <223> Synthetically generated peptide
<400> 257
   Gly Gly Val Ser Asp Val Lys Gly His Glu Asn Ser Leu Gln Ile Asp
    1               5                  10                  15
   Asp Leu Ala Arg Phe Ala Val Asp Asp His Asn Lys Lys Ala Asn Thr
               20                  25                  30
   Leu Leu Gln Phe Lys Lys Val Val Asn Ala Lys Gln Gln Val Val Ser
           35                  40                  45
   Gly Thr Ile Tyr Ile Leu Thr Leu Glu Val Glu Asp Gly Gly Lys Lys
       50                  55                  60
   Lys Val Tyr Glu Ala Lys Ile Trp Glu Lys Pro Trp Leu Asn Phe Lys
   65                  70                  75                  80
   Glu Val Gln Glu Phe Lys Leu Ile Gly
                   85
<210> 258
   <211> 89
   <212> PRT
   <213> Artificial Sequence
<220>
   <223> Synthetically generated peptide
<400> 258
   Gly Gly Leu Gln Asp Val Glu Gly Asp Ala Asn Asn Leu Glu Tyr Gln
    1               5                  10                  15
   Glu Leu Ala Arg Phe Ala Val Asp Glu His Asn Lys Lys Thr Asn Ala
               20                  25                  30
   Met Leu Gln Phe Lys Arg Val Val Asn Val Lys Gln Ala Val Val Glu
           35                  40                  45
   Gly Leu Lys Tyr Cys Ile Thr Leu Glu Ala Val Asp Gly His Lys Thr
       50                  55                  60
   Lys Val Tyr Glu Ala Glu Ile Trp Leu Lys Leu Trp Glu Asn Phe Arg
   65                  70                  75                  80
   Ser Leu Glu Gly Phe Lys Leu Leu Gly
                   85
<210> 259
   <211> 79
   <212> PRT
   <213> Artificial Sequence
<220>
   <223> Synthetically generated peptide
<400> 259
   Glu Ser Val Glu Ile Asp Ser Leu Ala Arg Phe Ala Val Glu Glu His
   1 5 10 15
   Asn Lys Lys Gln Asn Ala Leu Leu Glu Phe Gly Arg Val Val Ser Ala
   20 25 30
   Gln Gln Gln Val Val Ser Gly Thr Leu Tyr Thr Ile Thr Leu Glu Ala
   35 40 45
   Lys Asp Gly Gly Gln Lys Lys Val Tyr Glu Ala Lys Val Trp Glu Lys
   50 55 60
   Pro Trp Leu Asn Phe Lys Glu Leu Gln Glu Phe Lys Leu Val Gly
   65 70 75
<210> 260
   <211> 89
   <212> PRT
   <213> Artificial Sequence
<220>
   <223> Synthetically generated peptide
<400> 260
   Gly Gly Asn Arg Asp Val Ala Gly Asn Gln Asn Ser Leu Glu Ile Asp
   1 5 10 15
   Ser Leu Ala Arg Phe Ala Val Glu Glu His Asn Lys Lys Gln Asn Ala
   20 25 30
   Leu Leu Glu Phe Gly Arg Val Val Ser Ala Gln Gln Gln Val Val Ser
   35 40 45
   Gly Thr Leu Tyr Thr Ile Thr Leu Glu Ala Lys Asp Gly Gly Gln Lys
   50 55 60
   Lys Val Tyr Glu Ala Lys Val Trp Glu Lys Pro Trp Leu Asn Phe Lys
   65 70 75 80
   Glu Leu Gln Glu Phe Lys His Val Gly
   85
<210> 261
   <211> 77
   <212> PRT
   <213> Artificial Sequence
<220>
   <223> Synthetically generated peptide
<400> 261
   Asn Ser Val Glu Ile Asp Ala Arg Phe Ala Val Glu Glu His Asn Lys
   1 5 10 15
   Lys Gln Asn Ala Leu Leu Glu Phe Glu Lys Val Val Thr Ala Lys Gln
   20 25 30
   Gln Val Val Ser Gly Thr Leu Tyr Thr Ile Thr Leu Glu Ala Lys Asp
   35 40 45
   Gly Gly Gln Lys Lys Val Tyr Glu Ala Lys Val Trp Glu Lys Ser Trp
   50 55 60
   Leu Asn Phe Lys Glu Val Gln Glu Phe Lys Leu Val Gly
   65 70 75
<210> 262
   <211> 89
   <212> PRT
   <213> Artificial sequence
<220>
   <223> Synthetically generated peptide
<400> 262
   Gly Gly Ile Lys Glu Val Glu Glu Ser Ala Asn Ser Val Glu Ile Asp
   1               5                   10                  15
   Asn Leu Ala Arg Phe Ala Val Asp Asp Tyr Asn Lys Lys Gln Asn Ala
               20                  25                  30
   Leu Leu Glu Phe Lys Arg Val Val Ser Thr Lys Gln Gln Val Val Ala
           35                  40                  45
   Gly Thr Met Tyr Tyr Ile Thr Leu Glu Val Ala Asp Gly Gly Gln Thr
       50                  55                  60
   Lys Val Tyr Glu Ala Lys Val Trp Glu Lys Pro Trp Leu Asn Phe Lys
   65                  70                  75                  80
   Glu Val Gln Glu Phe Lys Pro Ile Gly
                   85
<210> 263
   <211> 89
   <212> PRT
   <213> Artificial Sequence
<220>
   <223> Synthetically generated peptide
<400> 263
   Gly Gly Val Arg Asp Val Pro Gly Asn Gln Asn Ser Leu Ala Ile Asp
   1               5                   10                  15
   Gly Leu Ala Arg Phe Ala Val Glu Glu His Asn Lys Lys Gln Asn Ala
               20                  25                  30
   Leu Leu Glu Phe Ser Arg Val Ile Ser Ala Lys Glu Gln Val Val Ala
           35                  40                  45
   Gly Thr Ile His His Ile Thr Leu Glu Val Lys Asp Gly Val Asn Lys
       50                  55                  60
   Lys Val Tyr Glu Ala Lys Val Trp Glu Lys Ser Trp Met Asn Phe Lys
   65                  70                  75                  80
   Glu Val Gln Glu Phe Lys Leu Val Glu
                   85
<210> 264
   <211> 89
   <212> PRT
   <213> Artificial Sequence
<220>
   <223> Synthetically generated peptide
<400> 264
   Gly Ala Pro Arg Glu Val Ala Gly Asn Glu Asn Ser Leu Glu Ile Asp
   1 5 10 15
   Ser Leu Ala Arg Phe Ala Val Asp Glu His Asn Lys Lys Gln Asn Gly
   20 25 30
   Leu Leu Glu Phe Lys Arg Val Ile Ser Ala Lys Gln Gln Val Val Ala
   35 40 45
   Gly Thr Leu His His Ile Thr Leu Glu Ala Ala Ser Gly Asp Ser Lys
   50 55 60
   Asn Val Tyr Glu Ala Lys Val Trp Glu Lys Pro Trp Met Asn Phe Lys
   65 70 75 80
   Glu Val Gln Glu Phe Lys Leu Ala Gly
   85
<210> 265
   <211> 89
   <212> PRT
   <213> Artificial Sequence
<220>
   <223> synthetically generated peptide
<400> 265
   Gly Gly Ile Thr Asp Val Pro Gly Ala Ala Asn Ser Val Glu Ile Ala
   1 5 10 15
   Asn Leu Ala Arg Phe Ala Val Asp Asp His Asn Lys Lys Gln Asn Gly
   20 25 30
   Val Leu Glu Phe Val Arg Val Ile Ser Ala Lys Gln Gln Val Val Ser
   35 40 45
   Gly Ile Leu Tyr Tyr Ile Thr Leu Glu Ala Lys Asp Gly Glu Thr Lys
   50 55 60
   Lys Val Tyr Lys Thr Lys Val Trp Val Arg Glu Trp Leu Asn Pro Lys
   65 70 75 80
   Glu Val Gln Glu Phe Asn Leu Val Thr
   85
<210> 266
   <211> 89
   <212> PRT
   <213> Artificial Sequence
<220>
   <223> Synthetically generated peptide
<400> 266
   Gly Gly Ile Thr Asp Val Pro Gly Ala Ala Asn Ser Val Glu Ile Ala
   1 5 10 15
   Asn Leu Ala Arg Phe Ala Val Asp Asp His Asn Lys Lys Gln Asn Gly
   20 25 30
   Val Leu Glu Phe Val Arg Val Ile Ser Ala Lys Gln Gln Val Val Ser
   35 40 45
   Gly Ile Leu Tyr Tyr Ile Thr Leu Glu Ala Lys Asp Gly Glu Thr Lys
   50 55 60
   Lys Val Tyr Lys Thr Lys Val Trp Val Arg Glu Trp Leu Asn Pro Lys
   65 70 75 80
   Glu Val Gln Glu Phe Asn Leu Val Thr
   85
<210> 267
   <211> 89
   <212> PRT
   <213> Artificial Sequence
<220>
   <223> Synthetically generated peptide
<400> 267
   Gly Gly Ile Ile Asp Val Ser Gly Ser Ala Asn Cys Leu Glu Ile Ala
   1               5                   10                  15
   Ile Leu Ala Arg Phe Ala Val Asp Asp His Asn Lys Lys Gln Asn Gly
               20                  25                  30
   Val Leu Glu Phe Val Arg Val Ile Ser Ala Lys Gln Gln Val Val Ser
           35                  40                  45
   Gly Ile Leu Tyr Tyr Ile Thr Leu Glu Ala Lys Asp Gly Glu Thr Lys
       50                  55                  60
   Lys Val Tyr Lys Thr Lys Val Trp Val Arg Glu Trp Leu Asn Pro Lys
   65                  70                  75                  80
   Glu Val Gln Glu Phe Asn Leu Val Thr
                   85
<210> 268
   <211> 89
   <212> PRT
   <213> Artificial sequence
<220>
   <223> Synthetically generated peptide
<400> 268
   Gly Gly Ile Thr Asp Val His Gly Ala Ala Asn Ser Val Glu Ile Asn
   1               5                   10                  15
   Asn Leu Ala Arg Phe Ala Val Glu Glu Gln Asn Lys Arg Glu Asn Ser
               20                  25                  30
   Val Leu Glu Phe Val Arg Val Ile Ser Ala Lys Gln Gln Val Val Ala
           35                  40                  45
   Gly Val Asn Tyr Tyr Ile Thr Leu Glu Ala Lys Asp Gly Leu Ile Lys
       50                  55                  60
   Asn Glu Tyr Glu Ala Lys Val Trp Val Arg Glu Trp Leu Asn Ser Lys
   65                  70                  75                  80
   Glu Leu Leu Glu Phe Lys Pro Val Asn
                   85
<210> 269
   <211> 87
   <212> PRT
   <213> Artificial sequence
<220>
   <223> Synthetically generated peptide
<400> 269
   Gly Gly Val Gly Asp Val Pro Ser Asp Thr Leu His Ile Glu Asn Leu
    1               5                  10                  15
   Ala Arg Phe Ala Val Asp Gln Tyr Asn Lys Asn Glu Asn Ala Asn Leu
               20                  25                  30
   Glu Phe Val Arg Val Ile Asp Ala Lys Glu Gln Val Val Glu Gly Phe
           35                  40                  45
   Ile Tyr Tyr Ile Thr Leu Glu Ala Lys Asp Gly Glu Ser Lys Asn Val
       50                  55                  60
   Tyr Glu Ala Lys Val Trp Glu Arg Ser Trp Leu Asn Ser Ile Glu Leu
   65                  70                  75                  80
   Leu Glu Phe Lys Pro Val Asp
                   85
<210> 270
   <211> 87
   <212> PRT
   <213> Artificial Sequence
<220>
   <223> Synthetically generated peptide
<400> 270
   Gly Gly Val Gly Asp Val Pro Ser Asp Thr Leu His Ile Glu Asn Leu
    1               5                  10                  15
   Ala Arg Phe Ala Val Asp Gln Tyr Asn Lys Asn Glu Asn Ala Asn Leu
               20                  25                  30
   Glu Phe Val Arg Val Ile Asp Ala Lys Glu Gln Val Val Glu Gly Phe
           35                  40                  45
   Ile Tyr Tyr Ile Thr Leu Glu Ala Lys Asp Gly Glu Ser Lys Asn Val
       50                  55                  60
   Tyr Glu Ala Lys Val Trp Glu Arg Ser Trp Leu Asn Ser Ile Glu Leu
   65                  70                  75                  80
   Leu Glu Phe Lys Pro Val Asp
                   85
<210> 271
   <211> 89
   <212> PRT
   <213> Artificial sequence
<220>
   <223> Synthetically generated peptide
<400> 271
   Gly Gly Phe Thr Asp Ile Thr Gly Ala Gln Asn Ser Ile Asp Ile Glu
    1               5                  10                  15
   Asn Leu Ala Arg Phe Ala Val Asp Glu His Asn Lys Lys Glu Asn Ala
               20                  25                  30
   Val Leu Glu Phe Val Arg Val Ile Ser Ala Lys Lys Gln Val Val Ser
           35                  40                  45
   Gly Thr Leu Tyr Tyr Ile Thr Leu Glu Ala Asn Asp Gly Val Thr Lys
       50                  55                  60
   Lys Val Tyr Glu Thr Lys Val Leu Glu Lys Pro Trp Leu Asn Ile Lys
   65                  70                  75                  80
   Glu Val Gln Glu Phe Lys Pro Ile Thr
                   85
<210> 272
   <211> 48
   <212> PRT
   <213> Artificial Sequence
<220>
   <223> Synthetically generated peptide
<400> 272
   Gly Phe Thr Asp Ile Thr Gly Ala Gln Asn Ser Ile Asp Ile Glu Asn
    1               5                  10                  15
   Leu Ala Arg Phe Ala Val Asp Glu His Asn Lys Lys Glu Asn Ala Val
               20                  25                  30
   Leu Glu Phe Val Arg Val Lys Ser Ala Lys Lys Gln Val Val Ser Gly
           35                  40                  45
<210> 273
   <211> 86
   <212> PRT
   <213> Artificial Sequence
<220>
   <223> Synthetically generated peptide
<400> 273
   Gly Gly Val Thr Glu Cys Lys Asn Phe Glu Asn Asn Val Glu Ile Glu
    1               5                  10                  15
   Thr Ile Ala Lys Phe Ala Val Glu Glu His Asn Lys Lys Glu Asn Ala
               20                  25                  30
   Thr Leu Glu Phe Val Lys Val Val Ser Ala Lys Glu Gln Val Val Ser
           35                  40                  45
   Gly Lys Ile Tyr Tyr Ile Thr Ile Glu Thr Asn Asp Gly Lys Thr Tyr
       50                  55                  60
   Glu Ala Lys Leu Trp Val Lys Pro Trp Glu Asn Phe Gln Glu Leu Gln
   65                  70                  75                  80
   Glu Phe Lys Pro Ala Ala
                   85
<210> 274
   <211> 88
   <212> PRT
   <213> Artificial Sequence
<220>
   <223> synthetically generated peptide
<400> 274
   Gly Gly Val Arg Asp Val Pro Asp His Asn Ser Ala Glu Thr Glu Glu
   1               5                   10                  15
   Leu Ala Arg Phe Ala Val Gln Glu His Asn Lys Lys Ala Asn Thr Arg
               20                  25                  30
   Leu Glu Phe Ser Arg Val Val Lys Ala Lys Glu Gln Val Val Ala Gly
           35                  40                  45
   Thr Met Tyr Tyr Ile Thr Leu Glu Val Val Glu Ala Gly Gln Lys Lys
       50                  55                  60
   Ile Tyr Glu Ala Lys Val Trp Val Lys Leu Trp Glu Asn Phe Lys Glu
   65                  70                  75                  80
   Leu Gln Glu Phe Lys Pro Val Gly
                   85
<210> 275
   <211> 57
   <212> PRT
   <213> Artificial Sequence
<220>
   <223> Synthetically generated peptide
<400> 275
   His Leu Glu Tyr Val Glu Asn Leu Asn Val Lys Glu Gln Leu Val Ala
   1               5                   10                  15
   Gly Thr Leu Tyr Tyr Ile Thr Leu Val Ala Thr Asp Ala Gly Lys Lys
               20                  25                  30
   Lys Ile Tyr Glu Thr Lys Ile Trp Val Lys Glu Trp Glu Asp Phe Lys
           35                  40                  45
   Lys Val Val Glu Phe Lys Leu Val Gly
       50                  55
<210> 276
   <211> 88
   <212> PRT
   <213> Artificial Sequence
<220>
   <223> Synthetically generated peptide
<400> 276
   Gly Asp Ile Val Asp Val Pro Asp Pro Asn Ile Pro Pro Leu Gln Asp
   1               5                   10                  15
   Leu Ala Arg Phe Ala Val Gln Asp Tyr Asn Lys Ala Gln Asn Ala His
               20                  25                  30
   Leu Glu Tyr Val Glu Asn Leu Asn Val Lys Glu Gln Leu Val Ala Gly
           35                  40                  45
   Thr Leu Tyr Tyr Ile Thr Leu Val Ala Thr Asp Ala Gly Lys Lys Lys
       50                  55                  60
   Ile Tyr Glu Thr Lys Ile Trp Val Lys Glu Trp Glu Asp Phe Lys Lys
   65                  70                  75                  80
   Val Val Glu Phe Lys Leu Val Gly
                   85
<210> 277
   <211> 88
   <212> PRT
   <213> Artificial Sequence
<220>
   <223> Synthetically generated peptide
<400> 277
   Gly Gly Phe Thr Asp Val Pro Phe Pro Asn Asn Pro Glu Phe Gln Asp
    1               5                  10                  15
   Leu Ala Arg Phe Ala Val Gln Asp Tyr Asn Lys Lys Glu Asn Ala His
               20                  25                  30
   Leu Glu Tyr Val Glu Asn Leu Asn Val Lys Glu Gln Leu Val Ala Gly
           35                  40                  45
   Met Ile Tyr Tyr Ile Thr Leu Val Ala Thr Asp Ala Gly Lys Lys Lys
       50                  55                  60
   Ile Tyr Glu Ala Lys Ile Trp Val Lys Glu Trp Glu Asp Phe Lys Lys
   65                  70                  75                  80
   Val Val Glu Phe Lys Leu Val Gly
                   85
<210> 278
   <211> 88
   <212> PRT
   <213> Artificial Sequence
<220>
   <223> Synthetically generated peptide
<400> 278
   Gly Gly Ile Thr Asp Val Pro Phe Pro Asn Asn Pro Glu Phe Gln Asp
    1               5                  10                  15
   Leu Ala Arg Phe Ala Ile Gln Val Tyr Asn Lys Lys Glu Asn Val His
               20                  25                  30
   Leu Glu Phe Val Glu Asn Leu Asn Val Lys Gln Gln Val Val Ala Gly
           35                  40                  45
   Met Met Tyr Tyr Ile Thr Leu Ala Ala Ile Asp Ala Gly Lys Lys Lys
       50                  55                  60
   Ile Tyr Glu Thr Lys Ile Trp Val Lys Glu Trp Glu Asp Phe Lys Lys
   65                  70                  75                  80
   Val Val Glu Phe Lys Leu Val Gly
                   85
<210> 279
   <211> 89
   <212> PRT
   <213> Artificial Sequence
<220>
   <223> Synthetically generated peptide
<400> 279
   Gly Gly Ile Val Asn Val Pro Asn Pro Asn Asn Thr Lys Phe Gln Glu
   1 5 10 15
   Leu Ala Arg Phe Ala Ile Gln Asp Tyr Asn Lys Lys Gln Asn Ala His
   20 25 30
   Leu Glu Phe Val Glu Asn Leu Asn Val Lys Glu Gln Val Val Ala Gly
   35 40 45
   Ile Met Tyr Tyr Ile Thr Leu Ala Ala Thr Asp Asp Ala Gly Lys Lys
   50 55 60
   Lys Ile Tyr Lys Ala Lys Ile Trp Val Lys Glu Trp Glu Asp Phe Lys
   65 70 75 80
   Lys Val Val Glu Phe Lys Leu Val Gly
   85
<210> 280
   <211> 58
   <212> PRT
   <213> Artificial Sequence
<220>
   <223> Synthetically generated peptide
<400> 280
   Gly Gly Ile Ile Ser Val Pro Phe Pro Asn Ser Pro Glu Phe Gln Asp
   1 5 10 15
   Leu Ala Arg Phe Ala Val Gln Asp Tyr Asn Asn Thr Gln Asn Ala His
   20 25 30
   Leu Glu Phe Val Glu Asn Leu Ser Val Lys Glu Gln Leu Val Ser Gly
   35 40 45
   Met Met Tyr Tyr Ile Thr Leu Ala Ala Thr
   50 55
<210> 281
   <211> 88
   <212> PRT
   <213> Artificial Sequence
<220>
   <223> Synthetically generated peptide
<400> 281
   Gly Gly Ile Ile Asn Val Pro Asn Pro Asn Ser Pro Glu Phe Gln Asp
   1 5 10 15
   Leu Ala Arg Phe Ala Val Gln Asp Tyr Asn Asn Thr Gln Asn Ala His
   20 25 30
   Leu Glu Phe Val Glu Asn Leu Asn Val Lys Glu Gln Leu Val Ser Gly
   35 40 45
   Met Met Tyr Tyr Ile Thr Leu Ala Ala Thr Asp Ala Gly Asn Lys Lys
   50 55 60
   Glu Tyr Glu Ala Lys Ile Trp Val Lys Glu Trp Glu Asp Phe Lys Lys
   65 70 75 80
   Val Ile Asp Phe Lys Leu Val Gly
   85
<210> 282
   <211> 88
   <212> PRT
   <213> Artificial Sequence
<220>
   <223> Synthetically generated peptide
<400> 282
   Gly Gly Ile Ile Asn Val Pro Phe Pro Asn Asn Pro Glu Phe Gln Asp
   1               5                   10                  15
   Leu Ala Arg Phe Ala Val Gln Asp Tyr Asn Lys Lys Glu Asn Ala His
               20                  25                  30
   Leu Glu Phe Val Glu Asn Leu Asn Val Lys Glu Gln Leu Val Ala Gly
           35                  40                  45
   Met Leu Tyr Tyr Ile Thr Leu Val Ala Ile Asp Ala Gly Lys Lys Lys
       50                  55                  60
   Ile Tyr Glu Ala Lys Ile Trp Val Lys Glu Trp Glu Asn Phe Lys Lys
   65                  70                  75                  80
   Val Ile Glu Phe Lys Leu Ile Gly
                   85
<210> 283
   <211> 88
   <212> PRT
   <213> Artificial Sequence
<220>
   <223> Synthetically generated peptide
<400> 283
   Gly Gly Ile Thr Asn Val Pro Phe Pro Asn Leu Pro Glu Phe Lys Asp
   1               5                   10                  15
   Leu Ala Arg Phe Ala Val Gln Asp Tyr Asn Lys Lys Glu Asn Ala His
               20                  25                  30
   Leu Glu Phe Val Glu Asn Leu Asn Val Lys Glu Gln Val Val Ala Gly
           35                  40                  45
   Ile Ile Tyr Tyr Ile Thr Leu Val Ala Thr Asp Ala Gly Lys Lys Lys
       50                  55                  60
   Ile Tyr Glu Thr Lys Ile Leu Val Lys Gly Trp Glu Asn Phe Lys Glu
   65                  70                  75                  80
   Val Gln Asp Phe Lys Leu Val Gly
                   85
<210> 284
   <211> 88
   <212> PRT
   <213> Artificial Sequence
<220>
   <223> Synthetically generated peptide
<400> 284
   Gly Gly Ile Thr Asn Val Pro Phe Pro Asn Leu Pro Gln Phe Lys Asp
   1 5 10 15
   Leu Ala Arg Phe Ala Val Gln Asp Tyr Asn Lys Lys Glu Asn Ala His
   20 25 30
   Leu Glu Phe Val Glu Asn Leu Asn Val Lys Glu Gln Val Val Ala Gly
   35 40 45
   Ile Ile Tyr Tyr Ile Thr Leu Val Ala Thr Asp Ala Gly Lys Lys Lys
   50 55 60
   Ile Tyr Glu Thr Lys Ile Leu Val Lys Gly Trp Glu Asn Phe Lys Glu
   65 70 75 80
   Val Gln Glu Phe Lys Leu Val Gly
   85
<210> 285
   <211> 88
   <212> PRT
   <213> Artificial Sequence
<220>
   <223> synthetically generated peptide
<400> 285
   Gly Gly Ile Ile Ile Val Pro Phe Pro Asn Ser Pro Glu Phe Gln Asp
   1 5 10 15
   Leu Ala Arg Phe Ala Val Gln Asp Phe Asn Lys Lys Glu Asn Gly His
   20 25 30
   Leu Glu Phe Val Glu Asn Leu Asn Val Lys Glu Gln Val Val Ala Gly
   35 40 45
   Met Met Tyr Tyr Ile Thr Leu Ala Ala Thr Asp Ala Arg Lys Lys Glu
   50 55 60
   Ile Tyr Glu Thr Lys Ile Leu Val Lys Glu Trp Glu Asn Phe Lys Glu
   65 70 75 80
   Val Gln Glu Phe Lys Leu Val Gly
   85
<210> 286
   <211> 58
   <212> PRT
   <213> Artificial Sequence
<220>
   <223> Synthetically generated peptide
<400> 286
   Gly Gly Leu Thr Asp Val Pro Phe Pro Asn Asn Pro Glu Phe Gln Asp
   1 5 10 15
   Leu Ala Arg Phe Ala Val Gln Asp Tyr Asn Lys Lys Glu Asn Gly His
   20 25 30
   Leu Glu Phe Val Glu Val Leu Asn Val Lys Glu Gln Val Val Ala Gly
   35 40 45
   Met Met Tyr Tyr Ile Thr Leu Ala Ala Thr
   50 55
<210> 287
   <211> 88
   <212> PRT
   <213> Artificial Sequence
<220>
   <223> Synthetically generated peptide
<400> 287
   Gly Gly Phe Thr Glu Val Pro Phe Pro Asn Ser Pro Glu Phe Gln Asp
   1               5                   10                  15
   Leu Thr Arg Phe Ala Val His Gln Tyr Asn Lys Asp Gln Asn Ala His
               20                  25                  30
   Leu Glu Phe Val Glu Asn Leu Asn Val Lys Lys Gln Val Val Ala Gly
           35                  40                  45
   Met Leu Tyr Tyr Ile Thr Phe Ala Ala Thr Asp Gly Gly Lys Lys Lys
       50                  55                  60
   Ile Tyr Glu Thr Lys Ile Trp Val Lys Val Trp Glu Asn Phe Lys Lys
   65                  70                  75                  80
   Val Val Glu Phe Lys Leu Val Gly
                   85
<210> 288
   <211> 57
   <212> PRT
   <213> Artificial Sequence
<220>
   <223> Synthetically generated peptide
<400> 288
   His Leu Glu Phe Val Glu Val Leu Asn Val Lys Glu Gln Val Val Ala
   1               5                   10                  15
   Gly Met Met Tyr Tyr Ile Thr Leu Ala Val Thr Asp Ala Gly Lys Lys
               20                  25                  30
   Lys Ile Tyr Glu Ala Asn Ile Trp Val Lys Glu Trp Glu His Phe Ile
           35                  40                  45
   Lys Val Val Glu Phe Lys Pro Val Ser
       50                  55
<210> 289
   <211> 59
   <212> PRT
   <213> Artificial Sequence
<220>
   <223> Synthetically generated peptide
<400> 289
   Asn Ala His Leu Glu Phe Val Glu Val Leu Asn Val Lys Glu Gln Val
   1               5                   10                  15
   Val Ala Gly Met Met Tyr Tyr Ile Thr Leu Val Ala Thr Asp Asp Gly
               20                  25                  30
   Tyr Lys Lys Ile Tyr Lys Thr Lys Ile Trp Val Lys Glu Trp Glu Asn
           35                  40                  45
   Phe Lys Glu Val Gln Glu Phe Lys Gln Ile Val
       50                  55
<210> 290
   <211> 88
   <212> PRT
   <213> Artificial Sequence
<220>
   <223> Synthetically generated peptide
<400> 290
   Gly Gly Leu Val Asp Val Pro Phe Glu Asn Lys Val Glu Phe Asp Asp
   1               5                   10                  15
   Leu Ala Arg Phe Ala Val Gln Asp Tyr Asn Gln Lys Asn Asp Ser Ser
               20                  25                  30
   Leu Glu Phe Lys Lys Val Leu Asn Val Lys Gln Gln Ile Val Ala Gly
           35                  40                  45
   Ile Met Tyr Tyr Ile Thr Phe Glu Ala Thr Glu Gly Gly Asn Lys Lys
       50                  55                  60
   Glu Tyr Glu Ala Lys Ile Leu Leu Arg Lys Trp Glu Asp Leu Lys Lys
   65                  70                  75                  80
   Val Val Gly Phe Lys Leu Val Gly
                   85
<210> 291
   <211> 35
   <212> PRT
   <213> Artificial Sequence
<220>
   <223> Synthetically generated peptide
<400> 291
   Arg Lys Ser Leu Glu His Phe Asn Glu Ile His Ser Thr Lys Tyr Glu
   1               5                   10                  15
   Phe Val Arg Phe Ile Lys Ala Asn His Gln Val Ser Ala Gly Met Met
               20                  25                  30
   Tyr Phe Ile
           35
<210> 292
   <211> 61
   <212> PRT
   <213> Artificial Sequence
<220>
   <223> synthetically generated peptide
<400> 292
   Gly Gly Trp Asn Pro Ile Pro Asp Val Ser Asp Ser His Ile Gln Glu
   1               5                   10                  15
   Leu Gly Gly Trp Ala Leu Gly Gln Ala Lys His Gln Lys Leu Ala Ala
               20                  25                  30
   Asp Gly Leu Arg Phe Arg Arg Val Val Arg Gly Glu Gln Gln Val Val
           35                  40                  45
   Ser Gly Met Asn Tyr Arg Leu Tyr Val Asp Ala Ala Asp
       50                  55                  60
<210> 293
   <211> 61
   <212> PRT
   <213> Artificial Sequence
<220>
   <223> Synthetically generated peptide
<400> 293
   Gly Gly Trp Asn Pro Ile Pro Asp Val Ser Asp Ser His Ile Gln Glu
   1               5                   10                  15
   Leu Gly Gly Trp Ala Leu Gly Gln Ala Lys His Gln Lys Leu Ala Ala
               20                  25                  30
   Asp Gly Leu Arg Phe Arg Arg Val Val Arg Gly Glu Gln Gln Val Val
           35                  40                  45
   Ser Gly Met Asn Tyr Arg Leu Tyr Val Asp Ala Ala Asp
       50                  55                  60
<210> 294
   <211> 64
   <212> PRT
   <213> Artificial Sequence
<220>
   <223> Synthetically generated peptide
<400> 294
   Gly Gly Trp Ser Pro Ile Arg Asn Val Ser Asp Pro His Ile Gln Glu
   1               5                   10                  15
   Leu Gly Gly Trp Ala Val Thr Glu His Val Arg Arg Ala Asn Asp Gly
               20                  25                  30
   Leu Arg Phe Gly Glu Val Thr Gly Gly Glu Glu Gln Val Val Ser Gly
           35                  40                  45
   Met Asn Tyr Lys Leu Val Leu Asp Ala Thr Asp Ala Asp Gly Lys Val
       50                  55                  60
<210> 295
   <211> 59
   <212> PRT
   <213> Artificial Sequence
<220>
   <223> Synthetically generated peptide
<400> 295
   Gly Gly Trp Lys Pro Ile Lys Asn Val Asn Asp Pro His Val Arg Glu
   1               5                   10                  15
   Ile Gly Arg Trp Ala Val Ser Glu His Ile Lys Thr Ala Asn Asp Gly
               20                  25                  30
   Leu Gly Phe Gly Arg Val Val Ser Gly Glu Glu Gln Ile Val Ala Gly
           35                  40                  45
   Lys Asn Tyr Arg Leu Arg Ile Gln Ala Thr Lys
       50                  55
<210> 296
   <211> 59
   <212> PRT
   <213> Artificial Sequence
<220>
   <223> synthetically generated peptide
<400> 296
   Gly Gly Trp Lys Pro Ile Lys Asn Val Asn Asp Pro His Val Gln Glu
   1               5                   10                  15
   Ile Gly Arg Trp Ala Val Ser Glu His Ile Lys Thr Ala Asn Asp Gly
               20                  25                  30
   Leu Gly Phe Gly Arg Val Val Ser Gly Glu Glu Gln Ile Val Ala Gly
           35                  40                  45
   Lys Asn Tyr Arg Leu Arg Ile Gln Ala Thr Lys
       50                  55
<210> 297
   <211> 58
   <212> PRT
   <213> Artificial Sequence
<220>
   <223> Synthetically generated peptide
<400> 297
   Gly Trp Glu Pro Ile Gly Asn Ile Asn Asp Gln His Ile Gln Glu Leu
   1               5                   10                  15
   Gly Arg Trp Ala Val Leu Glu Phe Gly Lys His Val Asn Cys Val Leu
               20                  25                  30
   Lys Phe Asn Lys Val Val Ser Gly Arg Gln Gln Leu Val Ser Gly Met
           35                  40                  45
   Asn Tyr Glu Leu Ile Ile Glu Ala Ser Asp
       50                  55
<210> 298
   <211> 64
   <212> PRT
   <213> Artificial Sequence
<220>
   <223> Synthetically generated peptide
<400> 298
   Gly Gly Trp Ser Pro Ile Ser Asp Ala Lys Asp Pro His Val Val Glu
   1               5                   10                  15
   Ile Gly Val Phe Ala Val Ser Glu Tyr Asp Lys Gln Ser Lys Ser Gly
               20                  25                  30
   Leu Lys Phe Val Thr Val Val Ser Gly Glu Ser Gln Val Ala Ala Gly
           35                  40                  45
   Thr Asn Tyr Arg Leu Ile Val Thr Val Asp Gly Ser Ile Gly Val Ala
       50                  55                  60
<210> 299
   <211> 92
   <212> PRT
   <213> Artificial Sequence
<220>
   <223> Synthetically generated peptide
<400> 299
   Gly Arg Trp Ser Pro Ile Ser Asn Val Lys Asp Pro His Val Val Glu
    1               5                  10                  15
   Val Gly Lys Phe Ala Val Ser Glu Tyr Asp Met Glu Ser Lys Ser Glu
               20                  25                  30
   Leu Lys Phe Val Val Val Val Ser Gly Glu Ser Lys Val Val Ala Gly
           35                  40                  45
   Thr Asn Tyr Arg Leu Ile Val Ala Val Asn Asp Gly Val Ala Gly Pro
       50                  55                  60
   Gly Ala Ser Lys Asn Tyr Glu Ala Ile Val Trp Glu Lys Gln Trp Leu
   65                  70                  75                  80
   Lys Ser Met Asn Leu Thr Ser Phe Lys Pro Val Val
                   85                  90
<210> 300
   <211> 94
   <212> PRT
   <213> Artificial Sequence

<220>
   <223> Synthetically generated peptide
<400> 300
   Gly Gly Trp Arg Pro Leu Ser Asp Val Asn Asp Pro His Val Val Glu
    1               5                  10                  15
   Ile Gly Lys Phe Ser Val Ser Glu Tyr Asn Lys Gln Ser Lys Ala Gly
               20                  25                  30
   Leu Lys Phe Val Ala Val Val Ser Gly Glu Ser Gln Val Val Ala Gly
           35                  40                  45
   Met Asn Tyr Arg Leu Ile Val Ala Val Asn Asp Gly Val Glu Thr Ala
       50                  55                  60
   Gly Ala Gly Ala Ser Lys Asn Tyr Glu Ala Ile Val Trp Glu Arg Ala
   65                  70                  75                  80
   Trp Leu Lys Ser Met Asn Leu Thr Ser Phe Lys Pro Ala Ile
                   85                  90
<210> 301
   <211> 89
   <212> PRT
   <213> Artificial sequence
<220>
   <223> synthetically generated peptide
<400> 301
   Gly Gly Trp Ser Pro Ile Ser Asn Val Thr Asp Pro Gln Val Val Glu
   1 5 10 15
   Ile Gly Glu Phe Ala Val Ser Glu Tyr Asn Lys Arg Ser Glu Ser Gly
   20 25 30
   Leu Lys Phe Glu Thr Val Val Ser Gly Glu Thr Gln Val Val Ser Gly
   35 40 45
   Thr Asn Tyr Arg Leu Lys Val Ala Ala Asn Asp Gly Asp Gly Val Ser
   50 55 60
   Lys Asn Tyr Leu Ala Ile Val Trp Asp Lys Pro Trp Met Lys Phe Arg
   65 70 75 80
   Asn Leu Thr Ser Phe Glu Pro Ala Asn
   85
<210> 302
   <211> 89
   <212> PRT
   <213> Artificial Sequence
<220>
   <223> synthetically generated peptide
<400> 302
   Gly Gly Trp Ser Pro Ile Ser Asn Val Thr Asp Pro Gln Val Val Glu
   1 5 10 15
   Ile Gly Glu Phe Ala Val Ser Glu Tyr Asn Lys Arg Ser Glu Ser Gly
   20 25 30
   Leu Lys Phe Glu Thr Val Val Ser Gly Glu Thr Gln Val Val Ser Gly
   35 40 45
   Thr Asn Tyr Arg Leu Lys Val Ala Ala Asn Asp Gly Asp Gly Val Ser
   50 55 60
   Lys Asn Tyr Leu Ala Ile Val Trp Asp Lys Pro Trp Met Lys Phe Arg
   65 70 75 80
   Asn Leu Thr Ser Phe Glu Pro Ala Asn
   85
<210> 303
   <211> 89
   <212> PRT
   <213> Artificial sequence
<220>
   <223> Synthetically generated peptide
<400> 303
   Gly Gly Trp Ser Pro Ile Ser Asn Val Thr Asp Pro Gln Val Val Glu
   1 5 10 15
   Ile Gly Glu Phe Ala Val Ser Glu Tyr Asn Lys Arg Ser Glu Ser Gly
   20 25 30
   Leu Lys Phe Glu Thr Val Val Ser Gly Glu Thr Gln Val Val Ser Gly
   35 40 45
   Thr Asn Tyr Arg Leu Lys Val Ala Ala Asn Asp Gly Asp Gly Val Ser
   50 55 60
   Lys Asn Tyr Leu Ala Ile Val Trp Val Lys Pro Trp Met Lys Phe Arg
   65 70 75 80
   Asn Leu Thr Ser Phe Glu Pro Ala Asn
   85
<210> 304
   <211> 88
   <212> PRT
   <213> Artificial Sequence
<220>
   <223> Synthetically generated peptide
<400> 304
   Gly Gly Trp Lys Pro Ile Glu Asp Pro Lys Glu Lys His Val Met Glu
    1               5                  10                  15
   Ile Gly Gln Phe Ala Val Thr Glu Tyr Asn Lys Gln Ser Lys Ser Ala
               20                  25                  30
   Leu Lys Phe Glu Ser Val Glu Lys Gly Glu Thr Gln Val Val Ser Gly
           35                  40                  45
   Thr Asn Tyr Arg Leu Ile Leu Val Val Lys Asp Gly Pro Ser Thr Lys
       50                  55                  60
   Lys Phe Glu Ala Val Val Trp Glu Lys Pro Trp Glu His Phe Lys Ser
   65                  70                  75                  80
   Leu Thr Ser Phe Lys Pro Met Val
                   85
<210> 305
   <211> 86
   <212> PRT
   <213> Artificial sequence
<220>
   <223> Synthetically generated peptide
<400> 305
   Gly Ser Arg Lys Pro Ile Lys Asn Val Ser Asp Pro Asp Val Val Ala
    1               5                  10                  15
   Val Ala Lys Tyr Ala Ile Glu Glu His Asn Lys Glu Ser Lys Glu Asn
               20                  25                  30
   Leu Val Phe Val Lys Val Val Glu Gly Thr Thr Gln Val Val Ser Gly
           35                  40                  45
   Thr Lys Tyr Asp Leu Lys Ile Ala Ala Lys Asp Gly Gly Gly Lys Ile
       50                  55                  60
   Lys Asn Tyr Glu Ala Val Val Val Glu Lys Leu Trp Leu His Ser Lys
   65                  70                  75                  80
   Ser Leu Glu Ser Phe Lys
                   85
<210> 306
   <211> 86
   <212> PRT
   <213> Artificial Sequence
<220>
   <223> Synthetically generated peptide
<400> 306
   Gly Ser Arg Lys Pro Ile Lys Asn Val Ser Asp Pro Asp Val Val Ala
   1 5 10 15
   Val Ala Lys Tyr Ala Ile Glu Glu His Asn Lys Glu Ser Lys Glu Lys
   20 25 30
   Leu Val Phe Val Lys Val Val Glu Gly Thr Thr Gln Val Val Ser Gly
   35 40 45
   Thr Lys Tyr Asp Leu Lys Ile Ala Ala Lys Asp Gly Gly Gly Lys Ile
   50 55 60
   Lys Asn Tyr Glu Ala Val Val Val Glu Lys Leu Trp Leu His Ser Lys
   65 70 75 80
   Ser Leu Glu Ser Phe Lys
   85
<210> 307
   <211> 56
   <212> PRT
   <213> Artificial Sequence
<220>
   <223> Synthetically generated peptide
<400> 307
   Gly Gln Tyr Val Lys Ile Glu Asn Val Lys Asp Pro Tyr Val Gln Gly
   1 5 10 15
   Val Gly Glu Trp Ala Val Lys Glu His Asn Arg Gln Thr Gly Glu Ser
   20 25 30
   Leu Gln Phe Ala Glu Val Val Ser Gly Met Glu Gln Val Val Ala Gly
   35 40 45
   Thr Asn Tyr Lys Leu Asn Leu Ala
   50 55
<210> 308
   <211> 60
   <212> PRT
   <213> Artificial sequence
<220>
   <223> Synthetically generated peptide
<400> 308
   Gly Gly Trp Arg Pro Ile Glu Ser Leu Asn Ser Ala Glu Val Gln Asp
   1 5 10 15
   Val Ala Gln Phe Ala Val Ser Glu His Asn Lys Gln Ala Asn Asp Glu
   20 25 30
   Leu Gln Tyr Gln Ser Val Val Arg Gly Tyr Thr Gln Val Val Ala Gly
   35 40 45
   Thr Asn Tyr Arg Leu Val Ile Ala Ala Lys Asp Gly
   50 55 60
<210> 309
   <211> 60
   <212> PRT
   <213> Artificial Sequence
<220>
   <223> Synthetically generated peptide
<400> 309
   Gly Gly Trp Arg Pro Ile Glu Asn Leu Asn Ser Ala Glu Val Gln Asp
   1 5 10 15
   Val Ala Gln Phe Ala Val Ser Glu His Asn Lys Gln Ala Asn Asp Glu
   20 25 30
   Leu Gln Tyr Gln Ser Val Val Arg Gly Tyr Thr Gln Val Val Ser Gly
   35 40 45
   Thr Asn Tyr Arg Leu Val Ile Ala Ala Lys Asp Gly
   50 55 60
<210> 310
   <211> 60
   <212> PRT
   <213> Artificial Sequence
<220>
   <223> Synthetically generated peptide
<400> 310
   Gly Gly Trp Arg Pro Ile Lys Asp Leu Asn Ser Ala Glu Val Gln Asp
   1 5 10 15
   Val Ala Gln Phe Ala Val Ser Glu His Asn Lys Gln Ala Asn Asp Lys
   20 25 30
   Leu Gln Tyr Gln Arg Val Val Arg Gly Tyr Ser Gln Val Val Ala Gly
   35 40 45
   Thr Asn Tyr Arg Leu Val Ile Ala Ala Lys Asp Gly
   50 55 60
<210> 311
   <211> 43
   <212> PRT
   <213> Artificial Sequence
<220>
   <223> Synthetically generated peptide
<400> 311
   Pro Gly Trp Gln Ser Val Pro Ala His Asp Pro Gln Val Gln Asp Ala
   1 5 10 15
   Ala Asn His Ala Ile Lys Thr Ile Gln Gln Arg Ser Asn Ser Leu Val
   20 25 30
   Pro Tyr Glu Leu His Glu Val Thr Asp Ala Lys
   35 40
<210> 312
   <211> 43
   <212> PRT
   <213> Artificial Sequence
<220>
   <223> synthetically generated peptide
<400> 312
   Pro Gly Trp Gln Ser Val Pro Ala His Asp Pro Gln Val Gln Asp Ala
   1 5 10 15
   Ala Asn His Ala Ile Lys Thr Ile Gln Gln Arg Ser Asn Ser Leu Val
   20 25 30
   Pro Tyr Glu Leu His Glu Val Thr Asp Ala Lys
   35 40
<210> 313
   <211> 43
   <212> PRT
   <213> Artificial Sequence
<220>
   <223> Synthetically generated peptide
<400> 313
   Pro Gly Trp Gln Ser Val Pro Pro His Asp Pro Gln Val Gln Asp Ala
   1 5 10 15
   Ala Asn His Ala Val Lys Ser Leu Gln Gln Arg Ser Asn Ser Leu Leu
   20 25 30
   Pro Tyr Glu Leu Gln Glu Val Val His Ala Gln
   35 40
<210> 314
   <211> 46
   <212> PRT
   <213> Artificial Sequence
<220>
   <223> Synthetically generated peptide
<400> 314
   Pro Gly Trp Gln Asp Val His Pro Gln Asp Pro Gln Val Gln Asp Ala
   1 5 10 15
   Ala Asn His Ala Val Lys Ser Leu Gln Gln Lys Ser Asn Ser Leu Phe
   20 25 30
   Pro Tyr Glu Leu Gln Glu Val Val His Ala Lys Ser Glu Val
   35 40 45
<210> 315
   <211> 47
   <212> PRT
   <213> Artificial sequence
<220>
   <223> Synthetically generated peptide
<400> 315
   Pro Gly Trp Arg Asp Val Pro Val His Asp Pro Val Val Lys Asp Ala
   1 5 10 15
   Ala Ser His Ala Val Lys Ser Ile Gln Gln Arg Ser Asn Ser Leu Leu
   20 25 30
   Pro Tyr Glu Leu Val Glu Ile Val Arg Ala Lys Ala Glu Val Val
   35 40 45
<210> 316
   <211> 47
   <212> PRT
   <213> Artificial Sequence
<220>
   <223> Synthetically generated peptide
<400> 316
   Pro Gly Trp Arg Asp Val Pro Val His Asp Pro Val Val Lys Asp Ala
   1 5 10 15
   Ala Asp His Ala Val Lys Ser Ile Gln Gln Arg Ser Asn Ser Leu Phe
   20 25 30
   Pro Tyr Glu Leu Leu Glu Ile Val Arg Ala Lys Ala Glu Val Val
   35 40 45
<210> 317
   <211> 47
   <212> PRT
   <213> Artificial Sequence
<220>
   <223> Synthetically generated peptide
<400> 317
   Pro Gly Trp Arg Glu Val Pro Val Glu Asp Pro Val Val Lys Asp Ala
   1 5 10 15
   Ala His His Ala Val Lys Ser Ile Gln Glu Arg Ser Asn Ser Leu Phe
   20 25 30
   Pro Tyr Glu Leu Leu Glu Ile Leu Arg Ala His Ala Gln Val Val
   35 40 45
<210> 318
   <211> 47
   <212> PRT
   <213> Artificial Sequence
<220>
   <223> Synthetically generated peptide
<400> 318
   Pro Gly Trp Arg Glu Val Pro Val Glu Asp Pro Val Val Lys Asp Ala
   1 5 10 15
   Ala His His Ala Val Lys Ser Ile Gln Glu Arg Ser Asn Ser Leu Phe
   20 25 30
   Pro Tyr Glu Leu Leu Glu Ile Leu Arg Ala His Ala Gln Val Val
   35 40 45
<210> 319
   <211> 89
   <212> PRT
   <213> Artificial Sequence
<220>
   <223> Synthetically generated peptide
<400> 319
   Ser Gly Trp Arg Glu Val Pro Gly Asp Asp Pro Glu Val Lys His Val
    1               5                  10                  15
   Ala Glu Gln Ala Val Lys Thr Ile Gln Gln Arg Ser Asn Ser Leu Phe
               20                  25                  30
   Pro Tyr Glu Leu Leu Glu Val Val His Ala Lys Ala Glu Val Thr Gly
           35                  40                  45
   Glu Ala Ala Lys Tyr Asn Met Leu Leu Lys Leu Lys Arg Gly Glu Lys
       50                  55                  60
   Glu Glu Lys Phe Lys Val Glu Val His Lys Asn His Glu Gly Ala Leu
   65                  70                  75                  80
   His Val Asn His Ala Glu Gln His His
                   85
<210> 320
   <211> 71
   <212> PRT
   <213> Artificial sequence
<220>
   <223> Synthetically generated peptide
<400> 320
   Ser Gly Trp Arg Glu Val Pro Gly Asp Asp Pro Glu Val Lys His Val
    1               5                  10                  15
   Ala Glu Gln Ala Val Lys Thr Ile Gln Gln Arg Ser Asn Ser Leu Phe
               20                  25                  30
   Pro Tyr Glu Leu Leu Glu Val Val His Ala Lys Ala Glu Val Thr Gly
           35                  40                  45
   Glu Ala Ala Lys Tyr Asn Met Leu Leu Lys Leu Lys Arg Gly Glu Lys
       50                  55                  60
   Glu Glu Lys Phe Lys Val Glu
   65                  70
<210> 321
   <211> 71
   <212> PRT
   <213> Artificial sequence
<220>
   <223> Synthetically generated peptide
<400> 321
   Ser Gly Trp Arg Glu Val Pro Gly Asp Asp Pro Glu Val Lys His Val
   1 5 10 15
   Ala Glu Gln Ala Val Lys Thr Ile Gln Gln Arg Ser Asn Ser Leu Phe
   20 25 30
   Pro Tyr Glu Leu Leu Glu Val Val His Ala Lys Ala Glu Val Thr Gly
   35 40 45
   Glu Ala Ala Lys Tyr Asn Met Leu Leu Lys Leu Lys Arg Gly Glu Lys
   50 55 60
   Glu Glu Lys Phe Lys Val Glu
   65 70
<210> 322
   <211> 71
   <212> PRT
   <213> Artificial Sequence
<220>
   <223> Synthetically generated peptide
<400> 322
   Ser Gly Trp Arg Glu Val Pro Gly Asp Asp Pro Glu Val Lys His Ala
   1 5 10 15
   Ala Glu Gln Ala Val Lys Thr Ile Gln Gln Arg Ser Asn Ser Leu Phe
   20 25 30
   Pro Tyr Glu Leu Leu Glu Val Val His Ala Lys Ala Glu Val Thr Gly
   35 40 45
   Glu Ala Ala Lys Tyr Asn Met Leu Leu Lys Leu Lys Arg Gly Glu Lys
   50 55 60
   Glu Glu Lys Phe Lys Val Glu
   65 70
<210> 323
   <211> 89
   <212> PRT
   <213> Artificial sequence
<220>
   <223> synthetically generated peptide
<400> 323
   Ser Gly Trp Arg Glu Val Pro Gly Asp Asp Pro Glu Val Gln His Val
   1 5 10 15
   Ala Asp His Ala Val Lys Ser Ile Gln Gln Arg Ser Asn Ser Leu Phe
   20 25 30
   Pro Tyr Glu Leu Gln Glu Val Val His Ala Asn Ala Glu Val Thr Gly
   35 40 45
   Glu Ala Ala Lys Tyr Asn Met Val Leu Lys Leu Lys Arg Gly Glu Lys
   50 55 60
   Glu Glu Lys Phe Lys Val Glu Val His Lys Asn His Glu Gly Val Leu
   65 70 75 80
   His Leu Asn His Met Glu Gln Gln His
   85
<210> 324
   <211> 89
   <212> PRT
   <213> Artificial sequence
<220>
   <223> Synthetically generated peptide
<400> 324
   Ser Gly Trp Arg Glu Val Pro Gly Asp Asp Pro Glu Val Gln His Val
   1               5                   10                  15
   Ala Asp His Ala Val Lys Thr Ile Gln Gln Arg Ser Asn Ser Leu Phe
               20                  25                  30
   Pro Tyr Glu Leu Gln Glu Val Val His Ala Asn Ala Glu Val Thr Gly
           35                  40                  45
   Glu Ala Ala Lys Tyr Asn Met Val Leu Lys Leu Lys Arg Gly Glu Lys
       50                  55                  60
   Glu Glu Lys Phe Lys Val Glu Val His Lys Asn His Glu Gly Val Leu
   65                  70                  75                  80
   His Leu Asn His Met Glu Gln Gln His
                   85
<210> 325
   <211> 89
   <212> PRT
   <213> Artificial sequence
<220>
   <223> Synthetically generated peptide
<400> 325
   Ser Gly Trp Arg Glu Val Pro Gly Asp Asp Pro Glu Val Gln His Val
   1               5                   10                  15
   Ala Asp His Ala Val Lys Thr Ile Gln Gln Arg Ser Asn Ser Leu Phe
               20                  25                  30
   Pro Tyr Glu Leu Gln Glu Val Val His Ala Asn Ala Glu Val Thr Gly
           35                  40                  45
   Glu Ala Ala Lys Tyr Asn Met Val Leu Lys Leu Lys Arg Gly Glu Lys
       50                  55                  60
   Glu Glu Lys Phe Lys Val Glu Val His Lys Asn His Glu Gly Val Leu
   65                  70                  75                  80
   His Leu Asn His Met Glu Gln His His
                   85
<210> 326
   <211> 89
   <212> PRT
   <213> Artificial Sequence
<220>
   <223> Synthetically generated peptide
<400> 326
   Ser Gly Trp Arg Glu Val Pro Gly Asp Asp Pro Glu Val Gln His Val
   1               5                   10                  15
   Ala Glu His Ala Val Lys Thr Ile Gln Gln Arg Ser Asn Ser Leu Phe
               20                  25                  30
   Pro Tyr Glu Leu Gln Glu Val Val His Ala Asn Ala Glu Val Thr Gly
           35                  40                  45
   Glu Thr Ala Lys Phe Asn Met Leu Leu Lys Leu Lys Arg Gly Glu Lys
       50                  55                  60
   Glu Glu Lys Phe Lys Val Glu Val His Lys Asn His Glu Gly Ala Leu
   65                  70                  75                      80
   His Leu Asn His Met Glu Gln His His
   85
<210> 327
   <211> 47
   <212> PRT
   <213> Artificial Sequence
<220>
   <223> Synthetically generated peptide
<400> 327
   Ser Gly Trp Arg Pro Val Pro Val His Asp Pro Val Val Gln Asp Ala
   1               5                   10                  15
   Ala His His Ala Ile Lys Thr Ile Gln Glu Arg Ser Asn Ser Leu Phe
               20                  25                  30
   Pro Tyr Glu Leu Ser Glu Val Val His Ala Asn Ala Glu Val Val
           35                  40                  45
<210> 328
   <211> 47
   <212> PRT
   <213> Artificial Sequence
<220>
   <223> Synthetically generated peptide
<400> 328
   Ala Glu Trp Lys Glu Val Ala Ala His Asp Pro Val Val Gln Asp Ala
   1               5                   10                  15
   Ala Thr His Ala Val Asn Thr Ile Gln Gln Arg Ser Asn Ser Leu Phe
               20                  25                  30
   Pro Tyr Gln Leu Gln Glu Ile Val His Ala Lys Ala Gln Val Val
           35                  40                  45
<210> 329
   <211> 63
   <212> PRT
   <213> Artificial Sequence
<220>
   <223> Synthetically generated peptide
<400> 329
   Phe Asp Trp Arg Ser Val Ser Thr Asn Asn Pro Glu Val Gln Glu Ala
   1 5 10 15
   Ala Lys His Ala Met Lys Ser Leu Gln Gln Lys Ser Asn Ser Leu Phe
   20 25 30
   Pro Tyr Lys Leu Ile Asp Ile Ile Leu Ala Arg Ala Lys Val Val Glu
   35 40 45
   Glu Arg Val Lys Phe Glu Leu Leu Leu Lys Leu Glu Arg Gly Asn
   50 55 60
<210> 330
   <211> 63
   <212> PRT
   <213> Artificial Sequence
<220>
   <223> Synthetically generated peptide
<400> 330
   Phe Asp Trp Arg Ser Val Ser Thr Asn Asn Pro Glu Val Gln Glu Ala
   1 5 10 15
   Ala Lys His Ala Met Lys Ser Leu Gln Gln Lys Ser Asn Ser Leu Phe
   20 25 30
   Pro Tyr Lys Leu Ile Asp Ile Ile Leu Ala Arg Ala Lys Val Val Glu
   35 40 45
   Glu Arg Val Lys Phe Glu Leu Leu Leu Lys Leu Glu Arg Gly Asn
   50 55 60
<210> 331
   <211> 78
   <212> PRT
   <213> Artificial Sequence
<220>
   <223> Synthetically generated peptide
<400> 331
   Leu Tyr Val Gln Val Ile Gln Ala Ile Asn Asn Ser Val Val Asn Pro
   1 5 10 15
   Ser Ala Arg Ile Cys Cys Pro Val Val Glu Ile Thr Leu Gly Asn Tyr
   20 25 30
   Lys Ser Ser Thr Lys Asn Leu Pro Met Gly Pro Asn Met Asp Trp Asn
   35 40 45
   Gln Val Phe Ala Phe Asp Lys Ser Lys Gly Asp Val Leu Ser Val Thr
   50 55 60
   Leu Lys Asp Gly Pro Thr Asn Thr Val Ile Asn Lys Arg Asn
   65 70 75
<210> 332
   <211> 66
   <212> PRT
   <213> Artificial Sequence
<220>
   <223> Synthetically generated peptide
<400> 332
   Leu Tyr Val Asp Val Ile Arg Ala Ile Lys Asn Ser Asp Val Asp Pro
   1               5                   10                  15
   Gly Pro Cys Asp Pro Val Val Glu Ile Thr Leu Gly Asn Tyr Lys Ser
               20                  25                  30
   Ser Thr Lys Asp Leu Pro Val Gly Pro Asn Met Asp Trp Asn Gln Val
           35                  40                  45
   Phe Ala Phe Asp Lys Thr Lys Gly Asp Val Leu Ser Val Thr Leu Lys
       50                  55                  60
   Asp Arg
   65
<210> 333
   <211> 67
   <212> PRT
   <213> Artificial Sequence
<220>
   <223> synthetically generated peptide
<400> 333
   Leu Tyr Val Arg Val Val Arg Arg Val Ala Ala Val Gly Glu Thr Val
   1               5                   10                  15
   Ala Glu Val Lys Leu Gly Asn Tyr Arg Gly Val Thr Pro Ala Thr Ala
               20                  25                  30
   Ala His His Trp Asp Gln Val Phe Ala Phe Ser Lys Glu Thr Ile Gln
           35                  40                  45
   Ser Ser Phe Val Glu Val Phe Val Arg Arg Ser Asp Asp His Val Gly
       50                  55                  60
   Arg Val Trp
   65
<210> 334
   <211> 75
   <212> PRT
   <213> Artificial sequence
<220>
   <223> Synthetically generated peptide
<400> 334
   Leu Tyr Val Asn Ile Val Lys Ala Lys Asp Leu Ser Val Leu Gly Glu
   1               5                   10                  15
   Val Val Ser Glu Val Lys Leu Gly Asn Tyr Arg Gly Val Thr Lys Lys
               20                  25                  30
   Val Ser Ser Asn Ser Ser Asn Pro Glu Trp Asn Gln Val Phe Val Phe
           35                  40                  45
   Ser Lys Glu Arg Ile Gln Ser Ser Val Val Glu Leu Phe Val Lys Glu
       50                  55                  60
   Gly Asn Lys Asp Glu Tyr Thr Gly Arg Val Leu
   65                  70                  75
<210> 335
   <211> 74
   <212> PRT
   <213> Artificial Sequence
<220>
   <223> Synthetically generated peptide
<400> 335
   Leu Tyr Val Arg Val Val Lys Arg Leu Lys Trp Ser Gly Glu Phe Asp
   1               5                   10                  15
   Pro Phe Ala Glu Leu Arg Leu Gly Gly Tyr Ser Cys Ile Thr Arg His
               20                  25                  30
   Val Glu Lys Thr Asp Glu Trp Asp Asp Val Phe Ala Phe Ser Arg Glu
           35                  40                  45
   Arg Ile His Ala Pro Phe Leu Asp Val Leu Val Arg Gly Arg Gly Phe
       50                  55                  60
   Ala Lys Asp Asp Tyr Val Gly Ser Thr Arg
   65                  70
<210> 336
   <211> 87
   <212> PRT
   <213> Artificial Sequence
<220>
   <223> Synthetically generated peptide
<400> 336
   Leu Tyr Val Arg Val Val Arg Arg Leu Thr Ala Ala Ala Ser Thr Val
   1               5                   10                  15
   Ala Gly Gly Gly Gly Cys Asn Pro Tyr Val Glu Val Arg Leu Gly Asn
               20                  25                  30
   Tyr Arg Gly Thr Thr Arg His His Glu Arg Lys Ala Ala Pro Glu Trp
           35                  40                  45
   Asn Gln Val Phe Ala Phe Ser Arg Glu Arg Val Gln Ala Ser Val Leu
       50                  55                  60
   Glu Val Phe Val Arg Asp Lys Asp Ala Val Ala Ala Val Ala Arg Asp
   65                  70                  75                  80
   Gly Tyr Val Gly Arg Val Ala
                   85
<210> 337
   <211> 81
   <212> PRT
   <213> Artificial sequence
<220>
   <223> Synthetically generated peptide
<400> 337
   Leu Tyr Val Arg Val Val Lys Ala Arg Glu Leu Pro Ile Met Asp Ile
   1 5 10 15
   Thr Gly Ser Val Asp Pro Phe Val Glu Val Arg Val Gly Asn Tyr Lys
   20 25 30
   Gly Ile Thr Arg His Phe Glu Lys Arg Gln His Pro Glu Trp Asn Gln
   35 40 45
   Val Phe Ala Phe Ala Lys Glu Arg Met Gln Ala Ser Val Leu Glu Val
   50 55 60
   Val Val Lys Asp Lys Asp Leu Leu Lys Asp Asp Tyr Val Gly Phe Val
   65 70 75 80
   Arg
<210> 338
   <211> 79
   <212> PRT
   <213> Artificial Sequence
<220>
   <223> Synthetically generated peptide
<400> 338
   Leu Phe Val Arg Val Val Lys Ala Arg Asp Leu Pro Asp Met Asp Val
   1 5 10 15
   Thr Gly Ser Leu Asp Pro Tyr Val Glu Val Arg Val Gly Asn Tyr Arg
   20 25 30
   Gly Ile Thr Arg His Phe Glu Lys Gln Lys Asn Pro Glu Trp Asn Ala
   35 40 45
   Val Phe Ala Phe Ser Arg Asp Arg Met Gln Ala Thr Ile Val Val Val
   50 55 60
   Lys Asp Lys Asp Leu Leu Lys Asp Asp Phe Val Gly Leu Val Arg
   65 70 75
<210> 339
   <211> 79
   <212> PRT
   <213> Artificial Sequence
<220>
   <223> Synthetically generated peptide
<400> 339
   Leu Phe Val Arg Val Val Lys Ala Arg Asp Leu Pro Asp Met Asp Val
   1 5 10 15
   Thr Gly Ser Leu Asp Pro Tyr Val Glu Val Arg Val Gly Asn Tyr Arg
   20 25 30
   Gly Ile Thr Arg His Phe Glu Lys Gln Lys Asn Pro Glu Trp Asn Ala
   35 40 45
   Val Phe Ala Phe Ser Arg Asp Arg Met Gln Ala Thr Ile Val Val Val
   50 55 60
   Lys Asp Lys Asp Leu Leu Lys Asp Asp Phe Val Gly Leu Val Arg
   65 70 75
<210> 340
   <211> 81
   <212> PRT
   <213> Artificial Sequence
<220>
   <223> synthetically generated peptide
<400> 340
   Leu Phe Val Arg Val Val Lys Ala Arg Asp Leu Pro His Met Asp Ile
    1               5                  10                  15
   Thr Gly Ser Leu Asp Pro Tyr Val Glu Val His Leu Gly Asn Tyr Lys
               20                  25                  30
   Met Lys Thr Arg His Phe Glu Lys Asn Gln Arg Pro Glu Trp Asp Glu
           35                  40                  45
   Val Phe Ala Phe Pro Arg Glu Val Met Gln Ser Thr Ser Leu Glu Val
       50                  55                  60
   Ile Val Lys Asp Lys Asp Phe Ile Arg Asp Asp Tyr Val Gly Arg Val
   65                  70                  75                  80
   Ser
<210> 341
   <211> 81
   <212> PRT
   <213> Artificial Sequence
<220>
   <223> synthetically generated peptide
<400> 341
   Leu Tyr Val Arg Val Val Lys Ala Arg Asp Leu Pro Asn Lys Asp Leu
    1               5                  10                  15
   Thr Gly Ser Leu Asp Pro Tyr Val Val Val Lys Ile Gly Asn Phe Lys
               20                  25                  30
   Gly Val Thr Thr His Phe Asn Lys Asn Thr Asp Pro Glu Trp Asn Gln
           35                  40                  45
   Val Phe Ala Phe Ala Lys Asp Asn Leu Gln Ser Asn Phe Leu Glu Val
       50                  55                  60
   Met Val Lys Asp Lys Asp Ile Leu Leu Asp Asp Phe Val Gly Ile Val
   65                  70                  75                  80
   Lys
<210> 342
   <211> 81
   <212> PRT
   <213> Artificial Sequence
<220>
   <223> synthetically generated peptide
<400> 342
   Leu Tyr Val Arg Val Val Lys Ala Arg Asp Leu Pro Asn Lys Asp Leu
   1 5 10 15
   Thr Gly Ser Leu Asp Pro Tyr Val Val Val Lys Ile Gly Asn Phe Lys
   20 25 30
   Gly Val Thr Thr His Phe Asn Lys Asn Thr Asp Pro Glu Trp Asn Gln
   35 40 45
   Val Phe Ala Phe Ala Lys Asp Asn Leu Gln Ser Asn Phe Leu Glu Val
   50 55 60
   Met Val Lys Asp Lys Asp Ile Leu Leu Asp Asp Phe Val Gly Ile Val
   65 70 75 80
   Lys
<210> 343
   <211> 81
   <212> PRT
   <213> Artificial Sequence
<220>
   <223> synthetically generated peptide
<400> 343
   Leu Phe Ile Arg Val Val Lys Ala Arg Asp Leu Pro Arg Met Asp Leu
   1 5 10 15
   Thr Gly Ser Leu Asp Pro Tyr Val Ile Val Lys Val Gly Asn Phe Lys
   20 25 30
   Gly Thr Thr Asn His Phe Glu Lys Asn Asn Ser Pro Glu Trp Asn Leu
   35 40 45
   Val Phe Ala Phe Ala Lys Glu Asn Gln Gln Ala Thr Thr Leu Glu Val
   50 55 60
   Val Ile Lys Asp Lys Asp Thr Ile His Asp Asp Phe Val Gly Thr Val
   65 70 75 80
   Arg
<210> 344
   <211> 82
   <212> PRT
   <213> Artificial Sequence
<220>
   <223> Synthetically generated peptide
<400> 344
   Leu Tyr Val Ser Val Val Lys Ala Arg Asp Leu Pro Val Met Asp Val
   1 5 10 15
   Ser Gly Ser Leu Asp Pro Tyr Val Glu Val Lys Leu Gly Asn Tyr Lys
   20 25 30
   Gly Leu Thr Lys His Leu Glu Lys Asn Ser Asn Pro Ile Trp Lys Gln
   35 40 45
   Ile Phe Ala Phe Ser Lys Glu Arg Leu Gln Ser Asn Leu Leu Glu Val
   50 55 60
   Thr Val Lys Asp Lys Asp Leu Leu Thr Lys Asp Asp Phe Val Gly Arg
   65 70 75 80
   Val His
<210> 345
   <211> 82
   <212> PRT
   <213> Artificial Sequence
<220>
   <223> Synthetically generated peptide
<400> 345
   Leu Tyr Val Ser Val Val Lys Ala Arg Asp Leu Pro Val Met Asp Val
    1               5                  10                  15
   Ser Gly Ser Leu Asp Pro Tyr Val Glu Val Lys Leu Gly Asn Tyr Lys
               20                  25                  30
   Gly Leu Thr Lys His Leu Glu Lys Asn Ser Asn Pro Ile Trp Lys Gln
           35                  40                  45
   Ile Phe Ala Phe Ser Lys Glu Arg Leu Gln Ser Asn Leu Leu Glu Val
       50                  55                  60
   Thr Val Lys Asp Lys Asp Leu Leu Thr Lys Asp Asp Phe Val Gly Arg
   65                  70                  75                  80
   Val His
<210> 346
   <211> 81
   <212> PRT
   <213> Artificial sequence
<220>
   <223> Synthetically generated peptide
<400> 346
   Leu Tyr Val Ser Val Val Lys Ala Arg Asp Leu Pro Asn Met Asp Ile
    1               5                  10                  15
   Thr Gly Ala Leu Asp Pro Tyr Val Glu Val Arg Leu Gly Asn Phe Lys
               20                  25                  30
   Gly Val Thr Arg His Leu Glu Lys Asn Pro Asn Pro Val Trp Arg Gln
           35                  40                  45
   Val Phe Ala Phe Ser Arg Asp His Leu Gln Ser Ser Gln Leu Glu Val
       50                  55                  60
   Val Val Lys Asp Lys Asp Val Leu Lys Asp Asp Phe Val Gly Arg Val
   65                  70                  75                  80
   Val
<210> 347
   <211> 81
   <212> PRT
   <213> Artificial sequence
<220>
   <223> Synthetically generated peptide
<400> 347
   Leu Phe Ile Lys Ile Val Lys Ala Arg Asn Leu Pro Ser Met Asp Leu
   1 5 10 15
   Thr Gly Ser Leu Asp Pro Tyr Ile Glu Val Lys Leu Gly Asn Tyr Thr
   20 25 30
   Gly Lys Thr Lys His Phe Glu Lys Asn Gln Asn Pro Val Trp Asn Glu
   35 40 45
   Val Phe Ala Phe Ser Lys Ser Asn Gln Gln Ser Asn Val Leu Glu Val
   50 55 60
   Ile Val Met Asp Lys Asp Met Val Lys Asp Asp Phe Val Gly Leu Ile
   65 70 75 80
   Arg
<210> 348
   <211> 82
   <212> PRT
   <213> Artificial Sequence
<220>
   <223> synthetically generated peptide
<400> 348
   Leu Tyr Val Arg Val Val Lys Ala Lys Asp Leu Pro Pro Gly Thr Ile
   1 5 10 15
   Thr Ser Ser Cys Asp Pro Tyr Val Glu Val Lys Leu Gly Asn Tyr Arg
   20 25 30
   Gly Arg Thr Lys His Leu Glu Lys Lys Leu Asn Pro Glu Trp Asn Gln
   35 40 45
   Val Phe Ala Phe Ser Lys Asp Arg Ile Gln Ser Ser Val Leu Glu Val
   50 55 60
   Phe Val Lys Asp Lys Glu Met Val Gly Arg Asp Asp Tyr Leu Gly Arg
   65 70 75 80
   Val Ile
<210> 349
   <211> 80
   <212> PRT
   <213> Artificial Sequence
<220>
   <223> Synthetically generated peptide
<400> 349
   Leu Tyr Val Arg Val Val Lys Ala Lys Asn Leu Thr Leu Asn Ser Leu
   1 5 10 15
   Thr Ser Thr Cys Asp Pro Tyr Val Glu Val Arg Leu Gly Asn Tyr Lys
   20 25 30
   Gly Arg Thr Lys His Leu Asp Lys Arg Ser Asn Pro Glu Trp Asn Gln
   35 40 45
   Val Tyr Ala Phe Ser Lys Asp Gln Ile Gln Ser Ser Ile Val Ile Val
   50 55 60
   Lys Asp Lys Glu Thr Val Gly Arg Asp Asp Tyr Ile Gly Arg Val Ala
   65 70 75 80
<210> 350
   <211> 82
   <212> PRT
   <213> Artificial Sequence
<220>
   <223> Synthetically generated peptide
<400> 350
   Leu Tyr Val Arg Val Val Lys Ala Lys Asp Leu Pro Pro Asn Pro Val
   1               5                   10                  15
   Thr Ser Asn Cys Asp Pro Tyr Val Glu Val Lys Ile Gly Asn Tyr Lys
               20                  25                  30
   Gly Lys Thr Lys His Phe Glu Lys Arg Thr Asn Pro Glu Trp Asn Gln
           35                  40                  45
   Val Phe Ala Phe Ser Lys Asp Lys Val Gln Ser Ser Thr Val Glu Val
       50                  55                  60
   Phe Val Arg Asp Lys Glu Met Val Thr Arg Asp Glu Tyr Ile Gly Lys
   65                  70                  75                  80
   Val Val
<210> 351
   <211> 83
   <212> PRT
   <213> Artificial sequence
<220>
   <223> Synthetically generated peptide
<400> 351
   Leu Tyr Val Arg Val Val Lys Ala Lys Glu Leu Pro Pro Gly Ser Ile
   1               5                   10                  15
   Thr Gly Gly Cys Asp Pro Tyr Val Glu Val Lys Leu Gly Asn Tyr Lys
               20                  25                  30
   Gly Arg Thr Lys Ile Phe Asp Arg Lys Thr Thr Ile Pro Glu Trp Asn
           35                  40                  45
   Gln Val Phe Ala Phe Thr Lys Glu Arg Ile Gln Ser Ser Val Leu Glu
       50                  55                  60
   Val Phe Val Lys Asp Lys Glu Thr Leu Gly Arg Asp Asp Ile Leu Gly
   65                  70                  75                  80
   Lys Val Val
<210> 352
   <211> 81
   <212> PRT
   <213> Artificial Sequence
<220>
   <223> Synthetically generated peptide
<400> 352
   Leu Tyr Val Arg Val Val Lys Ala Lys Glu Leu Pro Gly Lys Asp Met
   1 5 10 15
   Thr Gly Ser Cys Asp Pro Tyr Val Glu Val Lys Leu Gly Asn Tyr Lys
   20 25 30
   Gly Thr Thr Arg His Phe Glu Lys Lys Ser Asn Pro Glu Trp Asn Gln
   35 40 45
   Val Phe Ala Phe Ser Lys Asp Arg Ile Gln Ala Ser Phe Leu Glu Ala
   50 55 60
   Thr Val Lys Asp Lys Asp Phe Val Lys Asp Asp Leu Ile Gly Arg Val
   65 70 75 80
   Val
<210> 353
   <211> 81
   <212> PRT
   <213> Artificial Sequence
<220>
   <223> Synthetically generated peptide
<400> 353
   Leu Tyr Val Arg Val Val Lys Ala Lys Glu Leu Pro Gly Lys Asp Met
   1 5 10 15
   Thr Gly Ser Cys Asp Pro Tyr Val Glu Val Lys Leu Gly Asn Tyr Lys
   20 25 30
   Gly Thr Thr Arg His Phe Glu Lys Lys Ser Asn Pro Glu Trp Asn Gln
   35 40 45
   Val Phe Ala Phe Ser Lys Asp Arg Ile Gln Ala Ser Phe Leu Glu Ala
   50 55 60
   Thr Val Lys Asp Lys Asp Phe Val Lys Asp Asp Leu Ile Gly Arg Val
   65 70 75 80
   Val
<210> 354
   <211> 81
   <212> PRT
   <213> Artificial sequence
<220>
   <223> Synthetically generated peptide
<400> 354
   Leu Tyr Val Arg Val Val Lys Ala Lys Glu Leu Pro Gly Lys Asp Leu
   1 5 10 15
   Thr Gly Ser Cys Asp Pro Tyr Val Glu Val Lys Leu Gly Asn Tyr Arg
   20 25 30
   Gly Thr Thr Arg His Phe Glu Lys Lys Ser Asn Pro Glu Trp Asn Gln
   35 40 45
   Val Phe Ala Phe Ser Lys Asp Arg Val Gln Ala Ser Tyr Leu Glu Ala
   50 55 60
   Thr Val Lys Asp Lys Asp Leu Val Lys Asp Asp Leu Ile Gly Arg Val
   65 70 75 80
   Val
<210> 355
   <211> 79
   <212> PRT
   <213> Artificial sequence
<220>
   <223> Synthetically generated peptide
<400> 355
   Leu Tyr Val Arg Val Val Lys Ala Lys Glu Leu Pro Gly Lys Asp Val
    1               5                  10                  15
   Thr Gly Ser Cys Asp Pro Tyr Val Glu Val Lys Leu Gly Asn Tyr Arg
               20                  25                  30
   Gly Met Thr Lys His Phe Glu Lys Arg Ser Asn Pro Glu Trp Lys Gln
           35                  40                  45
   Val Phe Ala Phe Ser Lys Glu Arg Ile Gln Ala Ser Ile Val Val Val
       50                  55                  60
   Lys Asp Lys Asp Val Val Leu Asp Asp Leu Ile Gly Arg Ile Met
   65                  70                  75
<210> 356
   <211> 81
   <212> PRT
   <213> Artificial sequence
<220>
   <223> Synthetically generated peptide
<400> 356
   Leu Tyr Val Arg Val Val Lys Ala Lys Asp Leu Pro Ser Lys Asp Ile
    1               5                  10                  15
   Thr Gly Ser Cys Asp Pro Tyr Val Glu Val Lys Leu Gly Asn Tyr Lys
               20                  25                  30
   Gly Thr Thr Arg His Phe Glu Lys Lys Thr Asn Pro Glu Trp Asn Gln
           35                  40                  45
   Val Phe Ala Phe Ser Lys Glu Arg Ile Gln Ser Ser Val Val Glu Ile
       50                  55                  60
   Ile Val Lys Asp Lys Asp Phe Val Lys Asp Asp Phe Ile Gly Arg Val
   65                  70                  75                  80
   Leu
<210> 357
   <211> 82
   <212> PRT
   <213> Artificial Sequence
<220>
   <223> Synthetically generated peptide
<400> 357
   Leu Tyr Val His Val Val Lys Ala Lys Asp Leu Pro Ala Val Ser Ala
    1               5                  10                  15
   Ala Gly Thr Ile Asp Pro Phe Val Glu Val Lys Leu Gly Asn Phe Lys
               20                  25                  30
   Gly Thr Thr Pro Val Leu Gly Gly Asn His Asn Pro Ser Trp Lys Gln
           35                  40                  45
   Val Phe Ala Phe Ser Ala Thr His Leu Gln Ala His Val Leu Glu Val
       50                  55                  60
   Ala Val Lys Ala Lys Asp Leu Ala Gly Gly Asp Asp Leu Ile Gly Arg
   65                  70                  75                  80
   Val Gly
<210> 358
   <211> 73
   <212> PRT
   <213> Artificial Sequence
<220>
   <223> Synthetically generated peptide
<400> 358
   Leu Tyr Ile Arg Ile Val Lys Ala Arg Ala Leu Pro Ser Asn Asp Leu
    1               5                  10                  15
   Phe Val Glu Val Thr Ile Gly Arg Tyr Lys Gly Arg Thr Lys Arg Ser
               20                  25                  30
   Thr Asn Pro Tyr Pro Asn Leu Glu Phe Asp Glu Val Phe Ala Phe Asn
           35                  40                  45
   Ser Asp Arg Leu Gln Gly Asn Met Leu Glu Val Thr Met Lys Met Asn
       50                  55                  60
   Glu Glu Glu Ile Ile Gly Gln Cys Arg
   65                  70
<210> 359
   <211> 73
   <212> PRT
   <213> Artificial Sequence
<220>
   <223> Synthetically generated peptide
<400> 359
   Leu Tyr Ile Arg Ile Val Lys Ala Arg Ala Leu Pro Ser Asn Asp Leu
    1               5                  10                  15
   Phe Val Glu Val Thr Ile Gly Arg Tyr Lys Gly Arg Thr Lys Arg Ser
               20                  25                  30
   Thr Asn Pro Tyr Pro Asn Leu Glu Phe Asp Glu Val Phe Ala Phe Asn
           35                  40                  45
   Ser Asp Arg Leu Gln Gly Asn Met Leu Glu Val Thr Met Lys Met Asn
       50                  55                  60
   Glu Glu Glu Ile Ile Gly Gln Cys Arg
   65                  70
<210> 360
   <211> 75
   <212> PRT
   <213> Artificial Sequence
<220>
   <223> Synthetically generated peptide
<400> 360
   Leu Tyr Ala Arg Ile Val Arg Ala Arg Ala Leu Pro Val Asn Asp Ser
   1               5                   10                  15
   Phe Val Ala Val Lys Ile Gly Ser Tyr Lys Gly Arg Thr Lys Gln Ile
               20                  25                  30
   Leu Asn Ser Asn Pro Asn Pro Glu Phe His Glu Thr Phe Ala Phe Thr
           35                  40                  45
   Lys Thr Arg Leu Gln Gly Asp Ile Val Val Val Arg Asn Arg Asp Asn
       50                  55                  60
   Pro Asn Glu Asp Asp Ile Val Gly Lys Cys Lys
   65                  70                  75
<210> 361
   <211> 84
   <212> PRT
   <213> Artificial Sequence
<220>
   <223> Synthetically generated peptide
<400> 361
   Leu Phe Val Arg Val Ile Lys Ala Arg Lys Leu Pro Asp Met Asp Ala
   1               5                   10                  15
   Asn Gly Ser Leu Asp Pro Tyr Val Glu Val Lys Phe Gly Ala Tyr Asn
               20                  25                  30
   Arg Gly Val Thr Arg Cys Phe Lys Arg Asn Lys Asn Pro Glu Trp Asn
           35                  40                  45
   Glu Thr Phe Ala Phe Ser Phe Gln His Asp Lys Ile Pro Ser Pro Thr
       50                  55                  60
   Val Asp Ile Val Val Asn Asp Lys Asp Leu Val Arg Asp Asp Phe Val
   65                  70                  75                  80
   Gly Lys Leu His
<210> 362
   <211> 81
   <212> PRT
   <213> Artificial Sequence
<220>
   <223> Synthetically generated peptide
<400> 362
   Leu Phe Val Arg Ile Val Lys Arg Leu Pro Pro Asn Glu Ser Ala Tyr
   1               5                   10                  15
   Val Lys Val Arg Thr Ser Asn His Phe Val Arg Ser Lys Pro Ala Val
               20                  25                  30
   Asn Arg Pro Gly Glu Ser Val Asp Ser Pro Glu Trp Asn Gln Val Phe
           35                  40                  45
   Ala Leu Gly His Asn Arg Ser Asp Ser Ala Val Thr Gly Ala Thr Leu
       50                  55                  60
   Glu Ile Ser Ala Trp Asp Ala Ser Ser Glu Ser Phe Leu Gly Gly Val
   65                  70                  75                  80
   Cys
<210> 363
   <211> 81
   <212> PRT
   <213> Artificial Sequence
<220>
   <223> Synthetically generated peptide
<400> 363
   Leu Phe Val Arg Ile Val Lys Arg Leu Pro Pro Asn Glu Ser Ala Tyr
   1 5 10 15
   Val Lys Val Arg Thr Ser Asn His Phe Val Arg Ser Lys Pro Ala Val
   20 25 30
   Asn Arg Pro Gly Glu Ser Val Asp Ser Pro Glu Trp Asn Gln Val Phe
   35 40 45
   Ala Leu Gly His Asn Arg Ser Asp Ser Ala Val Thr Gly Ala Thr Leu
   50 55 60
   Glu Ile Ser Ala Trp Asp Ala Ser Ser Glu Ser Phe Leu Gly Gly Val
   65 70 75 80
   Cys
<210> 364
   <211> 80
   <212> PRT
   <213> Artificial Sequence
<220>
   <223> Synthetically generated peptide

<400> 364
   Leu Phe Val Arg Val Val Lys Val Arg Gly Ile Arg Ala Cys Glu Gly
   1 5 10 15
   Pro Tyr Val Lys Ile Gln Ala Gly Pro His Thr Leu Arg Ser Arg Pro
   20 25 30
   Gly Arg Asp Val Ser Gly Thr Gly Asn Pro Glu Trp Asn Gln Val Phe
   35 40 45
   Ala Ile Asn His Ala Lys Pro Glu Pro Thr Leu Glu Ile Ser Val Trp
   50 55 60
   Asp Gly Gly Ala Pro Ser Pro Ile Glu Ala Phe Leu Gly Gly Val Cys
   65 70 75 80
<210> 365
   <211> 85
   <212> PRT
   <213> Artificial Sequence
<220>
   <223> synthetically generated peptide
<400> 365
   Leu Phe Val Arg Val Val Arg Arg Leu Pro Ala Gly Ala His Pro His
   1 5 10 15
   Val Arg Val Ala Ala Gly Gly Arg His Ala Ser Thr Arg Glu Ala Arg
   20 25 30
   Arg Gly Ala Phe Phe Glu Trp Asp Gln Thr Phe Ala Phe Val Arg Asp
   35 40 45
   Pro Gly Ala Thr Asp Ser Pro Gly Pro Thr Leu Glu Val Ser Val Trp
   50 55 60
   Asp Leu Pro Pro Asp Ala Asp Val Ser Asp Ala Asp Asp Arg His Phe
   65 70 75 80
   Leu Gly Gly Leu Cys
   85
<210> 366
   <211> 79
   <212> PRT
   <213> Artificial Sequence
<220>
   <223> Synthetically generated peptide
<400> 366
   Val Phe Ile Arg Val Val Lys Ala Arg Ser Leu Pro Thr Ser Gly Ser
   1 5 10 15
   Pro Val Thr Lys Ile Ser Leu Ser Gly Thr Met Ile Gln Ser Lys Pro
   20 25 30
   Ala Arg Lys Thr Ser Cys Phe Glu Trp Asp Gln Thr Phe Ala Phe Leu
   35 40 45
   Arg Asp Ser Pro Asp Leu Ser Ser Ser Pro Ile Ile Ser Val Trp Asp
   50 55 60
   Ser Ser Thr Gly Ile Glu Thr Ser Gln Phe Leu Gly Gly Ile Cys
   65 70 75
<210> 367
   <211> 89
   <212> PRT
   <213> Artificial Sequence
<220>
   <223> Synthetically generated peptide
<400> 367
   Leu Glu Val His Leu Val Asp Ala Lys Gly Leu Thr Gly Asn Asp Phe
   1 5 10 15
   Leu Gly Glu Ile Gly Lys Ile Asp Pro Tyr Val Val Val Gln Tyr Arg
   20 25 30
   Ser Gln Glu Arg Lys Ser Ser Val Ala Arg Asp Gln Gly Lys Asn Pro
   35 40 45
   Ser Trp Asn Glu Val Phe Lys Phe Gln Ile Asn Ser Thr Ala Ala Thr
   50 55 60
   Gly Gln His Lys Leu Phe Leu Arg Leu Met Asp His Asp Thr Phe Ser
   65 70 75 80
   Arg Asp Asp Phe Leu Gly Glu Ala Thr
   85
<210> 368
   <211> 63
   <212> PRT
   <213> Artificial Sequence
<220>
   <223> Synthetically generated peptide
<400> 368
   Met Asp Pro Tyr Val Ile Leu Thr Cys Arg Thr Gln Glu Gln Lys Ser
   1               5                   10                  15
   Ser Val Ala Lys Gly Ala Gly Ser Glu Pro Glu Trp Asn Glu Thr Phe
               20                  25                  30
   Val Phe Thr Val Ser Asp Asp Val Pro Gln Leu Asn Val Lys Ile Met
           35                  40                  45
   Asp Ser Asp Ala Phe Ser Ala Asp Asp Phe Val Gly Glu Ala Asn
       50                  55                  60
<210> 369
   <211> 82
   <212> PRT
   <213> Artificial sequence
<220>
   <223> Synthetically generated peptide
<400> 369
   Leu Glu Val Leu Leu Val Cys Ala Lys Gly Leu Glu Asp Thr Asp Phe
   1               5                   10                  15
   Leu Asn Asp Met Asp Pro Tyr Val Ile Leu Thr Cys Arg Thr Gln Glu
               20                  25                  30
   Gln Lys Ser Ser Val Ala Lys Gly Ala Gly Ser Glu Pro Glu Trp Asn
           35                  40                  45
   Glu Thr Phe Val Phe Thr Val Ser Asp Asp Val Pro Gln Leu Asn Val
       50                  55                  60
   Lys Ile Met Asp Ser Asp Ala Phe Ser Ala Asp Asp Phe Val Gly Glu
   65                  70                  75                  80
   Ala Asn
<210> 370
   <211> 81
   <212> PRT
   <213> Artificial Sequence
<220>
   <223> Synthetically generated peptide
<400> 370
   Leu Glu Val Leu Leu Val Ser Ala Lys Gly Leu Glu Asp Thr Asp Phe
   1               5                   10                  15
   Leu Asn Asn Met Asp Pro Phe Val Ile Leu Thr Cys Arg Thr Gln Glu
                   20                  25                  30
   Gln Lys Ser Ser Val Ala Asn Gly Ala Gly Ser Glu Pro Glu Trp Asn
               35                  40                  45
   Glu Thr Phe Val Phe Thr Val Ser Asp Asp Thr Pro Gln Leu His Leu
       50                  55                  60
   Lys Ile Met Asp Ser Asp Leu Thr Asn Asp Asp Phe Val Gly Glu Arg
   65                  70                  75                  80
   Thr
<210> 371
   <211> 81
   <212> PRT
   <213> Artificial Sequence
<220>
   <223> Synthetically generated peptide
<400> 371
   Leu Glu Val Leu Leu Val Ser Ala Lys Gly Leu Glu Asp Thr Asp Phe
    1               5                  10                  15
   Leu Asn Asn Met Asp Pro Phe Val Ile Leu Thr Cys Arg Thr Gln Glu
                   20                  25                  30
   Gln Lys Ser Ser Val Ala Asn Gly Ala Gly Ser Glu Pro Glu Trp Asn
               35                  40                  45
   Glu Thr Phe Val Phe Thr Val Ser Asp Asp Thr Pro Gln Leu His Leu
       50                  55                  60
   Lys Ile Met Asp Ser Asp Leu Thr Asn Asp Asp Phe Val Gly Glu Ala
   65                  70                  75                  80
   Thr
<210> 372
   <211> 82
   <212> PRT
   <213> Artificial Sequence
<220>
   <223> Synthetically generated peptide
<400> 372
   Leu Glu Val Val Leu Val Ser Ala Lys Gly Leu Glu Asp Ala Asp Phe
    1               5                  10                  15
   Leu Asn Asn Met Asp Pro Tyr Val Gln Leu Thr Cys Arg Thr Gln Asp
                   20                  25                  30
   Gln Lys Ser Asn Val Ala Glu Gly Met Gly Thr Thr Pro Glu Trp Asn
               35                  40                  45
   Glu Thr Phe Ile Phe Thr Val Ser Glu Gly Thr Thr Glu Leu Lys Ala
       50                  55                  60
   Lys Ile Phe Asp Lys Asp Val Gly Thr Glu Asp Asp Ala Val Gly Glu
   65                  70                  75                  80
   Ala Thr
<210> 373
   <211> 82
   <212> PRT
   <213> Artificial Sequence
<220>
   <223> Synthetically generated peptide
<400> 373
   Leu Glu Val Leu Leu Val Gly Ala Lys Gly Leu Glu Asn Thr Asp Tyr
   1 5 10 15
   Leu Cys Asn Met Asp Pro Tyr Ala Val Leu Lys Cys Arg Ser Gln Glu
   20 25 30
   Gln Lys Ser Ser Val Ala Ser Gly Lys Gly Ser Asp Pro Glu Trp Asn
   35 40 45
   Glu Thr Phe Met Phe Ser Val Thr His Asn Ala Thr Glu Leu Ile Ile
   50 55 60
   Lys Leu Met Asp Ser Asp Ser Gly Thr Asp Asp Asp Phe Val Gly Glu
   65 70 75 80
   Ala Thr
<210> 374
   <211> 82
   <212> PRT
   <213> Artificial Sequence
<220>
   <223> synthetically generated peptide
<400> 374
   Leu Glu Val Leu Leu Val Gly Ala Lys Gly Leu Glu Asn Thr Asp Tyr
   1 5 10 15
   Leu Cys Asn Met Asp Pro Tyr Ala Val Leu Lys Cys Arg Ser Gln Glu
   20 25 30
   Gln Lys Ser Ser Val Ala Ser Gly Lys Gly Ser Asp Pro Glu Trp Asn
   35 40 45
   Glu Thr Phe Met Phe Ser Val Thr His Asn Ala Thr Glu Leu Ile Ile
   50 55 60
   Lys Leu Met Asp Ser Asp Ser Gly Thr Asp Asp Asp Phe Val Gly Glu
   65 70 75 80
   Ala Thr
<210> 375
   <211> 82
   <212> PRT
   <213> Artificial Sequence
<220>
   <223> synthetically generated peptide
<400> 375
   Leu Glu Val Leu Leu Val Gly Ala Lys Gly Leu Glu Asn Thr Asp Tyr
   1 5 10 15
   Leu Cys Asn Met Asp Pro Tyr Ala Ile Leu Lys Cys Arg Ser Gln Glu
   20 25 30
   Gln Arg Ser Ser Ile Ala Ser Gly Lys Gly Ser Asn Pro Glu Trp Asn
   35 40 45
   Glu Asn Phe Val Phe Thr Val Ser Asp Lys Ala Thr Glu Leu Leu Ile
   50 55 60
   Lys Leu Leu Asp Ser Asp Thr Gly Ser Ala Asp Asp Phe Val Gly Glu
   65 70 75 80
   Ala Thr
<210> 376
   <211> 82
   <212> PRT
   <213> Artificial Sequence
<220>
   <223> Synthetically generated peptide
<400> 376
   Leu Glu Val Val Leu Ile Ser Ala Lys Gly Leu Glu Asp Asn Asp Phe
   1 5 10 15
   Leu Ser Ser Ile Asp Pro Tyr Val Ile Leu Ser Tyr Arg Ala Gln Glu
   20 25 30
   His Lys Ser Thr Val Gln Glu Gly Ala Gly Ser Asn Pro Gln Trp Asn
   35 40 45
   Glu Thr Phe Leu Phe Thr Val Ser Asp Ser Ala Ser Glu Leu Asn Leu
   50 55 60
   Arg Ile Met Glu Lys Asp Asn Phe Asn Asn Asp Asp Asn Leu Gly Glu
   65 70 75 80
   Ala Ile
<210> 377
   <211> 87
   <212> PRT
   <213> Artificial Sequence
<220>
   <223> Synthetically generated peptide
<400> 377
   Met Glu Val His Leu Ile Ser Gly Lys Gly Leu Gln Ala His Asp Pro
   1 5 10 15
   Leu Asn Lys Pro Ile Asp Pro Tyr Ala Glu Ile Asn Phe Lys Gly Gln
   20 25 30
   Glu Arg Met Ser Lys Val Ala Lys Asn Ala Gly Pro Asn Pro Leu Trp
   35 40 45
   Asp Glu Lys Phe Lys Phe Leu Ala Glu Tyr Pro Gly Ser Gly Gly Asp
   50 55 60
   Phe His Ile Leu Phe Lys Val Met Asp His Asp Ala Ile Asp Gly Asp
   65 70 75 80
   Asp Tyr Ile Gly Asp Val Lys
   85
<210> 378
   <211> 87
   <212> PRT
   <213> Artificial Sequence
<220>
   <223> Synthetically generated peptide
<400> 378
   Met Glu Val His Leu Ile Ser Gly Lys Gly Leu Gln Ala His Asp Pro
    1              5                   10                  15
   Leu Asn Lys Pro Ile Asp Pro Tyr Ala Glu Ile Asn Phe Lys Gly Gln
               20                  25                  30
   Glu Arg Met Ser Lys Val Ala Lys Asn Ala Gly Pro Asp Pro Ile Trp
           35                  40                  45
   Asn Glu Lys Phe Lys Phe Leu Val Glu Tyr Pro Gly Ser Gly Gly Asp
       50                  55                  60
   Phe His Ile Leu Phe Lys Val Met Asp His Asp Ala Ile Asp Gly Asp
   65                  70                  75                  80
   Asp Tyr Ile Gly Asp Val Lys
                   85
<210> 379
   <211> 87
   <212> PRT
   <213> Artificial Sequence
<220>
   <223> Synthetically generated peptide
<400> 379
   Met Glu Val His Leu Ile Ser Gly Lys Gly Leu Gln Ala Leu Asp Pro
    1              5                   10                  15
   Leu Asn Lys Pro Ile Asp Pro Tyr Ala Glu Ile Asn Phe Lys Gly Gln
               20                  25                  30
   Glu Arg Met Ser Lys Val Ala Lys Asn Ala Gly Pro Asp Pro Ile Trp
           35                  40                  45
   Asn Glu Lys Phe Lys Phe Leu Val Glu Tyr Pro Gly Ser Gly Gly Asp
       50                  55                  60
   Phe His Ile Leu Phe Lys Val Met Asp His Asp Ala Ile Asp Gly Asp
   65                  70                  75                  80
   Asp Tyr Ile Gly Asp Val Ile
                   85
<210> 380
   <211> 87
   <212> PRT
   <213> Artificial sequence
<220>
   <223> Synthetically generated peptide
<400> 380
   Leu Glu Val His Leu Ile Ser Gly Lys Gly Leu Gln Ala His Asp Pro
   1 5 10 15
   Leu Asn Lys Pro Ile Asp Pro Tyr Val Glu Ile Asn Tyr Lys Gly Gln
   20 25 30
   Glu Arg Met Ser Lys Val Ala Lys Asn Ala Gly Pro Asp Pro Leu Trp
   35 40 45
   Asp Glu Lys Phe Lys Phe Leu Ala Glu Tyr Pro Gly Ser Gly Gly Asp
   50 55 60
   Phe His Val Leu Phe Lys Val Met Asp His Asp Ala Ile Asp Gly Asp
   65 70 75 80
   Asp Tyr Ile Gly Asp Val Lys
   85
<210> 381
   <211> 87
   <212> PRT
   <213> Artificial Sequence
<220>
   <223> Synthetically generated peptide
<400> 381
   Leu Glu Val His Leu Ile Ser Gly Lys Gly Leu Gln Ala His Asp Pro
   1 5 10 15
   Leu Asn Lys Pro Ile Asp Pro Tyr Val Glu Ile Asn Tyr Lys Gly Gln
   20 25 30
   Glu Arg Met Ser Lys Val Ala Lys Asn Ala Gly Pro Asp Pro Leu Trp
   35 40 45
   Asp Glu Lys Phe Lys Phe Leu Ala Glu Tyr Pro Gly Ser Gly Gly Asp
   50 55 60
   Phe His Ile Leu Phe Lys Val Met Asp His Asp Val Ile Asp Gly Asp
   65 70 75 80
   Asp Tyr Ile Gly Asp Val Lys
   85
<210> 382
   <211> 87
   <212> PRT
   <213> Artificial Sequence
<220>
   <223> Synthetically generated peptide
<400> 382
   Leu Glu Val His Leu Ile Ser Gly Lys Gly Leu Gln Ala His Asp Pro
   1 5 10 15
   Leu Asn Lys Pro Ile Asp Pro Tyr Ala Glu Ile Asn Tyr Lys Gly Gln
   20 25 30
   Glu Arg Met Ser Lys Val Ala Lys Asn Ala Gly Pro Asp Pro Leu Trp
   35 40 45
   Asp Glu Lys Phe Lys Phe Leu Ala Glu Tyr Pro Gly Ser Gly Gly Asp
   50 55 60
   Phe His Ile Phe Phe Lys Val Met Asp His Asp Ala Ile Asp Asp Asp
   65 70 75 80
   Asp Leu Ile Gly Glu Val Lys
   85
<210> 383
   <211> 87
   <212> PRT
   <213> Artificial Sequence
<220>
   <223> Synthetically generated peptide
<400> 383
   Leu Glu Val His Leu Ile Ser Gly Lys Gly Leu Gln Ala His Asp Pro
   1               5                   10                  15
   Leu Asn Lys Pro Ile Asp Pro Tyr Val Glu Ile Asn Tyr Lys Gly Gln
               20                  25                  30
   Glu Arg Met Ser Lys Val Ala Lys Asn Ala Gly Pro Asp Pro Val Trp
           35                  40                  45
   Asn Glu Lys Phe Lys Phe Leu Ala Glu Tyr Pro Gly Ser Gly Gly Asp
       50                  55                  60
   Phe Leu Ile Leu Phe Lys Val Met Asp His Asp Val Ile Asp Gly Asp
   65                  70                  75                  80
   Asp Tyr Ile Gly Asp Val Ser
                   85
<210> 384
   <211> 87
   <212> PRT
   <213> Artificial Sequence
<220>
   <223> Synthetically generated peptide
<400> 384
   Leu Glu Val His Leu Ile Ser Gly Lys Gly Leu Arg Ala His Asp Pro
   1               5                   10                  15
   Leu Asn Lys Pro Ile Asp Pro Tyr Val Glu Ile Asn Tyr Lys Gly Gln
               20                  25                  30
   Glu Arg Met Ser Lys Val Ala Lys Asn Ala Gly Pro Asp Pro Val Trp
           35                  40                  45
   Asn Glu Lys Phe Lys Phe Leu Ala Glu Tyr Pro Gly Ser Gly Gly Asp
       50                  55                  60
   Phe Leu Ile Leu Phe Lys Val Met Asp His Asp Val Ile Asp Gly Asp
   65                  70                  75                  80
   Asp Tyr Ile Gly Asp Val Ser
                   85
<210> 385
   <211> 87
   <212> PRT
   <213> Artificial sequence
<220>
   <223> Synthetically generated peptide
<400> 385
   Leu Glu Val Ser Leu Ile Ser Gly Lys Gly Leu Lys Arg Ser Asp Phe
    1               5                  10                  15
   Leu Gly Lys Ile Asp Pro Tyr Val Glu Ile Gln Tyr Lys Gly Gln Thr
               20                  25                  30
   Arg Lys Ser Ser Val Ala Lys Glu Asp Gly Gly Arg Asn Pro Thr Trp
           35                  40                  45
   Asn Asp Lys Leu Lys Trp Arg Ala Glu Phe Pro Gly Ser Gly Ala Asp
       50                  55                  60
   Tyr Lys Leu Ile Val Lys Val Met Asp His Asp Thr Phe Ser Ser Asp
   65                  70                  75                  80
   Asp Phe Ile Gly Glu Ala Thr
                   85
<210> 386
   <211> 77
   <212> PRT
   <213> Artificial Sequence
<220>
   <223> Synthetically generated peptide
<400> 386
   Val Gly Cys Gln Lys Leu Lys Asp Thr Glu Trp Phe Ser Arg Gln Asp
    1               5                  10                  15
   Pro Tyr Val Val Leu Glu Tyr Gly Gly Arg Ser His Arg Thr Arg Thr
               20                  25                  30
   Cys Thr Asp Gly Gly Lys Asn Ala Val Phe Gln Glu Lys Phe Ile Phe
           35                  40                  45
   Thr Leu Ile Glu Gly Leu Arg Asp Leu Lys Val Ala Val Trp Asn Ser
       50                  55                  60
   Asn Thr Leu Ser Thr Asp Asp Phe Ile Gly Asn Ala Thr
   65                  70                  75
<210> 387
   <211> 82
   <212> PRT
   <213> Artificial Sequence
<220>
   <223> Synthetically generated peptide
<400> 387
   Leu Glu Val Thr Val Val Gly Cys Gln Lys Leu Lys Asp Thr Glu Trp
    1               5                  10                  15
   Phe Ser Arg Gln Asp Pro Tyr Val Val Leu Glu Tyr Gly Gly Arg Ser
               20                  25                  30
   His Arg Thr Arg Thr Cys Thr Asp Gly Gly Lys Asn Ala Val Phe Gln
           35                  40                  45
   Glu Lys Phe Ile Phe Thr Leu Ile Glu Gly Leu Arg Asp Leu Lys Val
       50                  55                  60
   Ala Val Trp Asn Ser Asn Thr Leu Ser Thr Asp Asp Phe Ile Gly Asn
   65                  70                  75                  80
   Ala Thr
<210> 388
   <211> 80
   <212> PRT
   <213> Artificial Sequence
<220>
   <223> Synthetically generated peptide
<400> 388
   Val Asp Cys Thr Leu Val Ser Arg Ile Lys Asp Val Glu Ile Val Gly
   1               5                   10                  15
   Lys Gln Ser Pro Tyr Ala Val Leu Thr Val Gly Pro Lys Thr Phe Lys
               20                  25                  30
   Ser Gly Thr Ala Asn Gly Gly Gly Ser Asp Pro Val Trp Asn Gln Thr
           35                  40                  45
   Phe Ser Phe Thr Asn Val Thr Pro Asp Ser Ser Val Lys Leu Glu Ile
       50                  55                  60
   Phe Asn Ser Asn Val Val Leu Arg Asp Val Ala Ile Gly Gly Cys Lys
   65                  70                  75                  80
<210> 389
   <211> 104
   <212> PRT
   <213> Artificial Sequence
<220>
   <223> Synthetically generated peptide
<400> 389
   Leu Arg Ala Ser Val Ile Glu Ala His Asp Leu Arg Val Pro Ala Pro
   1               5                   10                  15
   Ser Pro Gly Leu Pro Phe Asp Val Arg Val Lys Ile Lys Ile Gly Phe
               20                  25                  30
   Gln Ser Ala Arg Thr Gln Arg Ser Val Ala Ser Thr Ser Ser Gly Ser
           35                  40                  45
   Ala Phe Ala Trp Glu Trp Glu Glu Asp Leu Met Phe Val Val Ser Glu
       50                  55                  60
   Pro Leu Asp Glu Ser Leu Ile Val Leu Val Lys Asp Arg Thr Met Ile
   65                  70                  75                  80
   Lys Glu Pro Ala Arg Arg Gly Ala Arg Pro Thr Ser Ala Leu Leu Pro
                   85                  90                  95
   Ala Lys Glu Ala Ala His Val Cys
               100
<210> 390
   <211> 88
   <212> PRT
   <213> Artificial Sequence
<220>
   <223> Synthetically generated peptide
<400> 390
   Leu Arg Ala Ser Val Ile Glu Ala Gln Asp Leu Arg Val Pro Ala Pro
   1               5                   10                  15
   Pro Pro Gly Leu Pro Phe Asp Val Arg Val Lys Ile Gln Val Gly Phe
               20                  25                  30
   Gln Ser Ala Arg Thr Arg Arg Ser Val Ala Ser Arg Ser Ser Gly Ser
           35                  40                  45
   Ala Phe Ala Trp Glu Glu Asp Leu Met Phe Val Val Ser Glu Pro Leu
       50                  55                  60
   Asp Glu Ser Leu Val Val Leu Val Glu Asp Arg Ser Met Ile Lys Glu
   65                  70                  75                  80
   Pro Ala Leu Leu Gly His Ala Thr
                   85
<210> 391
   <211> 87
   <212> PRT
   <213> Artificial Sequence
<220>
   <223> Synthetically generated peptide
<400> 391
   Leu Arg Val Thr Val Leu Glu Ala Gln Asp Leu His Ile Ala Pro Asn
   1               5                   10                  15
   Leu Pro Pro Leu Thr Ala Pro Glu Ile Arg Val Lys Ala Gln Leu Gly
               20                  25                  30
   Phe Gln Ser Ala Arg Thr Arg Arg Gly Ser Met Asn Asn His Ser Gly
           35                  40                  45
   Ser Phe His Trp His Glu Asp Met Ile Phe Val Ala Gly Glu Pro Leu
       50                  55                  60
   Glu Asp Cys Leu Val Leu Met Val Glu Asp Arg Thr Thr Lys Glu Ala
   65                  70                  75                  80
   Thr Leu Leu Gly His Ala Met
                   85
<210> 392
   <211> 87
   <212> PRT
   <213> Artificial Sequence
<220>
   <223> Synthetically generated peptide
<400> 392
   Leu Arg Val Thr Val Leu Glu Ala Gln Asp Leu His Ile Ala Pro Asn
   1               5                   10                  15
   Leu Pro Pro Leu Thr Ala Pro Glu Ile Arg Val Lys Ala Gln Leu Gly
               20                  25                  30
   Phe Gln Ser Ala Arg Thr Arg Arg Gly Ser Met Asn Asn His Ser Gly
           35                  40                  45
   Ser Phe His Trp His Glu Asp Met Ile Phe Val Ala Gly Glu Pro Leu
       50                  55                  60
   Glu Asp Cys Leu Val Leu Met Val Glu Asp Arg Thr Thr Lys Glu Ala
   65                  70                  75                  80
   Thr Leu Leu Gly His Ala Met
                   85
<210> 393
   <211> 83
   <212> PRT
   <213> Artificial sequence
<220>
   <223> Synthetically generated peptide
<400> 393
   Leu Arg Val Asn Val Ile Glu Ala Gln Asp Leu Val Leu Leu His Pro
    1               5                  10                  15
   Asn Arg Ile Asn Pro Glu Ile Leu Ile Lys Gly Phe Leu Gly Asn Val
               20                  25                  30
   Val Val Arg Ser Arg Ile Ser Gln Thr Lys Ser Val Ser Pro Val Trp
           35                  40                  45
   Asn Glu Asp Met Met Phe Val Ala Val Glu Pro Phe Asp Asp Ser Leu
       50                  55                  60
   Ile Leu Ser Val Glu Asp Lys Val Gly Pro Arg Glu Glu Cys Leu Gly
   65                  70                  75                  80
   Arg Cys Glu
<210> 394
   <211> 92
   <212> PRT
   <213> Artificial Sequence
<220>
   <223> Synthetically generated peptide
<400> 394
   Leu Arg Leu Ser Val Ile Gln Ala Gln Asp Leu Arg Leu Pro Ala Pro
    1               5                  10                  15
   Pro Asp Ala Lys Ala Lys Pro Met Gly Pro Ala Phe Pro Glu Leu Tyr
               20                  25                  30
   Val Lys Ala Gln Leu Gly Ala Gln Val Phe Lys Thr Cys Arg Val Gly
           35                  40                  45
   Ser Ala Ala Thr Gly Thr Ser Asn Pro Ser Trp Asn Glu Asp Leu Leu
       50                  55                  60
   Phe Val Ala Ala Glu Pro Phe Asp Pro Phe Leu Thr Val Val Val Glu
   65                  70                  75                  80
   Asp Ile Phe Ser Gly Gln Pro Val Gly Gln Ala Arg
                   85                  90
<210> 395
   <211> 93
   <212> PRT
   <213> Artificial Sequence
<220>
   <223> Synthetically generated peptide
<400> 395
   Leu Arg Leu Thr Val Ile Gln Thr Gln Asp Leu Gln Leu Gly Leu Gly
   1               5                   10                  15
   Ser Glu Ala Lys Ser Lys Ile Pro Thr Thr Glu Leu Tyr Val Lys Ala
               20                  25                  30
   Gln Leu Gly Pro Gln Val Phe Lys Thr Ala Arg Thr Ser Ile Gly Pro
           35                  40                  45
   Ser Ala Ser Ser Ser Gly Ser Gly Asn Pro Thr Trp Asn Glu Asp Leu
       50                  55                  60
   Val Phe Val Ala Ser Glu Pro Phe Glu Pro Phe Leu Ile Val Thr Val
   65                  70                  75                  80
   Glu Asp Ile Thr Asn Gly Gln Ser Ile Gly Gln Thr Lys
                   85                  90
<210> 396
   <211> 84
   <212> PRT
   <213> Artificial Sequence
<220>
   <223> Synthetically generated peptide
<400> 396
   Leu Arg Ala Thr Val Ile Glu Ala Gln Asp Leu Leu Pro Pro Gln Leu
   1               5                   10                  15
   Thr Ala Phe Lys Glu Ala Ser Phe Gln Leu Lys Ala Gln Leu Gly Ser
               20                  25                  30
   Gln Val Gln Lys Thr Lys Ser Ala Val Thr Arg Asn Gly Ala Pro Ser
           35                  40                  45
   Trp Asn Glu Asp Leu Leu Phe Val Ala Ala Glu Pro Phe Ser Asp Gln
       50                  55                  60
   Leu Val Phe Thr Leu Glu Tyr Arg Thr Ser Lys Gly Pro Val Thr Val
   65                  70                  75                  80
   Gly Met Ala Arg
<210> 397
   <211> 76
   <212> PRT
   <213> Artificial Sequence
<220>
   <223> Synthetically generated peptide
<400> 397
   Leu Arg Ile Ser Val Leu Glu Ala Gln Asp Val Val Pro Gly Ala Val
   1               5                   10                  15
   Ala Gly Ala Gly Gly Asp Lys Gly Arg His Gly Glu Ala Phe Val Val
               20                  25                  30
   Val Lys Val Gln Val Gly Gly Val Thr Leu Arg Thr Lys Pro Cys Cys
           35                  40                  45
   Arg Pro Thr Ser Pro Ser Trp Asn Glu Glu Leu Val Phe Val Val Ala
       50                  55                  60
   Glu Pro Phe Asp Glu Pro Ala Val Leu Val Ile Glu
   65                  70                  75
<210> 398
   <211> 96
   <212> PRT
   <213> Artificial sequence
<220>
   <223> synthetically generated peptide
<400> 398
   Leu Arg Ile Ser Val Ile Glu Ala Gln Asp Val Ala Ile Met Asp Lys
   1               5                   10                  15
   Gly Ser Ser Leu Met Arg Phe Pro Glu Leu Ser Ala Lys Leu Gln Val
               20                  25                  30
   Gly Ser Gln Ile Leu Arg Thr Ala Ile Ala Ser Ala Ile Pro Thr Lys
           35                  40                  45
   Ser Phe Ser Asn Pro Tyr Trp Asn Glu Asp Leu Met Phe Val Val Ala
       50                  55                  60
   Glu Pro Phe Glu Asp Cys Val Thr Val Val Val Glu Asp Arg Leu Asn
   65                  70                  75                  80
   Gly Gly Ala Ile Gly Gly Gln Asn Asp Val Ala Val Gly Arg Val Gln
                   85                  90                  95
<210> 399
   <211> 80
   <212> PRT
   <213> Artificial Sequence
<220>
   <223> Synthetically generated peptide
<400> 399
   Val Arg Val Asn Val Ile Gly Ala Gln Asp Ile Phe Pro Met Glu Asn
   1               5                   10                  15
   His Ile Pro Asp Val Phe Val Lys Val Arg Leu Gly His Gln Met Leu
               20                  25                  30
   Lys Trp Ala Arg Ser Pro Thr Arg Asn Phe Met Trp Asn Glu Glu Met
           35                  40                  45
   Met Phe Val Ala Ala Glu Pro Phe Glu Glu Asp Leu Ile Ile Gln Ile
       50                  55                  60
   Glu Asp Arg Val Ala Gln Asn Lys Asp Glu Val Ile Gly Glu Thr Met
   65                  70                  75                  80
<210> 400
   <211> 81
   <212> PRT
   <213> Artificial Sequence
<220>
   <223> Synthetically generated peptide
<400> 400
   Leu Arg Val Asn Val Ile Glu Ala Gln Asp Leu Val Ile Val Pro Asp
   1               5                   10                  15
   Arg Thr Arg Leu Pro Asn Pro Tyr Val Lys Ile Arg Leu Asn Asn Gln
               20                  25                  30
   Val Val Arg Thr Lys Pro Ser His Ser Leu Asn Pro Arg Trp Asn Glu
           35                  40                  45
   Glu Phe Thr Leu Val Ala Ala Glu Pro Phe Glu Asp Leu Ile Ile Ser
       50                  55                  60
   Ile Glu Asp Arg Val Ala Pro Asn Arg Glu Glu Thr Leu Gly Glu Val
   65                  70                  75                  80
   His
<210> 401
   <211> 72
   <212> PRT
   <213> Artificial Sequence
<220>
   <223> Synthetically generated peptide
<400> 401
   Leu Arg Val Lys Val Ile Glu Ala His Asp Leu Val Ser His Asp Asn
   1               5                   10                  15
   Lys Ser Arg Ala Pro Asp Ala Phe Val Lys Val Gln His Gly Asn Gln
               20                  25                  30
   Ile Phe Lys Thr Lys Pro Val Gln Ser Arg Ile Asn Asn Pro Arg Trp
           35                  40                  45
   Asp Gln Gly Thr Leu Phe Val Ala Ala Glu Pro Phe Glu Glu Pro Leu
       50                  55                  60
   Ile Ile Thr Val Glu Asp Lys Asp
   65                  70
<210> 402
   <211> 83
   <212> PRT
   <213> Artificial Sequence
<220>
   <223> Synthetically generated peptide
<400> 402
   Leu Arg Val Asn Val Ile Glu Ala Gln Asp Val Ile Ser Ser Asp Arg
   1               5                   10                  15
   Asn Arg Val Pro Glu Val Phe Ile Lys Ala Gln Met Gly Ser Gln Val
               20                  25                  30
   Leu Arg Thr Lys Val Cys Pro Thr Arg Ser Thr Thr Gln Ile Trp Asn
           35                  40                  45
   Glu Asp Leu Val Phe Val Ala Ala Glu Pro Phe Glu Glu Gln Leu Thr
       50                  55                  60
   Ile Thr Val Glu Asp Arg Val His Gly Ser Lys Asp Glu Val Leu Gly
   65                  70                  75                  80
   Lys Ile Met
<210> 403
   <211> 83
   <212> PRT
   <213> Artificial Sequence
<220>
   <223> Synthetically generated peptide
<400> 403
   Leu Arg Val Asn Val Ile Glu Ala Gln Asp Val Ile Pro Ser Asp Arg
    1               5                  10                  15
   Asn Arg Leu Pro Glu Val Ser Val Lys Ala His Leu Gly Cys Gln Val
               20                  25                  30
   Leu Lys Thr Lys Ile Cys Ser Thr Arg Thr Thr Ser Pro Leu Trp Asn
           35                  40                  45
   Glu Asp Leu Val Phe Val Ala Ala Glu Pro Phe Glu Glu Gln Leu Thr
       50                  55                  60
   Ile Thr Val Glu Asp His Val Gln Pro Ser Lys Asp Glu Val Leu Gly
   65                  70                  75                  80
   Arg Ile Ser
<210> 404
   <211> 83
   <212> PRT
   <213> Artificial Sequence
<220>
   <223> Synthetically generated peptide
<400> 404
   Leu Arg Val Asn Val Ile Glu Ala Gln Asp Met Ile Pro Ser Asp Arg
    1               5                  10                  15
   Asn Arg Leu Pro Asp Val Phe Val Lys Ala Ser Val Gly Met Gln Thr
               20                  25                  30
   Leu Lys Thr Ser Ile Cys Ser Ile Lys Thr Thr Asn Pro Leu Trp Lys
           35                  40                  45
   Glu Asp Leu Val Phe Val Val Ala Glu Pro Phe Glu Glu Gln Leu Val
       50                  55                  60
   Ile Ser Val Glu Asp Arg Val His Thr Ser Lys Asp Glu Val Ile Gly
   65                  70                  75                  80
   Lys Ile Thr
<210> 405
   <211> 83
   <212> PRT
   <213> Artificial Sequence
<220>
   <223> Synthetically generated peptide
<400> 405
   Leu Arg Val Asn Val Ile Glu Ala Gln Asp Val Glu Pro Ser Asp Arg
   1 5 10 15
   Ser Gln Pro Pro Gln Ala Phe Val Lys Val Gln Val Gly Asn Gln Ile
   20 25 30
   Leu Lys Thr Lys Leu Cys Pro Asn Lys Thr Thr Asn Pro Met Trp Asn
   35 40 45
   Glu Asp Leu Val Phe Val Ala Ala Glu Pro Phe Glu Glu Gln Phe Phe
   50 55 60
   Leu Thr Val Glu Asn Lys Val Thr Pro Ala Lys Asp Glu Val Met Gly
   65 70 75 80
   Arg Leu Ile
<210> 406
   <211> 83
   <212> PRT
   <213> Artificial sequence
<220>
   <223> Synthetically generated peptide
<400> 406
   Leu Arg Val Asn Val Ile Glu Ala Gln Asp Val Glu Pro Ser Asp Arg
   1 5 10 15
   Ser Gln Pro Pro Gln Ala Phe Val Lys Val Gln Val Gly Asn Gln Ile
   20 25 30
   Leu Lys Thr Lys Leu Cys Pro Asn Lys Thr Thr Asn Pro Met Trp Asn
   35 40 45
   Glu Asp Leu Val Phe Val Ala Ala Glu Pro Phe Glu Glu Gln Phe Phe
   50 55 60
   Leu Thr Val Glu Asn Lys Val Thr Pro Ala Lys Asp Glu Val Met Gly
   65 70 75 80
   Arg Leu Ile
<210> 407
   <211> 81
   <212> PRT
   <213> Artificial Sequence
<220>
   <223> Synthetically generated peptide
<400> 407
   Leu Arg Val Asn Val Ile Glu Ala Gln Asp Val Gln Pro Gln Arg Arg
   1 5 10 15
   Ala Pro Glu Val Phe Val Lys Ala Gln Val Gly Asn Gln Ile Leu Lys
   20 25 30
   Thr Ser Val Val Ala Ala Pro Thr Leu Asn Pro Arg Trp Asn Glu Asp
   35 40 45
   Leu Val Phe Val Val Ala Glu Pro Phe Glu Glu Gln Leu Leu Leu Thr
   50 55 60
   Val Glu Asp Arg Val Thr Pro Arg Lys Asp Asp Leu Leu Gly Arg Ala
   65 70 75 80
   Ala
<210> 408
   <211> 83
   <212> PRT
   <213> Artificial Sequence
<220>
   <223> Synthetically generated peptide
<400> 408
   Leu Arg Val Asn Ile Ile Glu Ala Gln Asp Ile Ala Ile Thr Asp Lys
    1               5                  10                  15
   Thr Arg Tyr Pro Asp Val Phe Val Arg Ala Gln Val Gly His Gln His
               20                  25                  30
   Gly Arg Thr Lys Pro Val Gln Ala Arg Asn Phe Asn Pro Phe Trp Asn
           35                  40                  45
   Glu Asp Leu Met Phe Val Ala Ala Glu Pro Phe Glu Asp His Leu Ile
       50                  55                  60
   Leu Ser Leu Glu Asp Arg Val Ala Pro Asn Lys Asp Glu Val Leu Gly
   65                  70                  75                  80
   Arg Val Ile
<210> 409
   <211> 83
   <212> PRT
   <213> Artificial Sequence
<220>
   <223> Synthetically generated peptide
<400> 409
   Leu Arg Val Asn Ile Ile Glu Ala Gln Asp Ile Ala Ile Thr Asp Lys
    1               5                  10                  15
   Thr Arg Tyr Pro Asp Val Phe Val Arg Ala Gln Val Gly His Gln His
               20                  25                  30
   Gly Arg Thr Lys Pro Val Gln Ala Arg Asn Phe Asn Pro Phe Trp Asn
           35                  40                  45
   Glu Asp Leu Met Phe Val Ala Ala Glu Pro Phe Glu Asp His Leu Ile
       50                  55                  60
   Leu Ser Leu Glu Asp Arg Val Ala Pro Asn Lys Asp Glu Val Leu Gly
   65                  70                  75                  80
   Arg Val Ile
<210> 410
   <211> 83
   <212> PRT
   <213> Artificial Sequence
<220>
   <223> Synthetically generated peptide
<400> 410
   Leu Arg Val Asn Val Ile Glu Ala Gln Asp Leu Ile Pro Thr Asp Lys
   1               5                   10                  15
   Gln Arg Tyr Pro Glu Val Tyr Val Lys Ala Ile Val Gly Asn Gln Ala
               20                  25                  30
   Leu Arg Thr Arg Val Ser Gln Ser Arg Thr Ile Asn Pro Met Trp Asn
           35                  40                  45
   Glu Asp Leu Met Phe Val Ala Ala Glu Pro Phe Glu Glu Pro Leu Ile
       50                  55                  60
   Leu Ser Val Glu Asp Arg Val Ala Pro Asn Lys Asp Glu Val Leu Gly
   65                  70                  75                  80
   Arg Cys Ala
<210> 411
   <211> 83
   <212> PRT
   <213> Artificial sequence
<220>
   <223> synthetically generated peptide
<400> 411
   Leu Arg Val Asn Val Ile Glu Ala Gln Asp Leu Ile Pro Thr Asp Lys
   1               5                   10                  15
   Gln Arg Tyr Pro Glu Val Tyr Val Lys Ala Ile Val Gly Asn Gln Ala
               20                  25                  30
   Leu Arg Thr Arg Val Ser Gln Ser Arg Thr Ile Asn Pro Met Trp Asn
           35                  40                  45
   Glu Asp Leu Met Phe Val Ala Ala Glu Pro Phe Glu Glu Pro Leu Ile
       50                  55                  60
   Leu Ser Val Glu Asp Arg Val Ala Pro Asn Lys Asp Glu Val Leu Gly
   65                  70                  75                  80
   Arg Cys Ala
<210> 412
   <211> 83
   <212> PRT
   <213> Artificial Sequence
<220>
   <223> Synthetically generated peptide
<400> 412
   Leu Arg Val Asn Val Ile Glu Ala Gln Asp Leu Ile Pro Ser Asp Lys
   1               5                   10                  15
   Gly Arg Tyr Pro Glu Val Phe Val Lys Val Ile Met Gly Asn Gln Ala
               20                  25                  30
   Leu Arg Thr Arg Val Ser Gln Ser Arg Ser Ile Asn Pro Met Trp Asn
           35                  40                  45
   Glu Asp Leu Met Phe Val Val Ala Glu Pro Phe Glu Glu Pro Leu Ile
       50                  55                  60
   Leu Ser Val Glu Asp Arg Val Ala Pro Asn Lys Asp Glu Val Leu Gly
   65                  70                  75                  80
   Arg Cys Ala
<210> 413
   <211> 83
   <212> PRT
   <213> Artificial Sequence
<220>
   <223> Synthetically generated peptide
<400> 413
   Val Arg Val Asn Val Ile Glu Ala Gln Asp Leu Ile Pro His Asp Lys
    1              5                   10                  15
   Thr Lys Phe Pro Glu Val Tyr Val Lys Ala Met Leu Gly Asn Gln Thr
               20                  25                  30
   Leu Arg Thr Arg Ile Ser Gln Thr Lys Thr Leu Asn Pro Met Trp Asn
           35                  40                  45
   Glu Asp Leu Met Phe Val Val Ala Glu Pro Phe Glu Glu Ala Leu Ile
       50                  55                  60
   Leu Ala Val Glu Asp Arg Val Ala Pro Asn Lys Asp Glu Thr Leu Gly
   65                  70                  75                  80
   Arg Cys Ala
<210> 414
   <211> 83
   <212> PRT
   <213> Artificial Sequence
<220>
   <223> Synthetically generated peptide
<400> 414
   Leu Arg Val Asn Val Ile Glu Ala Gln Asp Leu Ile Pro Asn Asp Arg
    1              5                   10                  15
   Thr Arg Phe Pro Asp Val Tyr Val Lys Ala Met Leu Gly Asn Gln Ala
               20                  25                  30
   Leu Arg Thr Arg Val Ser Pro Ser Arg Thr Leu Asn Pro Met Trp Asn
           35                  40                  45
   Glu Asp Leu Met Phe Val Ala Ala Glu Pro Phe Glu Glu His Leu Ile
       50                  55                  60
   Leu Ser Val Glu Asp Arg Ile Ala Pro Gly Lys Asp Asp Val Leu Gly
   65                  70                  75                  80
   Arg Thr Ile
<210> 415
   <211> 81
   <212> PRT
   <213> Artificial Sequence
<220>
   <223> Synthetically generated peptide
<400> 415
   Val Arg Val Asn Val Ile Glu Ala Gln Asp Leu Ile Pro Thr Asp Lys
   1 5 10 15
   Thr Arg Phe Pro Asp Val Tyr Val Lys Ala Gln Leu Gly Asn Gln Val
   20 25 30
   Met Lys Trp Cys Gln Ala Arg Thr Leu Gly Ala Val Trp Asn Glu Asp
   35 40 45
   Phe Leu Phe Val Val Ala Glu Pro Phe Glu Asp His Leu Val Leu Thr
   50 55 60
   Val Glu Asp Arg Val Ala Pro Gly Lys Asp Glu Ile Val Gly Arg Thr
   65 70 75 80
   Tyr
<210> 416
   <211> 81
   <212> PRT
   <213> Artificial Sequence
<220>
   <223> Synthetically generated peptide
<400> 416
   Val Arg Val Asn Val Ile Glu Ala Gln Asp Leu Ile Pro Thr Asp Lys
   1 5 10 15
   Thr Arg Phe Pro Asp Val Tyr Val Lys Ala Gln Leu Gly Asn Gln Val
   20 25 30
   Met Lys Trp Cys Gln Ala Arg Thr Leu Gly Ala Val Trp Asn Glu Asp
   35 40 45
   Phe Leu Phe Val Val Ala Glu Pro Phe Glu Asp His Leu Val Leu Thr
   50 55 60
   Val Glu Asp Arg Val Ala Pro Gly Lys Asp Glu Ile Val Gly Arg Thr
   65 70 75 80
   Tyr
<210> 417
   <211> 90
   <212> PRT
   <213> Artificial sequence
<220>
   <223> synthetically generated peptide
<400> 417
   Leu Arg Val Ser Val Ile Glu Ala Gln Asp Leu Ile Pro Met Asp Lys
   1 5 10 15
   Gly Pro Met Ala Ile Gly Arg Tyr Pro Glu Leu Phe Val Arg Ala Gln
   20 25 30
   Val Gly Ser Gln Met Leu Arg Trp Ala Pro Val Ala Ala Asn Arg Gly
   35 40 45
   Pro Ser Ser Pro Phe Trp Asn Glu Asp Leu Met Phe Val Val Ala Glu
   50 55 60
   Pro Phe Glu Glu Phe Leu Val Leu Ser Leu Glu Asp His Val Ser Pro
   65 70 75 80
   Gly Arg Asp Asp Val Leu Gly Arg Leu Val
   85 90
<210> 418
   <211> 68
   <212> PRT
   <213> Artificial Sequence
<220>
   <223> Synthetically generated peptide
<400> 418
   Asp Lys Ser Arg Val Pro Glu Val Phe Val Arg Val Lys Val Gly Asn
    1              5                  10                  15
   Gln Met Leu Arg Thr Lys Phe Pro Gln Arg Ser Asn Asn Pro Lys Trp
               20                  25                  30
   Gly Asp Glu Phe Thr Phe Val Val Ala Glu Pro Phe Glu Asp Asn Leu
           35                  40                  45
   Val Leu Ser Val Glu Asp His Thr Ala Pro Asn Arg Asp Glu Pro Val
       50                  55                  60
   Gly Lys Ala Val
   65
<210> 419
   <211> 81
   <212> PRT
   <213> Artificial Sequence
<220>
   <223> Synthetically generated peptide
<400> 419
   Leu Arg Val Gln Ile Ala Gln Asp Val Ile Ile Val Ser Asp Lys Ser
    1              5                  10                  15
   Arg Val Pro Glu Val Phe Val Arg Val Lys Val Gly Asn Gln Met Leu
               20                  25                  30
   Arg Thr Lys Phe Pro Gln Arg Ser Asn Asn Pro Lys Trp Gly Asp Glu
           35                  40                  45
   Phe Thr Phe Val Val Ala Glu Pro Phe Glu Asp Asn Leu Val Leu Ser
       50                  55                  60
   Val Glu Asp His Thr Ala Pro Asn Arg Asp Glu Pro Val Gly Lys Ala
   65                  70                  75                  80
   Val
<210> 420
   <211> 83
   <212> PRT
   <213> Artificial Sequence
<220>
   <223> synthetically generated peptide
<400> 420
   Leu Arg Ile His Val Met Glu Ala Gln Asp Leu Val Pro Ser Asp Lys
   1               5                   10                  15
   Gly Arg Val Pro Asp Ala Ile Val Lys Ile Gln Ala Gly Asn Gln Met
               20                  25                  30
   Arg Ala Thr Arg Thr Pro Gln Met Arg Thr Met Asn Pro Gln Trp His
           35                  40                  45
   Glu Glu Leu Met Phe Val Val Ser Glu Pro Phe Glu Asp Met Val Ile
       50                  55                  60
   Val Ser Val Asp Asp Arg Ile Gly Pro Gly Lys Asp Glu Ile Leu Gly
   65                  70                  75                  80
   Arg Val Phe
<210> 421
   <211> 83
   <212> PRT
   <213> Artificial Sequence
<220>
   <223> Synthetically generated peptide
<400> 421
   Leu Arg Ile His Val Met Glu Ala Gln Asp Leu Val Pro Ser Asp Lys
   1               5                   10                  15
   Gly Arg Val Pro Asp Ala Ile Val Lys Ile Gln Ala Gly Asn Gln Met
               20                  25                  30
   Arg Ala Thr Arg Thr Pro Gln Met Arg Thr Met Asn Pro Gln Trp His
           35                  40                  45
   Glu Glu Leu Met Phe Val Val Ser Glu Pro Phe Glu Asp Met Val Ile
       50                  55                  60
   Val Ser Val Asp Asp Arg Ile Gly Pro Gly Lys Asp Glu Ile Leu Gly
   65                  70                  75                  80
   Arg Val Phe
<210> 422
   <211> 82
   <212> PRT
   <213> Artificial sequence
<220>
   <223> Synthetically generated peptide
<400> 422
   Leu Lys Val Val Ala Ile Ala Ala Gln Asp Leu Ile Pro Ala Glu Lys
   1               5                   10                  15
   Gly Arg Pro Leu Ala Pro Ser Ile Val Lys Ile Gln Leu Gly Gly Gln
               20                  25                  30
   Thr Arg Arg Thr Arg Ser Gln Gly Ser Ala Asn Pro Met Trp Asn Glu
           35                  40                  45
   Glu Phe Leu Phe Val Ala Ala Glu Pro Phe Asp Glu Pro Leu Val Val
       50                  55                  60
   Thr Val Glu Glu Arg Val Ala Ala Gly Arg Asp Glu Pro Val Gly Arg
   65                  70                  75                  80
   Val Ile
<210> 423
   <211> 82
   <212> PRT
   <213> Artificial Sequence
<220>
   <223> synthetically generated peptide
<400> 423
   Leu Arg Val Ala Ala Ile Gly Ala Gln Asp Leu Val Pro Leu Asp Ala
   1               5                   10                  15
   Ser Arg Pro Ala Asn Phe Cys Val Lys Leu Gln Leu Ala Gly Gln Val
               20                  25                  30
   Arg Arg Trp Gly Ala Pro Pro Gly Thr Leu Asn Pro Ile Trp Asn Glu
           35                  40                  45
   Glu Phe Met Phe Val Val Ser Glu Pro Phe Asp Glu Pro Leu Phe Val
       50                  55                  60
   Thr Val Glu Asp Arg Val Gly Pro Gly Arg Asp Glu Pro Leu Gly Arg
   65                  70                  75                  80
   Ile Met
<210> 424
   <211> 82
   <212> PRT
   <213> Artificial Sequence

<220>
   <223> Synthetically generated peptide
<400> 424
   Val Arg Val Thr Ile Val Ser Gly His Asp Leu Ile Ser Thr Asp Arg
   1               5                   10                  15
   Asn Arg Thr Pro Ser Val Tyr Val Thr Ala Thr Leu Gly Gln Val Thr
               20                  25                  30
   Leu Lys Thr Glu Val Ser Ser Gly Thr Asn Pro Ser Trp Asn Lys Asp
           35                  40                  45
   Leu Ile Phe Val Ala Ser Glu Pro Leu Glu Gly Thr Val Tyr Ile Arg
       50                  55                  60
   Leu Ile Asp Arg Val Asp Asp Gln His Glu Glu Arg Ile Ile Gly Lys
   65                  70                  75                  80
   Leu Glu
<210> 425
   <211> 79
   <212> PRT
   <213> Artificial Sequence
<220>
   <223> Synthetically generated peptide
<400> 425
   Val Arg Val Thr Ile Val Ser Gly His Asp Leu Ile Ser Lys Asp Lys
   1 5 10 15
   Asn Lys Thr Pro Ser Val Tyr Val Thr Ala Thr Leu Gly Lys Val Lys
   20 25 30
   Thr Lys Val Ser Ser Gly Thr Asn Pro Ser Trp Asn Gln Asp Leu Ile
   35 40 45
   Phe Val Ala Ser Glu Pro Leu Glu Gly Thr Val Tyr Ile Arg Leu Ile
   50 55 60
   Asp Arg Glu Asp Glu Gln His Glu Gly Cys Ile Gly Thr Leu Lys
   65 70 75
<210> 426
   <211> 79
   <212> PRT
   <213> Artificial Sequence
<220>
   <223> synthetically generated peptide
<400> 426
   Ile Ile Val Thr Val Leu Ala Gly Lys Asn Leu Val Ser Lys Asp Lys
   1 5 10 15
   Ser Gly Lys Cys Asp Ala Ser Val Lys Leu Gln Tyr Gly Lys Ile Ile
   20 25 30
   Gln Lys Thr Lys Ile Val Asn Ala Ala Glu Cys Val Trp Asn Gln Lys
   35 40 45
   Phe Glu Phe Glu Glu Leu Ala Gly Glu Glu Tyr Leu Lys Val Lys Cys
   50 55 60
   Tyr Arg Glu Glu Met Leu Gly Thr Asp Asn Ile Gly Thr Ala Thr
   65 70 75
<210> 427
   <211> 79
   <212> PRT
   <213> Artificial Sequence
<220>
   <223> Synthetically generated peptide
<400> 427
   Ile Ile Val Thr Val Leu Ala Gly Lys Asn Leu Val Ser Lys Asp Lys
   1 5 10 15
   Ser Gly Lys Cys Asp Ala Ser Val Lys Leu Gln Tyr Gly Lys Ile Ile
   20 25 30
   Gln Lys Thr Lys Ile Val Asn Ala Ala Glu Cys Val Trp Asn Gln Lys
   35 40 45
   Phe Glu Phe Glu Glu Leu Ala Gly Glu Glu Tyr Leu Lys Val Lys Cys
   50 55 60
   Tyr Arg Glu Glu Met Leu Gly Thr Asp Asn Ile Gly Thr Ala Thr
   65 70 75
<210> 428
   <211> 100
   <212> PRT
   <213> Artificial sequence
<220>
   <223> Synthetically generated peptide
<400> 428
   Ile Tyr Val Thr Val Val Ser Gly Asn Asn Leu Asn Arg Arg Ile Leu
   1               5                   10                  15
   Arg Gly Ser Pro Ser Lys Ser Ser Glu Ile Gly Glu Gly Ser Ser Gly
               20                  25                  30
   Asn Ser Ser Ser Lys Pro Val Gln Thr Phe Val Glu Val Glu Leu Glu
           35                  40                  45
   Gln Leu Ser Arg Arg Thr Glu Met Lys Ser Gly Pro Asn Pro Ala Tyr
       50                  55                  60
   Gln Ser Thr Phe Asn Met Ile Leu His Asp Asn Thr Gly Thr Leu Lys
   65                  70                  75                  80
   Phe Asn Leu Tyr Glu Asn Asn Pro Gly Ser Val Arg Tyr Asp Ser Leu
                   85                  90                  95
   Ala Ser Cys Glu
               100
<210> 429
   <211> 100
   <212> PRT
   <213> Artificial Sequence
<220>
   <223> Synthetically generated peptide
<400> 429
   Ile Tyr Val Thr Val Val Ser Gly Asn Asn Leu Asn Arg Arg Ile Leu
   1               5                   10                  15
   Arg Gly Ser Pro Ser Lys Ser Ser Glu Ile Gly Glu Gly Ser Ser Gly
               20                  25                  30
   Asn Ser Ser Ser Lys Pro Val Gln Thr Phe Val Glu Val Glu Leu Glu
           35                  40                  45
   Gln Leu Ser Arg Arg Thr Glu Met Lys Ser Gly Pro Asn Pro Ala Tyr
       50                  55                  60
   Gln Ser Thr Phe Asn Met Ile Leu His Asp Asn Thr Gly Thr Leu Lys
   65                  70                  75                  80
   Phe Asn Leu Tyr Glu Asn Asn Pro Gly Ser Val Arg Tyr Asp Ser Leu
                   85                  90                  95
   Ala Ser Cys Glu
               100
<210> 430
   <211> 80
   <212> PRT
   <213> Artificial Sequence
<220>
   <223> Synthetically generated peptide
<400> 430
   Val Lys Leu Glu Ile Gly Ser Asp Met Lys Pro Ser Asp Met Asn Gly
   1               5                   10                  15
   Leu Ser Asp Pro Tyr Val Lys Gly Arg Leu Gly Pro Phe Lys Phe Gln
               20                  25                  30
   Thr Gln Ile Gln Lys Lys Thr Leu Ser Pro Lys Trp Phe Glu Glu Phe
           35                  40                  45
   Lys Ile Pro Ile Thr Ser Trp Glu Ser Leu Asn Glu Leu Ala Met Glu
       50                  55                  60
   Val Cys Asp Lys Asp His Met Phe Asp Asp Ser Leu Gly Thr Cys Thr
   65                  70                  75                  80
<210> 431
   <211> 80
   <212> PRT
   <213> Artificial Sequence
<220>
   <223> synthetically generated peptide
<400> 431
   Ala Lys Val Glu Ile Gly Ala Asp Met Lys Pro Ser Asp Pro Asn Gly
   1               5                   10                  15
   Leu Ala Asp Pro Tyr Val Lys Gly His Leu Gly Pro Tyr Arg Phe Gln
               20                  25                  30
   Thr Lys Ile His Lys Lys Thr Leu Asn Pro Lys Trp Met Glu Glu Phe
           35                  40                  45
   Lys Ile Pro Val Thr Ser Trp Ala Ala Leu Asn Leu Leu Ser Leu Gln
       50                  55                  60
   Val Arg Asp Lys Asp Pro Ile Phe Asp Asp Thr Leu Gly Asp Cys Ser
   65                  70                  75                  80
<210> 432
   <211> 82
   <212> PRT
   <213> Artificial Sequence
<220>
   <223> Synthetically generated peptide
<400> 432
   Ala Leu Val Glu Val Val Glu Ala Cys Asp Val Lys Pro Ser Asp Leu
   1               5                   10                  15
   Asn Gly Leu Ala Asp Pro Tyr Val Lys Gly Gln Leu Gly Ala Tyr Arg
               20                  25                  30
   Phe Lys Thr Lys Ile Leu Trp Lys Thr Leu Ala Pro Lys Trp Gln Glu
           35                  40                  45
   Glu Phe Lys Ile Pro Ile Cys Thr Trp Asp Ser Ala Asn Ile Leu Asn
       50                  55                  60
   Ile Glu Val Gln Asp Lys Asp Arg Phe Ser Asp Asp Ser Leu Gly Asp
   65                  70                  75                  80
   Cys Ser
<210> 433
   <211> 82
   <212> PRT
   <213> Artificial Sequence
<220>
   <223> Synthetically generated peptide
<400> 433
   Ala Leu Val Glu Val Val Glu Ala Cys Asp Val Lys Pro Ser Asp Leu
   1               5                   10                  15
   Asn Gly Leu Ala Asp Pro Tyr Val Lys Gly Gln Leu Gly Ala Tyr Arg
               20                  25                  30
   Phe Lys Thr Lys Ile Leu Trp Lys Thr Leu Ala Pro Lys Trp Gln Glu
           35                  40                  45
   Glu Phe Lys Ile Pro Ile Cys Thr Trp Asp Ser Ala Asn Ile Leu Asn
       50                  55                  60
   Ile Glu Val Gln Asp Lys Asp Arg Phe Ser Asp Asp Ser Leu Gly Asp
   65                  70                  75                  80
   Cys Ser
<210> 434
   <211> 82
   <212> PRT
   <213> Artificial Sequence
<220>
   <223> Synthetically generated peptide
<400> 434
   Val Leu Val Glu Val Phe Glu Ala Ser Asp Leu Lys Pro Ser Asp Leu
   1               5                   10                  15
   Asn Gly Leu Ala Asp Pro Tyr Val Lys Gly Lys Leu Gly Ala Tyr Arg
               20                  25                  30
   Phe Lys Thr Lys Ile Gln Lys Lys Thr Leu Ser Pro Lys Trp His Glu
           35                  40                  45
   Glu Phe Lys Ile Pro Ile Phe Thr Trp Asp Ser Pro Ser Ile Leu Asn
       50                  55                  60
   Ile Glu Val Gly Asp Lys Asp Arg Phe Val Asp Asp Thr Leu Gly Glu
   65                  70                  75                  80
   Cys Ser
<210> 435
   <211> 82
   <212> PRT
   <213> Artificial sequence
<220>
   <223> Synthetically generated peptide
<400> 435
   Val Leu Val Glu Val Phe Glu Ala Ser Asp Leu Lys Pro Ser Asp Leu
   1 5 10 15
   Asn Gly Leu Ala Asp Pro Tyr Val Lys Gly Lys Leu Gly Ala Tyr Arg
   20 25 30
   Phe Lys Thr Lys Ile Gln Lys Lys Thr Leu Ser Pro Lys Trp His Glu
   35 40 45
   Glu Phe Lys Ile Pro Ile Phe Thr Trp Asp Ser Pro Ser Ile Leu Asn
   50 55 60
   Ile Glu Val Gly Asp Lys Asp Arg Phe Val Asp Asp Thr Leu Gly Glu
   65 70 75 80
   Cys Ser
<210> 436
   <211> 82
   <212> PRT
   <213> Artificial Sequence
<220>
   <223> Synthetically generated peptide
<400> 436
   Val Leu Val Glu Val Phe Glu Ala Ser Asp Leu Lys Pro Ser Asp Leu
   1 5 10 15
   Asn Gly Leu Ala Asp Pro Tyr Val Lys Gly Lys Leu Gly Ala Tyr Arg
   20 25 30
   Phe Lys Thr Lys Ile Gln Lys Lys Thr Leu Ser Pro Lys Trp His Glu
   35 40 45
   Glu Phe Lys Ile Pro Ile Phe Thr Trp Asp Ser Pro Ser Ile Leu Asn
   50 55 60
   Ile Glu Val Gly Asp Lys Asp Arg Phe Val Asp Asp Thr Leu Gly Phe
   65 70 75 80
   Ala Pro
<210> 437
   <211> 79
   PRT
   <213> Artificial Sequence
<220>
   <223> Synthetically generated peptide
<400> 437
   Leu Cys Val His Val Leu Glu Arg Leu Gln Ala Ala Tyr Leu Thr Gly
   1 5 10 15
   His Ser Asp Pro Tyr Val Arg Leu Gln Met Gly Arg Arg Arg Ala Lys
   20 25 30
   Thr Thr Val Val Lys Arg Cys Leu Ser Pro Leu Trp Asp Glu Glu Phe
   35 40 45
   Gly Phe Ala Val Gly Asp Ala Glu Glu Glu Leu Val Val Ser Val Leu
   50 55 60
   Asn Glu Glu Gly Tyr Phe Gly Gly Gly Phe Leu Gly Arg Val Lys
   65 70 75
<210> 438
   <211> 79
   <212> PRT
   <213> Artificial sequence
<220>
   <223> Synthetically generated peptide
<400> 438
   Leu Gln Val Arg Val Val Glu Arg Leu Pro Ala Val Arg Val Asp Gly
    1               5                  10                  15
   Thr Ser Asp Pro Phe Val Lys Leu Gln Leu Gly Lys Arg Arg Ala Lys
               20                  25                  30
   Thr Ala Val Ala Arg Arg Thr Leu Ala Pro Ala Trp Asp Glu Glu Phe
           35                  40                  45
   Ser Phe Leu Val Gly Asp Ile Ala Glu Glu Leu Val Val Ser Val Leu
       50                  55                  60
   Asn Glu Asp Lys Tyr Phe Ser Asn Asp Leu Leu Gly Lys Val Arg
   65                  70                  75
<210> 439
   <211> 81
   <212> PRT
   <213> Artificial sequence
<220>
   <223> Synthetically generated peptide
<400> 439
   Leu Gln Val Arg Val Val Glu Ala Arg Asn Leu Pro Ala Met Asp Leu
    1               5                  10                  15
   Asn Gly Phe Ser Asp Pro Tyr Val Arg Leu Gln Leu Gly Lys Gln Arg
               20                  25                  30
   Ser Arg Thr Lys Val Val Lys Lys Asn Leu Asn Pro Lys Trp Thr Glu
           35                  40                  45
   Asp Phe Ser Phe Gly Val Asp Asp Leu Asn Asp Glu Leu Val Val Ser
       50                  55                  60
   Val Leu Asp Glu Asp Lys Tyr Phe Asn Asp Asp Phe Val Gly Gln Val
   65                  70                  75                  80
   Arg
<210> 440
   <211> 81
   <212> PRT
   <213> Artificial Sequence
<220>
   <223> Synthetically generated peptide
<400> 440
   Leu Asn Val Arg Val Ile Glu Ala Arg Asn Leu Arg Ala Met Asp Ser
    1               5                  10                  15
   Asn Gly Phe Ser Asp Pro Tyr Val Lys Leu Gln Leu Gly Lys Gln Arg
               20                  25                  30
   Phe Lys Thr Lys Val Val Lys Lys Asn Leu Asn Pro Ala Trp Asp Gln
           35                  40                  45
   Glu Phe Ser Phe Ser Val Gly Asp Val Arg Asp Val Leu Lys Leu Tyr
       50                  55                  60
   Val Tyr Asp Glu Asp Met Ile Gly Ile Asp Asp Phe Leu Gly Gln Val
   65                  70                  75                  80
   Lys
<210> 441
   <211> 82
   <212> PRT
   <213> Artificial Sequence
<220>
   <223> Synthetically generated peptide
<400> 441
   Leu Leu Val Gln Val Ser Glu Ala Arg Asn Leu Pro Ala Ile Asp Gly
    1               5                  10                  15
   Gly Gly Gly Leu Ser Asp Pro Tyr Ala Lys Leu Gln Leu Gly Arg Gln
               20                  25                  30
   Arg Gly Lys Thr Arg Val Ala Lys Arg Thr Leu Ser Pro Thr Trp Asp
           35                  40                  45
   Glu Glu Phe Ala Phe Arg Val Val Asp Leu Lys Asp Glu Leu Val Val
       50                  55                  60
   Val Val Val Asp Glu Asp Arg Tyr Phe Ser Asp Asp Phe Leu Gly Gln
   65                  70                  75                  80
   Val Arg
<210> 442
   <211> 65
   <212> PRT
   <213> Artificial Sequence
<220>
   <223> Synthetically generated peptide
<400> 442
   Leu Cys Val Ser Val Ile Glu Ala Arg Asn Val Gln Ala Asp Asp Phe
    1               5                  10                  15
   Ala Gly Lys Asn Phe Tyr Val Lys Leu Arg Val Gly Ser Asn Glu Val
               20                  25                  30
   Lys Thr Asp Val Val Lys Gly Ser Leu Ala Pro Lys Phe Leu Lys Asp
           35                  40                  45
   Cys Arg Leu Ala Val Pro Thr Pro Glu Ser Asp Tyr Leu Arg Ile Glu
       50                  55                  60
   Leu
   65
<210> 443
   <211> 81
   <212> PRT
   <213> Artificial Sequence
<220>
   <223> Synthetically generated peptide
<400> 443
   Val Lys Val Glu Leu Leu Ala Ala Lys Asn Leu Ile Gly Ala Asn Leu
   1               5                   10                  15
   Asn Gly Thr Ser Asp Pro Tyr Ala Ile Val Asn Cys Gly Ser Glu Lys
               20                  25                  30
   Arg Phe Ser Ser Met Val Pro Gly Ser Arg Asn Pro Met Trp Gly Glu
           35                  40                  45
   Glu Phe Asn Phe Pro Thr Asp Glu Leu Pro Ala Lys Ile Asn Val Thr
       50                  55                  60
   Ile His Asp Trp Asp Ile Ile Trp Lys Ser Thr Val Leu Gly Ser Val
   65                  70                  75                  80
   Thr
<210> 444
   <211> 81
   <212> PRT
   <213> Artificial Sequence
<220>
   <223> Synthetically generated peptide
<400> 444
   Val Lys Val Glu Leu Leu Ala Ala Lys Asn Leu Ile Gly Ala Asn Leu
   1               5                   10                  15
   Asn Gly Thr Ser Asp Pro Tyr Ala Ile Val Asn Cys Gly Ser Glu Lys
               20                  25                  30
   Arg Phe Ser Ser Met Val Pro Gly Ser Arg Asn Pro Met Trp Gly Glu
           35                  40                  45
   Glu Phe Asn Phe Pro Thr Asp Glu Leu Pro Ala Lys Ile Asn Val Thr
       50                  55                  60
   Ile His Asp Trp Asp Ile Ile Trp Lys Ser Thr Val Leu Gly Ser Val
   65                  70                  75                  80
   Thr
<210> 445
   <211> 81
   <212> PRT
   <213> Artificial sequence
<220>
   <223> Synthetically generated peptide
<400> 445
   Val Lys Leu Glu Leu Leu Ala Ala Lys Asn Leu Ile Ala Ala Asn Leu
   1               5                   10                  15
   Asn Gly Thr Ser Asp Pro Tyr Ala Leu Ile Thr Cys Gly Glu Glu Lys
               20                  25                  30
   Arg Phe Ser Ser Met Val Pro Gly Ser Arg Asn Pro Met Trp Gly Glu
           35                  40                  45
   Glu Phe Asn Phe Phe Val Asp Ser Leu Pro Val Lys Ile Asn Val Thr
       50                  55                  60
   Ile Tyr Asp Trp Asp Ile Val Trp Lys Ser Thr Val Leu Gly Ser Val
   65                  70                  75                  80
   Ile
<210> 446
   <211> 91
   <212> PRT
   <213> Artificial sequence
<220>
   <223> Synthetically generated peptide
<400> 446
   Ile Lys Leu Glu Leu Leu Cys Ala Lys Tyr Leu Ile Gly Ala Asn Leu
   1               5                   10                  15
   Asn Gly Ser Ser Asp Pro Tyr Ala Val Ile Ser Cys Gly Glu Gln Arg
               20                  25                  30
   Arg Phe Ser Ser Met Val Pro Ser Ser Arg Asn Pro Leu Trp Gly Glu
           35                  40                  45
   Glu Phe Asn Phe Leu Val Arg Glu Leu Pro Val Glu Phe Cys Thr Ala
       50                  55                  60
   Pro Val Asn Asp Ser Lys Val Thr Ile Thr Met Tyr Asp Trp Asp Thr
   65                  70                  75                  80
   Val Cys Lys Cys Lys Val Ile Gly Ser Val Thr
                   85                  90
<210> 447
   <211> 31
   <212> PRT
   <213> Artificial Sequence
<220>
   <223> Synthetically generated peptide
<400> 447
   Leu Glu Val His Leu Leu Glu Ala His Gly Leu Leu Asp Thr Asp Thr
   1               5                   10                  15
   Phe Gly Lys Ser Asp Pro Tyr Ala Ile Val Tyr Cys Gln Lys Glu
               20                  25                  30
<210> 448
   <211> 79
   <212> PRT
   <213> Artificial Sequence
<220>
   <223> Synthetically generated peptide
<400> 448
   Ile Glu Leu Val Leu Val Glu Ala Arg Asp Leu Val Ala Ala Asp Ile
   1               5                   10                  15
   Arg Gly Thr Ser Asp Pro Tyr Val Arg Val Gln Tyr Gly Glu Lys Lys
               20                  25                  30
   Gln Arg Thr Lys Val Ile Tyr Lys Thr Leu Gln Pro Lys Trp Asn Gln
           35                  40                  45
   Thr Met Glu Phe Pro Asp Asp Gly Ser Ser Leu Glu Leu His Val Lys
       50                  55                  60
   Asp Tyr Asn Thr Leu Leu Pro Thr Ser Ser Ile Gly Asn Cys Val
   65                  70                  75
<210> 449
   <211> 79
   <212> PRT
   <213> Artificial sequence
<220>
   <223> Synthetically generated peptide
<400> 449
   Ile Glu Leu Val Leu Val Glu Ala Arg Asp Leu Val Ala Ala Asp Ile
   1               5                   10                  15
   Arg Gly Thr Ser Asp Pro Tyr Val Arg Val Gln Tyr Gly Glu Lys Lys
               20                  25                  30
   Gln Arg Thr Lys Val Ile Tyr Lys Thr Leu Gln Pro Lys Trp Asn Gln
           35                  40                  45
   Thr Met Glu Phe Pro Asp Asp Gly Ser Ser Leu Glu Leu His Val Lys
       50                  55                  60
   Asp Tyr Asn Thr Leu Leu Pro Thr Ser Ser Ile Gly Asn Cys Val
   65                  70                  75
<210> 450
   <211> 83
   <212> PRT
   <213> Artificial Sequence
<220>
   <223> Synthetically generated peptide
<400> 450
   Leu Val Val Ile Val His Glu Ala Gln Asp Val Glu Gly Lys His His
   1 5 10 15
   Thr Asn Pro Gln Ala Arg Leu Ile Phe Arg Gly Glu Glu Lys Lys Thr
   20 25 30
   Lys Arg Ile Lys Lys Asn Arg Asp Pro Arg Trp Glu Asp Glu Phe Gln
   35 40 45
   Phe Ile Ala Glu Glu Pro Pro Thr Asn Asp Lys Leu His Val Glu Val
   50 55 60
   Val Ser Ser Ser Ser Arg Thr Leu Leu His Gln Lys Glu Ser Leu Gly
   65 70 75 80
   Tyr Val Asp
<210> 451
   <211> 83
   <212> PRT
   <213> Artificial Sequence
<220>
   <223> Synthetically generated peptide
<400> 451
   Leu Val Val Ile Val His Glu Ala Gln Asp Val Glu Gly Lys His His
   1 5 10 15
   Thr Asn Pro Tyr Val Arg Ile Val Phe Arg Gly Glu Glu Arg Lys Thr
   20 25 30
   Lys His Ile Lys Lys Asn Arg Asp Pro Arg Trp Glu Gln Glu Phe Gln
   35 40 45
   Phe Val Cys Glu Glu Pro Pro Ile Asn Asp Lys Met Gln Ile Glu Val
   50 55 60
   Ile Ser Arg Pro Pro Ser Ile Gly Ile His Ser Lys Glu Asn Leu Gly
   65 70 75 80
   Tyr Val Val
<210> 452
   <211> 82
   <212> PRT
   <213> Artificial Sequence
<220>
   <223> Synthetically generated peptide
<400> 452
   Leu Val Val Ile Val His Glu Ala Glu Asp Leu Glu Gly Lys Tyr His
   1 5 10 15
   Thr Asn Pro Ser Val Arg Leu Leu Phe Arg Gly Glu Glu Arg Lys Thr
   20 25 30
   Lys Arg Val Lys Lys Asn Arg Glu Pro Arg Trp Asp Glu Asp Phe Gln
   35 40 45
   Phe Pro Leu Asp Glu Pro Pro Ile Asn Asp Lys Leu His Val Glu Val
   50 55 60
   Ile Ser Ser Ser Ser Arg Leu Ile His Pro Lys Glu Thr Leu Gly Tyr
   65 70 75 80
   Val Val
<210> 453
   <211> 65
   <212> PRT
   <213> Artificial Sequence
<220>
   <223> Synthetically generated peptide
<400> 453
   Phe Val Val Ile Val His Ser Ala Glu Asp Val Glu Gly Lys His His
    1               5                  10                  15
   Thr Asn Pro Tyr Val His Ile Tyr Phe Lys Gly Glu Glu Arg Lys Thr
               20                  25                  30
   Lys His Val Lys Lys Asn Lys Asp Pro Lys Trp Asn Glu Glu Phe Ser
           35                  40                  45
   Phe Met Leu Glu Glu Pro Pro Ile His Glu Lys Met His Val Lys Val
       50                  55                  60
   Phe
   65
<210> 454
   <211> 65
   <212> PRT
   <213> Artificial Sequence
<220>
   <223> Synthetically generated peptide
<400> 454
   Phe Val Val Ile Val His Ser Ala Glu Asp Val Glu Gly Lys His His
    1               5                  10                  15
   Thr Asn Pro Tyr Val His Ile Tyr Phe Lys Gly Glu Glu Arg Lys Thr
               20                  25                  30
   Lys His Val Lys Lys Asn Lys Asp Pro Lys Trp Asn Glu Glu Phe Ser
           35                  40                  45
   Phe Met Leu Glu Glu Pro Pro Ile His Glu Lys Met His Val Lys Val
       50                  55                  60
   Phe
   65
<210> 455
   <211> 65
   <212> PRT
   <213> Artificial sequence
<220>
   <223> synthetically generated peptide
<400> 455
   Leu Val Val Ile Val His Ser Ala Glu Asp Val Glu Gly Lys His His
   1               5                   10                  15
   Thr Asn Pro Tyr Val His Ile Tyr Phe Lys Gly Glu Glu Arg Lys Thr
               20                  25                  30
   Lys Asn Val Lys Lys Asn Lys Asp Pro Lys Trp Asn Glu Glu Phe Ser
           35                  40                  45
   Phe Met Leu Glu Glu Pro Pro Val His Glu Lys Leu His Val Glu Val
       50                  55                  60
   Phe
   65
<210> 456
   <211> 84
   <212> PRT
   <213> Artificial Sequence
<220>
   <223> Synthetically generated peptide
<220>
   <221> VARIANT
   <222> 58
   <223> xaa = any amino acid
<400> 456
   Leu Ala Val Ile Val His Ser Ala Glu Asp Val Glu Gly Lys His His
   1               5                   10                  15
   Thr Asn Pro Tyr Val Arg Ile Tyr Phe Lys Gly Glu Glu Arg Lys Thr
               20                  25                  30
   Lys His Val Lys Lys Asn Arg Asp Pro Arg Trp Glu Glu Glu Phe Thr
           35                  40                  45
   Phe Met Leu Glu Glu Pro Pro Val Arg Xaa Lys Leu His Val Glu Val
       50                  55                  60
   Leu Ser Thr Ser Ser Arg Ile Gly Leu Leu His Pro Lys Glu Thr Leu
   65                  70                  75                  80
   Gly Tyr Val Asp
<210> 457
   <211> 84
   <212> PRT
   <213> Artificial Sequence
<220>
   <223> Synthetically generated peptide
<400> 457
   Leu Val Val Ile Val His Ser Ala Glu Asp Val Glu Gly Lys His His
   1               5                   10                  15
   Thr Asn Pro Tyr Val Arg Ile Tyr Phe Lys Gly Glu Glu Arg Lys Thr
               20                  25                  30
   Lys His Val Lys Lys Asn Arg Asp Pro Arg Trp Asn Glu Glu Phe Thr
           35                  40                  45
   Phe Met Leu Glu Glu Pro Pro Val Arg Glu Lys Leu His Val Glu Val
       50                  55                  60
   Leu Ser Thr Ser Ser Arg Ile Gly Leu Leu His Pro Lys Glu Thr Leu
   65 70 75 80
   Gly Tyr Val Asp
<210> 458
   <211> 84
   <212> PRT
   <213> Artificial Sequence
<220>
   <223> Synthetically generated peptide
<400> 458
   Leu Tyr Val Val Val His Glu Ala Gln Asp Leu Glu Gly Lys His His
   1 5 10 15
   Thr Asn Pro Tyr Ala Lys Ile Ile Phe Lys Gly Glu Glu Lys Lys Thr
   20 25 30
   Lys Val Ile Lys Lys Asn Arg Asp Pro Arg Trp Glu Asp Glu Phe Glu
   35 40 45
   Phe Val Cys Glu Glu Pro Pro Val Asn Asp Lys Leu His Ile Glu Val
   50 55 60
   Leu Ser Lys Ala Ser Lys Lys Gly Leu Ile His Gly Lys Glu Thr Leu
   65 70 75 80
   Gly Tyr Ile Asp
<210> 459
   <211> 64
   <212> PRT
   <213> Artificial Sequence
<220>
   <223> Synthetically generated peptide
<400> 459
   Leu Tyr Val Val Val His Glu Ala Lys Glu Leu Glu Gly Lys Cys Asn
   1 5 10 15
   Thr Asn Pro Tyr Val Lys Leu Thr Phe Lys Gly Val Glu Lys Lys Thr
   20 25 30
   Lys Val Val Lys Glu Asn Arg Asn Pro Arg Trp Lys Glu Glu Phe Glu
   35 40 45
   Phe Glu Cys Glu Glu Thr Pro Ala Asn Asp Lys Leu His Val Glu Val
   50 55 60
<210> 460
   <211> 84
   <212> PRT
   <213> Artificial Sequence
<220>
   <223> Synthetically generated peptide
<400> 460
   Leu Ser Val Ala Val Gln Ser Ala Lys Asp Val Glu Gly Lys Lys Lys
   1 5 10 15
   His Ser Asn Pro Tyr Ala Val Val Leu Phe Arg Gly Glu Lys Lys Lys
   20 25 30
   Thr Lys Met Leu Lys Lys Thr Arg Asp Pro Arg Trp Asn Glu Glu Phe
   35 40 45
   Gln Phe Thr Leu Glu Glu Pro Pro Val Lys Glu Ser Ile Arg Val Glu
   50 55 60
   Val Met Ser Lys Gly Thr Gly Phe His Phe Arg Ser Lys Glu Glu Leu
   65 70 75 80
   Gly His Val Asp
<210> 461
   <211> 84
   <212> PRT
   <213> Artificial sequence
<220>
   <223> Synthetically generated peptide
<400> 461
   Leu Ser Val Ala Val Gln Ser Ala Lys Asp Val Glu Gly Lys Lys Lys
   1 5 10 15
   His Ser Asn Pro Tyr Ala Val Val Leu Phe Arg Gly Glu Lys Lys Lys
   20 25 30
   Thr Lys Met Leu Lys Lys Thr Arg Asp Pro Arg Trp Asn Glu Glu Phe
   35 40 45
   Gln Phe Thr Leu Glu Glu Pro Pro Val Lys Glu Ser Ile Arg Val Glu
   50 55 60
   Val Met Ser Lys Gly Thr Gly Phe His Phe Arg Ser Lys Glu Glu Leu
   65 70 75 80
   Gly His Val Asp
<210> 462
   <211> 84
   <212> PRT
   <213> Artificial sequence
<220>
   <223> Synthetically generated peptide
<400> 462
   Leu Ser Val Ala Val Gln Ser Ala Lys Asp Val Glu Gly Lys Lys Lys
   1 5 10 15
   His Ser Asn Pro Tyr Ala Val Val Leu Phe Arg Gly Glu Lys Lys Lys
   20 25 30
   Thr Lys Met Leu Lys Lys Thr Arg Asp Pro Arg Trp Asn Glu Glu Phe
   35 40 45
   Gln Phe Thr Leu Glu Glu Pro Pro Val Lys Glu Ser Ile Arg Val Glu
   50 55 60
   Val Met Ser Lys Gly Thr Gly Phe His Phe Arg Ser Lys Glu Glu Leu
   65 70 75 80
   Gly His Val Asp
<210> 463
   <211> 85
   <212> PRT
   <213> Artificial Sequence
<220>
   <223> Synthetically generated peptide
<400> 463
   Leu Leu Val Ser Val Glu Asn Ala Glu Asp Val Glu Gly Lys Arg His
   1               5                   10                  15
   Thr Asn Pro Tyr Ala Val Val His Phe Arg Gly Glu Arg Lys Glu Thr
               20                  25                  30
   Lys Ile Ile Lys Lys Thr Arg Asp Pro Arg Trp Asn Glu Glu Phe Gln
           35                  40                  45
   Phe Met Val Asp Glu Ala Pro Val Asp Asp Lys Ile His Ile Glu Val
       50                  55                  60
   Val Ser Lys Arg Arg Gly Leu Arg Leu Pro Phe Arg Asn Lys Glu Ser
   65                  70                  75                  80
   Leu Gly His Val Asp
                   85
<210> 464
   <211> 87
   <212> PRT
   <213> Artificial Sequence
<220>
   <223> Synthetically generated peptide
<400> 464
   Leu Gly Val Glu Val Ala Ser Ala His Asp Leu Met Pro Lys Asp Gly
   1               5                   10                  15
   Gln Gly Ser Ala Ser Ala Cys Val Glu Leu Thr Phe Asp Gly Gln Arg
               20                  25                  30
   Phe Arg Thr Ala Ile Lys Asp Lys Asp Leu Asn Pro Val Trp Asn Glu
           35                  40                  45
   Arg Phe Tyr Phe Asn Val Ser Asp Pro Ser Asn Leu Pro Glu Leu Ala
       50                  55                  60
   Leu Glu Ala Tyr Val Tyr Asn Ile Asn Arg Ser Ile Asp Gly Ser Arg
   65                  70                  75                  80
   Ser Phe Leu Gly Lys Val Arg
                   85
<210> 465
   <211> 87
   <212> PRT
   <213> Artificial Sequence
<220>
   <223> Synthetically generated peptide
<400> 465
   Leu Gly Val Glu Val Ala Ser Ala His Asp Leu Met Pro Lys Asp Gly
   1 5 10 15
   Gln Gly Ser Ala Ser Ala Cys Val Glu Leu Thr Phe Asp Gly Gln Arg
   20 25 30
   Phe Arg Thr Ala Ile Lys Asp Lys Asp Leu Asn Pro Val Trp Asn Glu
   35 40 45
   Arg Phe Tyr Phe Asn Val Ser Asp Pro Ser Asn Leu Pro Glu Leu Ala
   50 55 60
   Leu Glu Ala Tyr Val Tyr Asn Ile Asn Arg Ser Val Asp Gly Ser Arg
   65 70 75 80
   Ser Phe Leu Gly Lys Val Arg
   85
<210> 466
   <211> 86
   <212> PRT <213> Artificial Sequence
<220>
   <223> Synthetically generated peptide
<400> 466
   Leu Gly Val Asp Val Ile Gly Ala His Asn Leu Phe Pro Lys Asp Gly
   1 5 10 15
   Gln Gly Thr Ser Asn Ala Tyr Val Glu Leu Tyr Phe Asp Gly Gln Lys
   20 25 30
   His Arg Thr Thr Ile Lys Asp Arg Asp Leu Asn Pro Val Trp Asn Glu
   35 40 45
   Ser Phe Phe Phe Asn Ile Ser Asp Pro Ser Arg Leu His Tyr Leu Asn
   50 55 60
   Leu Glu Ala Gln Ala Tyr Ser His Asn Arg Ser Thr Asn Gly Arg Ser
   65 70 75 80
   Phe Leu Gly Lys Val Ser
   85
<210> 467
   <211> 86
   <212> PRT
   <213> Artificial Sequence
<220>
   <223> Synthetically generated peptide
<400> 467
   Leu Gly Val Asp Val Ile Gly Ala His Asn Leu Phe Pro Lys Asp Gly
   1 5 10 15
   Gln Gly Thr Ser Asn Ala Tyr Val Glu Leu Tyr Phe Asp Gly Gln Lys
   20 25 30
   His Arg Thr Thr Ile Lys Asp Arg Asp Leu Asn Pro Val Trp Asn Glu
   35 40 45
   Ser Phe Phe Phe Asn Ile Ser Asp Pro Ser Arg Leu His Tyr Leu Asn
   50 55 60
   Leu Glu Ala Gln Ala Tyr Ser His Asn Arg Ser Thr Asn Gly Arg Ser
   65 70 75 80
   Phe Leu Gly Lys Val Ser
   85
<210> 468
   <211> 83
   <212> PRT
   <213> Artificial Sequence
<220>
   <223> Synthetically generated peptide
<400> 468
   Leu Gly Val Glu Val Thr Ser Ala His Asp Leu Leu Pro Lys Glu Gln
    1               5                   10                  15
   Gly Thr Cys Asn Pro Tyr Val Glu Ile Glu Phe Asp Asp Gln Lys Phe
               20                  25                  30
   Arg Thr Ala Ile Lys Glu Arg Asp Ile Asn Pro Val Trp Asn Glu Gln
           35                  40                  45
   Phe Tyr Phe Asn Ile Ser Asp Pro Ser Arg Leu Thr Glu Lys Asp Leu
       50                  55                  60
   Glu Ala Tyr Val Tyr His Ala Asn Arg Asn Ser Lys Thr Cys Leu Gly
   65                  70                  75                  80
   Lys Val Arg
<210> 469
   <211> 86
   <212> PRT
   <213> Artificial Sequence
<220>
   <223> synthetically generated peptide
<400> 469
   Leu Cys Val Glu Val Val Gly Ala His Asp Leu Val Ala Lys Asp Gly
    1               5                   10                  15
   Glu Gly Ser Ser Thr Thr Phe Val Glu Leu Glu Phe Asp Asp Gln Lys
               20                  25                  30
   Phe Arg Thr Thr Thr Lys Asp Lys Asp Leu Ser Pro Tyr Trp Asn Glu
           35                  40                  45
   Ile Phe Tyr Phe Asn Ile Thr Asp Pro Ser Lys Leu Ser Asn Leu Asn
       50                  55                  60
   Leu Glu Ala Cys Ile Asn His Tyr Asn Lys Thr Asn Gly Ser Lys Ile
   65                  70                  75                  80
   Pro Leu Gly Lys Val Lys
                   85
<210> 470
   <211> 59
   <212> PRT
   <213> Artificial Sequence
<220>
   <223> Synthetically generated peptide
<400> 470
   Leu Gly Val Glu Val Ile Ser Ala Gln Gly Leu Leu Gln Arg Asp Lys
   1               5                   10                  15
   His Asn Ser Cys Ser Pro Phe Val Glu Leu Lys Phe Asp Asn Gln Ile
               20                  25                  30
   Phe Arg Ala Thr Thr Lys His Asn Asp Pro Asn Pro Val Trp His Glu
           35                  40                  45
   Cys Phe Tyr Phe Val Val Ser Asp Pro Ser Val
       50                  55
<210> 471
   <211> 86
   <212> PRT
   <213> Artificial Sequence
<220>
   <223> Synthetically generated peptide
<400> 471
   Leu Gly Val Glu Val Ile Ser Ala Gln Gly Leu Leu Gln Arg Asp Lys
   1               5                   10                  15
   His Asn Ser Cys Ser Pro Phe Val Glu Leu Lys Phe Asp Asn Gln Ile
               20                  25                  30
   Phe Arg Ala Thr Thr Lys His Asn Asp Pro Asn Pro Val Trp His Glu
           35                  40                  45
   Cys Phe Tyr Phe Val Val Ser Asp Pro Ser Val Leu Ser Thr Arg Thr
       50                  55                  60
   Leu Glu Ala His Val Tyr Ser Tyr Gln Asn Glu Phe Asp Ala Lys Pro
   65                  70                  75                  80
   Phe Leu Gly Lys Val Arg
                   85
<210> 472
   <211> 84
   <212> PRT
   <213> Artificial Sequence
<220>
   <223> Synthetically generated peptide
<400> 472
   Leu Gly Val Glu Val Ile Ser Ala Arg Leu Lys Pro Arg Glu Asp Tyr
   1               5                   10                  15
   Gly Gly Val Asn Ala Tyr Val Glu Leu Arg Phe Asp Asp Gln Lys Val
               20                  25                  30
   Ile Thr Met Thr Lys Ile Asp Asp Ser Ser Pro Val Trp Asn Glu Lys
           35                  40                  45
   Phe Phe Phe Asn Ile Ser Asp Thr Glu Asp Leu Ser Asn Gln Phe Leu
       50                  55                  60
   Asp Ala Tyr Val Tyr Asn Lys Thr Ser Ser Ile Thr Lys Ser Cys Leu
   65                  70                  75                  80
   Gly Lys Ile Arg
<210> 473
   <211> 82
   <212> PRT
   <213> Artificial Sequence
<220>
   <223> Synthetically generated peptide
<400> 473
   Leu Val Val Glu Val Ile Ser Ala Asp Ile Pro Ser Ser Ser Asn Thr
    1               5                  10                  15
   Ser Gln Thr Asn Tyr Ser Val Glu Leu His Phe Asn Ser Gln Ser Lys
               20                  25                  30
   Ser Thr Thr Ile Lys Glu Asn Val Ala Val Trp Asn Glu Arg Phe Ser
           35                  40                  45
   Phe Asp Met Arg Gln Arg Glu Asp Pro Ser Gly Asn Leu Ile Ala Ala
       50                  55                  60
   Val Tyr Cys Phe Asp Gln Met Ser Asn Ser Lys Ser Leu Leu Gly Lys
   65                  70                  75                  80
   Val Leu
<210> 474
   <211> 92
   <212> PRT
   <213> Artificial Sequence
<220>
   <223> Synthetically generated peptide
<400> 474
   Val Val Val Glu Val Cys Asn Ala Arg Asn Leu Met Pro Lys Asp Gly
    1               5                  10                  15
   Gln Gly Thr Ala Ser Ala Tyr Ala Val Val Asp Phe Asp Gly Gln Arg
               20                  25                  30
   Arg Arg Thr Ala Trp Arg Asp Leu Asn Pro Gln Trp Gly Glu Arg Leu
           35                  40                  45
   Glu Phe Leu Val His Asp Pro Asp Ala Met Cys Ala Glu Thr Leu Glu
       50                  55                  60
   Leu Asn Leu Tyr Asn Asp Lys Lys Ala Ile Ala Ala Thr Gly Gly Gly
   65                  70                  75                  80
   Gly Arg Arg Gly Gly Thr Phe Leu Gly Lys Val Lys
                   85                  90
<210> 475
   <211> 84
   <212> PRT
   <213> Artificial sequence
<220>
   <223> Synthetically generated peptide
<400> 475
   Leu Ile Val Glu Ile Cys Ser Ala Arg Asn Leu Met Pro Lys Asp Gly
    1               5                  10                  15
   Gln Gly Thr Ala Ser Ala Tyr Ala Ile Val Asp Phe Asp Gly Gln Arg
               20                  25                  30
   Arg Arg Thr Lys Thr Lys Phe Arg Asp Leu Asn Pro Gln Trp Asp Glu
           35                  40                  45
   Lys Leu Glu Phe Phe Val His Asp Val Ala Thr Met Gly Glu Glu Ile
       50                  55                  60
   Ile Asn Leu Cys Asn Asp Lys Lys Thr Gly Lys Arg Ser Thr Phe Leu
   65                  70                  75                  80
   Gly Lys Val Lys
<210> 476
   <211> 90
   <212> PRT
   <213> Artificial sequence
<220>
   <223> Synthetically generated peptide
<400> 476
   Leu Val Val Glu Val Val Glu Ala Arg Asn Ile Leu Pro Lys Asp Gly
    1               5                  10                  15
   Gln Gly Ser Ser Ser Ala Tyr Val Val Val Asp Phe Asp Ala Gln Lys
               20                  25                  30
   Lys Arg Thr Ser Thr Lys Phe Arg Asp Leu Asn Pro Ile Trp Asn Glu
           35                  40                  45
   Met Leu Asp Phe Ala Val Ser Asp Pro Lys Asn Met Asp Tyr Asp Glu
       50                  55                  60
   Leu Asp Ile Glu Val Tyr Asn Asp Lys Arg Phe Gly Asn Gly Gly Gly
   65                  70                  75                  80
   Arg Lys Asn His Phe Leu Gly Arg Val Lys
                   85                  90
<210> 477
   <211> 90
   <212> PRT
   <213> Artificial Sequence
<220>
   <223> Synthetically generated peptide
<400> 477
   Leu Val Val Glu Val Val Glu Ala Arg Asn Ile Leu Pro Lys Asp Gly
    1               5                  10                  15
   Gln Gly Ser Ser Ser Ala Tyr Val Val Val Asp Phe Asp Ala Gln Lys
               20                  25                  30
   Lys Arg Thr Ser Thr Lys Phe Arg Asp Leu Asn Pro Ile Trp Asn Glu
           35                  40                  45
   Met Leu Asp Phe Ala Val Ser Asp Pro Lys Asn Met Asp Tyr Asp Glu
       50                  55                  60
   Leu Asp Ile Glu Val Tyr Asn Asp Lys Arg Phe Gly Asn Gly Gly Gly
   65                  70                  75                  80
   Arg Lys Asn His Phe Leu Gly Arg Val Lys
                   85                  90
<210> 478
   <211> 95
   <212> PRT
   <213> Artificial Sequence
<220>
   <223> Synthetically generated peptide
<400> 478
   Leu Ala Val Glu Val Val Asp Ala Arg Asp Leu Val Pro Lys Asp Gly
   1 5 10 15
   Leu Gly Thr Ser Ser Ala Phe Ala Val Val Asp Phe Asp Gly Gln Arg
   20 25 30
   Lys Arg Thr Arg Thr Val Pro Arg Asp Leu Ser Pro Gln Trp His Glu
   35 40 45
   Arg Leu Glu Phe Ala Val His Asp Pro Ala Ala Met His Ala Glu Ala
   50 55 60
   Leu Asp Val Ser Leu Tyr His Asp Arg Arg Phe Asn Pro Ser Gly Gly
   65 70 75 80
   Gly Gly Gly Gly Gly Gly Lys Asn His Phe Leu Gly Arg Val Arg
   85 90 95
<210> 479
   <211> 87
   <212> PRT
   <213> Artificial Sequence
<220>
   <223> Synthetically generated peptide
<400> 479
   Leu Ile Val Glu Val Ile Asn Ala His Asp Leu Met Pro Lys Asp Gly
   1 5 10 15
   Glu Gly Ser Ala Ser Thr Phe Val Glu Val Asp Phe Glu Asn Gln Leu
   20 25 30
   Ser Arg Thr Arg Thr Val Pro Lys Asn Leu Asn Pro Thr Trp Asn Gln
   35 40 45
   Lys Leu Val Phe Asn Leu Asp Thr Thr Lys Pro Tyr His His Lys Thr
   50 55 60
   Ile Glu Val Ser Val Tyr Asn Asp Arg Arg Gln Pro Asn Pro Gly Arg
   65 70 75 80
   Asn Phe Leu Gly Arg Val Arg
   85
<210> 480
   <211> 85
   <212> PRT
   <213> Artificial Sequence
<220>
   <223> synthetically generated peptide
<400> 480
   Leu Val Val His Val Val Asp Ala Gln Tyr Leu Met Pro Arg Asp Gly
   1 5 10 15
   Gln Gly Ser Asp Phe Val Glu Val Asp Phe Leu Asn Gln Leu Ser Lys
   20 25 30
   Thr Arg Thr Val Pro Lys Ser Leu Asn Pro Val Trp Asn Gln Lys Leu
   35 40 45
   Tyr Phe Asp Tyr Asp Gln Ser Val Ile Asn Gln His Asn Gln His Ile
   50 55 60
   Glu Val Ser Val Tyr His Glu Arg Arg Pro Ile Pro Gly Arg Ser Phe
   65 70 75 80
   Leu Gly Arg Val Lys
   85
<210> 481
   <211> 97
   <212> PRT
   <213> Artificial Sequence
<220>
   <223> Synthetically generated peptide
<400> 481
   Leu Val Val Glu Val Val Ala Ala His Asn Leu Met Pro Lys Asp Gly
   1 5 10 15
   Gln Gly Ser Ser Ser Ala Tyr Val Glu Val Glu Phe Glu His Gln Arg
   20 25 30
   Arg Arg Thr Arg Ala Arg Pro Lys Glu Leu Asn Pro Val Trp Asn Glu
   35 40 45
   Arg Leu Val Phe Ala Val Ala Asp Pro Asp Asp Leu Pro Tyr Arg Ala
   50 55 60
   Ile Asp Val Gly Val Tyr Asn Asp Arg Ala Ala Ser Gly Gly Val Ala
   65 70 75 80
   Gly Gly Gly Gly Ala Ala Pro His Gly Arg Asn Phe Leu Gly Lys Val
   85 90 95
   Arg
<210> 482
   <211> 86
   <212> PRT
   <213> Artificial Sequence
<220>
   <223> Synthetically generated peptide
<400> 482
   Leu Val Val Glu Ile Val Gly Ala His Asn Leu Met Pro Lys Asp Gly
   1               5                   10                  15
   Glu Asp Ser Ser Ser Pro Phe Val Glu Val Gln Phe Glu Asn Gln Arg
               20                  25                  30
   Leu Arg Thr Lys Val Lys Pro Lys Asp Leu Asn Pro Ile Trp Asn Glu
           35                  40                  45
   Lys Leu Val Phe His Val Ile Asp Val Asn Asp Leu Arg His Lys Ala
       50                  55                  60
   Leu Glu Ile Asn Val Tyr Asn Glu Lys Arg Ser Ser Asn Ser Arg Asn
   65                  70                  75                  80
   Phe Leu Gly Lys Val Arg
                   85
<210> 483
   <211> 84
   <212> PRT
   <213> Artificial Sequence
<220>
   <223> Synthetically generated peptide
<400> 483
   Leu Val Val Glu Ile Val Asp Ala Ser Asp Leu Met Pro Lys Asp Gly
   1               5                   10                  15
   Gln Gly Ser Asp Phe Val Glu Val Glu Phe Asp Glu Gln Arg Gln Arg
               20                  25                  30
   Thr Gln Thr Arg Phe Lys Asp Leu Asn Pro Gln Trp Asn Glu Lys Leu
           35                  40                  45
   Val Phe Asn Val Gly Asp Leu Lys Arg Leu Asn Asn Lys Thr Val Asp
       50                  55                  60
   Val Thr Val Tyr Asp Asp Arg Arg Asp Asn Gln Pro Gly Lys Phe Leu
   65                  70                  75                  80
   Gly Arg Val Lys
<210> 484
   <211> 91
   <212> PRT
   <213> Artificial Sequence
<220>
   <223> Synthetically generated peptide
<400> 484
   Val Gly Val Glu Ile Leu Asp Ala Ser Glu Leu Ala Pro Lys Asp Gly
   1               5                   10                  15
   Ala Gly Ala Cys Asn Ala Phe Val Glu Val Glu Phe Asp Gly Gln Lys
               20                  25                  30
   Gln Arg Thr Pro Thr Lys Pro Ala Asp Arg Ser Pro Gln Trp Asn His
           35                  40                  45
   Thr Leu Val Phe Asp Val Arg Asp Pro Ser Arg Leu Pro Ser Leu Pro
   50 55 60
   Val Asp Val Ser Val His His Asp Arg Ser Leu Thr Asp His His Ala
   65 70 75 80
   Thr Arg Leu His Thr Phe Leu Gly Arg Val Arg
   85 90

<210> 485
   <211> 90
   <212> PRT
   <213> Artificial Sequence
<220>
   <223> Synthetically generated peptide
<400> 485
   Leu Val Val Glu Val Val Asp Ala Lys Asp Leu Thr Pro Lys Asp Gly
   1 5 10 15
   His Gly Thr Ser Ser Pro Tyr Val Val Leu Asp Tyr Tyr Gly Gln Arg
   20 25 30
   Arg Arg Thr Arg Thr Ile Val Arg Asp Leu Asn Pro Val Trp Asn Glu
   35 40 45
   Thr Leu Glu Phe Ser Leu Ala Lys Arg Pro Ser His Gln Leu Phe Thr
   50 55 60
   Asp Val Leu Glu Leu Asp Met Tyr His Asp Lys Asn Phe Gly Gln Thr
   65 70 75 80
   Arg Arg Asn Asn Phe Leu Gly Arg Ile Arg
   85 90
<210> 486
   <211> 82
   <212> PRT
   <213> Artificial Sequence
<220>
   <223> Synthetically generated peptide
<400> 486
   Leu Asn Val Arg Val Val Arg Gly Ser Asn Leu Ala Ile Cys Asp Pro
   1 5 10 15
   Leu Thr His Thr Ser Asp Pro Tyr Val Val Leu His Tyr Gly Ala Gln
   20 25 30
   Lys Val Lys Thr Ser Val Gln Lys Lys Asn Pro Asn Pro Val Trp Asn
   35 40 45
   Glu Val Leu Gln Leu Ser Val Thr Asn Pro Thr Lys Pro Val His Leu
   50 55 60
   Glu Val Phe Asp Glu Asp Lys Phe Thr Ala Asp Asp Ser Met Gly Val
   65 70 75 80
   Ala Glu
<210> 487
   <211> 82
   <212> PRT
   <213> Artificial Sequence
<220>
   <223> Synthetically generated peptide
<400> 487
   Leu Asn Val Arg Val Val Arg Gly Ser Asn Leu Ile Ile Ala Asp Pro
   1               5                   10                  15
   Leu Thr His Thr Ser Asp Pro Tyr Val Val Leu Ser Tyr Gly Pro Gln
               20                  25                  30
   Lys Val Lys Thr Ser Val Gln Lys Lys Asn Ser Asn Pro Val Trp Asn
           35                  40                  45
   Glu Val Leu Gln Leu Ala Val Thr Asn Pro Thr Lys Pro Val Lys Leu
       50                  55                  60
   Glu Val Phe Asp Glu Asp Lys Phe Thr Ala Asp Asp Ser Met Gly Val
   65                  70                  75                  80
   Ala Glu
<210> 488
   <211> 80
   <212> PRT
   <213> Artificial Sequence
<220>
   <223> Synthetically generated peptide
<400> 488
   Leu Lys Val Arg Val Met Arg Gln Leu Ala Ile Cys Asp Pro Leu Thr
   1               5                   10                  15
   His Ser Ser Asp Pro Tyr Val Val Leu Arg His Gly Ser Gln Lys Val
               20                  25                  30
   Lys Ser Ser Ile Arg Tyr His Ser Ile Asn Pro Glu Trp Asn Glu Glu
           35                  40                  45
   Leu Thr Leu Ser Ile Thr Asn Met Met Leu Pro Val Lys Ile Glu Val
       50                  55                  60
   Phe Asp Lys Asp Thr Phe Thr Lys Asp Asp Ser Met Gly Asp Ala Glu
   65                  70                  75                  80
<210> 489
   <211> 80
   <212> PRT
   <213> Artificial Sequence
<220>
   <223> Synthetically generated peptide
<400> 489
   Leu Lys Ile Arg Val Val Arg Gly Ile Asn Leu Ala Tyr Arg Asp Thr
   1               5                   10                  15
   Arg Gly Ser Asp Pro Tyr Val Val Leu Arg Leu Gly Lys Gln Lys Val
               20                  25                  30
   Lys Thr Ser Val Lys Lys Lys Ser Val Asn Pro Ile Trp His Glu Glu
           35                  40                  45
   Leu Thr Leu Ser Ile Met Asn Pro Ile Ala Pro Ile Lys Leu Gly Val
       50                  55                  60
   Phe Asp Lys Asp Thr Phe Ser Arg Asp Asp Pro Met Gly Asp Ala Glu
   65                  70                  75                  80
<210> 490
   <211> 79
   <212> PRT
   <213> Artificial Sequence
<220>
   <223> synthetically generated peptide
<400> 490
   Ser Val Arg Val Leu Arg Gly Val Asn Leu Val Ser Arg Asp Ala Gly
   1               5                   10                  15
   Gly Ser Asp Pro Tyr Val Val Leu His Leu Asp Asn Gln Lys Leu Lys
               20                  25                  30
   Thr Gly Val Val Lys Lys Thr Thr Asn Pro Val Trp Asn Glu Glu Leu
           35                  40                  45
   Thr Leu Ala Val Arg Asn Pro Glu Thr Pro Ile Gln Leu Glu Val Phe
       50                  55                  60
   Asp Lys Asp Thr Phe Ser Lys Asp Asp Gln Met Gly Asp Ala Glu
   65                  70                  75
<210> 491
   <211> 79
   <212> PRT
   <213> Artificial Sequence
<220>
   <223> Synthetically generated peptide
<400> 491
   Val Lys Val Arg Val Val Arg Gly Val Asn Leu Ala Val Arg Asp Leu
   1               5                   10                  15
   Arg Ser Ser Asp Pro Tyr Val Ile Val Arg Met Gly Lys Gln Lys Leu
               20                  25                  30
   Lys Thr Arg Val Ile Lys Lys Thr Thr Asn Pro Glu Trp Asn Asp Glu
           35                  40                  45
   Leu Thr Leu Ser Ile Glu Asp Pro Ala Val Pro Val Arg Leu Glu Val
       50                  55                  60
   Tyr Asp Lys Asp Thr Phe Ile Asp Asp Ala Met Gly Asn Ala Glu
   65                  70                  75
<210> 492
   <211> 80
   <212> PRT
   <213> Artificial Sequence
<220>
   <223> Synthetically generated peptide
<400> 492
   Leu Arg Ile Arg Val Lys Arg Gly Ile Asn Leu Ala Gln Arg Asp Thr
   1 5 10 15
   Leu Gly Ser Asp Pro Phe Val Val Ile Thr Met Gly Ser Gln Lys Leu
   20 25 30
   Lys Thr Arg Val Val Glu Asn Asn Cys Asn Pro Glu Trp Asn Glu Glu
   35 40 45
   Leu Thr Leu Ala Leu Arg His Pro Asp Glu Pro Val Asn Leu Ile Val
   50 55 60
   Tyr Asp Lys Asp Thr Phe Thr Ser His Asp Lys Met Gly Asp Ala Lys
   65 70 75 80
<210> 493
   <211> 80
   <212> PRT
   <213> Artificial Sequence
<220>
   <223> Synthetically generated peptide
<400> 493
   Leu Arg Ile Arg Val Lys Arg Gly Ile Asn Leu Ala Gln Arg Asp Thr
   1 5 10 15
   Leu Ser Ser Asp Pro Phe Val Val Ile Thr Met Gly Ser Gln Lys Leu
   20 25 30
   Lys Thr Arg Val Val Glu Asn Asn Cys Asn Pro Glu Trp Asn Glu Glu
   35 40 45
   Leu Thr Leu Ala Leu Arg His Pro Asp Glu Pro Val Asn Leu Ile Val
   50 55 60
   Tyr Asp Lys Asp Thr Phe Thr Ser His Asp Lys Met Gly Asp Ala Lys
   65 70 75 80
<210> 494
   <211> 32
   <212> PRT
   <213> Artificial Sequence
<220>
   <223> Synthetically generated peptide
<400> 494
   Leu Arg Ile Arg Val Lys Arg Gly Ile Asn Leu Ala Gln Arg Asp Thr
   1 5 10 15
   Leu Ser Ser Asp Pro Phe Val Val Ile Thr Met Gly Ser Gln Val Phe
   20 25 30
<210> 495
   <211> 80
   <212> PRT
   <213> Artificial sequence
<220>
   <223> synthetically generated peptide
<400> 495
   Val Arg Ile Leu Val Lys Arg Gly Ile Asp Leu Ala Arg Arg Asp Ala
   1 5 10 15
   Leu Ser Ser Asp Pro Phe Val Val Ile Thr Met Gly Pro Gln Lys Leu
   20 25 30
   Lys Ser Phe Thr Val Lys Asn Asn Cys Asn Pro Glu Trp Asn Glu Glu
   35 40 45
   Leu Thr Leu Ala Ile Glu Asp Pro Asn Glu Pro Val Lys Leu Met Val
   50 55 60
   Tyr Asp Lys Asp Thr Phe Thr Ala Asp Asp Lys Met Gly Asp Ala Gln
   65 70 75 80
<210> 496
   <211> 80
   <212> PRT
   <213> Artificial Sequence
<220>
   <223> synthetically generated peptide
<400> 496
   Leu Arg Ile Arg Val Lys Arg Gly Ile Asn Leu Val Ser Arg Asp Ser
   1 5 10 15
   Asn Thr Ser Asp Pro Phe Val Val Val Thr Met Gly Ser Gln Lys Leu
   20 25 30
   Lys Thr Arg Gly Val Glu Asn Ser Cys Asn Pro Glu Trp Asp Asp Glu
   35 40 45
   Leu Thr Leu Gly Ile Asn Asp Pro Asn Gln His Val Thr Leu Glu Val
   50 55 60
   Tyr Asp Lys Asp Thr Phe Thr Ser His Asp Pro Met Gly Asp Ala Glu
   65 70 75 80
<210> 497
   <211> 80
   <212> PRT
   <213> Artificial Sequence
<220>
   <223> synthetically generated peptide
<400> 497
   Leu Arg Val His Val Lys Arg Gly Ile Asn Leu Ala Ile Arg Asp Ala
   1 5 10 15
   Thr Thr Ser Asp Pro Tyr Val Val Ile Thr Leu Ala Asn Gln Lys Leu
   20 25 30
   Lys Thr Arg Val Ile Asn Asn Asn Cys Asn Pro Val Trp Asn Glu Gln
   35 40 45
   Leu Thr Leu Ser Ile Lys Asp Val Asn Asp Pro Ile Arg Leu Thr Val
   50 55 60
   Phe Asp Lys Asp Arg Phe Ser Gly Asp Asp Lys Met Gly Asp Ala Glu
   65 70 75 80
<210> 498
   <211> 80
   <212> PRT
   <213> Artificial Sequence
<220>
   <223> Synthetically generated peptide
<400> 498
   Leu Thr Ile His Val Lys Arg Gly Ile Asn Leu Ala Ile Arg Asp His
   1 5 10 15
   Arg Ser Ser Asp Pro Tyr Ile Val Leu Asn Val Ala Asp Gln Thr Leu
   20 25 30
   Lys Thr Arg Val Val Lys Lys Asn Cys Asn Pro Val Trp Asn Glu Glu
   35 40 45
   Met Thr Val Ala Ile Lys Asp Pro Asn Val Pro Ile Arg Leu Thr Val
   50 55 60
   Phe Asp Trp Asp Lys Phe Thr Gly Asp Asp Lys Met Gly Asp Ala Asn
   65 70 75 80
<210> 499
   <211> 81
   <212> PRT
   <213> Artificial Sequence
<220>
   <223> Synthetically generated peptide
<400> 499
   Leu Lys Leu Arg Ile Lys Arg Gly Ile Asn Leu Ala Ile Arg Asp Ser
   1 5 10 15
   Asn Ser Ser Asp Pro Tyr Val Val Val Asn Ile Gly His Glu Gln Lys
   20 25 30
   Leu Lys Thr Arg Val Val Lys Asn Asn Cys Asn Pro Glu Trp Asn Glu
   35 40 45
   Glu Leu Thr Leu Ser Ile Arg Asp Val Arg Val Pro Ile Cys Leu Thr
   50 55 60
   Val Phe Asp Lys Asp Thr Phe Phe Val Asp Asp Lys Met Gly Asp Ala
   65 70 75 80
   Glu
<210> 500
   <211> 80
   <212> PRT
   <213> Artificial Sequence
<220>
   <223> Synthetically generated peptide
<400> 500
   Leu Arg Ile Arg Ile Lys Arg Gly Val Asn Leu Ala Val Arg Asp Ile
   1 5 10 15
   Ser Ser Ser Asp Pro Tyr Val Val Val Lys Met Gly Lys Gln Lys Leu
   20 25 30
   Lys Thr Arg Val Ile Asn Lys Asp Val Asn Pro Glu Trp Asn Glu Asp
   35 40 45
   Leu Thr Leu Ser Val Thr Asp Ser Asn Leu Thr Val Leu Leu Thr Val
   50 55 60
   Tyr Asp His Asp Met Phe Ser Lys Asp Asp Lys Met Gly Asp Ala Glu
   65 70 75 80
<210> 501
   <211> 80
   <212> PRT
   <213> Artificial Sequence
<220>
   <223> Synthetically generated peptide
<400> 501
   Leu Arg Ile Arg Ile Lys Arg Gly Val Asn Leu Ala Val Arg Asp Leu
   1 5 10 15
   Asn Ser Ser Asp Pro Tyr Val Val Val Lys Met Ala Lys Gln Lys Leu
   20 25 30
   Lys Thr Arg Val Ile Tyr Lys Asn Val Asn Pro Glu Trp Asn Glu Asp
   35 40 45
   Leu Thr Leu Ser Val Ser Asp Pro Asn Leu Thr Val Leu Leu Thr Val
   50 55 60
   Tyr Asp Tyr Asp Thr Phe Thr Lys Asp Asp Lys Met Gly Asp Ala Glu
   65 70 75 80
<210> 502
   <211> 80
   <212> PRT
   <213> Artificial Sequence
<220>
   <223> Synthetically generated peptide
<400> 502
   Leu Arg Ile Arg Ile Lys Arg Gly Val Asn Leu Ala Val Arg Asp Val
   1 5 10 15
   Asn Thr Ser Asp Pro Tyr Ala Val Val Lys Met Gly Lys Gln Arg Leu
   20 25 30
   Lys Thr His Val Ile Lys Lys Asp Val Asn Pro Glu Trp Asn Glu Asp
   35 40 45
   Leu Thr Leu Ser Ile Thr Asp Pro Val Val Pro Phe Lys Leu Thr Val
   50 55 60
   Tyr Asp Tyr Asp Thr Phe Ser Lys Asp Asp Lys Met Gly Asp Ala Glu
   65 70 75 80
<210> 503
   <211> 80
   <212> PRT
   <213> Artificial Sequence
<220>
   <223> Synthetically generated peptide
<400> 503
   Leu Arg Ile His Val Lys Arg Gly Val Asn Leu Ala Ile Arg Asp Ile
   1 5 10 15
   Ser Ser Ser Asp Pro Tyr Ile Val Val His Cys Gly Lys Gln Lys Leu
   20 25 30
   Lys Thr Arg Val Val Lys His Ser Val Asn Pro Glu Trp Asn Asp Asp
   35 40 45
   Leu Thr Leu Ser Val Thr Asp Pro Asn Leu Pro Ile Lys Leu Thr Val
   50 55 60
   Tyr Asp Tyr Asp Leu Leu Ser Ala Asp Asp Lys Met Gly Glu Ala Glu
   65 70 75 80
<210> 504
   <211> 80
   <212> PRT
   <213> Artificial Sequence
<220>
   <223> Synthetically generated peptide
<400> 504
   Leu Arg Leu His Val Ile Arg Gly Val Asn Leu Ala Ile Arg Asp Ser
   1 5 10 15
   Gln Ser Ser Asp Pro Tyr Val Ile Val Arg Met Gly Lys Gln Lys Leu
   20 25 30
   Arg Thr Arg Val Met Lys Lys Asn Leu Asn Thr Glu Trp Asn Glu Asp
   35 40 45
   Leu Thr Leu Ser Val Thr Asp Pro Thr Leu Pro Val Lys Ile Met Val
   50 55 60
   Tyr Asp Arg Asp Arg Phe Ser Arg Asp Asp Lys Met Gly Asp Ala Ile
   65 70 75 80
<210> 505
   <211> 80
   <212> PRT
   <213> Artificial sequence
<220>
   <223> Synthetically generated peptide
<400> 505
   Leu Arg Val Arg Val Gln Arg Gly Val Asn Leu Ala Val Arg Asp Val
   1 5 10 15
   Ser Ser Ser Asp Pro Tyr Val Val Leu Lys Leu Gly Arg Gln Lys Leu
   20 25 30
   Lys Thr Lys Val Val Lys Gln Asn Val Asn Pro Gln Trp Gln Glu Asp
   35 40 45
   Leu Ser Phe Thr Val Thr Asp Pro Asn Leu Pro Leu Thr Leu Ile Val
   50 55 60
   Tyr Asp His Asp Phe Phe Ser Lys Asp Asp Lys Met Gly Asp Ala Glu
   65 70 75 80
<210> 506
   <211> 80
   <212> PRT
   <213> Artificial Sequence
<220>
   <223> Synthetically generated peptide
<400> 506
   Leu Lys Val Thr Ile Lys Lys Gly Thr Asn Leu Ala Ile Arg Asp Met
    1               5                  10                  15
   Met Ser Ser Asp Pro Tyr Val Val Leu Asn Leu Gly Lys Gln Lys Leu
               20                  25                  30
   Gln Thr Thr Val Met Asn Ser Asn Leu Asn Pro Val Trp Asn Gln Glu
           35                  40                  45
   Leu Met Leu Ser Val Pro Glu Ser Tyr Gly Pro Val Lys Leu Gln Val
       50                  55                  60
   Tyr Asp Tyr Asp Thr Phe Ser Ala Asp Asp Ile Met Gly Glu Ala Asp
   65                  70                  75                  80
<210> 507
   <211> 80
   <212> PRT
   <213> Artificial Sequence
<220>
   <223> Synthetically generated peptide
<400> 507
   Leu Lys Val Thr Ile Lys Lys Gly Thr Asn Leu Ala Ile Arg Asp Met
    1               5                  10                  15
   Met Ser Ser Asp Pro Tyr Val Val Leu Asn Leu Gly Lys Gln Lys Leu
               20                  25                  30
   Gln Thr Thr Val Met Asn Ser Asn Leu Asn Pro Val Trp Asn Gln Glu
           35                  40                  45
   Leu Met Leu Ser Val Pro Glu Ser Tyr Gly Pro Val Lys Leu Gln Val
       50                  55                  60
   Tyr Asp Tyr Asp Thr Phe Ser Ala Asp Asp Ile Met Gly Glu Ala Asp
   65                  70                  75                  80
<210> 508
   <211> 80
   <212> PRT
   <213> Artificial Sequence
<220>
   <223> Synthetically generated peptide
<400> 508
   Leu Lys Val Thr Ile Lys Lys Gly Thr Asn Met Ala Ile Arg Asp Met
    1               5                  10                  15
   Met Ser Ser Asp Pro Tyr Val Val Leu Thr Leu Gly Gln Gln Lys Ala
               20                  25                  30
   Gln Ser Thr Val Val Lys Ser Asn Leu Asn Pro Val Trp Asn Glu Glu
           35                  40                  45
   Leu Met Leu Ser Val Pro His Asn Tyr Gly Ser Val Lys Leu Gln Val
       50                  55                  60
   Phe Asp Tyr Asp Thr Phe Ser Ala Asp Asp Ile Met Gly Glu Ala Glu
   65                  70                  75                  80
<210> 509
   <211> 80
   <212> PRT
   <213> Artificial Sequence
<220>
   <223> Synthetically generated peptide
<400> 509
   Leu Lys Val Thr Ile Lys Lys Gly Thr Asn Met Ala Ile Arg Asp Met
   1               5                   10                  15
   Met Ser Ser Asp Pro Tyr Val Val Leu Thr Leu Gly Gln Gln Lys Ala
               20                  25                  30
   Gln Ser Thr Val Val Lys Ser Asn Leu Asn Pro Val Trp Asn Glu Glu
           35                  40                  45
   Leu Met Leu Ser Val Pro His Asn Tyr Gly Ser Val Lys Leu Gln Val
       50                  55                  60
   Phe Asp Tyr Asp Thr Phe Ser Ala Asp Asp Ile Met Gly Glu Ala Glu
   65                  70                  75                  80
<210> 510
   <211> 80
   <212> PRT
   <213> Artificial Sequence
<220>
   <223> Synthetically generated peptide
<400> 510
   Leu Lys Val Thr Ile Lys Lys Gly Thr Asn Met Ala Ile Arg Asp Met
   1               5                   10                  15
   Met Ser Ser Asp Pro Tyr Val Val Leu Thr Leu Gly Gln Gln Lys Ala
               20                  25                  30
   Gln Ser Thr Val Val Lys Ser Asn Leu Asn Pro Val Trp Asn Glu Glu
           35                  40                  45
   Leu Met Leu Ser Val Pro His Asn Tyr Gly Ser Val Lys Leu Gln Val
       50                  55                  60
   Phe Asp Tyr Asp Thr Phe Ser Ala Asp Asp Ile Met Gly Glu Ala Glu
   65                  70                  75                  80
<210> 511
   <211> 80
   <212> PRT
   <213> Artificial Sequence
<220>
   <223> Synthetically generated peptide
<400> 511
   Leu Lys Val Lys Val Val Lys Gly Thr Asn Leu Ala Ile Arg Asp Met
   1 5 10 15
   Arg Thr Ser Asp Pro Tyr Val Val Leu Lys Leu Gly Gln Gln Thr Val
   20 25 30
   Gln Thr Thr Val Ile Arg Ser Asn Leu Asn Pro Val Trp Asn Glu Glu
   35 40 45
   Leu Met Leu Ser Val Pro Gln Gln Phe Gly Pro Ile Ser Leu Glu Val
   50 55 60
   Phe Asp His Asp Leu Phe Ser Ala Asp Asp Ile Met Gly Glu Ala Gln
   65 70 75 80
<210> 512
   <211> 80
   <212> PRT
   <213> Artificial Sequence
<220>
   <223> Synthetically generated peptide
<400> 512
   Leu Asn Val Lys Val Lys Gly Gly Thr Asn Leu Ala Ile Arg Asp Met
   1 5 10 15
   Ser Ser Ser Asp Pro Tyr Val Val Leu Thr Leu Gly Gln Gln Lys Ala
   20 25 30
   Gln Thr Ser Val Ile Lys Ala Asn Leu Asn Pro Val Trp Asn Glu Glu
   35 40 45
   Leu Lys Leu Ser Val Pro Gln Gln Tyr Gly Pro Leu Lys Leu Gln Ala
   50 55 60
   Phe Asp His Asp Met Leu Ser Lys Asp Asp Leu Met Gly Glu Ala Glu
   65 70 75 80
<210> 513
   <211> 80
   <212> PRT
   <213> Artificial Sequence
<220>
   <223> Synthetically generated peptide
<400> 513
   Leu Asn Val Lys Val Lys Gly Gly Thr Asn Leu Ala Ile Arg Asp Met
   1 5 10 15
   Ser Ser Ser Asp Pro Tyr Val Val Leu Thr Leu Gly Gln Gln Lys Ala
   20 25 30
   Gln Thr Ser Val Ile Lys Ala Asn Leu Asn Pro Val Trp Asn Glu Glu
   35 40 45
   Leu Lys Leu Ser Val Pro Gln Gln Tyr Gly Pro Leu Lys Leu Gln Val
   50 55 60
   Phe Asp His Asp Met Leu Ser Lys Asp Asp Leu Met Gly Glu Ala Glu
   65 70 75 80
<210> 514
   <211> 80
   <212> PRT
   <213> Artificial Sequence
<220>
   <223> Synthetically generated peptide
<400> 514
   Ile Lys Val Lys Val Ile Arg Gly Thr Lys Leu Ala Val Arg Asp Ile
    1               5                  10                  15
   Leu Ser Ser Asp Pro Tyr Val Val Leu Thr Leu Gly Gln Gln Lys Ala
               20                  25                  30
   Lys Thr Lys Val Ile Lys Ser Asn Leu Asn Pro Val Trp Asn Glu Val
           35                  40                  45
   Leu Thr Leu Ser Val Pro Gln Lys Tyr Gly Pro Leu Lys Leu Gln Val
       50                  55                  60
   Tyr Asp His Asp Val Leu Ser Arg Asp Asp Ile Met Gly Glu Ala Glu
   65                  70                  75                  80
<210> 515
   <211> 80
   <212> PRT
   <213> Artificial Sequence
<220>
   <223> Synthetically generated peptide
<400> 515
   Leu Asn Ile Thr Val Val Arg Gly Ile Gln Leu Ala Val Arg Asp Met
    1               5                  10                  15
   Leu Thr Ser Asp Pro Tyr Val Val Leu Thr Leu Gly Glu Gln Lys Ala
               20                  25                  30
   Gln Thr Thr Val Lys Pro Ser Asp Leu Asn Pro Val Trp Asn Glu Val
           35                  40                  45
   Leu Lys Ile Ser Ile Pro Arg Asn Tyr Gly Pro Leu Lys Leu Glu Val
       50                  55                  60
   Tyr Asp His Asp Thr Phe Ser Ala Asp Asp Ile Met Gly Glu Ala Glu
   65                  70                  75                  80
<210> 516
   <211> 80
   <212> PRT
   <213> Artificial Sequence
<220>
   <223> Synthetically generated peptide
<400> 516
   Ile Lys Val Asp Ile Arg Arg Gly Thr Asn Leu Ala Val Arg Asp Val
   1               5                   10                  15
   Met Ser Ser Asp Pro Tyr Val Met Leu Asn Leu Gly His Gln Thr Met
               20                  25                  30
   Lys Thr Lys Val Ile Lys Asn Thr Leu Asn Pro Val Trp Asn Glu Arg
           35                  40                  45
   Leu Met Leu Ser Ile Pro His Pro Val Pro Pro Leu Lys Leu Gln Val
       50                  55                  60
   Phe Asp Lys Asp Thr Phe Ser Ser Asp Asp Arg Met Gly Asp Val Glu
   65                  70                  75                  80
<210> 517
   <211> 80
   <212> PRT
   <213> Artificial Sequence
<220>
   <223> Synthetically generated peptide
<400> 517
   Ile Lys Val Asn Val Ile Arg Gly Thr Asn Leu Ala Val Arg Asp Met
   1               5                   10                  15
   Met Ser Ser Asp Pro Tyr Val Ile Leu Asn Leu Gly His Gln Ser Met
               20                  25                  30
   Lys Thr Lys Val Ile Lys Ser Ser Leu Asn Pro Val Trp Asn Glu Arg
           35                  40                  45
   Ile Leu Leu Ser Ile Pro Asp Pro Ile Pro Met Leu Lys Leu Gln Val
       50                  55                  60
   Tyr Asp Lys Asp Thr Phe Thr Thr Asp Asp Arg Met Gly Glu Ala Glu
   65                  70                  75                  80
<210> 518
   <211> 80
   <212> PRT
   <213> Artificial Sequence
<220>
   <223> Synthetically generated peptide
<400> 518
   Ile Lys Val Asn Val Val Lys Gly Thr Asn Leu Ala Val Arg Asp Val
   1               5                   10                  15
   Met Thr Ser Asp Pro Tyr Val Ile Leu Ala Leu Gly Gln Gln Ser Val
               20                  25                  30
   Lys Thr Arg Val Ile Lys Asn Asn Leu Asn Pro Val Trp Asn Glu Thr
           35                  40                  45
   Leu Met Leu Ser Ile Pro Glu Pro Met Pro Pro Leu Lys Val Leu Val
       50                  55                  60
   Tyr Asp Lys Asp Thr Phe Ser Thr Asp Asp Phe Met Gly Glu Ala Glu
   65                  70                  75                  80
<210> 519
   <211> 80
   <212> PRT
   <213> Artificial Sequence
<220>
   <223> Synthetically generated peptide
<400> 519
   Ile Lys Val Asn Val Val Lys Gly Thr Asn Leu Ala Val Arg Asp Val
   1 5 10 15
   Met Thr Ser Asp Pro Tyr Val Ile Leu Ala Leu Gly Gln Gln Ser Val
   20 25 30
   Lys Thr Arg Val Ile Lys Asn Asn Leu Asn Pro Val Trp Asn Glu Thr
   35 40 45
   Leu Met Leu Ser Ile Pro Glu Pro Met Pro Pro Leu Lys Val Leu Val
   50 55 60
   Tyr Asp Lys Asp Thr Phe Ser Thr Asp Asp Phe Met Gly Glu Ala Glu
   65 70 75 80
<210> 520
   <211> 80
   <212> PRT
   <213> Artificial Sequence
<220>
   <223> Synthetically generated peptide
<400> 520
   Ile Lys Val Asn Val Arg Lys Gly Thr His Leu Ala Ile Arg Asp Val
   1 5 10 15
   Val Thr Ser Asp Pro Tyr Val Ile Leu Ser Leu Gly His Gln Ser Val
   20 25 30
   Lys Thr Arg Val Ile Arg Asn Asn Leu Asn Pro Val Trp Asn Glu Ser
   35 40 45
   Leu Met Leu Ser Ile Pro Glu Asn Ile Pro Pro Leu Lys Val Leu Val
   50 55 60
   Tyr Asp Lys Asp Thr Phe Ser Thr Asp Asp Phe Met Gly Glu Ala Glu
   65 70 75 80
<210> 521
   <211> 80
   <212> PRT
   <213> Artificial Sequence
<220>
   <223> Synthetically generated peptide
<400> 521
   Leu Gln Val Thr Val Ile Gln Gly Lys Lys Leu Val Ile Arg Asp Phe
   1 5 10 15
   Lys Ser Ser Asp Pro Tyr Val Ile Val Lys Leu Gly Asn Glu Ser Ala
   20 25 30
   Lys Thr Lys Val Ile Asn Asn Cys Leu Asn Pro Val Trp Asn Glu Glu
   35 40 45
   Leu Asn Phe Thr Leu Lys Asp Pro Ala Ala Val Leu Ala Leu Glu Val
   50 55 60
   Phe Asp Lys Asp Arg Phe Lys Ala Asp Asp Lys Met Gly His Ala Ser
   65 70 75 80
<210> 522
   <211> 80
   <212> PRT
   <213> Artificial Sequence
<220>
   <223> Synthetically generated peptide
<400> 522
   Leu Gln Val Thr Val Ile Arg Gly Lys Lys Leu Ala Ile Arg Asp Phe
   1               5                   10                  15
   Lys Ser Ser Asp Pro Tyr Val Ile Val Lys Leu Gly Asn Glu Ser Ala
               20                  25                  30
   Lys Thr Lys Val Ile Asn Asn Cys Leu Asn Pro Val Trp Asp Glu Glu
           35                  40                  45
   Leu Ser Phe Thr Leu Lys Asp Pro Ala Ala Val Leu Ser Leu Glu Val
       50                  55                  60
   Phe Asp Lys Asp Arg Phe Lys Ala Asp Asp Lys Met Gly His Ala Thr
   65                  70                  75                  80
<210> 523
   <211> 80
   <212> PRT
   <213> Artificial Sequence
<220>
   <223> Synthetically generated peptide
<400> 523
   Leu Lys Val Ile Val Val Gln Gly Lys Arg Leu Val Ile Arg Asp Phe
   1               5                   10                  15
   Lys Thr Ser Asp Pro Tyr Val Val Leu Lys Leu Gly Asn Gln Thr Ala
               20                  25                  30
   Lys Thr Lys Val Ile Asn Ser Cys Leu Asn Pro Val Trp Asn Glu Glu
           35                  40                  45
   Leu Asn Phe Thr Leu Thr Glu Pro Leu Gly Val Leu Asn Leu Glu Val
       50                  55                  60
   Phe Asp Lys Asp Leu Leu Lys Ala Asp Asp Lys Met Gly Asn Ala Phe
   65                  70                  75                  80
<210> 524
   <211> 80
   <212> PRT
   <213> Artificial Sequence
<220>
   <223> Synthetically generated peptide
<400> 524
   Leu Lys Val Val Val Ala Ser Gly Thr Asn Leu Ala Val Arg Asp Phe
   1 5 10 15
   Thr Ser Ser Asp Pro Tyr Val Val Val Arg Leu Ala Ala Met Asn Lys
   20 25 30
   Lys Thr Lys Val Ile Asn Ser Cys Leu Asn Pro Val Trp Asn Glu Glu
   35 40 45
   Met Ser Phe Ser Ile Glu Glu Pro Ala Gly Val Ile Lys Phe Glu Val
   50 55 60
   Phe Asp Trp Asp Arg Phe Lys Tyr Asp Asp Lys Met Gly His Ala Phe
   65 70 75 80
<210> 525
   <211> 80
   <212> PRT
   <213> Artificial Sequence
<220>
   <223> Synthetically generated peptide
<400> 525
   Val Lys Val Lys Val Val Arg Gly Thr Asn Leu Ala Val Arg Asp Val
   1 5 10 15
   Phe Ser Ser Asp Pro Tyr Val Val Leu Lys Leu Gly Asn Gln Glu Val
   20 25 30
   Arg Thr Arg Thr Val Arg Lys Asn Thr Asn Pro Val Trp Asn Glu Asp
   35 40 45
   Leu Thr Leu Ile Val Gln Asp Leu Asn His Leu Leu Val Thr Leu Glu
   50 55 60
   Val Tyr Asp Arg Asp Pro Phe Val Asp Asp Pro Met Gly Ala Ala Phe
   65 70 75 80
<210> 526
   <211> 94
   <212> PRT
   <213> Artificial Sequence
<220>
   <223> Synthetically generated peptide
<400> 526
   Leu Glu Leu Gly Ile Leu Gly Ala Gln Gly Ile Val Pro Met Lys Thr
   1 5 10 15
   Arg Asp Gly Lys Gly Ser Ser Asp Thr Tyr Cys Val Ala Lys Tyr Gly
   20 25 30
   Ser Lys Trp Val Arg Thr Arg Thr Ile Val Asn Asn Pro Gly Pro Lys
   35 40 45
   Phe Asn Glu Gln Tyr Thr Trp Glu Val Tyr Asp Pro Ala Thr Val Leu
   50 55 60
   Thr Val Gly Val Phe Asp Asn Gly Gln Leu Gly Glu Lys Gly Gly Glu
   65 70 75 80
   Lys Thr Ser Ser Ser Lys Asp Ala Lys Ile Gly Lys Val Arg
   85 90
<210> 527
   <211> 94
   <212> PRT
   <213> Artificial Sequence
<220>
   <223> Synthetically generated peptide
<400> 527
   Leu Glu Leu Gly Ile Leu Gly Ala Gln Gly Ile Val Pro Met Lys Thr
   1               5                   10                  15
   Arg Asp Gly Lys Gly Ser Ser Asp Thr Tyr Cys Val Ala Lys Tyr Gly
               20                  25                  30
   Ser Lys Trp Val Arg Thr Arg Thr Ile Val Asn Asn Pro Gly Pro Lys
           35                  40                  45
   Phe Asn Glu Gln Tyr Thr Trp Glu Val Tyr Asp Pro Ala Thr Val Leu
       50                  55                  60
   Thr Val Gly Val Phe Asp Asn Gly Gln Leu Gly Glu Lys Gly Gly Glu
   65                  70                  75                  80
   Lys Thr Ser Ser Ser Lys Asp Ala Lys Ile Gly Lys Val Arg
                   85                  90
<210> 528
   <211> 88
   <212> PRT
   <213> Artificial Sequence
<220>
   <223> Synthetically generated peptide
<400> 528
   Leu Glu Leu Gly Ile Leu Asn Ala Val Gly Leu His Pro Met Lys Thr
   1               5                   10                  15
   Arg Glu Gly Arg Gly Thr Ser Asp Thr Phe Cys Val Gly Lys Tyr Gly
               20                  25                  30
   Gln Lys Trp Val Arg Thr Arg Thr Met Val Asp Asn Leu Cys Pro Lys
           35                  40                  45
   Tyr Asn Glu Gln Tyr Thr Trp Glu Val Phe Asp Pro Ala Thr Val Leu
       50                  55                  60
   Thr Val Gly Val Phe Asp Asn Gly Gln Leu Gly Glu Lys Gly Asn Arg
   65                  70                  75                  80
   Asp Val Lys Ile Gly Lys Ile Arg
                   85
<210> 529
   <211> 88
   <212> PRT
   <213> Artificial Sequence
<220>
   <223> Synthetically generated peptide
<400> 529
   Leu Glu Leu Gly Ile Leu Asn Ala Val Gly Leu His Pro Met Lys Thr
   1               5                   10                  15
   Arg Glu Gly Arg Gly Thr Ser Asp Thr Phe Cys Val Gly Lys Tyr Gly
                   20                      25                      30
   Gln Lys Trp Val Arg Thr Arg Thr Met Val Asp Asn Leu Cys Pro Lys
           35                      40                      45
   Tyr Asn Glu Gln Tyr Thr Trp Glu Val Phe Asp Pro Ala Thr Val Leu
       50                      55                      60
   Thr Val Gly Val Phe Asp Asn Gly Gln Leu Gly Glu Lys Gly Asn Arg
   65                  70                      75                  80
   Asp Val Lys Ile Gly Lys Ile Arg
                   85
<210> 530
   <211> 50
   <212> PRT
   <213> Artificial Sequence
<220>
   <223> Synthetically generated peptide
<400> 530
   Arg Thr Ile Ser Asn Ser Leu Asp Pro Lys Tyr His Glu Gln Tyr Thr
    1               5                  10                  15
   Trp Glu Val Phe Asp Pro Ala Thr Val Leu Thr Val Gly Val Phe Asp
               20                  25                  30
   Asn Cys Gln Val Asn Gly Pro Asp Asn Lys Asp Leu Leu Ile Gly Lys
           35                  40                  45
   Val Arg
       50
<210> 531
   <211> 73
   <212> PRT
   <213> Artificial Sequence
<220>
   <223> Synthetically generated peptide
<400> 531
   Arg Asp Gly Arg Gly Ala Ala Asp Val Tyr Cys Val Ala Lys Tyr Gly
    1               5                  10                  15
   His Lys Trp Val Arg Thr Arg Thr Ile Val Gly Ser Leu Ser Pro Lys
               20                  25                  30
   Phe His Glu Gln Tyr Tyr Trp Glu Val Tyr Asp Pro Ser Thr Val Leu
           35                  40                  45
   Thr Leu Gly Val Phe Asn Asn Gly Gln Leu Asn Asp Ser Asn Asp Ser
       50                  55                  60
   Asn Asp Ser Lys Ile Gly Lys Val Arg
   65                  70
<210> 532
   <211> 91
   <212> PRT
   <213> Artificial Sequence
<220>
   <223> Synthetically generated peptide
<400> 532
   Leu Glu Ile Gly Ile Leu Ser Ala Thr Gly Leu Met Pro Met Lys Val
   1               5                   10                  15
   Arg Asp Gly Lys Cys Gly Gly Ile Ala Asp Ser Tyr Cys Val Ala Lys
               20                  25                  30
   Tyr Gly Pro Lys Trp Val Arg Thr Arg Thr Val Val Asp Ser Leu Cys
           35                  40                  45
   Pro Lys Trp Asn Glu Gln Tyr Thr Trp Glu Val Tyr Asp Pro Cys Thr
       50                  55                  60
   Val Val Thr Val Gly Val Phe Asp Asn Ala Arg Val Asn Glu Asn Asn
   65                  70                  75                  80
   Asn Ser Arg Asp Val Arg Ile Gly Lys Val Arg
                   85                  90
<210> 533
   <211> 71
   <212> PRT
   <213> Artificial Sequence
<220>
   <223> Synthetically generated peptide
<400> 533
   Leu Val Leu Gly Val Ile Ser Ala Ser Gly Ser Ile Pro Met Lys Ser
   1               5                   10                  15
   Arg Asp Gly Arg Gly Thr Thr Asp Ala Tyr Cys Val Ala Lys Tyr Gly
               20                  25                  30
   Gln Lys Trp Val Arg Thr Arg Thr Ile Val Asp Ser Leu Ser Pro Lys
           35                  40                  45
   Trp Ser Glu Gln Tyr Thr Trp Glu Val Tyr Asp Pro Tyr Thr Val Ile
       50                  55                  60
   Thr Val Ala Val Phe Asp Asn
   65                  70
<210> 534
   <211> 93
   <212> PRT
   <213> Artificial Sequence
<220>
   <223> Synthetically generated peptide
<400> 534
   Leu Glu Leu Gly Val Leu Asn Ala Thr Gly Leu Met Pro Met Lys Ser
   1 5 10 15
   Arg Gly Gly Arg Gly Thr Thr Asp Ala Tyr Cys Val Ala Lys Tyr Gly
   20 25 30
   Thr Lys Trp Val Arg Thr Arg Thr Ile Val Asp Thr Phe Asp Pro Lys
   35 40 45
   Trp Asn Glu Gln Tyr Thr Trp Glu Val Tyr Asp Pro Tyr Thr Val Ile
   50 55 60
   Thr Ile Gly Val Phe Asp Asn Leu Lys Leu Phe Gly Ala Gly Asn Glu
   65 70 75 80
   Asn Arg Leu Ile Asn Asp Ser Arg Ile Gly Lys Ile Arg
   85 90
<210> 535
   <211> 92
   <212> PRT
   <213> Artificial Sequence
<220>
   <223> Synthetically generated peptide
<400> 535
   Leu Glu Leu Gly Ile Leu Asn Ala Thr Gly Leu Met Pro Met Lys Thr
   1 5 10 15
   Lys Asp Gly Arg Gly Thr Thr Asp Ala Tyr Cys Val Ala Lys Tyr Gly
   20 25 30
   Gln Lys Trp Ile Arg Thr Arg Thr Ile Ile Asp Ser Phe Thr Pro Arg
   35 40 45
   Trp Asn Glu Gln Tyr Thr Trp Glu Val Phe Asp Pro Cys Thr Val Val
   50 55 60
   Thr Val Gly Val Phe Asp Asn Cys His Leu His Gly Gly Glu Lys Ile
   65 70 75 80
   Gly Gly Ala Lys Asp Ser Arg Ile Gly Lys Val Arg
   85 90
<210> 536
   <211> 92
   <212> PRT
   <213> Artificial Sequence
<220>
   <223> Synthetically generated peptide
<400> 536
   Leu Glu Leu Gly Ile Leu Asn Ala Thr Gly Leu Met Pro Met Lys Thr
   1 5 10 15
   Lys Asp Gly Arg Gly Thr Thr Asp Ala Tyr Cys Val Ala Lys Tyr Gly
   20 25 30
   Gln Lys Trp Ile Arg Thr Arg Thr Ile Ile Asp Ser Phe Thr Pro Arg
   35 40 45
   Trp Asn Glu Gln Tyr Thr Trp Glu Val Phe Asp Pro Cys Thr Val Val
   50 55 60
   Thr Val Gly Val Phe Asp Asn Cys His Leu His Gly Gly Glu Lys Ile
   65 70 75 80
   Gly Gly Ala Lys Asp Ser Arg Ile Gly Lys Val Arg
   85 90
<210> 537
   <211> 94
   <212> PRT
   <213> Artificial Sequence
<220>
   <223> Synthetically generated peptide
<400> 537
   Leu Glu Leu Gly Val Leu Asn Ala Thr Gly Leu Met Pro Met Lys Ala
   1 5 10 15
   Lys Glu Gly Gly Arg Gly Thr Thr Asp Ala Tyr Cys Val Ala Lys Tyr
   20 25 30
   Gly Gln Lys Trp Ile Arg Thr Arg Thr Ile Ile Asp Ser Phe Thr Pro
   35 40 45
   Arg Trp Asn Glu Gln Tyr Thr Trp Glu Val Phe Asp Pro Cys Thr Val
   50 55 60
   Val Thr Val Gly Val Phe Asp Asn Cys His Leu His Gly Gly Asp Lys
   65 70 75 80
   Asn Asn Gly Gly Gly Lys Asp Ser Arg Ile Gly Lys Val Arg
   85 90
<210> 538
   <211> 92
   <212> PRT
   <213> Artificial Sequence
<220>
   <223> Synthetically generated peptide
<400> 538
   Leu Glu Leu Gly Ile Leu Thr Ala Gln Gly Leu Leu Pro Met Lys Thr
   1 5 10 15
   Lys Asp Gly Arg Gly Thr Thr Asp Ala Tyr Cys Val Ala Lys Tyr Gly
   20 25 30
   Gln Lys Trp Val Arg Thr Arg Thr Ile Ile Asp Ser Phe Thr Pro Lys
   35 40 45
   Trp Asn Glu Gln Tyr Thr Trp Glu Val Tyr Asp Pro Cys Thr Val Ile
   50 55 60
   Thr Ile Gly Val Phe Asp Asn Cys His Leu Asn Gly Gly Glu Lys Ala
   65 70 75 80
   Asn Gly Ala Arg Asp Thr Arg Ile Gly Lys Val Arg
   85 90
<210> 539
   <211> 94
   <212> PRT
   <213> Artificial Sequence
<220>
   <223> Synthetically generated peptide
<400> 539
   Leu Glu Val Gly Ile Leu Ser Ala Gln Gly Leu Ser Pro Met Lys Thr
   1 5 10 15
   Lys Asp Gly Lys Ala Thr Thr Asp Pro Tyr Cys Val Ala Lys Tyr Gly
   20 25 30
   Gln Lys Trp Val Arg Thr Arg Thr Ile Ile Asp Ser Ser Ser Pro Lys
   35 40 45
   Trp Asn Glu Gln Tyr Thr Trp Glu Val Tyr Asp Pro Cys Thr Val Ile
   50 55 60
   Thr Leu Gly Val Phe Asp Asn Cys His Leu Gly Gly Ser Glu Lys Ser
   65 70 75 80
   Asn Ser Gly Ala Lys Val Asp Ser Arg Ile Gly Lys Val Arg
   85 90
<210> 540
   <211> 94
   <212> PRT
   <213> Artificial Sequence
<220>
   <223> Synthetically generated peptide
<400> 540
   Leu Glu Val Gly Ile Leu Ser Ala Gln Gly Leu Ser Pro Met Lys Thr
   1 5 10 15
   Lys Asp Gly Lys Ala Thr Thr Asp Pro Tyr Cys Val Ala Lys Tyr Gly
   20 25 30
   Gln Lys Trp Val Arg Thr Arg Thr Ile Ile Asp Ser Ser Ser Pro Lys
   35 40 45
   Trp Asn Glu Gln Tyr Thr Trp Glu Val Tyr Asp Pro Cys Thr Val Ile
   50 55 60
   Thr Leu Gly Val Phe Asp Asn Cys His Leu Gly Gly Ser Glu Lys Ser
   65 70 75 80
   Asn Ser Gly Ala Lys Val Asp Ser Arg Ile Gly Lys Val Arg
   85 90
<210> 541
   <211> 88
   <212> PRT
   <213> Artificial Sequence
<220>
   <223> Synthetically generated peptide
<400> 541
   Leu Glu Val Gly Ile Ile Ser Ala His Gly Leu Met Pro Met Lys Ser
   1 5 10 15
   Lys Asp Gly Lys Gly Thr Thr Asp Ala Tyr Cys Val Ala Lys Tyr Gly
   20 25 30
   Gln Lys Trp Ile Arg Thr Arg Thr Ile Val Asp Ser Phe Thr Pro Lys
   35 40 45
   Trp Asn Glu Gln Tyr Thr Trp Glu Val Phe Asp Thr Cys Thr Val Ile
   50 55 60
   Thr Phe Gly Ala Phe Asp Asn Gly His Ile Pro Gly Gly Ser Gly Lys
   65 70 75 80
   Asp Leu Arg Ile Gly Lys Val Arg
   85
<210> 542
   <211> 93
   <212> PRT
   <213> Artificial Sequence
<220>
   <223> Synthetically generated peptide
<400> 542
   Leu Glu Met Gly Ile Leu Gly Ala Lys Gly Leu Leu Pro Met Lys Met
   1 5 10 15
   Lys Asp Gly His Gly Ser Thr Asp Ala Tyr Cys Val Ala Lys Tyr Gly
   20 25 30
   Gln Lys Trp Ile Arg Thr Arg Thr Leu Leu Asp Thr Phe Ser Pro Lys
   35 40 45
   Trp Asn Glu Gln Tyr Thr Trp Glu Val Tyr Asp Pro Cys Thr Val Ile
   50 55 60
   Thr Leu Gly Val Phe Asp Asn Cys His Leu Gly Glu Lys Ala Pro Ser
   65 70 75 80
   Gly Ser Ser Ile Lys Asp Ser Arg Ile Gly Lys Val Arg
   85 90
<210> 543
   <211> 98
   <212> PRT
   <213> Artificial Sequence
<220>
   <223> synthetically generated peptide
<400> 543
   Leu Glu Val Gly Ile Leu Gly Ala Gln Lys Leu Leu Pro Met Lys Met
   1               5                   10                  15
   Asn Asn Ser Arg Gly Ser Thr Asp Ala Tyr Cys Val Ala Lys Tyr Gly
               20                  25                  30
   Gln Lys Trp Ile Arg Thr Arg Thr Ile Leu Asp Thr Phe Ser Pro Lys
           35                  40                  45
   Trp Asn Glu Gln Tyr Thr Trp Glu Val Tyr Asp Pro Cys Thr Val Ile
       50                  55                  60
   Thr Leu Gly Val Phe Asp Asn Cys His Leu Gly Gly Gly Gly Glu Lys
   65                  70                  75                  80
   Ala Pro Ser Gly Gly Ser Asn Ala Ala Arg Asp Ser Arg Ile Gly Lys
                   85                  90                  95
   Val Arg
<210> 544
   <211> 93
   <212> PRT
   <213> Artificial Sequence
<220>
   <223> Synthetically generated peptide
<400> 544
   Leu Glu Ile Gly Ile Leu Gly Ala Asn Gly Leu Val Pro Met Lys Leu
   1               5                   10                  15
   Lys Asp Gly Arg Gly Ser Thr Asn Ala Tyr Cys Val Ala Lys Tyr Gly
               20                  25                  30
   Gln Lys Trp Val Arg Thr Arg Thr Ile Leu Asp Thr Leu Ser Pro Arg
           35                  40                  45
   Trp Asn Glu Gln Tyr Thr Trp Glu Val Tyr Asp Pro Cys Thr Val Ile
       50                  55                  60
   Thr Leu Gly Val Phe Asp Asn Ser His Leu Gly Ser Ala Gln Ser Gly
   65                  70                  75                  80
   Thr Ala Asp Ser Arg Asp Ala Arg Ile Gly Lys Val Arg
                   85                  90
<210> 545
   <211> 103
   <212> PRT
   <213> Artificial Sequence
<220>
   <223> synthetically generated peptide
<400> 545
   Leu Glu Val Gly Ile Leu Gly Ala Ala Gly Leu Gln Pro Met Lys Asn
   1               5                   10                  15
   Arg Asp Gly Arg Gly Thr Thr Asp Ala Tyr Cys Val Ala Lys Tyr Gly
               20                  25                  30
   Gln Lys Trp Val Arg Thr Arg Thr Met Leu Gly Thr Phe Ser Pro Thr
           35                  40                  45
   Trp Asn Glu Gln Tyr Thr Trp Glu Val Phe Asp Pro Cys Thr Val Ile
       50                  55                  60
   Thr Ile Gly Val Phe Asp Asn Asn His Leu Gly Asn Gly Asn Gly Asn
   65                  70                  75                  80
   Gly Asn Asn Ala Gly Gly Gly Gly Gly Gly Ser Pro Pro Ala Arg Asp
                   85                  90                  95
   Ala Arg Val Gly Lys Ile Arg
               100
<210> 546
   <211> 86
   <212> PRT
   <213> Artificial Sequence
<220>
   <223> Synthetically generated peptide
<400> 546
   Leu Glu Met Gly Ile Leu Gly Ala His Gly Leu Pro Pro Met Lys Ser
   1               5                   10                  15
   Lys Asp Gly Trp Thr Thr Thr Asp Ala Tyr Cys Val Ala Lys Phe Gly
               20                  25                  30
   Thr Lys Trp Val Arg Thr Arg Thr Ile Thr Asn Asn Phe His Pro Lys
           35                  40                  45
   Trp Asn Glu Gln Tyr Thr Trp Glu Val Phe Asp Pro Cys Ser Ile Ile
       50                  55                  60
   Thr Ile Gly Val Phe Asp Asn Asn Phe His Leu Gln Gly Gly Asp Lys
   65                  70                  75                  80
   Arg Ile Gly Lys Val Arg
                   85
<210> 547
   <211> 92
   <212> PRT
   <213> Artificial Sequence
<220>
   <223> synthetically generated peptide
<400> 547
   Leu Glu Val Gly Val Leu Gly Ala Gln Gly Leu Pro Pro Met Lys Thr
    1                       5                                  10                          15
   Ala Ala Asp Gly Gly Arg Gly Thr Thr Asp Ala Tyr Cys Val Ala Lys
                   20                      25                      30
   Tyr Gly His Lys Trp Val Arg Thr Arg Thr Val Val Asp Ser Ser Thr
           35                      40                      45
   Pro Arg Trp Asn Glu Gln Tyr Thr Trp Glu Val Tyr Asp Pro Cys Thr
       50                      55                      60
   Val Leu Thr Leu Ala Val Phe Asp Asn Cys Asn Leu Gly Asn Gly Gly
   65                      70                      75                      80
   Gly Gly Gly Lys Asp Gln Arg Ile Gly Lys Val Arg
                   85                      90

<210> 548
   <211> 96
   <212> PRT
   <213> Artificial Sequence
<220>
   <223> synthetically generated peptide
<400> 548
   Leu Glu Leu Gly Val Leu Gly Ala Thr Gly Leu Ile Pro Met Lys Ala
    1               5                  10                  15
   Arg Asp Gly Arg Gly Ala Thr Ser Asp Ala Tyr Cys Val Ala Lys Tyr
               20                  25                  30
   Gly Gln Lys Trp Ile Arg Thr Arg Thr Val Val Asp Ser Val Cys Pro
           35                  40                  45
   Arg Trp Asn Glu Gln Tyr Thr Trp Glu Val Phe Asp Pro Cys Thr Val
       50                  55                  60
   Ile Thr Val Gly Val Phe Asp Asn Cys His Val Asp Lys Pro Ala Ser
   65                  70                  75                  80
   Gly Asn Thr Thr Leu Ala Val Arg Asp Asn Cys Ile Gly Lys Val Arg
                   85                  90                  95
<210> 549
   <211> 86
   <212> PRT
   <213> Artificial Sequence
<220>
   <223> Synthetically generated peptide
<400> 549
   Leu Glu Leu Gly Ile Leu Ser Ala Arg Asn Leu Met Pro Met Lys Gly
    1               5                  10                  15
   Lys Asp Gly Arg Met Thr Asp Pro Tyr Cys Val Ala Lys Tyr Gly Asn
               20                  25                  30
   Lys Trp Val Arg Thr Arg Thr Leu Leu Asp Ala Pro Lys Trp Asn Glu
           35                  40                  45
   Gln Tyr Thr Trp Glu Val His Asp Pro Cys Thr Val Ile Thr Ile Gly
       50                  55                  60
   Val Phe Asp Asn Ser His Val Asn Asp Gly Gly Asp Phe Lys Asp Gln
   65                  70                  75                  80
   Arg Ile Gly Lys Val Arg
                   85
<210> 550
   <211> 86
   <212> PRT
   <213> Artificial Sequence
<220>
   <223> synthetically generated peptide
<400> 550
   Leu Glu Leu Gly Ile Leu Ser Ala Arg Asn Leu Met Pro Met Lys Gly
   1               5                   10                  15
   Lys Asp Gly Arg Met Thr Asp Pro Tyr Cys Val Ala Lys Tyr Gly Asn
               20                  25                  30
   Lys Trp Val Arg Thr Arg Thr Leu Leu Asp Ala Pro Lys Trp Asn Glu
           35                  40                  45
   Gln Tyr Thr Trp Glu Val His Asp Pro Cys Thr Val Ile Thr Ile Gly
       50                  55                  60
   Val Phe Asp Asn Ser His Val Asn Asp Gly Gly Asp Phe Lys Asp Gln
   65                  70                  75                  80
   Arg Ile Gly Lys Val Arg
                   85
<210> 551
   <211> 88
   <212> PRT
   <213> Artificial Sequence
<220>
   <223> Synthetically generated peptide
<400> 551
   Leu Glu Leu Gly Ile Leu Gly Ala Arg Asn Leu Ile Pro Met Lys Gly
   1               5                   10                  15
   Lys Asp Gly Arg Thr Thr Asp Ala Tyr Cys Val Ala Lys Tyr Gly Pro
               20                  25                  30
   Lys Trp Val Arg Thr Arg Thr Ile Leu Asn Thr Leu Asn Pro Gln Trp
           35                  40                  45
   Asn Glu Gln Tyr Thr Trp Glu Val Phe Asp Pro Cys Thr Val Ile Thr
       50                  55                  60
   Val Val Val Phe Asp Asn Asn Gln Ile Gly Lys Asn Gly Asp Ala Arg
   65                  70                  75                  80
   Asp Glu Ser Ile Gly Lys Val Arg
                   85
<210> 552
   <211> 81
   <212> PRT
   <213> Artificial Sequence
<220>
   <223> synthetically generated peptide
<400> 552
   Leu Glu Leu Gly Ile Leu Gly Ala Arg Asn Leu Ala Gly Gly Lys Ser
   1 5 10 15
   Pro Tyr Cys Val Ala Lys Tyr Gly Ala Lys Trp Val Arg Thr Arg Thr
   20 25 30
   Leu Val Gly Thr Ala Ala Pro Arg Trp Asn Glu Gln Tyr Thr Trp Glu
   35 40 45
   Val Phe Asp Leu Cys Thr Val Val Thr Val Ala Val Phe Asp Asn Cys
   50 55 60
   His Leu Thr Gly Gly Gly Asp Ala Lys Asp Gln Arg Ile Gly Lys Val
   65 70 75 80
   Arg
<210> 553
   <211> 73
   <212> PRT
   <213> Artificial Sequence
<220>
   <223> Synthetically generated peptide
<400> 553
   Arg Lys Gly Thr Ser Asp Thr Tyr Val Val Ala Lys Tyr Gly His Lys
   1 5 10 15
   Trp Val Arg Ser Arg Thr Val Ile Asn Ser Met Asn Pro Lys Tyr Asn
   20 25 30
   Glu Gln Tyr Thr Trp Glu Val Phe Asp Pro Ala Thr Val Leu Thr Ile
   35 40 45
   Cys Val Phe Asp Asn Ala His Phe Ala Ala Gly Asp Gly Gly Asn Lys
   50 55 60
   Arg Asp Gln Pro Ile Gly Lys Val Arg
   65 70
<210> 554
   <211> 91
   <212> PRT
   <213> Artificial Sequence
<220>
   <223> synthetically generated peptide
<400> 554
   Leu Glu Leu Gly Ile Leu Asn Ala Asn Val Phe His Ser Met Lys Thr
   1 5 10 15
   Arg Glu Gly Lys Gly Thr Ser Asp Thr Tyr Val Val Ala Lys Tyr Gly
   20 25 30
   His Lys Trp Val Arg Ser Arg Thr Val Ile Asn Ser Met Asn Pro Lys
   35 40 45
   Tyr Asn Glu Gln Tyr Thr Trp Glu Val Phe Asp Pro Ala Thr Val Leu
   50 55 60
   Thr Ile Cys Val Phe Asp Asn Ala His Phe Ala Ala Gly Asp Gly Gly
   65 70 75 80
   Asn Lys Arg Asp Gln Pro Ile Gly Lys Val Arg
   85 90
<210> 555
   <211> 87
   <212> PRT
   <213> Artificial Sequence
<220>
   <223> synthetically generated peptide
<400> 555
   Leu Glu Leu Gly Ile Leu Arg Ile Glu Gln Leu Ser Gln Glu Gly Lys
   1               5                   10                  15
   Lys Glu Thr Val Asp Ala Tyr Cys Val Ala Lys Tyr Gly Thr Lys Trp
               20                  25                  30
   Val Arg Thr Arg Thr Val Thr Asn Cys Leu Asn Pro Arg Phe Asn Glu
           35                  40                  45
   Gln Tyr Thr Trp Glu Val Tyr Glu Pro Ala Thr Val Ile Thr Ile Gly
       50                  55                  60
   Val Phe Asp Asn Asn Gln Ile Asn Ser Gly Asn Gly Asn Lys Gly Asp
   65                  70                  75                  80
   Gly Lys Ile Gly Lys Ile Arg
                   85
<210> 556
   <211> 91
   <212> PRT
   <213> Artificial Sequence
<220>
   <223> Synthetically generated peptide
<400> 556
   Leu Glu Val Gly Ile Leu Ser Ala Asn Gln Pro Thr Lys Thr Lys His
   1               5                   10                  15
   Glu Arg Gly Ser Cys Asp Ala Tyr Cys Val Ala Lys Tyr Gly Gln Lys
               20                  25                  30
   Trp Val Arg Thr Arg Thr Ile Val Asp Asn Leu Asn Pro Arg Phe Asn
           35                  40                  45
   Glu Gln Tyr Thr Trp Asp Val Phe Asp His Gly Thr Val Leu Thr Ile
       50                  55                  60
   Gly Leu Phe Asp Asn Cys His Ala Asp Ser Asn His Ser Ser Ser Pro
   65                  70                  75                  80
   Gly His Met Asp Lys Pro Ile Gly Lys Val Arg
                   85                  90
<210> 557
   <211> 79
   <212> PRT
   <213> Artificial Sequence
<220>
   <223> synthetically generated peptide
<400> 557
   Arg Asp Gly Arg Gly Ser Cys Asp Ala Tyr Cys Val Ala Lys Tyr Gly
   1               5                   10                  15
   Val Lys Trp Tyr Arg Thr Arg Thr Val Thr Asp Ser Ile Ser Pro Arg
               20                  25                  30
   Phe His Gln Gln Tyr His Trp Glu Val His Asp His Cys Thr Val Leu
           35                  40                  45
   Thr Val Ala Val Phe His Asn Ser Gln Ile Gly Asp Lys Gly Gly Leu
       50                  55                  60
   Val Ala Gly Asp Pro Val Lys Asp Val Leu Leu Gly Lys Val Arg
   65                  70                  75
<210> 558
   <211> 94
   <212> PRT
   <213> Artificial Sequence
<220>
   <223> synthetically generated peptide
<400> 558
   Val Glu Leu Gly Ile Ile Gly Cys Lys Asn Leu Leu Pro Met Lys Thr
   1               5                   10                  15
   Val Asn Gly Lys Gly Ser Thr Asp Ala Tyr Thr Val Ala Lys Tyr Gly
               20                  25                  30
   Ser Lys Trp Val Arg Thr Arg Thr Val Ser Asp Ser Leu Asp Pro Lys
           35                  40                  45
   Trp Asn Glu Gln Tyr Thr Trp Lys Val Tyr Asp Pro Cys Thr Val Leu
       50                  55                  60
   Thr Ile Gly Val Phe Asp Ser Trp Gly Val Tyr Glu Val Asp Gly Gly
   65                  70                  75                  80
   Lys Glu Ala Thr Arg Gln Asp Leu Arg Ile Gly Lys Val Arg
                   85                  90
<210> 559
   <211> 98
   <212> PRT
   <213> Artificial Sequence
<220>
   <223> Synthetically generated peptide
<400> 559
   Val Glu Leu Gly Ile Val Gly Cys Lys Gly Leu Leu Pro Met Arg Thr
   1               5                   10                  15
   Ala Asp Gly Lys Gly Cys Thr Asp Ala Tyr Ala Val Ala Lys Tyr Gly
               20                  25                  30
   Pro Lys Trp Ala Arg Thr Arg Thr Ile Ser Asp Ser Phe Asp Pro Ala
           35                  40                  45
   Trp Asn Glu Gln Tyr Thr Trp Pro Val Tyr Asp Pro Cys Thr Val Leu
       50                  55                  60
   Thr Val Gly Val Phe Asp Asp Pro Pro Pro Pro Ser Pro Ser Gln Leu
   65                  70                  75                  80
   Pro Asp Gly Ala Lys Asp Ala Ala Ala Phe Ser Arg Pro Met Gly Lys
                   85                  90                  95
   Val Arg
<210> 560
   <211> 90
   <212> PRT
   <213> Artificial Sequence
<220>
   <223> synthetically generated peptide
<400> 560
   Leu Glu Leu Gly Ile Leu Gly Arg Leu Leu Pro Met Lys Ala Lys Asn
   1               5                   10                  15
   Gly Gly Lys Gly Ser Thr Asp Ala Tyr Cys Val Ala Lys Tyr Gly Lys
               20                  25                  30
   Lys Trp Val Arg Thr Arg Thr Ile Thr Asp Ser Phe Asp Pro Arg Trp
           35                  40                  45
   His Glu Gln Tyr Thr Trp Gln Val Tyr Asp Pro Cys Thr Val Leu Thr
       50                  55                  60
   Val Gly Val Phe Asp Asn Trp Arg Met Phe Ser Asp Ala Ser Asp Asp
   65                  70                  75                  80
   Arg Pro Asp Thr Arg Ile Gly Lys Ile Arg
                   85                  90
<210> 561
   <211> 90
   <212> PRT
   <213> Artificial Sequence
<220>
   <223> synthetically generated peptide
<400> 561
   Leu Glu Leu Gly Ile Leu Gly Arg Leu Leu Pro Met Lys Ala Lys Asn
   1               5                   10                  15
   Gly Gly Lys Gly Ser Thr Asp Ala Tyr Cys Val Ala Lys Tyr Gly Lys
               20                  25                  30
   Lys Trp Val Arg Thr Arg Thr Ile Thr Asp Ser Phe Asp Pro Arg Trp
           35                  40                  45
   His Glu Gln Tyr Thr Trp Gln Val Tyr Asp Pro Cys Thr Val Leu Thr
       50                  55                  60
   Val Gly Val Phe Asp Asn Trp Arg Met Phe Ser Asp Ala Ser Asp Asp
   65                  70                  75                  80
   Arg Pro Asp Thr Arg Ile Gly Lys Ile Arg
                   85                  90
<210> 562
   <211> 95
   <212> PRT
   <213> Artificial Sequence
<220>
   <223> synthetically generated peptide
<400> 562
   Leu Glu Leu Gly Ile Ile Gly Ala Cys Gly Leu Leu Pro Met Lys Thr
   1               5                   10                  15
   Lys Gly Gly Ala Lys Gly Ser Thr Asp Ala Tyr Cys Val Ala Lys Tyr
               20                  25                  30
   Gly Lys Lys Trp Val Arg Thr Arg Thr Val Thr Asp Ser Leu Asn Pro
           35                  40                  45
   Arg Trp Asn Glu Gln Tyr Thr Trp Gln Val Tyr Asp Pro Cys Thr Val
       50                  55                  60
   Leu Thr Val Ala Val Phe Asp Asn Trp Arg Met Phe Ala Phe Ala Gly
   65                  70                  75                  80
   Ala Gly Asp Glu Gln Arg Gln Asp Tyr Arg Ile Gly Lys Val Arg
                   85                  90                  95
<210> 563
   <211> 98
   <212> PRT
   <213> Artificial Sequence
<220>
   <223> Synthetically generated peptide
<400> 563
   Leu Glu Val Gly Ile Arg Gly Ala Ala Asn Leu Val Pro Met Lys Ile
   1               5                   10                  15
   Ala Lys Asp Gly Ala Ser Gly Ser Thr Asp Ala Tyr Val Val Leu Lys
               20                  25                  30
   Tyr Gly Pro Lys Trp Ala Arg Thr Arg Thr Ile Leu Asp Gln Phe Asn
           35                  40                  45
   Pro Arg Trp Asn Glu Gln Tyr Ala Trp Asp Val Phe Asp Pro Cys Thr
       50                  55                  60
   Val Leu Thr Ile Ala Val Phe Asp Asn Val Arg Tyr Arg Ser Ala Glu
   65                  70                  75                  80
   Ala Ser Gly Asp Ala Gly Lys Leu Pro Lys Asp Ala Arg Ile Gly Lys
                   85                  90                  95
   Leu Arg
<210> 564
   <211> 93
   <212> PRT
   <213> Artificial Sequence
<220>
   <223> Synthetically generated peptide
<400> 564
   Leu Glu Val Gly Ile Arg Gly Ala Thr Asn Leu Leu Pro Val Lys Thr
   1               5                   10                  15
   Arg Asp Gly Thr Arg Gly Thr Thr Asp Ala Tyr Val Val Ala Lys Tyr
               20                  25                  30
   Gly Pro Lys Trp Ile Arg Thr Arg Thr Ile Leu Asp Arg Phe Asn Pro
           35                  40                  45
   Arg Trp Asn Glu Gln Tyr Thr Trp Asp Val Tyr Asp Pro Cys Thr Val
       50                  55                  60
   Leu Thr Ile Gly Val Phe Asp Asn Gly Arg Tyr Lys Arg Asp Glu Ser
   65                  70                  75                  80
   Gly Lys Gln Gly Arg Asp Val Arg Val Gly Lys Ile Arg
                   85                  90
<210> 565
   <211> 87
   <212> PRT
   <213> Artificial Sequence
<220>
   <223> Synthetically generated peptide
<400> 565
   Leu Glu Ile Gly Ile Leu Gly Ala Thr Gly Leu Lys Gly Ser Asp Glu
   1               5                   10                  15
   Arg Lys Gln Gly Ile Asp Ser Tyr Val Val Ala Lys Tyr Gly Asn Lys
               20                  25                  30
   Trp Ala Arg Thr Arg Thr Val Val Asn Ser Val Thr Pro Lys Trp Asn
           35                  40                  45
   Glu Gln Tyr Ser Trp Asp Asp Tyr Glu Lys Cys Thr Val Leu Thr Leu
       50                  55                  60
   Gly Ile Tyr Asp Asn Arg Gln Ile Phe Lys Glu Asp Gln Ala Asn Asp
   65                  70                  75                  80
   Val Pro Ile Gly Lys Val Arg
                   85
<210> 566
   <211> 86
   <212> PRT
   <213> Artificial Sequence
<220>
   <223> Synthetically generated peptide
<400> 566
   Leu Glu Ile Gly Ile Leu Gly Ala Thr Gly Leu Lys Gly Ser Asp Glu
   1               5                   10                  15
   Lys Lys Gln Thr Ile Asp Ser Tyr Val Val Ala Lys Tyr Gly Asn Lys
               20                  25                  30
   Trp Ala Arg Thr Arg Thr Val Val Asn Ser Val Ser Pro Lys Trp Asn
           35                  40                  45
   Glu Gln Tyr Ser Trp Asp Val Tyr Glu Lys Cys Thr Val Leu Thr Leu
       50                  55                  60
   Gly Ile Tyr Asp Asn Arg Gln Ile Asp Lys Asn Lys Ala Asn Asp Val
   65                  70                  75                  80
   Pro Ile Gly Lys Val Arg
                   85
<210> 567
   <211> 83
   <212> PRT
   <213> Artificial Sequence
<220>
   <223> Synthetically generated peptide
<400> 567
   Val His Leu Gly Ile Leu Arg Ala Thr Gly Leu Pro Leu Arg Met Gly
   1               5                   10                  15
   Lys Ser Thr Val Asn Pro Tyr Cys Val Ala Lys Tyr Gly Asp Lys Trp
               20                  25                  30
   Val Arg Thr Arg Thr Ile Leu Asp Gly Pro Glu His Val Phe Asn Glu
           35                  40                  45
   Gln His Thr Trp Ser Val Tyr Asp Ile Ala Thr Val Leu Thr Ala Gly
       50                  55                  60
   Val Phe Asp His Phe Pro His Thr Arg Lys Ala His Arg Glu Ile Gly
   65                  70                  75                  80
   Lys Val Gln
<210> 568
   <211> 83
   <212> PRT
   <213> Artificial Sequence
<220>
   <223> synthetically generated peptide
<400> 568
   Leu Ser Val Thr Val Ile Ser Gly Glu Asp Leu Pro Ala Met Asp Met
   1               5                   10                  15
   Asn Gly Lys Ser Asp Pro Tyr Val Val Leu Ser Leu Lys Lys Ser Lys
               20                  25                  30
   Thr Lys Tyr Lys Thr Arg Val Val Ser Glu Ser Leu Asn Pro Val Trp
           35                  40                  45
   Asn Gln Thr Phe Asp Phe Val Val Glu Asp Gly Leu His Asp Met Leu
       50                  55                  60
   Met Leu Glu Val Tyr Asp His Asp Thr Phe Ser Arg Asp Tyr Met Gly
   65                  70                  75                  80
   Arg Cys Ile
<210> 569
   <211> 83
   <212> PRT
   <213> Artificial Sequence
<220>
   <223> synthetically generated peptide
<400> 569
   Leu Ser Val Thr Val Ile Ser Gly Glu Asp Leu Pro Ala Met Asp Met
    1               5                  10                  15
   Asn Gly Lys Ser Asp Pro Tyr Val Val Leu Ser Leu Lys Lys Ser Lys
               20                  25                  30
   Thr Lys Tyr Lys Thr Arg Val Val Ser Glu Ser Leu Asn Pro Val Trp
           35                  40                  45
   Asn Gln Thr Phe Asp Phe Val Val Glu Asp Gly Leu His Asp Met Leu
       50                  55                  60
   Met Leu Glu Val Tyr Asp His Asp Thr Phe Ser Arg Asp Tyr Met Gly
   65                  70                  75                  80
   Arg Cys Ile
<210> 570
   <211> 46
   <212> PRT
   <213> Artificial Sequence
<220>
   <223> Synthetically generated peptide
<400> 570
   Arg Val Val Asn Glu Ser Leu Asn Pro Val Trp Asn Gln Thr Phe Asp
    1               5                  10                  15
   Phe Val Val Glu Asp Gly Leu His Asp Met Leu Val Leu Glu Val Tyr
               20                  25                  30
   Asp His Asp Thr Phe Ser Arg Asp Tyr Met Gly Arg Cys Ile
           35                  40                  45
<210> 571
   <211> 83
   <212> PRT
   <213> Artificial Sequence
<220>
   <223> synthetically generated peptide
<400> 571
   Leu Ser Val Thr Val Ile Ser Ala Glu Glu Ile Pro Ile Gln Asp Leu
    1               5                  10                  15
   Met Gly Lys Ala Asp Pro Tyr Val Val Leu Ser Met Lys Lys Ser Gly
               20                  25                  30
   Ala Lys Ser Lys Thr Arg Val Val Asn Asp Ser Leu Asn Pro Val Trp
           35                  40                  45
   Asn Gln Thr Phe Asp Phe Val Val Glu Asp Gly Leu His Asp Met Leu
       50                  55                  60
   Val Leu Glu Val Trp Asp His Asp Thr Phe Gly Lys Asp Tyr Ile Gly
   65                  70                  75                  80
   Arg Cys Ile
<210> 572
   <211> 83
   <212> PRT
   <213> Artificial Sequence
<220>
   <223> Synthetically generated peptide
<400> 572
   Leu Ser Val Thr Val Ile Ser Ala Glu Glu Ile Pro Ile Gln Asp Leu
   1               5                   10                  15
   Met Gly Lys Ala Asp Pro Tyr Val Val Leu Ser Met Lys Lys Ser Gly
               20                  25                  30
   Ala Lys Ser Lys Thr Arg Val Val Asn Asp Ser Leu Asn Pro Val Trp
           35                  40                  45
   Asn Gln Thr Phe Asp Phe Val Val Glu Asp Gly Leu His Asp Met Leu
       50                  55                  60
   Val Leu Glu Val Trp Asp His Asp Thr Phe Gly Lys Asp Tyr Ile Gly
   65                  70                  75                  80
   Arg Cys Ile
<210> 573
   <211> 83
   <212> PRT
   <213> Artificial Sequence
<220>
   <223> Synthetically generated peptide
<400> 573
   Leu Ser Val Thr Val Ile Ser Ala Glu Asp Leu Pro Ile Val Asp Phe
   1               5                   10                  15
   Met Gly Lys Ala Asp Pro Phe Val Val Leu Ala Leu Lys Lys Ser Glu
               20                  25                  30
   Lys Lys Gln Lys Thr Arg Val Val Asn Glu Thr Leu Asn Pro Val Trp
           35                  40                  45
   Asn Gln Thr Phe Asp Phe Val Val Glu Asp Gly Leu His Asp Met Leu
       50                  55                  60
   Ile Val Glu Leu Trp Asp His Asp Thr Phe Gly Lys Glu Lys Met Gly
   65                  70                  75                  80
   Lys Val Ile
<210> 574
   <211> 83
   <212> PRT
   <213> Artificial Sequence
<220>
   <223> synthetically generated peptide
<400> 574
   Leu Ser Val Thr Val Ile Ser Ala Glu Asp Leu Pro Ala Val Asp Phe
    1               5                  10                  15
   Met Gly Lys Ser Asp Pro Phe Val Val Leu Thr Leu Lys Lys Ala Glu
               20                  25                  30
   Thr Lys Asn Lys Thr Arg Val Val Asn Asn Ser Leu Asn Pro Val Trp
           35                  40                  45
   Asn Gln Thr Phe Asp Phe Val Val Glu Asp Gly Leu His Asp Met Leu
       50                  55                  60
   Leu Val Glu Val Tyr Asp His Asp Thr Phe Gly Lys Asp Tyr Met Gly
   65                  70                  75                  80
   Arg Val Ile
<210> 575
   <211> 83
   <212> PRT
   <213> Artificial Sequence
<220>
   <223> synthetically generated peptide
<400> 575
   Leu Ser Val Thr Val Ile Ser Ala Glu Asp Leu Pro Pro Met Asp Val
    1               5                  10                  15
   Met Gly Lys Ala Asp Pro Phe Val Val Leu Tyr Leu Lys Lys Gly Glu
               20                  25                  30
   Thr Lys Lys Lys Thr Arg Val Val Thr Glu Thr Leu Asn Pro Ile Trp
           35                  40                  45
   Asn Gln Thr Phe Asp Phe Val Val Glu Asp Ala Leu His Asp Leu Leu
       50                  55                  60
   Met Val Glu Val Trp Asp His Asp Thr Phe Gly Lys Asp Tyr Ile Gly
   65                  70                  75                  80
   Arg Cys Ile
<210> 576
   <211> 83
   <212> PRT
   <213> Artificial Sequence
<220>
   <223> Synthetically generated peptide
<400> 576
   Leu Ser Val Thr Val Ile Ser Ala Glu Asp Leu Pro Pro Met Asp Val
    1               5                  10                  15
   Met Gly Lys Ala Asp Pro Phe Val Val Leu Tyr Leu Lys Lys Gly Glu
               20                  25                  30
   Thr Lys Lys Lys Thr Arg Val Val Thr Glu Thr Leu Asn Pro Ile Trp
           35                  40                  45
   Asn Gln Thr Phe Asp Phe Val Val Glu Asp Ala Leu His Asp Leu Leu
       50                  55                  60
   Met Val Glu Val Trp Asp His Asp Thr Phe Gly Lys Asp Tyr Ile Gly
   65                  70                  75                  80
   Arg Cys Ile
<210> 577
   <211> 83
   <212> PRT
   <213> Artificial Sequence
<220>
   <223> synthetically generated peptide
<400> 577
   Leu Ser Val Thr Val Val Ala Ala Glu Asp Leu Pro Ala Val Asp Phe
   1               5                   10                  15
   Met Gly Lys Ala Asp Ala Phe Val Val Ile Thr Leu Lys Lys Ser Glu
               20                  25                  30
   Thr Lys Ser Lys Thr Arg Val Val Pro Asp Ser Leu Asn Pro Val Trp
           35                  40                  45
   Asn Gln Thr Phe Asp Phe Val Val Glu Asp Ala Leu His Asp Leu Leu
       50                  55                  60
   Thr Leu Glu Val Trp Asp His Asp Lys Phe Gly Lys Asp Lys Ile Gly
   65                  70                  75                  80
   Arg Val Ile
<210> 578
   <211> 82
   <212> PRT
   <213> Artificial Sequence
<220>
   <223> Synthetically generated peptide
<400> 578
   Leu Ile Val Thr Val Val Lys Ala Thr Asn Leu Lys Asn Lys Glu Leu
   1               5                   10                  15
   Ile Gly Lys Ser Asp Pro Tyr Ala Thr Ile Tyr Ile Arg Pro Val Phe
               20                  25                  30
   Lys Tyr Lys Thr Asn Ala Ile Asp Asn Asn Leu Asn Pro Val Trp Asp
           35                  40                  45
   Gln Thr Phe Glu Leu Ile Ala Glu Asp Lys Glu Thr Gln Ser Leu Thr
       50                  55                  60
   Val Glu Val Phe Asp Lys Asp Val Gly Gln Asp Glu Arg Leu Gly Leu
   65                  70                  75                  80
   Val Lys
<210> 579
   <211> 82
   <212> PRT
   <213> Artificial Sequence
<220>
   <223> synthetically generated peptide
<400> 579
   Leu Ile Val Thr Val Val Lys Ala Thr Asn Leu Lys Asn Lys Glu Leu
   1               5                   10                  15
   Ile Gly Lys Ser Asp Pro Tyr Ala Thr Ile Tyr Ile Arg Pro Val Phe
               20                  25                  30
   Lys Tyr Lys Thr Lys Ala Ile Glu Asn Asn Leu Asn Pro Val Trp Asp
           35                  40                  45
   Gln Thr Phe Glu Leu Ile Ala Glu Asp Lys Glu Thr Gln Ser Leu Thr
       50                  55                  60
   Val Glu Val Phe Asp Lys Asp Val Gly Gln Asp Glu Arg Leu Gly Leu
   65                  70                  75                  80
   Val Lys
<210> 580
   <211> 44
   <212> PRT
   <213> Artificial Sequence
<220>
   <223> synthetically generated peptide
<400> 580
   Ile Glu Asn Asn Leu Asn Pro Val Trp Asp Gln Thr Phe Glu Leu Ile
   1               5                   10                  15
   Val Glu Asp Lys Glu Thr Gln Ser Leu Thr Val Glu Val Phe Asp Lys
               20                  25                  30
   Asp Val Gly Gln Asp Glu Arg Leu Gly Leu Val Lys
           35                  40
<210> 581
   <211> 79
   <212> PRT
   <213> Artificial Sequence
<220>
   <223> Synthetically generated peptide
<400> 581
   Thr Val Val Lys Ala Thr Asn Leu Lys Asn Lys Glu Phe Ile Gly Lys
   1               5                   10                  15
   Ser Asp Pro Tyr Ala Thr Ile His Ile Arg Pro Val Phe Lys Tyr Asn
               20                  25                  30
   Thr Lys Ala Ile Glu Asn Asn Leu Asn Pro Val Trp Asp Gln Thr Phe
           35                  40                  45
   Asp Leu Ile Ala Glu Asp Lys Glu Thr Gln Ser Leu Thr Ile Glu Val
       50                  55                  60
   Phe Asp Lys Asp Val Gly Gln Asp Glu Arg Leu Gly Leu Val Lys
   65                  70                  75
<210> 582
   <211> 83
   <212> PRT
   <213> Artificial Sequence
<220>
   <223> synthetically generated peptide
<400> 582
   Leu Thr Val Thr Ile Val Lys Ala Asn Gly Leu Lys Asn His Glu Met
   1 5 10 15
   Ile Gly Lys Ser Asp Pro Tyr Ala Val Val His Ile Arg Pro Leu Phe
   20 25 30
   Lys Val Lys Thr Lys Thr Ile Asp Asn Asn Leu Asn Pro Val Trp Asp
   35 40 45
   Gln Thr Phe Glu Leu Ile Ala Glu Asp Lys Glu Thr Gln Ser Leu Phe
   50 55 60
   Ile Glu Val Phe Asp Lys Asp Asn Ile Gly Gln Asp Gln Arg Met Gly
   65 70 75 80
   Val Ala Lys
<210> 583
   <211> 83
   <212> PRT
   <213> Artificial Sequence
<220>
   <223> synthetically generated peptide
<400> 583
   Leu Thr Val Thr Val Val Lys Ala Thr Ser Leu Lys Asn Lys Glu Leu
   1 5 10 15
   Ile Gly Lys Ser Asp Pro Tyr Val Ile Leu Tyr Val Arg Pro Met Phe
   20 25 30
   Lys Val Lys Thr Lys Val Ile Asp Asp Asn Leu Asn Pro Glu Trp Asn
   35 40 45
   Glu Thr Phe Pro Leu Ile Val Glu Asp Lys Glu Thr Gln Ser Val Ile
   50 55 60
   Phe Glu Val Tyr Asp Glu Asp Arg Leu Gln Gln Asp Lys Lys Leu Gly
   65 70 75 80
   Val Ala Lys
<210> 584
   <211> 85
   <212> PRT
   <213> Artificial Sequence
<220>
   <223> synthetically generated peptide
<400> 584
   Leu Glu Val Lys Leu Val Glu Ala Arg Asp Leu Thr Asn Lys Asp Leu
   1 5 10 15
   Val Gly Lys Ser Asp Pro Phe Ala Val Leu Tyr Ile Arg Pro Leu Gln
   20 25 30
   Asp Lys Met Lys Lys Ser Lys Thr Ile Asn Asn Asp Leu Asn Pro Ile
   35 40 45
   Trp Asn Glu His Tyr Glu Phe Val Val Glu Asp Thr Ser Thr Gln Arg
   50 55 60
   Leu Thr Val Lys Ile Tyr Asp Asp Glu Gly Leu Gln Ala Ser Glu Leu
   65 70 75 80
   Ile Gly Cys Ala Arg
   85
<210> 585
   <211> 85
   <212> PRT
   <213> Artificial Sequence
<220>
   <223> Synthetically generated peptide
<400> 585
   Leu Glu Val Lys Leu Val Glu Ala Arg Asp Leu Thr Asn Lys Asp Leu
   1 5 10 15
   Val Gly Lys Ser Asp Pro Phe Ala Val Leu Tyr Ile Arg Pro Leu Gln
   20 25 30
   Asp Lys Met Lys Lys Ser Lys Thr Ile Asn Asn Asp Leu Asn Pro Ile
   35 40 45
   Trp Asn Glu His Tyr Glu Phe Val Val Glu Asp Thr Ser Thr Gln Arg
   50 55 60
   Leu Thr Val Lys Ile Tyr Asp Asp Glu Gly Leu Gln Ala Ser Glu Leu
   65 70 75 80
   Ile Gly Cys Ala Arg
   85
<210> 586
   <211> 85
   <212> PRT
   <213> Artificial Sequence
<220>
   <223> synthetically generated peptide
<400> 586
   Leu Glu Val Lys Leu Val Gln Ala Lys Asn Leu Thr Asn Lys Asp Leu
   1 5 10 15
   Val Gly Lys Ser Asp Pro Phe Ala Lys Met Phe Ile Arg Pro Leu Arg
   20 25 30
   Glu Lys Thr Lys Arg Ser Lys Thr Ile Asn Asn Asp Leu Asn Pro Ile
   35 40 45
   Trp Asn Glu His Phe Glu Phe Val Val Glu Asp Ala Ser Thr Gln His
   50 55 60
   Leu Val Val Arg Ile Tyr Asp Asp Glu Gly Val Gln Ala Ser Glu Leu
   65 70 75 80
   Ile Gly Cys Ala Gln
   85
<210> 587
   <211> 85
   <212> PRT
   <213> Artificial Sequence
<220>
   <223> Synthetically generated peptide
<400> 587
   Leu Glu Val Lys Leu Val Gln Ala Lys Asn Leu Thr Asn Lys Asp Leu
   1 5 10 15
   Val Gly Lys Ser Asp Pro Phe Ala Lys Met Phe Ile Arg Pro Leu Arg
   20 25 30
   Glu Lys Thr Lys Arg Ser Lys Thr Ile Asn Asn Asp Leu Asn Pro Ile
   35 40 45
   Trp Asn Glu His Phe Glu Phe Val Val Glu Asp Ala Ser Thr Gln His
   50 55 60
   Leu Val Val Arg Ile Tyr Asp Asp Glu Gly Val Gln Ala Ser Glu Leu
   65 70 75 80
   Ile Gly Cys Ala Gln
   85
<210> 588
   <211> 85
   <212> PRT
   <213> Artificial Sequence
<220>
   <223> synthetically generated peptide
<400> 588
   Leu Glu Val Lys Leu Val Gln Ala Arg Asp Leu Thr Asn Lys Asp Leu
   1 5 10 15
   Ile Gly Lys Ser Asp Pro Phe Ala Ile Val Tyr Val Arg Pro Leu Pro
   20 25 30
   Asp Lys Met Lys Arg Ser Lys Thr Ile Asn Asn Asp Leu Asn Pro Ile
   35 40 45
   Trp Asn Glu His Phe Glu Phe Ile Val Glu Asp Ala Asp Thr Gln Thr
   50 55 60
   Val Thr Val Lys Ile Tyr Asp Asp Asp Gly Ile Gln Glu Ser Glu Leu
   65 70 75 80
   Ile Gly Cys Ala Gln
   85
<210> 589
   <211> 85
   <212> PRT
   <213> Artificial Sequence
<220>
   <223> Synthetically generated peptide
<400> 589
   Leu Glu Val Lys Leu Val Gln Ala Arg Asp Leu Thr Asn Lys Asp Leu
   1 5 10 15
   Ile Gly Lys Ser Asp Pro Phe Ala Ile Val Tyr Val Arg Pro Leu Pro
   20 25 30
   Asp Lys Met Lys Arg Ser Lys Thr Ile Asn Asn Asp Leu Asn Pro Ile
   35 40 45
   Trp Asn Glu His Phe Glu Phe Ile Val Glu Asp Ala Asp Thr Gln Thr
   50 55 60
   Val Thr Val Lys Ile Tyr Asp Asp Asp Gly Ile Gln Glu Ser Glu Leu
   65 70 75 80
   Ile Gly Cys Ala Gln
   85
<210> 590
   <211> 85
   <212> PRT
   <213> Artificial Sequence
<220>
   <223> synthetically generated peptide
<400> 590
   Leu Glu Val Lys Leu Val Gln Ala Lys Glu Leu Thr Asn Lys Asp Ile
   1 5 10 15
   Ile Gly Lys Ser Asp Pro Tyr Ala Val Leu Tyr Ile Arg Pro Leu Arg
   20 25 30
   Asn Arg Thr Lys Lys Ser Lys Thr Ile Asn Asn Asp Leu Asn Pro Ile
   35 40 45
   Trp Asn Glu His Phe Glu Phe Ile Val Glu Asp Ala Ser Thr Gln His
   50 55 60
   Leu Phe Val Lys Val Tyr Asp Asp Glu Gly Leu Gln Ser Ser Glu Leu
   65 70 75 80
   Ile Gly Cys Thr Asp
   85
<210> 591
   <211> 85
   <212> PRT
   <213> Artificial Sequence
<220>
   <223> synthetically generated peptide
<400> 591
   Leu Asp Val Lys Leu Val Gln Ala Lys Asn Leu Ser Asn Lys Asp Ile
   1 5 10 15
   Ile Gly Lys Ser Asp Pro Phe Ala Val Val Phe Val Arg Pro Leu Arg
   20 25 30
   Asp Lys Thr Lys Thr Ser Lys Ile Ile Asn Asn Gln Leu Asn Pro Ile
   35 40 45
   Trp Asn Glu His Phe Glu Phe Ile Ile Glu Asp Glu Ser Thr Gln His
   50 55 60
   Leu Thr Ile Arg Ile Phe Asp Asp Glu Gly Ile Gln Ala Ala Glu Leu
   65 70 75 80
   Ile Gly Cys Ala Gln
   85
<210> 592
   <211> 85
   <212> PRT
   <213> Artificial Sequence
<220>
   <223> synthetically generated peptide
<400> 592
   Leu Asp Val Lys Val Val Gln Ala Lys Asp Leu Ala Asn Lys Asp Met
   1 5 10 15
   Ile Gly Lys Ser Asp Pro Tyr Ala Ile Val Phe Ile Arg Pro Leu Pro
   20 25 30
   Asp Arg Thr Lys Lys Thr Lys Thr Ile Ser Asn Ser Leu Asn Pro Ile
   35 40 45
   Trp Asn Glu His Phe Glu Phe Ile Val Glu Asp Val Ser Thr Gln His
   50 55 60
   Leu Thr Val Arg Val Phe Asp Asp Glu Gly Val Gly Ser Ser Gln Leu
   65 70 75 80
   Ile Gly Ala Ala Gln
   85
<210> 593
   <211> 82
   <212> PRT
   <213> Artificial Sequence
<220>
   <223> synthetically generated peptide
<400> 593
   Lys Val Val Arg Ala Val Gly Leu Arg Lys Lys Asp Met Met Gly Gly
   1 5 10 15
   Ala Asp Pro Tyr Val Lys Ile Lys Leu Ser Glu Asp Lys Ile Pro Ser
   20 25 30
   Lys Lys Thr Thr Val Lys His Lys Asn Leu Asn Pro Glu Trp Asn Glu
   35 40 45
   Glu His Lys Phe Ser Val Arg Asp Pro Gln Thr Gln Val Leu Glu Phe
   50 55 60
   Ser Val Tyr Asp Trp Gly Gln Leu Gly Lys His Asp Lys Met Gly Met
   65 70 75 80
   Asn Val
<210> 594
   <211> 85
   <212> PRT
   <213> Artificial Sequence
<220>
   <223> synthetically generated peptide
<400> 594
   Val His Val Lys Val Val Arg Ala Val Gly Leu Arg Lys Lys Asp Leu
   1               5                   10                  15
   Met Gly Gly Ala Asp Pro Phe Val Lys Ile Lys Leu Ser Glu Asp Lys
               20                  25                  30
   Ile Pro Ser Lys Lys Thr Thr Val Lys His Lys Asn Leu Asn Pro Glu
           35                  40                  45
   Trp Asn Glu Glu Phe Lys Phe Ser Val Arg Asp Pro Gln Thr Gln Val
       50                  55                  60
   Leu Glu Phe Ser Val Tyr Asp Trp Glu Gln Val Gly Asn Pro Glu Lys
   65                  70                  75                  80
   Met Gly Met Asn Val
                   85
<210> 595
   <211> 68
   <212> PRT
   <213> Artificial Sequence
<220>
   <223> synthetically generated peptide
<400> 595
   Gly Met Ile Asn Pro Tyr Val Gln Ile Glu Leu Ser Glu Asp Lys Ile
   1               5                   10                  15
   Ser Ser Lys Lys Thr Thr Val Lys His Lys Asn Leu Asn Pro Glu Trp
               20                  25                  30
   Asn Glu Glu Phe Lys Phe Ser Val Arg Asp Pro Lys Thr Gln Val Leu
           35                  40                  45
   Glu Phe Asn Val Tyr Gly Trp Glu Lys Ile Gly Lys His Asp Lys Met
       50                  55                  60
   Gly Met Asn Val
   65
<210> 596
   <211> 85
   <212> PRT
   <213> Artificial Sequence
<220>
   <223> synthetically generated peptide
<400> 596
   Leu His Val Lys Val Leu His Ala Met Lys Leu Lys Lys Lys Asp Leu
   1 5 10 15
   Leu Gly Ala Ser Asp Pro Tyr Val Lys Leu Lys Leu Thr Asp Asp Lys
   20 25 30
   Met Pro Ser Lys Lys Thr Thr Val Lys His Lys Asn Leu Asn Pro Glu
   35 40 45
   Trp Asn Glu Glu Phe Asn Leu Val Val Lys Asp Pro Glu Thr Gln Val
   50 55 60
   Leu Gln Leu Asn Val Tyr Asp Trp Glu Gln Val Gly Lys His Asp Lys
   65 70 75 80
   Met Gly Met Asn Val
   85
<210> 597
   <211> 85
   <212> PRT
   <213> Artificial Sequence
<220>
   <223> Synthetically generated peptide
<400> 597
   Leu Ser Val Lys Val Ile Lys Ala Ile Lys Leu Lys Lys Lys Asp Leu
   1 5 10 15
   Leu Gly Gly Ser Asp Pro Tyr Val Lys Leu Thr Leu Ser Gly Asp Lys
   20 25 30
   Val Pro Gly Lys Lys Thr Val Val Lys His Ser Asn Leu Asn Pro Glu
   35 40 45
   Trp Asn Glu Glu Phe Asp Leu Val Val Lys Glu Pro Glu Ser Gln Glu
   50 55 60
   Leu Gln Leu Ile Val Tyr Asp Trp Glu Gln Val Gly Lys His Asp Lys
   65 70 75 80
   Ile Gly Met Asn Val
   85
<210> 598
   <211> 85
   <212> PRT
   <213> Artificial Sequence
<220>
   <223> Synthetically generated peptide
<400> 598
   Leu His Val Asn Ile Val Arg Ala Val Lys Leu Thr Lys Lys Asp Phe
   1 5 10 15
   Leu Gly Lys Ser Asp Pro Tyr Val Lys Leu Lys Leu Thr Glu Glu Lys
   20 25 30
   Leu Pro Ser Lys Lys Thr Ser Val Lys Arg Ser Asn Leu Asn Pro Glu
   35 40 45
   Trp Asn Glu Asp Phe Lys Leu Val Val Lys Asp Pro Glu Ser Gln Ala
   50 55 60
   Leu Glu Leu Thr Val Tyr Asp Trp Glu Gln Val Gly Lys His Asp Lys
   65 70 75 80
   Ile Gly Met Ser Val
   85
<210> 599
   <211> 85
   <212> PRT
   <213> Artificial Sequence
<220>
   <223> Synthetically generated peptide
<400> 599
   Leu His Val Ser Ile Leu Arg Ala Arg Asn Leu Leu Lys Lys Asp Leu
   1               5                   10                  15
   Leu Gly Thr Ser Asp Pro Tyr Val Lys Leu Ser Leu Thr Gly Glu Lys
               20                  25                  30
   Leu Pro Ala Lys Lys Thr Thr Ile Lys Lys Arg Asn Leu Asn Pro Glu
           35                  40                  45
   Trp Asn Glu His Phe Lys Leu Ile Val Lys Asp Pro Asn Ser Gln Val
       50                  55                  60
   Leu Gln Leu Glu Val Phe Asp Trp Asp Lys Val Gly Gly His Asp Arg
   65                  70                  75                  80
   Leu Gly Met Gln Met
                   85
<210> 600
   <211> 85
   <212> PRT
   <213> Artificial Sequence
<220>
   <223> Synthetically generated peptide
<400> 600
   Leu His Val Ser Ile Leu Arg Ala Arg Asn Leu Leu Lys Lys Asp Leu
   1               5                   10                  15
   Leu Gly Thr Ser Asp Pro Tyr Val Lys Leu Ser Leu Thr Gly Glu Lys
               20                  25                  30
   Leu Pro Ala Lys Lys Thr Thr Ile Lys Lys Arg Asn Leu Asn Pro Glu
           35                  40                  45
   Trp Asn Glu His Phe Lys Leu Ile Val Lys Asp Pro Asn Ser Gln Val
       50                  55                  60
   Leu Gln Leu Glu Val Phe Asp Trp Asp Lys Val Gly Gly His Asp Arg
   65                  70                  75                  80
   Leu Gly Met Gln Met
                   85
<210> 601
   <211> 85
   <212> PRT
   <213> Artificial Sequence
<220>
   <223> synthetically generated peptide
<400> 601
   Leu His Val Ser Ile Leu Arg Ala Arg Asn Leu Leu Lys Lys Asp Leu
   1               5                   10                  15
   Leu Gly Thr Ser Asp Pro Tyr Val Lys Leu Ser Leu Thr Gly Glu Lys
               20                  25                  30
   Leu Pro Ala Lys Lys Thr Thr Ile Lys Lys Arg Asn Leu Asn Pro Glu
           35                  40                  45
   Trp Asn Glu His Phe Lys Leu Ile Val Lys Asp Pro Asn Ser Gln Val
       50                  55                  60
   Leu Gln Leu Glu Val Phe Asp Trp Asp Lys Val Gly Gly His Asp Arg
   65                  70                  75                  80
   Leu Gly Met Gln Met
                   85
<210> 602
   <211> 83
   <212> PRT
   <213> Artificial Sequence
<220>
   <223> synthetically generated peptide
<400> 602
   Leu His Val Lys Val Ile Arg Ala Met Asn Leu Leu Lys Met Asp Leu
   1               5                   10                  15
   Leu Gly Lys Ser Asp Pro Tyr Val Lys Leu Arg Leu Ser Gly Glu Lys
               20                  25                  30
   Leu Pro Ser Lys Lys Thr Ser Ile Lys Met Ser Asn Leu Asn Pro Glu
           35                  40                  45
   Trp Asn Glu His Phe Arg Phe Ile Val Lys Asp Pro Glu Thr Gln Ile
       50                  55                  60
   Leu Arg Met Phe Asp Trp Glu Lys Val Lys Met His Asp Lys Leu Gly
   65                  70                  75                  80
   Met Gln Val
<210> 603
   <211> 79
   <212> PRT
   <213> Artificial Sequence
<220>
   <223> Synthetically generated peptide
<400> 603
   Leu Leu Val Lys Val Leu Arg Ala Gln Asn Leu Arg Glu Lys Gly Pro
   1               5                   10                  15
   Leu Gly Lys Arg Asp Pro Tyr Val Lys Leu Lys Met Ser Gly Ser Lys
               20                  25                  30
   Leu Pro Ser Lys Lys Thr Ala Val Lys His Ser Asn Leu Asn Pro Glu
           35                  40                  45
   Trp Asn Gln Glu Phe Lys Phe Val Ile Arg Asp Pro Glu Thr Gln Glu
       50                  55                  60
   Leu Asp Ile Asn Phe Gly Lys Asp Glu Lys Leu Gly Met Cys Lys
   65                  70                  75
<210> 604
   <211> 85
   <212> PRT
   <213> Artificial Sequence
<220>
   <223> Synthetically generated peptide
<400> 604
   Leu Leu Val Lys Val Leu Arg Ala Gln Asn Leu Arg Lys Lys Asp Leu
   1               5                   10                  15
   Leu Gly Lys Ser Asp Pro Tyr Val Lys Leu Lys Met Ser Asp Asp Lys
               20                  25                  30
   Leu Pro Ser Lys Lys Thr Thr Val Lys Arg Ser Asn Leu Asn Pro Glu
           35                  40                  45
   Trp Asn Glu Asp Phe Lys Phe Val Val Thr Asp Pro Glu Thr Gln Ala
       50                  55                  60
   Leu Glu Ile Asn Val Phe Asp Trp Glu Gln Val Gly Lys His Glu Lys
   65                  70                  75                  80
   Met Gly Met Asn Asn
                   85
<210> 605
   <211> 86
   <212> PRT
   <213> Artificial Sequence
<220>
   <223> synthetically generated peptide
<400> 605
   Leu Ser Val Thr Leu Val Asp Ala Gln Lys Leu Arg Tyr Met Phe Phe
   1               5                   10                  15
   Gly Lys Thr Asp Pro Tyr Ala Ile Leu Arg Leu Gly Asp Gln Val Ile
               20                  25                  30
   Arg Ser Lys Arg Asn Ser Gln Thr Thr Val Ile Gly Ala Pro Gly Gln
           35                  40                  45
   Pro Ile Trp Asn Gln Asp Phe Gln Phe Leu Val Ser Asn Pro Arg Glu
       50                  55                  60
   Gln Val Leu Gln Ile Glu Val Asn Asp Arg Leu Gly Phe Ala Asp Met
   65                  70                  75                  80
   Ala Ile Gly Thr Gly Glu
                   85
<210> 606
   <211> 86
   <212> PRT
   <213> Artificial Sequence
<220>
   <223> Synthetically generated peptide
<400> 606
   Leu Ser Val Thr Leu Val Asp Ala Gln Lys Leu Arg Tyr Met Phe Phe
    1               5                  10                  15
   Gly Lys Thr Asp Pro Tyr Ala Ile Leu Arg Leu Gly Asp Gln Val Ile
               20                  25                  30
   Arg Ser Lys Arg Asn Ser Gln Thr Thr Val Ile Gly Ala Pro Gly Gln
           35                  40                  45
   Pro Ile Trp Asn Gln Asp Phe Gln Phe Leu Val Ser Asn Pro Arg Glu
       50                  55                  60
   Gln Val Leu Gln Ile Glu Val Asn Asp Arg Leu Gly Phe Ala Asp Met
   65                  70                  75                  80
   Ala Ile Gly Thr Gly Glu
                   85
<210> 607
   <211> 86
   <212> PRT
   <213> Artificial Sequence
<220>
   <223> Synthetically generated peptide

<400> 607
   Leu Ser Val Thr Leu Val Asn Ala Gln Lys Leu Pro Tyr Met Phe Ser
    1               5                  10                  15
   Gly Arg Thr Asp Pro Tyr Val Ile Leu Arg Ile Gly Asp Gln Val Ile
               20                  25                  30
   Arg Ser Lys Lys Asn Ser Gln Thr Thr Val Ile Gly Ala Pro Gly Gln
           35                  40                  45
   Pro Ile Trp Asn Gln Asp Phe Gln Phe Leu Val Ser Asn Pro Arg Glu
       50                  55                  60
   Gln Val Leu Gln Ile Glu Val Asn Asp Cys Leu Gly Phe Ala Asp Met
   65                  70                  75                  80
   Ala Ile Gly Ile Gly Glu
                   85
<210> 608
   <211> 42
   <212> PRT
   <213> Artificial Sequence
<220>
   <223> Synthetically generated peptide
<400> 608
   Leu Ser Val Thr Leu Val Asp Ala Arg Lys Leu Pro Tyr Phe Phe Gly
    1               5                  10                  15
   Lys Thr Asp Pro Tyr Val Ile Leu Ser Leu Gly Asp Gln Thr Ile Arg
               20                  25                  30
   Ser Lys Lys Asn Ser Gln Thr Thr Val Ile
           35                  40
<210> 609
   <211> 86
   <212> PRT
   <213> Artificial Sequence
<220>
   <223> Synthetically generated peptide
<400> 609
   Leu Ser Val Thr Leu Val Asp Ala Arg Lys Leu Ser Phe Val Leu Phe
   1 5 10 15
   Gly Lys Thr Asp Pro Tyr Val Val Met Ile Leu Gly Asp Gln Glu Ile
   20 25 30
   Lys Ser Lys Lys Asn Ser Gln Thr Thr Val Ile Gly Gln Pro Gly Glu
   35 40 45
   Pro Ile Trp Asn Gln Asp Phe His Met Leu Val Ala Asn Pro Arg Lys
   50 55 60
   Gln Lys Leu Cys Ile Gln Val Lys Asp Ser Val Gly Leu Thr Asp Val
   65 70 75 80
   Thr Ile Gly Thr Gly Glu
   85
<210> 610
   <211> 84
   <212> PRT
   <213> Artificial Sequence
<220>
   <223> synthetically generated peptide
<400> 610
   Phe Arg Cys Val Asn Leu Asp Asn Lys Asp Leu Phe Ser Lys Ser Asp
   1 5 10 15
   Pro Phe Leu Arg Ile Ser Arg Val Val Glu Thr Ser Ala Ala Val Pro
   20 25 30
   Ile Cys Arg Thr Glu Val Val Asp Asn Asn Leu Asn Pro Met Trp Arg
   35 40 45
   Pro Val Cys Leu Thr Met Gln Gln Phe Gly Ser Lys Asp Thr Pro Leu
   50 55 60
   Val Ile Glu Cys Leu Asp Phe Asn Thr Ser Gly Asn His Glu Leu Ile
   65 70 75 80
   Gly Lys Thr Glu
<210> 611
   <211> 84
   <212> PRT
   <213> Artificial Sequence
<220>
   <223> Synthetically generated peptide
<400> 611
   Phe Arg Cys Val Asn Leu Asp Asn Lys Asp Leu Phe Ser Lys Ser Asp
   1 5 10 15
   Pro Phe Leu Arg Ile Ser Arg Val Val Glu Thr Ser Ala Ala Val Pro
   20 25 30
   Ile Cys Arg Thr Glu Val Val Asp Asn Asn Leu Asn Pro Met Trp Arg
   35 40 45
   Pro Val Cys Leu Thr Met Gln Gln Phe Gly Ser Lys Asp Thr Pro Leu
   50 55 60
   Val Ile Glu Cys Leu Asp Phe Asn Thr Ser Gly Asn His Glu Leu Ile
   65 70 75 80
   Gly Lys Thr Glu
<210> 612
   <211> 84
   <212> PRT
   <213> Artificial Sequence
<220>
   <223> synthetically generated peptide
<400> 612
   Phe Arg Cys Val Asn Leu Asp Asn Lys Asp Leu Phe Ser Lys Ser Asp
   1 5 10 15
   Pro Phe Leu Arg Ile Ser Arg Val Val Glu Thr Ser Ala Ala Val Pro
   20 25 30
   Ile Cys Arg Thr Glu Val Val Asp Asn Asn Leu Asn Pro Met Trp Arg
   35 40 45
   Pro Val Cys Leu Thr Met Gln Gln Phe Gly Ser Lys Asp Thr Pro Leu
   50 55 60
   Val Ile Glu Cys Leu Asp Phe Asn Thr Ser Gly Asn His Glu Leu Met
   65 70 75 80
   Lys Asp Arg Glu
<210> 613
   <211> 83
   <212> PRT
   <213> Artificial Sequence
<220>
   <223> synthetically generated peptide
<400> 613
   Val Phe Arg Gln Leu Glu Ser Lys Asp Thr Phe Ser Lys Ser Asp Pro
   1 5 10 15
   Phe Leu Val Ile Ser Lys Ile Val Glu His Gly Thr Pro Ile Pro Val
   20 25 30
   Ser Lys Thr Glu Val Leu Lys Asn Asp Pro Asn Pro Leu Trp Lys Pro
   35 40 45
   Val Ser Leu Ser Val Gln Gln Val Gly Ser Lys Asp Ser Pro Leu Val
   50 55 60
   Ile Glu Cys Leu Asp Phe Asn Gly Asn Gly Asn His Asp Leu Ile Gly
   65 70 75 80
   Lys Val Gln
<210> 614
   <211> 83
   <212> PRT
   <213> Artificial Sequence
<220>
   <223> synthetically generated peptide
<400> 614
   Val Phe Arg Gln Leu Glu Ser Lys Asp Thr Phe Ser Lys Ser Asp Pro
   1 5 10 15
   Phe Leu Val Ile Ser Lys Ile Val Glu His Gly Thr Pro Ile Pro Val
   20 25 30
   Ser Lys Thr Glu Val Leu Lys Asn Asp Pro Asn Pro Leu Trp Lys Pro
   35 40 45
   Val Ser Leu Ser Val Gln Gln Val Gly Ser Lys Asp Ser Pro Leu Val
   50 55 60
   Ile Glu Cys Leu Asp Phe Asn Gly Asn Gly Asn His Asp Leu Ile Gly
   65 70 75 80
   Lys Val Gln
<210> 615
   <211> 83
   <212> PRT
   <213> Artificial Sequence
<220>
   <223> Synthetically generated peptide
<400> 615
   Val Phe Arg Gln Leu Glu Ser Lys Asp Thr Phe Ser Lys Ser Asp Pro
   1 5 10 15
   Phe Leu Val Ile Ser Lys Ile Val Glu His Gly Thr Pro Ile Pro Val
   20 25 30
   Ser Lys Thr Glu Val Leu Lys Asn Asp Pro Asn Pro Leu Trp Lys Pro
   35 40 45
   Val Ser Leu Ser Val Gln Gln Val Gly Ser Lys Asp Ser Pro Leu Val
   50 55 60
   Ile Glu Cys Leu Asp Phe Asn Gly Asn Gly Asn His Asp Leu Ile Gly
   65 70 75 80
   Lys Val Gln
<210> 616
   <211> 85
   <212> PRT
   <213> Artificial Sequence
<220>
   <223> synthetically generated peptide
<400> 616
   Val Phe Arg Cys Ser Asn Leu Glu Ser Lys Asp Leu Phe Ser Lys Ser
   1 5 10 15
   Asp Pro Phe Leu Val Val Ser Lys Ile Val Glu His Gly Thr Pro Ile
               20                  25                  30
   Pro Val Ser Lys Thr Glu Val Arg Lys Asn Asp Leu Asn Pro Ile Trp
           35                  40                  45
   Lys Pro Val Phe Leu Ser Val Gln Gln Val Gly Ser Lys Asp Ser Pro
       50                  55                  60
   Val Ile Ile Glu Cys Ser Asp Phe Asn Ser Asn Gly Lys His Ser Leu
   65                  70                  75                  80
   Ile Gly Lys Val Gln
                   85
<210> 617
   <211> 85
   <212> PRT
   <213> Artificial Sequence
<220>
   <223> Synthetically generated peptide
<400> 617
   Val Phe Arg Cys Ser Asn Leu Glu Ser Lys Asp Leu Phe Ser Lys Ser
    1               5                  10                  15
   Asp Pro Phe Leu Val Val Ser Lys Ile Val Glu His Gly Thr Pro Ile
               20                  25                  30
   Pro Val Ser Lys Thr Glu Val Arg Lys Asn Asp Leu Asn Pro Ile Trp
           35                  40                  45
   Lys Pro Val Phe Leu Ser Val Gln Gln Val Gly Ser Lys Asp Ser Pro
       50                  55                  60
   Val Ile Ile Glu Cys Ser Asp Phe Asn Ser Asn Gly Lys His Ser Leu
   65                  70                  75                  80
   Ile Gly Lys Val Gln
                   85
<210> 618
   <211> 89
   <212> PRT
   <213> Artificial Sequence
<220>
   <223> Synthetically generated peptide
<400> 618
   Ile Met Glu Met Val Phe Arg Cys Ser Asp Leu Glu Ile Lys Asp Leu
    1               5                  10                  15
   Leu Ser Lys Ser Asp Pro Phe Leu Leu Ile Ser Arg Ile Ser Glu Ser
               20                  25                  30
   Gly Val Pro Val Pro Ile Cys Lys Thr Glu Val Arg Lys Asn Asp Leu
           35                  40                  45
   Asn Pro Lys Trp Lys Pro Val Ile Leu Asn Leu Gln Gln Ile Gly Ser
       50                  55                  60
   Lys Glu Asn Pro Leu Ile Ile Glu Cys Phe Asn Phe Ser Ser Asn Gly
   65                  70                  75                  80
   Lys His Asp Leu Ile Gly Lys Ile Val
                   85
<210> 619
   <211> 92
   <212> PRT
   <213> Artificial Sequence
<220>
   <223> synthetically generated peptide
<400> 619
   Phe Ser Asn Leu Arg Asp Arg Asp Val Ile Ser Lys Ser Asp Ala Met
   1               5                   10                  15
   Val Val Val Tyr Thr Lys Gly Arg Asp Gly Thr Leu Ala Glu Leu Phe
               20                  25                  30
   Arg Ser Glu Val Val Leu Asn Ser Leu Asn Pro Lys Trp Ile Lys Asn
           35                  40                  45
   Phe Thr Ile Gly Tyr Gln Phe Glu Ile Val Gln Thr Leu Leu Phe Arg
       50                  55                  60
   Val Tyr Asp Ile Asp Thr Gln Phe Gln Asn Ser Lys Glu Glu Leu Leu
   65                  70                  75                  80
   Lys Leu Asp Glu Gln Gln Phe Leu Gly Glu Ala Thr
                   85                  90
<210> 620
   <211> 92
   <212> PRT
   <213> Artificial Sequence
<220>
   <223> synthetically generated peptide
<400> 620
   Phe Ser Asn Leu Arg Asp Arg Asp Val Ile Ser Lys Ser Asp Ala Met
   1               5                   10                  15
   Val Val Val Tyr Thr Lys Gly Arg Asp Gly Thr Leu Ala Glu Leu Phe
               20                  25                  30
   Arg Ser Glu Val Val Leu Asn Ser Leu Asn Pro Lys Trp Ile Lys Asn
           35                  40                  45
   Phe Thr Ile Gly Tyr Gln Phe Glu Ile Val Gln Thr Leu Leu Phe Arg
       50                  55                  60
   Val Tyr Asp Ile Asp Thr Gln Phe Gln Asn Ser Lys Glu Glu Leu Leu
   65                  70                  75                  80
   Lys Leu Asp Glu Gln Gln Phe Leu Gly Glu Ala Thr
                   85                  90
<210> 621
   <211> 92
   <212> PRT
   <213> Artificial Sequence
<220>
   <223> Synthetically generated peptide
<400> 621
   Phe Ser Asn Leu Arg Asp Arg Asp Val Ile Ser Lys Ser Asp Ala Met
   1 5 10 15
   Val Val Val Tyr Thr Lys Gly Arg Asp Gly Thr Leu Ala Glu Leu Phe
   20 25 30
   Arg Ser Glu Val Val Leu Asn Ser Leu Asn Pro Lys Trp Ile Lys Asn
   35 40 45
   Phe Thr Ile Gly Tyr Gln Phe Glu Ile Val Gln Thr Leu Leu Phe Arg
   50 55 60
   Val Tyr Asp Ile Asp Thr Gln Phe Gln Asn Ser Lys Glu Glu Leu Leu
   65 70 75 80
   Lys Leu Asp Glu Gln Gln Phe Leu Gly Glu Ala Thr
   85 90
<210> 622
   <211> 92
   <212> PRT
   <213> Artificial Sequence
<220>
   <223> Synthetically generated peptide
<400> 622
   Phe Ser Asn Leu Arg Asp Arg Asp Val Leu Ser Lys Ser Asp Pro Met
   1 5 10 15
   Val Val Val Tyr Gln Lys Glu Lys Asp Ala Thr Leu Ser Glu Val Phe
   20 25 30
   Arg Ser Glu Val Val Leu Asn Ser Leu Ala Pro Lys Trp Ile Lys Lys
   35 40 45
   Phe Ile Val Ala Tyr His Phe Glu Thr Val Gln Thr Leu Val Phe Arg
   50 55 60
   Val Tyr Asp Val Asp Thr Lys Phe Gln Asn Ser Arg Glu Glu Met Leu
   65 70 75 80
   Lys Leu Asp Glu Gln Gln Phe Leu Gly Glu Ala Thr
   85 90
<210> 623
   <211> 92
   <212> PRT
   <213> Artificial Sequence
<220>
   <223> Synthetically generated peptide
<400> 623
   Phe Ser Asn Leu Arg Asp Arg Asp Val Leu Ser Lys Ser Asp Pro Met
   1 5 10 15
   Val Val Val Tyr Gln Lys Glu Lys Asp Ala Thr Leu Ser Glu Val Phe
   20 25 30
   Arg Ser Glu Val Val Leu Asn Ser Leu Ala Pro Lys Trp Ile Lys Lys
   35 40 45
   Phe Ile Val Ala Tyr His Phe Glu Thr Val Gln Thr Leu Val Phe Arg
   50 55 60
   Val Tyr Asp Val Asp Thr Lys Phe Gln Asn Ser Arg Glu Glu Met Leu
   65 70 75 80
   Lys Leu Asp Glu Gln Gln Phe Leu Gly Glu Ala Thr
   85 90
<210> 624
   <211> 92
   <212> PRT
   <213> Artificial Sequence
<220>
   <223> Synthetically generated peptide
<400> 624
   Leu Ser Asn Leu Gly Asp Gln Glu Phe Phe Thr Lys Ser Asn Pro Met
   1               5                   10                  15
   Val Ile Val Tyr Ser Lys Ser Lys Glu Gly Ala Leu Glu Glu Leu Gly
               20                  25                  30
   Arg Thr Glu Val Ile Leu Asn Ser Leu Asn Pro Ser Trp Asn Ala Arg
           35                  40                  45
   Ile Asn Val His Tyr Gln Phe Glu Val Leu Gln Pro Ile Val Phe Gln
       50                  55                  60
   Val Tyr Asp Ile Asp Pro Gln Phe His Asp Val Asn Glu Lys Met Leu
   65                  70                  75                  80
   Lys Leu Glu Glu Gln Gln Phe Leu Gly Glu Ala Val
                   85                  90
<210> 625
   <211> 92
   <212> PRT
   <213> Artificial Sequence
<220>
   <223> synthetically generated peptide
<400> 625
   Leu Ser Asn Leu Leu Asp Cys Asp Ile Thr Ser Lys Ser Asp Pro Met
   1               5                   10                  15
   Ala Val Met Tyr Leu Arg Lys Lys Asp Gly Arg Leu Glu Glu Ile Gly
               20                  25                  30
   Arg Thr Glu Val Ile Leu Asn Asn Leu Asn Pro Lys Trp Ile Glu Lys
           35                  40                  45
   Ile Thr Val Ser Phe Gln Phe Glu Ala Val Gln Thr Leu Val Phe His
       50                  55                  60
   Val Tyr Asp Val Asp Thr Arg Tyr His Asn Val Pro Val Lys Thr Leu
   65                  70                  75                  80
   Lys Leu Lys Asp Gln Asp Phe Leu Gly Glu Gly Thr
                   85                  90
<210> 626
   <211> 92
   <212> PRT
   <213> Artificial Sequence
<220>
   <223> synthetically generated peptide
<400> 626
   Leu Ser Asn Leu Leu Asp Cys Asp Ile Thr Ser Lys Ser Asp Pro Met
   1 5 10 15
   Ala Val Met Tyr Leu Arg Lys Lys Asp Gly Arg Leu Glu Glu Ile Gly
   20 25 30
   Arg Thr Glu Val Ile Leu Asn Asn Leu Asn Pro Lys Trp Ile Glu Lys
   35 40 45
   Ile Thr Val Ser Phe Gln Phe Glu Ala Val Gln Thr Leu Val Phe His
   50 55 60
   Val Tyr Asp Val Asp Thr Arg Tyr His Asn Val Pro Val Lys Thr Leu
   65 70 75 80
   Lys Leu Lys Asp Gln Asp Phe Leu Gly Glu Gly Thr
   85 90
<210> 627
   <211> 86
   <212> PRT
   <213> Artificial Sequence
<220>
   <223> synthetically generated peptide
<400> 627
   Val Asn Gln Leu Thr Leu Ser Asn Leu Leu Asp Cys Asp Ile Thr Ser
   1 5 10 15
   Lys Ser Asp Pro Met Ala Val Met Tyr Leu Arg Lys Lys Asp Gly Arg
   20 25 30
   Leu Glu Glu Ile Gly Arg Thr Glu Val Ile Leu Asn Asn Leu Asn Pro
   35 40 45
   Lys Trp Ile Glu Lys Ile Thr Val Ser Phe Gln Phe Glu Ala Val Gln
   50 55 60
   Thr Leu Val Phe His Val Tyr Asp Val Asp Thr Arg Tyr His Asn Val
   65 70 75 80
   Pro Val Lys Val Ile Phe
   85
<210> 628
   <211> 93
   <212> PRT
   <213> Artificial Sequence
<220>
   <223> Synthetically generated peptide
<400> 628
   Phe Ser Ala Ser Lys Leu Arg Asn Met Asp Ala Phe Ser Lys Ser Asp
   1 5 10 15
   Pro Met Leu Val Ile Tyr Ile Arg Lys Asp Ala Arg Leu Glu Glu Ile
   20 25 30
   Gly Arg Thr Glu Val Ile Leu Asn Ser Leu Glu Pro Ser Trp Ile Thr
   35 40 45
   Lys Ala Thr Ile Ser Tyr Gln Phe Glu Ile Ile Gln Pro Leu Val Phe
   50 55 60
   Lys Ile Tyr Asp Ile Asp Thr Arg Tyr His Asn Thr Pro Val Lys Thr
   65 70 75 80
   Leu Asn Leu Ala Gln Gln Asp Phe Leu Gly Glu Ala Cys
   85 90
<210> 629
   <211> 80
   <212> PRT
   <213> Artificial Sequence
<220>
   <223> synthetically generated peptide
<400> 629
   Met Thr Val Ile Glu Gly Thr Gly Ile Thr Asn Ser Asn Ser Lys Glu
   1               5                   10                  15
   Leu Phe Asp Met Tyr Ala Val Phe Thr Cys Asn Ala Lys Arg Lys Thr
               20                  25                  30
   Ser Ser Val Lys Phe Gln Thr Ser Glu Pro Lys Trp Asn Glu Ile Tyr
           35                  40                  45
   Glu Phe Asp Ala Met Asp Asp Pro Pro Ser Arg Met Asp Val Ala Ile
       50                  55                  60
   His Asp Ala Asn Gly Pro Phe Asp Gln Ser Pro Ile Gly His Ala Glu
   65                  70                  75                  80
<210> 630
   <211> 80
   <212> PRT
   <213> Artificial Sequence
<220>
   <223> Synthetically generated peptide
<400> 630
   Leu Thr Val Ile Glu Gly Ser Gly Val Val Gly Ser Gly Thr Pro Gly
   1               5                   10                  15
   Leu Pro Asp Pro Tyr Val Val Phe Thr Cys Asn Gly Lys Arg Lys Thr
               20                  25                  30
   Ser Ser Val Lys Phe Gln Thr Ser Glu Pro Lys Trp Asn Glu Ile Phe
           35                  40                  45
   Glu Phe Asn Ala Met Asp Asp Pro Pro Ser Arg Leu Glu Val Val Val
       50                  55                  60
   His Asp Ser Glu Gly Pro His Asn Lys Ile Pro Ile Gly Gln Thr Glu
   65                  70                  75                  80
<210> 631
   <211> 81
   <212> PRT
   <213> Artificial Sequence
<220>
   <223> Synthetically generated peptide
<400> 631
   Leu Thr Val Ile Glu Gly Thr Lys Leu Ala Pro Val Asp Ala Thr Gly
    1               5                   10                  15
   Phe Ser Asp Pro Tyr Val Val Phe Thr Cys Asn Gly Lys Ser Lys Thr
               20                  25                  30
   Ser Ser Ile Lys Phe Gln Thr Leu Glu Pro Gln Trp Asn Asp Ile Phe
           35                  40                  45
   Glu Phe Asp Ala Met Asp Asp Pro Pro Ser Val Met Asn Val His Val
       50                  55                  60
   Tyr Asp Phe Asp Gly Pro Phe Asp Glu Val Thr Ser Leu Gly His Ala
   65                  70                  75                  80
   Glu
<210> 632
   <211> 81
   <212> PRT
   <213> Artificial Sequence
<220>
   <223> Synthetically generated peptide
<400> 632
   Leu Thr Val Ile Asp Gly Thr Asn Leu Ala Ala Thr Lys Ser Ser Gly
    1               5                  10                  15
   Tyr Ser Asp Pro Tyr Val Val Phe Thr Cys Asn Gly Lys Thr Lys Thr
               20                  25                  30
   Ser Ser Ile Lys Phe His Thr Leu Glu Pro Arg Trp Asn Glu Ile Phe
           35                  40                  45
   Glu Phe Asp Ala Met Glu Asp Pro Pro Ser Val Met Lys Ile Asn Val
       50                  55                  60
   Tyr Asp Phe Asp Gly Pro Phe Asp Glu Val Glu Ser Leu Gly His Ala
   65                  70                  75                  80
   Glu
<210> 633
   <211> 81
   <212> PRT
   <213> Artificial Sequence
<220>
   <223> Synthetically generated peptide
<400> 633
   Leu Thr Val Ile Glu Gly Val Asp Leu Ala Ala Val Asp Pro Ser Gly
    1               5                  10                  15
   His Cys Asp Pro Tyr Ile Val Phe Thr Ser Asn Gly Lys Thr Arg Thr
               20                  25                  30
   Ser Ser Ile Lys Phe Gln Lys Ser Asn Pro Gln Trp Asn Glu Ile Phe
           35                  40                  45
   Glu Phe Asp Ala Met Ala Asp Pro Pro Ser Val Leu Asn Val Glu Val
       50                  55                  60
   Phe Asp Phe Asp Gly Pro Phe Asp Glu Ala Val Ser Leu Gly His Ala
   65                  70                  75                  80
   Glu
<210> 634
   <211> 83
   <212> PRT
   <213> Artificial Sequence
<220>
   <223> synthetically generated peptide
<400> 634
   Leu Thr Ile Ala Leu Ile Lys Gly Thr Asn Leu Ala Ser Val Glu Ala
    1               5                  10                  15
   Thr Glu Leu Phe Asp Pro Tyr Val Val Phe Thr Cys Asn Gly Lys Thr
               20                  25                  30
   Arg Thr Ser Ser Val Lys Leu Gln Ala Gln Asp Pro Gln Trp Asn Glu
           35                  40                  45
   Val Ile Glu Phe Asp Ala Met Glu Glu Pro Pro Ser Val Leu Asp Val
       50                  55                  60
   Glu Val Phe Asp Phe Asp Gly Pro Phe Asp Gln Gly Ala Ser Leu Gly
   65                  70                  75                  80
   His Ala Glu
<210> 635
   <211> 80
   <212> PRT
   <213> Artificial Sequence
<220>
   <223> synthetically generated peptide
<400> 635
   Leu Thr Val Leu Glu Ala Thr Ser Leu Pro Pro Val Ser Ser Gly Ser
    1               5                  10                  15
   Val Asp Pro Tyr Val Val Phe Ser Cys Asn Gly Ile Thr Arg Thr Ser
               20                  25                  30
   Ser Val Gln Leu Gln Thr His Asp Pro Gln Trp Asn Glu Ile Met Glu
           35                  40                  45
   Phe Asp Ala Met Glu Glu Pro Pro Ala Thr Leu Asp Val Glu Val Phe
       50                  55                  60
   Asn Phe Asp Gly Pro Phe Asp Leu Ala Val Ser Leu Gly His Ala Glu
   65                  70                  75                  80
<210> 636
   <211> 44
   <212> PRT
   <213> Artificial Sequence
<220>
   <223> Synthetically generated peptide
<400> 636
   Arg Arg Thr Lys Glu Pro Thr Trp Asn Glu Glu Phe Thr Phe Asn Ile
    1               5                  10                  15
   Ser Leu Ser Arg Glu Asn Leu Leu Gln Val Ala Ala Trp Asp Ala Asn
               20                  25                  30
   Leu Val Thr Pro His Lys Arg Met Gly Asn Ala Gly
           35                  40
<210> 637
   <211> 65
   <212> PRT
   <213> Artificial Sequence
<220>
   <223> Synthetically generated peptide
<400> 637
   Gly Thr Ser Asp Pro Tyr Val Val Met Asp Leu Asp Gly Gln Val Ala
   1               5                   10                  15
   Lys Ser Lys Thr Lys Trp Gly Thr Lys Glu Pro Lys Trp Asn Glu Asp
               20                  25                  30
   Phe Val Phe Asn Ile Lys Leu Pro Pro Ala Lys Lys Ile Glu Ile Ala
           35                  40                  45
   Ala Trp Asp Ala Asn Leu Val Thr Pro His Lys Arg Met Gly Asn Ser
       50                  55                  60
   Glu
   65
<210> 638
   <211> 94
   <212> PRT
   <213> Artificial Sequence
<220>
   <223> synthetically generated peptide
<400> 638
   Leu Lys Val Lys Ile Tyr Thr Gly Glu Gly Trp Asp Leu Asp Phe His
   1               5                   10                  15
   His Thr His Phe Asp Gln Tyr Ser Pro Pro Asp Phe Phe Val Lys Ile
               20                  25                  30
   Gly Ile Ala Gly Val Pro Arg Asp Thr Val Ser Tyr Arg Thr Glu Thr
           35                  40                  45
   Ala Val Asp Gln Trp Phe Pro Ile Trp Gly Asn Asp Glu Phe Leu Phe
       50                  55                  60
   Gln Leu Ser Val Pro Glu Leu Ala Leu Leu Trp Phe Lys Val Gln Asp
   65                  70                  75                  80
   Tyr Asp Asn Asp Thr Gln Asn Asp Phe Ala Gly Gln Thr Cys
                   85                  90
<210> 639
   <211> 94
   <212> PRT
   <213> Artificial Sequence
<220>
   <223> synthetically generated peptide
<400> 639
   Leu Lys Val Lys Ile Tyr Thr Gly Glu Gly Trp Asp Leu Asp Phe His
   1               5                   10                  15
   His Thr His Phe Asp Gln Tyr Ser Pro Pro Asp Phe Phe Val Lys Ile
               20                  25                  30
   Gly Ile Ala Gly Val Pro Arg Asp Thr Val Ser Tyr Arg Thr Glu Thr
           35                  40                  45
   Ala Val Asp Gln Trp Phe Pro Ile Trp Gly Asn Asp Glu Phe Leu Phe
       50                  55                  60
   Gln Leu Ser Val Pro Glu Leu Ala Leu Leu Trp Phe Lys Val Gln Asp
   65                  70                  75                  80
   Tyr Asp Asn Asp Thr Gln Asn Asp Phe Ala Gly Gln Thr Cys
                   85                  90
<210> 640
   <211> 94
   <212> PRT
   <213> Artificial Sequence
<220>
   <223> Synthetically generated peptide
<400> 640
   Leu Lys Val Lys Ile Tyr Thr Gly Glu Gly Trp Asp Leu Asp Phe His
   1               5                   10                  15
   His Thr His Phe Asp Gln Tyr Ser Pro Pro Asp Phe Phe Val Lys Ile
               20                  25                  30
   Gly Ile Ala Gly Val Pro Arg Asp Thr Val Ser Tyr Arg Thr Glu Thr
           35                  40                  45
   Ala Val Asp Gln Trp Phe Pro Ile Trp Gly Asn Asp Glu Phe Leu Phe
       50                  55                  60
   Gln Leu Ser Val Pro Glu Leu Ala Leu Leu Trp Phe Lys Val Gln Asp
   65                  70                  75                  80
   Tyr Asp Asn Asp Thr Gln Asn Asp Phe Ala Gly Gln Thr Cys
                   85                  90
<210> 641
   <211> 93
   <212> PRT
   <213> Artificial Sequence
<220>
   <223> synthetically generated peptide
<400> 641
   Leu Lys Val Lys Ile Tyr Thr Gly Glu Gly Trp Asn Met Asp Phe Pro
   1 5 10 15
   Leu Asp His Phe Asp Arg Tyr Ser Pro Pro Asp Phe Tyr Ala Lys Val
   20 25 30
   Gly Ile Ala Gly Val Pro Leu Asp Thr Ala Ser Tyr Arg Thr Glu Ile
   35 40 45
   Asp Lys Asp Glu Trp Phe Pro Ile Trp Asp Lys Glu Phe Glu Phe Pro
   50 55 60
   Leu Arg Val Pro Glu Leu Ser Leu Leu Cys Ile Thr Val Lys Asp Tyr
   65 70 75 80
   Asp Ser Asn Thr Gln Asn Asp Phe Ala Gly Gln Thr Cys
   85 90
<210> 642
   <211> 93
   <212> PRT
   <213> Artificial Sequence
<220>
   <223> Synthetically generated peptide
<400> 642
   Leu Lys Val Lys Ile Tyr Thr Gly Glu Gly Trp Asn Met Asp Phe Pro
   1 5 10 15
   Leu Asp His Phe Asp Arg Tyr Ser Pro Pro Asp Phe Tyr Ala Lys Val
   20 25 30
   Gly Ile Ala Gly Val Pro Leu Asp Thr Ala Ser Tyr Arg Thr Glu Ile
   35 40 45
   Asp Lys Asp Glu Trp Phe Pro Ile Trp Asp Lys Glu Phe Glu Phe Pro
   50 55 60
   Leu Arg Val Pro Glu Leu Ser Leu Leu Cys Ile Thr Val Lys Asp Tyr
   65 70 75 80
   Asp Ser Asn Thr Gln Asn Asp Phe Ala Gly Gln Thr Cys
   85 90
<210> 643
   <211> 93
   <212> PRT
   <213> Artificial Sequence
<220>
   <223> Synthetically generated peptide
<400> 643
   Leu Lys Val Lys Val Cys Met Gly Asp Gly Trp Leu Leu Asp Phe Lys
   1 5 10 15
   Lys Thr His Phe Asp Ser Tyr Ser Pro Pro Asp Phe Phe Val Arg Val
   20 25 30
   Gly Ile Ala Gly Ala Pro Val Asp Glu Val Met Glu Lys Thr Lys Ile
   35 40 45
   Glu Tyr Asp Thr Trp Thr Pro Ile Trp Asn Lys Glu Phe Thr Phe Pro
   50 55 60
   Leu Ala Val Pro Glu Leu Ala Leu Leu Arg Val Glu Val His Glu His
   65 70 75 80
   Asp Val Asn Glu Lys Asp Asp Phe Gly Gly Gln Thr Cys
   85 90
<210> 644
   <211> 93
   <212> PRT
   <213> Artificial Sequence
<220>
   <223> synthetically generated peptide
<400> 644
   Leu Lys Val Lys Val Cys Met Gly Asp Gly Trp Leu Leu Asp Phe Lys
   1               5                   10                  15
   Lys Thr His Phe Asp Ser Tyr Ser Pro Pro Asp Phe Phe Val Arg Val
               20                  25                  30
   Gly Ile Ala Gly Ala Pro Val Asp Glu Val Met Glu Lys Thr Lys Ile
           35                  40                  45
   Glu Tyr Asp Thr Trp Thr Pro Ile Trp Asn Lys Glu Phe Thr Phe Pro
       50                  55                  60
   Leu Ala Val Pro Glu Leu Ala Leu Leu Arg Val Glu Val His Glu His
   65                  70                  75                  80
   Asp Val Asn Glu Lys Asp Asp Phe Gly Gly Gln Thr Cys
                   85                  90
<210> 645
   <211> 93
   <212> PRT
   <213> Artificial Sequence
<220>
   <223> synthetically generated peptide
<400> 645
   Leu Lys Val Lys Val Cys Met Gly Asp Gly Trp Leu Leu Asp Phe Lys
   1               5                   10                  15
   Lys Thr His Phe Asp Ser Tyr Ser Pro Pro Asp Phe Phe Val Arg Val
               20                  25                  30
   Gly Ile Ala Gly Ala Pro Val Asp Glu Val Met Glu Lys Thr Lys Ile
           35                  40                  45
   Glu Tyr Asp Thr Trp Thr Pro Ile Trp Asn Lys Glu Phe Thr Phe Pro
       50                  55                  60
   Leu Ala Val Pro Glu Leu Ala Leu Leu Arg Val Glu Val His Glu His
   65                  70                  75                  80
   Asp Val Asn Glu Lys Asp Asp Phe Gly Gly Gln Thr Cys
                   85                  90
<210> 646
   <211> 93
   <212> PRT
   <213> Artificial Sequence
<220>
   <223> Synthetically generated peptide
<400> 646
   Leu Lys Val Thr Val Tyr Met Gly Asp Gly Trp Arg Phe Asp Phe Arg
   1 5 10 15
   Lys Thr His Phe Asp Lys Cys Ser Pro Pro Asp Phe Tyr Ala Arg Val
   20 25 30
   Gly Ile Ala Gly Val Glu Ala Asp Thr Arg Met Glu Gln Thr Lys Val
   35 40 45
   Lys Met Asp Thr Trp Ile Pro Ala Trp Asp His Glu Phe Glu Phe Pro
   50 55 60
   Leu Ser Val Pro Glu Leu Ala Leu Leu Arg Val Glu Val His Glu Ser
   65 70 75 80
   Asp Asn His Gln Lys Asp Asp Phe Gly Gly Gln Thr Cys
   85 90
<210> 647
   <211> 93
   <212> PRT
   <213> Artificial Sequence
<220>
   <223> Synthetically generated peptide
<400> 647
   Leu Arg Val Thr Val Tyr Met Gly Glu Gly Trp Tyr Phe Asp Phe Arg
   1 5 10 15
   His Thr His Phe Asp Gln Tyr Ser Pro Pro Asp Phe Tyr Thr Arg Val
   20 25 30
   Gly Ile Ala Gly Val Pro Gly Asp Thr Val Met Lys Lys Thr Lys Thr
   35 40 45
   Leu Glu Asp Asn Trp Ile Pro Ala Trp Asp Glu Val Phe Glu Phe Pro
   50 55 60
   Leu Thr Val Pro Glu Leu Ala Leu Leu Arg Leu Glu Val His Glu Tyr
   65 70 75 80
   Asp Met Ser Glu Lys Asp Asp Phe Gly Gly Gln Thr Cys
   85 90
<210> 648
   <211> 93
   <212> PRT
   <213> Artificial Sequence
<220>
   <223> synthetically generated peptide
<400> 648
   Leu Arg Val Thr Val Tyr Met Gly Glu Gly Trp Tyr Phe Asp Phe Arg
   1 5 10 15
   His Thr His Phe Asp Gln Tyr Ser Pro Pro Asp Phe Tyr Thr Arg Val
   20 25 30
   Gly Ile Ala Gly Val Pro Gly Asp Thr Val Met Lys Lys Thr Lys Thr
   35 40 45
   Leu Glu Asp Asn Trp Ile Pro Ala Trp Asp Glu Val Phe Glu Phe Pro
   50 55 60
   Leu Thr Val Pro Glu Leu Ala Leu Leu Arg Leu Glu Val His Glu Tyr
   65 70 75 80
   Asp Met Ser Glu Lys Asp Asp Phe Gly Gly Gln Thr Cys
   85 90
<210> 649
   <211> 93
   <212> PRT
   <213> Artificial Sequence
<220>
   <223> synthetically generated peptide
<400> 649
   Leu Arg Val Thr Val Tyr Met Gly Glu Gly Trp Tyr Phe Asp Phe Arg
   1 5 10 15
   His Thr His Phe Asp Gln Tyr Ser Pro Pro Asp Phe Tyr Thr Arg Val
   20 25 30
   Gly Ile Ala Gly Val Pro Gly Asp Thr Val Met Lys Lys Thr Lys Thr
   35 40 45
   Leu Glu Asp Asn Trp Ile Pro Ala Trp Asp Glu Val Phe Glu Phe Pro
   50 55 60
   Leu Thr Val Pro Glu Leu Ala Leu Leu Arg Leu Glu Val His Glu Tyr
   65 70 75 80
   Asp Met Ser Glu Lys Asp Asp Phe Gly Gly Gln Thr Cys
   85 90
<210> 650
   <211> 93
   <212> PRT
   <213> Artificial Sequence
<220>
   <223> synthetically generated peptide
<400> 650
   Leu Arg Val Thr Ile Tyr Met Gly Glu Gly Trp Tyr Phe Asp Phe Arg
   1 5 10 15
   His Thr His Phe Asp Gln Tyr Ser Pro Pro Asp Phe Tyr Thr Arg Val
   20 25 30
   Gly Ile Ala Gly Val Pro Ala Asp Thr Val Met Lys Lys Thr Lys Thr
   35 40 45
   Leu Glu Asp Asn Trp Val Pro Ser Trp Asp Glu Val Phe Glu Phe Pro
   50 55 60
   Leu Thr Val Pro Glu Leu Ala Leu Leu Arg Leu Glu Val His Glu Tyr
   65 70 75 80
   Asp Met Ser Glu Lys Asp Asp Phe Gly Gly Gln Thr Cys
   85 90
<210> 651
   <211> 93
   <212> PRT
   <213> Artificial Sequence
<220>
   <223> Synthetically generated peptide
<400> 651
   Leu Arg Val Thr Ile Tyr Met Gly Glu Gly Trp Tyr Tyr Asp Phe Pro
   1 5 10 15
   His Thr His Phe Asp Arg Tyr Ser Pro Pro Asp Phe Tyr Thr Arg Val
   20 25 30
   Gly Ile Ala Gly Val Pro Ala Asp Thr Val Met Lys Lys Thr Lys Thr
   35 40 45
   Leu Glu Asp Asn Trp Ile Pro Ala Trp Asp Glu Val Phe Glu Phe Pro
   50 55 60
   Leu Thr Val Pro Glu Leu Ala Leu Leu Arg Ile Glu Val His Glu Tyr
   65 70 75 80
   Asp Met Ser Glu Lys Asp Asp Phe Gly Gly Gln Ile Cys
   85 90
<210> 652
   <211> 93
   <212> PRT
   <213> Artificial Sequence
<220>
   <223> Synthetically generated peptide
<400> 652
   Leu Lys Val Thr Val Tyr Met Gly Glu Gly Trp Tyr Tyr Asp Phe Lys
   1 5 10 15
   His Thr His Phe Asp Gln Tyr Ser Pro Pro Asp Phe Tyr Thr Arg Val
   20 25 30
   Gly Ile Ala Gly Val Pro Asn Asp Thr Ile Met Lys Arg Thr Lys Ala
           35                  40                  45
   Ile Glu Asp Asn Trp Leu Pro Thr Trp Asn Glu Ala Phe Glu Phe Pro
       50                  55                  60
   Leu Thr Val Pro Glu Leu Ala Leu Leu Arg Ile Glu Val His Glu Tyr
   65                  70                  75                  80
   Asp Met Ser Glu Lys Asp Asp Phe Gly Gly Gln Thr Cys
                   85                  90
<210> 653
   <211> 93
   <212> PRT
   <213> Artificial sequence
<220>
   <223> Synthetically generated peptide
<400> 653
   Leu Lys Val Thr Val Tyr Met Gly Glu Gly Trp Tyr Tyr Asp Phe Lys
   1               5                   10                  15
   His Thr His Phe Asp Gln Tyr Ser Pro Pro Asp Phe Tyr Thr Arg Val
               20                  25                  30
   Gly Ile Ala Gly Val Pro Asn Asp Thr Ile Met Lys Arg Thr Lys Ala
           35                  40                  45
   Ile Glu Asp Asn Trp Leu Pro Thr Trp Asn Glu Val Phe Glu Phe Pro
       50                  55                  60
   Leu Thr Val Pro Glu Leu Ala Leu Leu Arg Ile Glu Val His Glu Tyr
   65                  70                  75                  80
   Asp Met Ser Glu Lys Asp Asp Phe Gly Gly Gln Ala Cys
                   85                  90
<210> 654
   <211> 93
   <212> PRT
   <213> Artificial sequence
<220>
   <223> Synthetically generated peptide
<400> 654
   Leu Lys Val Thr Val Tyr Met Gly Glu Gly Trp Tyr Tyr Asp Phe Lys
   1               5                   10                  15
   His Thr His Phe Asp Gln Tyr Ser Pro Pro Asp Phe Tyr Thr Arg Val
               20                  25                  30
   Gly Ile Ala Gly Val Pro Ser Asp Thr Val Met Lys Lys Thr Lys Ala
           35                  40                  45
   Asn Lys Asp Asn Trp Leu Pro Thr Trp Asn Glu Thr Phe Glu Phe Pro
       50                  55                  60
   Leu Ser Val Pro Glu Leu Ala Leu Leu Arg Ile Glu Val His Glu Tyr
   65                  70                  75                  80
   Asp Met Ser Glu Lys Asp Asp Phe Gly Gly Gln Thr Cys
                   85                  90
<210> 655
   <211> 93
   <212> PRT
   <213> Artificial Sequence
<220>
   <223> Synthetically generated peptide
<400> 655
   Leu Lys Val Thr Val Tyr Met Gly Glu Gly Trp Tyr Tyr Asp Phe Lys
   1               5                   10                  15
   His Thr His Phe Asp Gln Phe Ser Pro Pro Asp Phe Tyr Ala Arg Ile
               20                  25                  30
   Gly Ile Ala Gly Val Pro Phe Asp Thr Val Met Lys Lys Thr Lys Ser
           35                  40                  45
   Ile Glu Asp Ser Trp Leu Pro Ser Trp Asn Glu Val Phe Glu Phe Pro
       50                  55                  60
   Leu Ser Val Pro Glu Leu Ala Leu Leu Arg Ile Glu Val His Glu Tyr
   65                  70                  75                  80
   Asp Met Ser Glu Lys Asp Asp Phe Gly Gly Gln Thr Cys
                   85                  90
<210> 656
   <211> 93
   <212> PRT
   <213> Artificial sequence
<220>
   <223> Synthetically generated peptide
<400> 656
   Leu Lys Val Thr Val Tyr Met Gly Glu Gly Trp Tyr Tyr Asp Phe Lys
   1               5                   10                  15
   His Thr His Phe Asp Gln Phe Ser Pro Pro Asp Phe Tyr Ala Arg Val
               20                  25                  30
   Gly Ile Ala Gly Val Pro Phe Asp Thr Val Met Lys Lys Thr Lys Ser
           35                  40                  45
   Ile Glu Asp Ser Trp Leu Pro Ser Trp Asn Glu Val Phe Glu Phe Pro
       50                  55                  60
   Leu Ser Val Pro Glu Leu Ala Leu Leu Arg Ile Glu Val His Glu Tyr
   65                  70                  75                  80
   Asp Met Ser Glu Lys Asp Asp Phe Gly Gly Gln Thr Cys
                   85                  90
<210> 657
   <211> 93
   <212> PRT
   <213> Artificial Sequence
<220>
   <223> Synthetically generated peptide
<400> 657
   Leu Lys Val Thr Val Tyr Met Gly Glu Gly Trp Tyr Tyr Asp Phe Asp
   1               5                   10                  15
   His Thr His Phe Asp Gln Phe Ser Pro Pro Asp Phe Tyr Ala Arg Val
               20                  25                  30
   Gly Ile Ala Gly Val Pro Phe Asp Thr Ile Met Lys Lys Thr Lys Thr
           35                  40                  45
   Val Glu Asp Ser Trp Leu Pro Ser Trp Asn Glu Val Phe Glu Phe Pro
       50                  55                  60
   Leu Ser Val Pro Glu Leu Ala Leu Leu Arg Ile Glu Val His Glu Tyr
   65                  70                  75                  80
   Asp Met Ser Glu Lys Asp Asp Phe Gly Gly Gln Thr Cys
                   85                  90
<210> 658
   <211> 93
   <212> PRT
   <213> Artificial sequence
<220>
   <223> synthetically generated peptide
<400> 658
   Leu Lys Val Thr Val Tyr Met Gly Glu Gly Trp Tyr Tyr Asp Phe Asp
   1               5                   10                  15
   His Thr His Phe Asp Gln Phe Ser Pro Pro Asp Phe Tyr Ala Arg Val
               20                  25                  30
   Gly Ile Ala Gly Val Pro Phe Asp Thr Ile Met Lys Lys Thr Lys Thr
           35                  40                  45
   Val Glu Asp Ser Trp Leu Pro Ser Trp Asn Glu Val Phe Glu Phe Pro
       50                  55                  60
   Leu Ser Val Pro Glu Leu Ala Leu Leu Arg Ile Glu Val His Glu Tyr
   65                  70                  75                  80
   Asp Met Ser Glu Lys Asp Asp Phe Gly Gly Gln Thr Cys
                   85                  90
<210> 659
   <211> 93
   <212> PRT
   <213> Artificial Sequence
<220>
   <223> Synthetically generated peptide
<400> 659
   Leu Lys Val Thr Ile Tyr Met Gly Glu Gly Trp Phe Leu Asp Phe Lys
   1               5                   10                  15
   His Thr His Phe Asp Lys Phe Ser Pro Pro Asp Phe Tyr Ala Arg Val
               20                  25                  30
   Gly Ile Ala Gly Val Pro Asn Asp Thr Val Met Lys Lys Thr Glu Lys
           35                  40                  45
   Val Glu Asp Asn Trp Ser Pro Ser Trp Asn Gln Val Phe Lys Phe Pro
       50                  55                  60
   Leu Ala Val Pro Glu Leu Ala Leu Leu Arg Val Glu Val His Glu Tyr
   65                  70                  75                  80
   Asp Met Ser Glu Lys Asp Asp Phe Gly Gly Gln Thr Cys
                   85                  90
<210> 660
   <211> 93
   <212> PRT
   <213> Artificial sequence
<220>
   <223> Synthetically generated peptide
<400> 660
   Leu Lys Val Thr Ile Tyr Met Gly Glu Gly Trp Phe His Asp Phe Lys
   1               5                   10                  15
   His Thr His Phe Asp Gln Tyr Ser Pro Pro Asp Phe Tyr Ala Arg Val
               20                  25                  30
   Gly Ile Ala Gly Val Pro Tyr Asp Thr Val Met Lys Lys Thr Lys Ser
           35                  40                  45
   Val Glu Asp Asn Trp Ser Pro Ser Trp Asn Glu Glu Phe Lys Phe Pro
       50                  55                  60
   Leu Ser Val Pro Glu Leu Ala Leu Leu Arg Val Glu Val His Glu Tyr
   65                  70                  75                  80
   Asp Met Ser Glu Lys Asp Asp Phe Gly Gly Gln Thr Cys
                   85                  90
<210> 661
   <211> 93
   <212> PRT
   <213> Artificial Sequence
<220>
   <223> Synthetically generated peptide
<400> 661
   Leu Lys Val Thr Val Phe Met Gly Glu Gly Trp Tyr Ile Asp Phe Lys
   1 5 10 15
   His Thr His Phe Asp Ala Tyr Thr Pro Pro Asp Phe Tyr Ala Lys Ile
   20 25 30
   Gly Ile Ala Gly Val Pro Ala Asp Asn Val Met Lys Lys Thr Lys Thr
   35 40 45
   Leu Glu Asp Met Asp Thr Pro Thr Trp Asp Glu Lys Phe Glu Phe Pro
   50 55 60
   Leu Thr Val Pro Glu Leu Ala Leu Leu Arg Val Glu Val His Glu Tyr
   65 70 75 80
   Asp Met Ser Glu Lys Asp Asp Phe Ala Gly Gln Thr Cys
   85 90
<210> 662
   <211> 93
   <212> PRT
   <213> Artificial Sequence
<220>
   <223> Synthetically generated peptide
<400> 662
   Leu Lys Val Thr Val Phe Met Gly Glu Gly Trp Tyr Tyr Asp Phe Asn
   1 5 10 15
   His Thr His Phe Asp Ala Tyr Ala Pro Pro Asp Phe Tyr Ala Lys Ile
   20 25 30
   Gly Ile Ala Gly Val Pro Ala Asp Asn Val Met Lys Lys Thr Arg Thr
   35 40 45
   Leu Glu Asn Asn Trp Ile Pro Thr Trp Asp Glu Lys Phe Glu Phe Pro
   50 55 60
   Leu Thr Val Pro Glu Leu Ala Leu Leu Arg Val Glu Val His Val Tyr
   65 70 75 80
   Asp Met Ser Glu Lys Asp Asp Phe Ala Gly Gln Thr Cys
   85 90
<210> 663
   <211> 93
   <212> PRT
   <213> Artificial Sequence
<220>
   <223> Synthetically generated peptide
<400> 663
   Leu Lys Val Thr Val Phe Met Gly Glu Gly Trp Tyr Tyr Asp Phe Glu
   1 5 10 15
   His Thr His Phe Asp Ala Tyr Ser Pro Pro Asp Phe Tyr Ala Arg Ile
   20 25 30
   Gly Ile Ala Gly Val Asp Ala Asp Ile Val Met Lys Lys Thr Lys Thr
   35 40 45
   Leu Glu Asp Asn Trp Ile Pro Thr Trp Asp Glu Gln Phe Glu Phe Pro
   50 55 60
   Leu Thr Val Pro Glu Leu Ala Leu Leu Arg Val Glu Val His Glu Tyr
   65 70 75 80
   Asp Met Ser Glu Lys Asp Asp Phe Ala Gly Gln Thr Cys
   85 90
<210> 664
   <211> 93
   <212> PRT
   <213> Artificial Sequence
<220>
   <223> Synthetically generated peptide
<400> 664
   Leu Lys Val Lys Val Tyr Met Gly Lys Gly Trp His Leu Asp Phe Lys
   1 5 10 15
   Arg Thr His Phe Asp Ala Tyr Ser Pro Pro Asp Phe Tyr Val Lys Ile
   20 25 30
   Gly Ile Ala Gly Val Ala Ala Asp Ser Arg Val Lys Lys Thr Lys Ala
   35 40 45
   Ile Glu Asp Asn Trp Ile Pro Ile Trp Asn Asp Glu Phe Glu Phe Pro
   50 55 60
   Leu Thr Val Pro Glu Leu Ala Leu Leu Arg Val Glu Val His Glu Tyr
   65 70 75 80
   Asp Met Ser Glu Ile Asp Asp Phe Gly Gly Gln Thr Cys
   85 90
<210> 665
   <211> 93
   <212> PRT
   <213> Artificial Sequence
<220>
   <223> Synthetically generated peptide
<400> 665
   Leu Lys Val Lys Val Tyr Met Gly Asp Gly Trp Arg Met Asp Phe Ser
   1 5 10 15
   Lys Thr His Phe Asp Thr Phe Ser Pro Pro Asp Phe Tyr Thr Arg Val
   20 25 30
   Gly Ile Ala Gly Val Arg Ala Asp Cys Val Met Lys Lys Thr Arg Thr
   35 40 45
   Ile Glu Asp Gln Trp Val Pro Met Trp Asp Glu Glu Phe Thr Phe Pro
   50 55 60
   Leu Thr Val Pro Glu Leu Ala Val Leu Arg Ile Glu Val His Glu Tyr
   65 70 75 80
   Asp Met Ser Glu Lys His Asp Phe Gly Gly Gln Thr Cys
   85 90
<210> 666
   <211> 93
   <212> PRT
   <213> Artificial Sequence
<220>
   <223> Synthetically generated peptide
<400> 666
   Leu Lys Val Lys Val Tyr Met Gly Asp Gly Trp Arg Met Asp Phe Ser
   1 5 10 15
   Lys Thr His Phe Asp Thr Phe Ser Pro Pro Asp Phe Tyr Thr Arg Val
   20 25 30
   Gly Ile Ala Gly Val Arg Ala Asp Cys Val Met Lys Lys Thr Arg Thr
   35 40 45
   Ile Glu Asp Gln Trp Val Pro Met Trp Asp Glu Glu Phe Thr Phe Pro
   50 55 60
   Leu Thr Val Pro Glu Leu Ala Val Leu Arg Ile Glu Val His Glu Tyr
   65 70 75 80
   Asp Met Ser Glu Lys His Asp Phe Gly Gly Gln Thr Cys
   85 90
<210> 667
   <211> 93
   <212> PRT
   <213> Artificial Sequence
<220>
   <223> Synthetically generated peptide

<400> 667
   Leu Lys Val Lys Val Tyr Met Gly Asp Arg Trp Arg Met Asp Phe Ser
   1 5 10 15
   Lys Thr His Phe Asp Ala Phe Ser Pro Pro Asp Phe Tyr Thr Lys Val
   20 25 30
   Gly Ile Ala Gly Val Lys Ala Asp Ser Val Met Lys Lys Thr Arg Val
   35 40 45
   Ile Glu Asp Gln Trp Val Pro Met Trp Asp Glu Glu Phe Thr Phe Leu
   50 55 60
   Leu Thr Val Pro Glu Leu Ala Leu Leu Arg Val Glu Val Gln Glu Tyr
   65 70 75 80
   Asp Met Ser Glu Lys His Asp Phe Gly Gly Gln Thr Val
   85 90
<210> 668
   <211> 93
   <212> PRT
   <213> Artificial sequence
<220>
   <223> Synthetically generated peptide
<400> 668
   Leu Lys Val Lys Val Tyr Met Gly Asp Gly Trp Arg Met Asp Phe Thr
   1 5 10 15
   Gln Thr His Phe Asp Gln Tyr Ser Pro Pro Asp Phe Tyr Ala Arg Val
   20 25 30
   Gly Ile Ala Gly Val Pro Ala Asp Ser Val Met Lys Arg Thr Arg Ala
   35 40 45
   Ile Glu Asp Asn Trp Val Pro Val Trp Glu Glu Asp Phe Thr Phe Lys
   50 55 60
   Leu Thr Val Pro Glu Ile Ala Leu Leu Arg Val Glu Val His Glu Tyr
   65 70 75 80
   Asp Met Ser Glu Lys Asp Asp Phe Gly Gly Gln Thr Val
   85 90
<210> 669
   <211> 93
   <212> PRT
   <213> Artificial Sequence
<220>
   <223> Synthetically generated peptide
<400> 669
   Leu Lys Val Lys Val Tyr Met Gly Asp Gly Trp Arg Met Asp Phe Thr
   1               5                   10                  15
   Gln Thr His Phe Asp Gln Tyr Ser Pro Pro Asp Phe Tyr Ala Arg Val
               20                  25                  30
   Gly Ile Ala Gly Val Pro Ala Asp Ser Val Met Lys Arg Thr Arg Ala
           35                  40                  45
   Ile Glu Asp Asn Trp Val Pro Val Trp Glu Glu Asp Phe Thr Phe Lys
       50                  55                  60
   Leu Thr Val Pro Glu Ile Ala Leu Leu Arg Val Glu Val His Glu Tyr
   65                  70                  75                  80
   Asp Met Ser Glu Lys Asp Asp Phe Gly Gly Gln Thr Val
                   85                  90
<210> 670
   <211> 93
   <212> PRT
   <213> Artificial sequence
<220>
   <223> Synthetically generated peptide
<400> 670
   Leu Lys Val Lys Val Tyr Met Gly Asp Gly Trp Arg Met Asp Phe Lys
   1               5                   10                  15
   Ser Thr His Phe Asp Thr Tyr Ser Pro Pro Asp Phe Tyr Thr Arg Val
               20                  25                  30
   Gly Ile Ala Gly Val Pro Ala Asp Cys Thr Met Lys Lys Thr Arg Thr
           35                  40                  45
   Ile Glu Asp Asp Trp Thr Pro Val Trp Asp Glu Glu Leu Val Phe Pro
       50                  55                  60
   Leu Thr Val Pro Glu Leu Ala Leu Leu Arg Ile Glu Val His Glu Tyr
   65                  70                  75                  80
   Asp Met Ser Asp Lys Asp Asp Phe Gly Gly Gln Thr Cys
                   85                  90
<210> 671
   <211> 93
   <212> PRT
   <213> Artificial Sequence
<220>
   <221> VARIANT
   <222> 41
   <223> Xaa = any amino acid
<220>
   <223> Synthetically generated peptide
<400> 671
   Leu Lys Val Lys Val Tyr Met Gly Asp Gly Trp Arg Met Asp Phe Lys
   1               5                   10                  15
   Gln Thr Tyr Phe Asp Ala Tyr Ser Pro Pro Asp Phe Tyr Thr Arg Ile
               20                  25                  30
   Gly Ile Ala Gly Val Pro Ala Asp Xaa Val Met Lys Arg Thr Lys Ala
           35                  40                  45
   Ile Glu Asp Asp Trp Thr Pro Val Trp Asn Glu Glu Phe Val Phe Pro
       50                  55                  60
   Leu Thr Val Pro Glu Ile Ala Leu Leu Arg Ile Glu Val His Glu Tyr
   65                  70                  75                  80
   Asp Met Ser Glu Lys Asp Asp Phe Gly Gly Gln Thr Cys
                   85                  90
<210> 672
   <211> 91
   <212> PRT
   <213> Artificial Sequence
<220>
   <223> Synthetically generated peptide
<400> 672
   Leu Lys Val Lys Val Tyr Met Gly Asp Gly Trp His Leu Asp Phe Lys
   1               5                   10                  15
   Gln Thr His Phe Asp Lys Pro Pro Asp Phe Tyr Thr Arg Val Gly Ile
               20                  25                  30
   Ala Gly Val Pro Ala Asp Glu Ile Met Lys Lys Thr Lys Thr Lys Glu
           35                  40                  45
   Asp Lys Trp Thr Pro Val Trp Asp Glu Ala Phe Thr Phe Pro Leu Thr
       50                  55                  60
   Val Pro Glu Leu Ala Leu Leu Arg Ile Glu Val His Glu Tyr Asp Met
   65                  70                  75                  80
   Ser Glu Lys Asp Asp Phe Ala Gly Gln Thr Cys
                   85                  90
<210> 673
   <211> 91
   <212> PRT
   <213> Artificial sequence
<220>
   <223> Synthetically generated peptide
<400> 673
   Leu Lys Val Lys Val Tyr Met Gly Asp Gly Trp His Leu Asp Phe Lys
   1               5                   10                  15
   Gln Thr His Phe Asp Lys Pro Pro Asp Phe Tyr Thr Arg Val Gly Ile
               20                  25                  30
   Ala Gly Val Pro Ala Asp Glu Ile Met Lys Lys Thr Lys Thr Lys Glu
           35                  40                  45
   Asp Lys Trp Thr Pro Val Trp Asp Glu Glu Phe Thr Phe Pro Leu Thr
       50                  55                  60
   Val Pro Glu Leu Ala Leu Leu Arg Ile Glu Val His Glu Tyr Asp Met
   65                  70                  75                  80
   Ser Glu Lys Asp Asp Phe Ala Gly Gln Thr Cys
                   85                  90
<210> 674
   <211> 89
   <212> PRT
   <213> Artificial Sequence
<220>
   <223> Synthetically generated peptide
<400> 674
   Leu Gln Val Arg Val Tyr Met Gly Asp Gly Trp Arg Leu Asp Phe Ser
    1               5                  10                  15
   His Thr His Phe Asp Ala Tyr Ser Pro Pro Asp Phe Tyr Thr Lys Val
               20                  25                  30
   Ile Gly Val Pro Ala Asp Ser Arg Lys Lys Lys Thr Arg Ile Asp Asp
           35                  40                  45
   Trp Cys Pro Val Trp Asp Glu Glu Phe Asn Phe Pro Leu Thr Val Pro
       50                  55                  60
   Glu Leu Ala Leu Leu Arg Ile Glu Val Arg Glu Tyr Asp Met Ser Glu
   65                  70                  75                  80
   Lys Asp Asp Phe Gly Gly Gln Thr Cys
                   85
<210> 675
   <211> 93
   <212> PRT
   <213> Artificial Sequence
<220>
   <223> Synthetically generated peptide
<400> 675
   Leu Lys Val Lys Val Tyr Met Gly Asp Gly Trp Arg Met Asp Phe Ser
    1               5                  10                  15
   His Thr His Phe Asp Ala Tyr Ser Pro Pro Asp Phe Tyr Thr Lys Met
               20                  25                  30
   Phe Ile Val Gly Val Pro Ala Asp Asn Ala Lys Lys Lys Thr Lys Ile
           35                  40                  45
   Ile Glu Asp Asn Trp Tyr Pro Ile Trp Asp Glu Glu Phe Ser Phe Pro
       50                  55                  60
   Leu Thr Val Pro Glu Leu Ala Leu Leu Arg Ile Glu Val Arg Glu Tyr
   65                  70                  75                  80
   Asp Met Ser Glu Lys Asp Asp Phe Gly Gly Gln Thr Cys
                   85                  90
<210> 676
   <211> 92
   <212> PRT
   <213> Artificial sequence
<220>
   <223> Synthetically generated peptide
<400> 676
   Leu Lys Val Lys Val Tyr Met Gly Glu Gly Trp His Lys Asp Phe Lys
   1 5 10 15
   Gln Thr His Phe Asp Thr Tyr Ser Pro Pro Asp Phe Tyr Val Glu Val
   20 25 30
   Gly Ile Ala Gly Val Pro Leu Asp Ser Val Met Arg Lys Thr Lys Ala
   35 40 45
   Val Glu Asp Asn Trp Val Pro Val Trp Glu Glu Glu Phe Ala Phe Pro
   50 55 60
   Leu Thr Val Pro Glu Ile Ala Val Leu Arg Val Glu Val His Glu Gln
   65 70 75 80
   Asp Val Ser Glu Asp Asp Phe Gly Gly Gln Thr Ala
   85 90
<210> 677
   <211> 93
   <212> PRT
   <213> Artificial Sequence
<220>
   <223> Synthetically generated peptide
<400> 677
   Leu Lys Val Thr Val Tyr Met Gly Asp Gly Trp Asp Lys Asp Phe Asp
   1 5 10 15
   Gln Thr His Phe Asp Thr Tyr Ser Pro Pro Asp Phe Tyr Ala Lys Leu
   20 25 30
   Gly Ile Ala Gly Val Pro Ala Asp Glu Val Lys Lys Arg Thr Lys Thr
   35 40 45
   Met Asp Asp Asn Trp Ile Pro Ser Trp Asp Glu Gln Phe Glu Phe Pro
   50 55 60
   Leu Thr Val Pro Glu Leu Ala Leu Leu Arg Ile Lys Val Leu Asp Tyr
   65 70 75 80
   Asn Leu Ser Asp Lys Asp Glu Phe Ala Gly Gln Thr Cys
   85 90
<210> 678
   <211> 93
   <212> PRT
   <213> Artificial Sequence
<220>
   <223> Synthetically generated peptide
<400> 678
   Leu Lys Val Thr Val Tyr Met Gly Asp Gly Trp Asp Lys Asp Phe Asp
   1 5 10 15
   Gln Thr Asp Phe Asp Thr Tyr Ser Pro Pro Asp Phe Tyr Ala Lys Leu
   20 25 30
   Gly Ile Ala Gly Val Pro Ala Asp Glu Val Lys Lys Arg Thr Glu Thr
   35 40 45
   Ile Asp Asp Asn Trp Ile Pro Ser Trp Asn Glu Gln Phe Glu Phe Pro
   50 55 60
   Leu Thr Val Pro Glu Leu Ala Leu Leu Arg Ile Lys Val Leu Asp Tyr
   65 70 75 80
   Asn Leu Ser Asp Lys Asp Glu Phe Ala Gly Gln Thr Cys
   85 90
<210> 679
   <211> 93
   <212> PRT
   <213> Artificial sequence
<220>
   <223> Synthetically generated peptide
<400> 679
   Leu Lys Val Lys Val Tyr Leu Gly Lys Gly Trp Ser Leu Asp Phe Ser
   1               5                   10                  15
   Pro Ser Asp Phe Asp Ser Tyr Ser Pro Pro Asp Phe Tyr Val Lys Val
               20                  25                  30
   Cys Ile Val Gly Val Pro Ala Asp Met Ile Lys Lys Lys Thr Ser Val
           35                  40                  45
   Ile Ser Asn Asn Trp Phe Pro Val Trp Asn Glu Glu Phe Asp Phe Pro
       50                  55                  60
   Leu Thr Val Pro Glu Leu Ala Leu Leu Gly Ile Glu Val Arg Glu Asp
   65                  70                  75                  80
   Asp Lys His Gln Lys Asp Asp Phe Gly Gly Gln Thr Cys
                   85                  90
<210> 680
   <211> 93
   <212> PRT
   <213> Artificial Sequence
<220>
   <223> Synthetically generated peptide
<400> 680
   Leu Lys Val Lys Val Tyr Leu Gly Lys Gly Trp Ser Leu Asp Phe Ser
   1               5                   10                  15
   Pro Ser Asp Phe Asp Ser Tyr Ser Pro Pro Asp Phe Tyr Val Lys Val
               20                  25                  30
   Cys Ile Val Gly Val Pro Ala Asp Met Ile Lys Lys Lys Thr Ser Val
           35                  40                  45
   Ile Ser Asn Asn Trp Phe Pro Val Trp Asn Glu Glu Phe Asp Phe Pro
       50                  55                  60
   Leu Thr Val Pro Glu Leu Ala Leu Leu Arg Ile Glu Val Arg Glu Glu
   65                  70                  75                  80
   Asp Lys His Gln Lys Asp Asp Phe Gly Gly Gln Thr Cys
                   85                  90
<210> 681
   <211> 93
   <212> PRT
   <213> Artificial Sequence
<220>
   <223> Synthetically generated peptide
<400> 681
   Leu Lys Val Lys Val Tyr Met Gly Asn Gly Trp Ser Ser Asp Phe Ser
   1               5                   10                  15
   Lys Thr His Phe Asp Ser Phe Ser Pro Pro Asp Phe Tyr Thr Lys Val
               20                  25                  30
   Cys Ile Val Gly Val Pro Ala Asp Lys Ala Asn Lys Lys Thr Lys Val
           35                  40                  45
   Ile Gln Asp Asn Trp Phe Pro Val Trp Asp Glu Glu Phe Glu Phe Pro
       50                  55                  60
   Leu Thr Val Pro Glu Leu Ala Leu Leu Arg Ile Glu Val Arg Glu Tyr
   65                  70                  75                  80
   Asp Lys His Glu Lys Asp Asp Phe Gly Gly Gln Thr Cys
                   85                  90
<210> 682
   <211> 92
   <212> PRT
   <213> Artificial sequence
<220>
   <223> Synthetically generated peptide
<400> 682
   Leu Lys Val Lys Val Tyr Lys Gly Val Gly Trp Arg Ser Asp Phe Ser
   1               5                   10                  15
   Pro Thr His Phe Asp Arg Phe Ser Pro Pro Asp Phe Tyr Thr Lys Val
               20                  25                  30
   Cys Ile Ala Gly Val Gly Ala Asp Ser Val Lys Met Lys Thr Ser Val
           35                  40                  45
   Lys Met Asp Asn Trp Tyr Pro Val Trp Asp Glu Glu Phe Glu Phe Gln
       50                  55                  60
   Leu Thr Val Pro Glu Leu Ala Leu Leu Arg Ile Glu Val Lys Asp Lys
   65                  70                  75                  80
   Asp Lys Gly Ser Asp Asp Phe Ala Gly Gln Thr Cys
                   85                  90
<210> 683
   <211> 92
   <212> PRT
   <213> Artificial Sequence
<220>
   <223> Synthetically generated peptide
<400> 683
   Leu Lys Val Lys Val Tyr Lys Gly Val Gly Trp Ser Ser Asp Phe Ser
   1               5                   10                  15
   Pro Thr His Phe Asp Arg Phe Ser Pro Pro Asp Phe Tyr Thr Lys Val
               20                  25                  30
   Ser Ile Ala Gly Val Arg Ala Asp Cys Ala Lys Lys Lys Thr Ser Val
           35                  40                  45
   Lys Met Asp Asn Trp Asn Pro Ile Trp Asp Glu Glu Phe Glu Phe Arg
       50                  55                  60
   Leu Thr Val Pro Glu Leu Ala Leu Leu Arg Ile Glu Val Lys Asp Lys
   65                  70                  75                  80
   Asp Gln Thr Lys Asp Asp Phe Ala Gly Gln Thr Cys
                   85                  90
<210> 684
   <211> 93
   <212> PRT
   <213> Artificial sequence
<220>
   <223> synthetically generated peptide
<400> 684
   Leu Lys Val Lys Val Tyr Met Gly Lys Gly Trp Asp Ser Gly Phe Gln
   1               5                   10                  15
   Arg Thr Cys Phe Asn Thr Trp Ser Ser Pro Asn Phe Tyr Thr Arg Val
               20                  25                  30
   Gly Ile Thr Gly Val Arg Gly Asp Lys Val Met Lys Lys Thr Lys Lys
           35                  40                  45
   Glu Gln Lys Thr Trp Glu Pro Phe Trp Asn Glu Glu Phe Glu Phe Gln
       50                  55                  60
   Leu Thr Val Pro Glu Leu Ala Leu Leu Arg Ile Glu Val His Asp Tyr
   65                  70                  75                  80
   Asn Met Pro Glu Lys Asp Asp Phe Ser Gly Gln Thr Cys
                   85                  90
<210> 685
   <211> 93
   <212> PRT
   <213> Artificial sequence
<220>
   <223> Synthetically generated peptide
<400> 685
   Leu Lys Val Lys Val Tyr Met Gly Lys Gly Trp Asp Ser Gly Phe Gln
   1               5                   10                  15
   Arg Thr Cys Phe Asn Thr Trp Ser Ser Pro Asn Phe Tyr Thr Arg Val
               20                  25                  30
   Gly Ile Thr Gly Val Arg Gly Asp Lys Val Met Lys Lys Thr Lys Lys
           35                  40                  45
   Glu Gln Lys Thr Trp Glu Pro Phe Trp Asn Glu Glu Phe Glu Phe Gln
       50                  55                  60
   Leu Thr Val Pro Glu Leu Ala Leu Leu Arg Ile Glu Val His Asp Tyr
   65                  70                  75                  80
   Asn Met Pro Glu Lys Asp Asp Phe Ser Gly Gln Thr Cys
                   85                  90
<210> 686
   <211> 73
   <212> PRT
   <213> Artificial sequence
<220>
   <223> Synthetically generated peptide
<400> 686
   Asp Leu Phe Ser Pro Pro Asp Phe Phe Thr Arg Leu Leu Val Thr Gly
   1               5                   10                  15
   Val Pro Ala Asp Val Ala Lys Trp Lys Thr Ser Val Ile Asp Asp Val
               20                  25                  30
   Trp Glu Pro His Trp Asn Glu Asp His Glu Phe Tyr Leu Lys Cys Pro
           35                  40                  45
   Glu Leu Ala Leu Leu Arg Ile Glu Val Arg Asp His Asp Glu Gly Ser
       50                  55                  60
   Gln Asp Glu Phe Glu Gly Gln Ala Cys
   65                  70
<210> 687
   <211> 92
   <212> PRT
   <213> Artificial Sequence
<220>
   <223> Synthetically generated peptide
<400> 687
   Leu Lys Val Lys Ile Tyr Met Gly Asp Gly Trp Ile Val Asp Phe Lys
   1               5                   10                  15
   Lys Arg Ile Gly Arg Leu Ser Lys Pro Asp Leu Tyr Val Arg Ile Ser
               20                  25                  30
   Ile Ala Gly Val Pro His Asp Glu Lys Ile Met Asn Thr Thr Val Lys
           35                  40                  45
   Asn Asn Glu Trp Lys Pro Thr Trp Gly Glu Glu Phe Thr Phe Pro Leu
       50                  55                  60
   Thr Tyr Pro Asp Leu Ala Leu Ile Ser Phe Glu Val Tyr Asp Tyr Glu
   65                  70                  75                  80
   Val Ser Thr Pro Asp Tyr Phe Cys Gly Gln Thr Cys
                   85                  90
<210> 688
   <211> 92
   <212> PRT
   <213> Artificial sequence
<220>
   <223> Synthetically generated peptide
<400> 688
   Leu Lys Val Lys Ile Tyr Met Gly Asp Gly Trp Ile Val Asp Phe Lys
   1               5                   10                  15
   Lys Arg Ile Gly Arg Leu Ser Lys Pro Asp Leu Tyr Val Arg Ile Ser
               20                  25                  30
   Ile Ala Gly Val Pro His Asp Glu Lys Ile Met Asn Thr Thr Val Lys
           35                  40                  45
   Asn Asn Glu Trp Lys Pro Thr Trp Gly Glu Glu Phe Thr Phe Pro Leu
       50                  55                  60
   Thr Tyr Pro Asp Leu Ala Leu Ile Ser Phe Glu Val Tyr Asp Tyr Glu
   65                  70                  75                  80
   Val Ser Thr Pro Asp Tyr Phe Cys Gly Gln Thr Cys
                   85                  90
<210> 689
   <211> 92
   <212> PRT
   <213> Artificial Sequence
<220>
   <223> Synthetically generated peptide
<400> 689
   Leu Lys Val Lys Ile Tyr Met Gly Asp Gly Trp Ile Val Asp Phe Lys
   1               5                   10                  15
   Lys Arg Ile Gly Arg Leu Ser Lys Pro Asp Leu Tyr Val Arg Ile Ser
               20                  25                  30
   Ile Ala Gly Val Pro His Asp Glu Asn Ile Met Lys Thr Thr Val Lys
           35                  40                  45
   Asn Asn Glu Trp Thr Pro Thr Trp Gly Glu Glu Phe Thr Phe Pro Leu
       50                  55                  60
   Thr Tyr Pro Asp Leu Ala Leu Ile Ser Phe Glu Val Tyr Asp Tyr Glu
   65                  70                  75                  80
   Val Ser Thr Ala Asp Ala Phe Cys Gly Gln Thr Cys
                   85                  90
<210> 690
   <211> 92
   <212> PRT
   <213> Artificial Sequence
<220>
   <223> Synthetically generated peptide
<400> 690
   Leu Lys Val Lys Ile Tyr Met Gly Asp Gly Trp Ile Val Asp Phe Lys
   1               5                   10                  15
   Lys Arg Ile Gly Arg Leu Ser Lys Pro Asp Leu Tyr Val Arg Ile Ser
               20                  25                  30
   Ile Ala Gly Val Pro His Asp Glu Asn Ile Met Lys Thr Thr Val Lys
           35                  40                  45
   Asn Asn Glu Trp Thr Pro Thr Trp Gly Glu Glu Phe Thr Phe Pro Leu
       50                  55                  60
   Thr Tyr Pro Asp Leu Ala Leu Ile Ser Phe Glu Val Tyr Asp Tyr Glu
   65                  70                  75                  80
   Val Ser Thr Ala Asp Ala Phe Cys Gly Gln Thr Cys
                   85                  90
<210> 691
   <211> 89
   <212> PRT
   <213> Artificial Sequence
<220>
   <223> synthetically generated peptide
<400> 691
   Leu Lys Val Thr Val Leu Leu Gly Thr Asp Trp His Lys Asn Tyr Asp
   1               5                   10                  15
   Val Phe Lys Lys Pro Gly Tyr Phe Val Lys Val Ala Ile His Gly Met
               20                  25                  30
   His Asp Asp Glu Gln Lys Phe Lys Thr His Val Cys Lys Arg Ser Arg
           35                  40                  45
   Glu Pro His Trp Glu Val Glu Glu Phe Val Phe Gln Ile Arg Val Pro
       50                  55                  60
   Lys Leu Ala Ile Leu Arg Leu Glu Val Arg Glu Tyr Asp Arg Ile Val
   65                  70                  75                  80
   Arg Asp Asp Met Val Gly Gln Ser Cys
                   85
<210> 692
   <211> 68
   <212> PRT
   <213> Artificial sequence
<220>
   <223> synthetically generated peptide
<400> 692
   Lys Gln Ser Val Gly Asn Pro Ser Val Phe Cys Lys Ile Thr Leu Gly
   1               5                   10                  15
   Asn Asn Pro Pro Arg Gln Thr Lys Val Ile Ser Thr Gly Pro Asn Pro
               20                  25                  30
   Glu Trp Asp Glu Ser Phe Ser Trp Ser Phe Glu Ser Pro Pro Lys Gly
           35                  40                  45
   Gln Lys Leu His Cys Lys Asn Lys Ser Lys Met Gly Lys Ser Ser Phe
       50                  55                  60
   Gly Lys Val Thr
   65
<210> 693
   <211> 79
   <212> PRT
   <213> Artificial Sequence
<220>
   <223> Synthetically generated peptide
<400> 693
   Leu Val Val Thr Ile Lys Arg Gly Asn Asn Met Lys Gln Ser Val Gly
   1               5                   10                  15
   Asn Pro Ser Val Phe Cys Lys Ile Thr Leu Gly Asn Asn Pro Pro Arg
               20                  25                  30
   Gln Thr Lys Val Ile Ser Thr Gly Pro Asn Pro Glu Trp Asp Glu Ser
           35                  40                  45
   Phe Ser Trp Ser Phe Glu Ser Pro Pro Lys Gly Gln Lys Leu His Cys
       50                  55                  60
   Lys Asn Lys Ser Lys Met Gly Lys Ser Ser Phe Gly Lys Val Thr
   65                  70                  75
<210> 694
   <211> 79
   <212> PRT
   <213> Artificial sequence
<220>
   <223> Synthetically generated peptide
<400> 694
   Leu Val Val Ile Ile Lys Arg Gly Asn Asn Met Lys Gln Ser Val Gly
    1               5                  10                  15
   Asn Pro Ser Val Tyr Cys Lys Ile Thr Leu Gly Asn Asn Pro Pro Arg
               20                  25                  30
   Leu Thr Lys Val Val Ser Thr Gly Pro Asn Pro Glu Trp Asp Glu Ser
           35                  40                  45
   Phe Ser Trp Ser Phe Glu Ser Pro Pro Lys Gly Gln Lys Leu His Cys
       50                  55                  60
   Lys Asn Lys Ser Lys Val Gly Lys Ser Lys Phe Gly Lys Val Thr
   65                  70                  75
<210> 695
   <211> 79
   <212> PRT
   <213> Artificial Sequence
<220>
   <223> Synthetically generated peptide
<400> 695
   Leu Val Val Ile Val Lys Arg Gly Asn Asn Met Arg Gln Ser Val Gly
    1               5                  10                  15
   Ile Pro Ser Val Tyr Cys Lys Ile Thr Leu Gly Asn Ser Pro Pro Lys
               20                  25                  30
   Leu Thr Lys Val Val Ser Thr Gly Pro Asn Pro Glu Trp Glu Glu Ser
           35                  40                  45
   Phe Thr Trp Ser Phe Glu Ser Pro Pro Lys Gly Gln Lys Leu His Cys
       50                  55                  60
   Lys Asn Lys Ser Lys Val Gly Lys Ser Lys Phe Gly Lys Val Thr
   65                  70                  75
<210> 696
   <211> 79
   <212> PRT
   <213> Artificial sequence
<220>
   <223> Synthetically generated peptide
<400> 696
   Leu Thr Val Thr Ile Lys Arg Gly Asn Asn Leu Arg Gln Ser Val Gly
    1               5                  10                  15
   Asn Pro Ser Ala Phe Cys Lys Leu Thr Leu Gly Asn Asn Pro Pro Arg
               20                  25                  30
   Leu Thr Lys Ile Val Ser Thr Gly Ala Thr Pro Glu Trp Asp Glu Ala
           35                  40                  45
   Phe Ala Trp Ala Phe Asp Ser Pro Pro Lys Gly Gln Lys Leu His Cys
       50                  55                  60
   Lys Asn Asn Ser Lys Phe Gly Lys Lys Ser Phe Gly Lys Val Thr
   65                  70                  75
<210> 697
   <211> 81
   <212> PRT
   <213> Artificial Sequence
<220>
   <223> Synthetically generated peptide
<400> 697
   Leu Thr Val Asn Val Met Arg Ala Asn Asn Leu Lys Gln Ser Met Ala
   1               5                   10                  15
   Thr Thr Asn Ala Phe Cys Gln Leu Thr Ile Gly Asn Cys Pro Pro Arg
               20                  25                  30
   Gln Thr Lys Val Val Ser Asn Ser Thr Thr Pro Glu Trp Lys Glu Gly
           35                  40                  45
   Phe Thr Trp Ala Phe Asp Val Pro Pro Lys Gly Gln Lys Leu His Ile
       50                  55                  60
   Ile Cys Lys Ser Lys Ser Thr Phe Gly Lys Thr Thr Leu Gly Arg Val
   65                  70                  75                  80
   Thr
<210> 698
   <211> 81
   <212> PRT
   <213> Artificial Sequence
<220>
   <223> Synthetically generated peptide
<400> 698
   Leu Thr Val Asn Val Met Arg Ala Asn Asn Leu Lys Gln Ser Met Ala
   1               5                   10                  15
   Thr Thr Asn Ala Phe Cys Gln Leu Thr Ile Gly Asn Cys Pro Pro Arg
               20                  25                  30
   Gln Thr Lys Val Val Ser Asn Ser Thr Thr Pro Glu Trp Lys Glu Gly
           35                  40                  45
   Phe Thr Trp Ala Phe Asp Val Pro Pro Lys Gly Gln Lys Leu His Ile
       50                  55                  60
   Ile Cys Lys Ser Lys Ser Thr Phe Gly Lys Thr Thr Leu Gly Arg Val
   65                  70                  75                  80
   Thr
<210> 699
   <211> 81
   <212> PRT
   <213> Artificial Sequence
<220>
   <223> synthetically generated peptide
<400> 699
   Leu Thr Val Thr Ile Leu Arg Gly Asn Asn Leu Lys Gln Thr Met Gly
   1               5                   10                  15
   Ser Thr Asn Ala Phe Cys Cys Leu Gln Ile Gly Asn Gly Pro Pro Arg
               20                  25                  30
   Gln Thr Lys Val Val Asn Asn Ser Ile Cys Pro Val Trp Asn Glu Gly
           35                  40                  45
   Phe Thr Trp Leu Phe Asp Ile Pro Pro Lys Gly Gln Lys Leu Tyr Ile
       50                  55                  60
   Leu Cys Lys Ser Lys Asn Thr Phe Gly Lys Ser Thr Leu Gly Arg Val
   65                  70                  75                  80
   Thr
<210> 700
   <211> 77
   <212> PRT
   <213> Artificial Sequence
<220>
   <223> synthetically generated peptide
<400> 700
   Leu Thr Val Ala Ile Lys Arg Gly Asp Asn Leu Lys Arg Ser Asn Ala
   1               5                   10                  15
   Phe Cys Arg Leu Ile Ile Asp Asn Cys Pro Thr Lys Lys Thr Lys Val
               20                  25                  30
   Val Lys Arg Ser Ser Ser Pro Val Trp Lys Glu Ser Phe Thr Trp Asp
           35                  40                  45
   Phe Ala Ala Pro Pro Arg Gly Gln Phe Leu Glu Ile Val Cys Lys Ser
       50                  55                  60
   Asn Asn Ile Phe Arg Asn Lys Asn Leu Gly Lys Val Arg
   65                  70                  75
<210> 701
   <211> 88
   <212> PRT
   <213> Artificial Sequence
<220>
   <223> Synthetically generated peptide
<400> 701
   Leu Glu Val Tyr Val His Gln Ala Arg Asp Ile His Asn Ile Cys Ile
   1               5                   10                  15
   Tyr His Lys Gln Asp Val Tyr Ala Lys Leu Cys Leu Thr Ser Asp Pro
               20                  25                  30
   Asp Lys Ser Val Ser Thr Lys Ile Ile Asn Gly Gly Gly Arg Asn Pro
           35                  40                  45
   Val Phe Asp Asp Asn Val Lys Leu Asp Val Arg Val Leu Asp Thr Ser
       50                  55                  60
   Leu Lys Cys Glu Ile Tyr Met Met Ser Arg Val Lys Asn Tyr Leu Glu
   65                  70                  75                  80
   Asp Gln Leu Leu Gly Phe Thr Leu
                   85
<210> 702
   <211> 88
   <212> PRT
   <213> Artificial Sequence
<220>
   <223> Synthetically generated peptide
<400> 702
   Leu Glu Val Tyr Val His Gln Ala Arg Asp Ile His Asn Ile Cys Ile
    1               5                  10                  15
   Tyr His Lys Gln Asp Val Tyr Ala Lys Leu Cys Leu Thr Ser Asp Pro
               20                  25                  30
   Asp Lys Ser Val Ser Thr Lys Ile Ile Asn Gly Gly Gly Arg Asn Pro
           35                  40                  45
   Val Phe Asp Asp Asn Val Lys Leu Asp Val Arg Val Leu Asp Thr Ser
       50                  55                  60
   Leu Lys Cys Glu Ile Tyr Met Met Ser Arg Val Lys Asn Tyr Leu Glu
   65                  70                  75                  80
   Asp Gln Leu Leu Gly Phe Thr Leu
                   85
<210> 703
   <211> 88
   <212> PRT
   <213> Artificial sequence
<220>
   <223> Synthetically generated peptide
<400> 703
   Leu Glu Val Tyr Val His Gln Ala Arg Asp Ile His Asn Ile Cys Ile
    1               5                  10                  15
   Tyr His Lys Gln Asp Val Tyr Ala Lys Leu Cys Leu Thr Ser Asp Pro
               20                  25                  30
   Asp Lys Ser Val Ser Thr Lys Ile Ile Asn Gly Gly Gly Arg Asn Pro
           35                  40                  45
   Val Phe Asp Asp Asn Val Lys Leu Asp Val Arg Val Leu Asp Thr Ser
       50                  55                  60
   Leu Lys Cys Glu Ile Tyr Met Met Ser Arg Val Lys Asn Tyr Leu Glu
   65                  70                  75                  80
   Asp Gln Leu Leu Gly Phe Thr Leu
                   85
<210> 704
   <211> 88
   <212> PRT
   <213> Artificial Sequence
<220>
   <223> Synthetically generated peptide
<400> 704
   Leu Glu Val Phe Val His Gln Ala Arg Asp Ile His Asn Ile Cys Ile
   1               5                   10                  15
   Tyr His Lys Gln Asp Val Tyr Ala Lys Leu Cys Leu Thr Asn Asp Pro
               20                  25                  30
   Glu Asn Ser Leu Ser Thr Lys Ile Ile Asn Gly Gly Gly Gln Asn Pro
           35                  40                  45
   Val Phe Asp Asp Thr Leu Gln Phe Asp Val Lys Asn Leu Asp Cys Ser
       50                  55                  60
   Leu Lys Cys Glu Ile Phe Met Met Ser Arg Val Lys Asn Tyr Leu Glu
   65                  70                  75                  80
   Asp Gln Leu Leu Gly Phe Ser Leu
                   85
<210> 705
   <211> 88
   <212> PRT
   <213> Artificial Sequence
<220>
   <223> Synthetically generated peptide
<400> 705
   Leu Glu Val Phe Val His Gln Ala Arg Asp Ile His Asn Ile Cys Ile
   1               5                   10                  15
   Tyr His Lys Gln Asp Val Tyr Ala Lys Leu Cys Leu Thr Asn Asp Pro
               20                  25                  30
   Glu Asn Ser Leu Ser Thr Lys Ile Ile Asn Gly Gly Gly Gln Asn Pro
           35                  40                  45
   Val Phe Asp Asp Thr Leu Gln Phe Asp Val Lys Asn Leu Asp Cys Ser
       50                  55                  60
   Leu Lys Cys Glu Ile Phe Met Met Ser Arg Val Lys Asn Tyr Leu Glu
   65                  70                  75                  80
   Asp Gln Leu Leu Gly Phe Ser Leu
                   85
<210> 706
   <211> 88
   <212> PRT
   <213> Artificial Sequence
<220>
   <223> synthetically generated peptide
<400> 706
   Leu Glu Val Phe Val His Gln Ala Arg Asp Ile His Asn Ile Cys Ile
   1               5                   10                  15
   Tyr His Lys Gln Asp Val Tyr Ala Lys Leu Cys Leu Thr Asn Asp Pro
               20                  25                  30
   Glu Asn Ser Leu Ser Thr Lys Ile Ile Asn Gly Gly Gly Gln Asn Pro
           35                  40                  45
   Val Phe Asp Asp Thr Leu Gln Phe Asp Val Lys Asn Leu Asp Cys Ser
       50                  55                  60
   Leu Lys Cys Glu Ile Phe Met Met Ser Arg Val Lys Asn Tyr Leu Glu
   65                  70                  75                  80
   Asp Gln Leu Leu Gly Phe Ser Leu
                   85
<210> 707
   <211> 88
   <212> PRT
   <213> Artificial Sequence
<220>
   <223> Synthetically generated peptide
<400> 707
   Leu Asp Val Phe Val His Gln Ala Arg Asp Ile His Asn Ile Cys Ile
   1               5                   10                  15
   Tyr His Lys Gln Asp Val Tyr Ala Lys Leu Cys Leu Thr Ser Asp Pro
               20                  25                  30
   Asp Val Ser Cys Ser Thr Lys Val Ile Asn Gly Gly Gly Arg Asn Pro
           35                  40                  45
   Val Phe Asp Asp Gly Leu Arg Leu Asp Val Arg Thr Val Asp Ala Ser
       50                  55                  60
   Leu Lys Cys Glu Ile Trp Met Leu Ser Arg Val Arg Asn Tyr Leu Glu
   65                  70                  75                  80
   Asp Gln Leu Leu Gly Phe Ala Leu
                   85
<210> 708
   <211> 88
   <212> PRT
   <213> Artificial Sequence
<220>
   <223> synthetically generated peptide
<400> 708
   Val Asp Val Tyr Ile His Gln Ala Arg Asp Ile His Lys Ile Cys Ile
   1               5                   10                  15
   Tyr His Lys Gln Asp Val Tyr Ala Lys Ile Ser Leu Thr Ser Asp Pro
               20                  25                  30
   Glu Asn Ser Val Asn Thr Lys Ile Ile Asn Gly Gly Gly Arg Asn Pro
           35                  40                  45
   Val Phe Asn Asp Asn Leu Arg Leu Ser Val Arg Thr Val Asp Ser Ser
       50                  55                  60
   Leu Lys Cys Glu Ile Trp Met Leu Ser Arg Val Lys Asn Tyr Leu Glu
   65                  70                  75                  80
   Asp Gln Leu Leu Gly Phe Ala Leu
                   85
<210> 709
   <211> 90
   <212> PRT
   <213> Artificial sequence
<220>
   <223> Synthetically generated peptide
<400> 709
   Val Asp Val His Val Gln Ser Ala Arg Asp Ile Gln Asn Ile Cys Ile
   1 5 10 15
   Tyr His Lys Gln Asp Val Tyr Ala Arg Leu Ser Leu Pro Gly Glu Gly
   20 25 30
   Ala Pro Ala Ala Ser Thr Gln Val Ile Asn Gly Gly Gly Arg Asn Pro
   35 40 45
   Val Phe Asp Gln Ser Leu Arg Leu Gly Val Arg Ala Gly Asp Val Asp
   50 55 60
   Gly Ala Leu Arg Cys Glu Val Trp Met Leu Ser Arg Val Lys Asn Tyr
   65 70 75 80
   Leu Gln Asp Gln Leu Leu Gly Phe Ala Leu
   85 90
<210> 710
   <211> 91
   <212> PRT
   <213> Artificial Sequence
<220>
   <223> Synthetically generated peptide
<400> 710
   Leu Gln Val Tyr Val His Asn Ala Arg Asn Ile Asn Asn Ile Cys Ile
   1 5 10 15
   Tyr Asp Asn Gln Asp Val Tyr Ala Lys Phe Ser Leu Thr Tyr Asn Pro
   20 25 30
   Asp Asp Thr Ile Ser Thr Arg Ile Ile His Arg Ala Gly Lys Asn Pro
   35 40 45
   Glu Phe Asn Gln Lys Leu Met Ile Asp Val Thr Gln Ile Asp Ala His
   50 55 60
   Ala Ala Val Leu Lys Cys Glu Ile Trp Met Met Ser Arg Ala Arg His
   65 70 75 80
   Tyr Met Glu Asp Gln Leu Leu Gly Phe Ala Leu
   85 90
<210> 711
   <211> 92
   <212> PRT
   <213> Artificial Sequence
<220>
   <223> Synthetically generated peptide
<400> 711
   Leu Asp Ile Tyr Val His Gly Arg Ile His Asn Ile Cys Ile Tyr Ala
   1 5 10 15
   Ala Gln Asp Val Tyr Ala Arg Leu Ala Leu Thr Ser Ser Pro Asp Asp
   20 25 30
   Ala Pro Ala Leu Asp Thr Arg Val Ala Ala Gly Gly Gly Ala Asn Pro
   35 40 45
   Arg Phe Asp Glu Arg Leu Pro Pro Leu Arg Val Arg Arg Ala Arg Leu
   50 55 60
   Gly Thr Asp Val Leu Lys Cys Glu Ile Trp Met Arg Ser Cys Ala Arg
   65 70 75 80
   Arg Leu Leu Asp Asp Gln Leu Leu Gly Phe Ala Leu
   85 90
<210> 712
   <211> 85
   <212> PRT
   <213> Artificial Sequence
<220>
   <223> Synthetically generated peptide
<400> 712
   Leu Glu Ile Glu Val Ile Ser Ala Glu Gly Leu Lys Val Asp Arg Lys
   1 5 10 15
   Pro Leu Lys Lys Lys Thr Tyr Ser Val Val Arg Ile Asp Glu Lys Ser
   20 25 30
   Trp Ala Ser Lys Val Asp Glu Leu Gly Gly Ser Tyr Pro Ile Trp Lys
   35 40 45
   Asp Arg Phe Asp Met Glu Met Pro Ile Asn Ala Ser Val Arg Phe Ile
   50 55 60
   Ser Ile Glu Val Tyr Tyr Arg Thr Ser Gly Ser Gly Arg Asp Lys Asn
   65 70 75 80
   Val Gly Tyr Ala Lys
   85
<210> 713
   <211> 85
   <212> PRT
   <213> Artificial Sequence
<220>
   <223> synthetically generated peptide
<400> 713
   Leu Glu Ile Glu Val Ile Ser Ala Glu Gly Leu Lys Val Asp Arg Lys
   1 5 10 15
   Pro Leu Lys Lys Lys Thr Tyr Ser Val Val Arg Ile Asp Glu Lys Ser
   20 25 30
   Trp Ala Ser Lys Val Asp Glu Leu Gly Gly Ser Tyr Pro Ile Trp Lys
   35 40 45
   Asp Arg Phe Asp Met Glu Met Pro Ile Asn Ala Ser Val Arg Phe Ile
   50 55 60
   Ser Ile Glu Val Tyr Tyr Arg Thr Ser Gly Ser Gly Arg Asp Lys Asn
   65 70 75 80
   Val Gly Tyr Ala Lys
   85
<210> 714
   <211> 85
   <212> PRT
   <213> Artificial Sequence
<220>
   <223> Synthetically generated peptide
<220>
   <221> VARIANT
   <222> 26
   <223> Xaa = any amino acid
<400> 714
   Leu Glu Ile Glu Val Ile Ser Ala Glu Gly Leu Lys Val Asp Arg Lys
   1 5 10 15
   Pro Leu Lys Lys Lys Thr Tyr Ser Val Xaa Arg Ile Asp Glu Lys Ser
   20 25 30
   Trp Ala Ser Lys Val Asp Glu Leu Gly Gly Ser Tyr Pro Ile Trp Lys
   35 40 45
   Asp Arg Phe Asp Met Glu Met Pro Ile Asn Ala Ser Val Arg Phe Ile
   50 55 60
   Ser Ile Glu Val Tyr Tyr Arg Thr Ser Gly Ser Gly Arg Asp Lys Asn
   65 70 75 80
   Val Gly Tyr Ala Lys
   85
<210> 715
   <211> 82
   <212> PRT
   <213> Artificial Sequence
<220>
   <223> Synthetically generated peptide
<400> 715
   Leu Glu Ile Asp Leu Arg Ser Ala Glu Gly Leu Lys Leu Asn Arg Arg
   1 5 10 15
   Pro Ile Lys Lys Lys Thr Phe Ala Val Val Lys Ile Asp Glu Lys Cys
   20 25 30
   Arg Lys Ser Asn Leu Asp Glu Ser Arg Arg Ser Asn Pro Thr Trp Asn
   35 40 45
   Tyr Lys Ser Glu Met Pro Ile Asn Gly Asn Glu Gln Phe Ile Phe Ile
   50 55 60
   Glu Val Phe Tyr Arg Thr Gly Ser Gly His Asp Lys Lys Ile Gly Glu
   65 70 75 80
   Ala Lys
<210> 716
   <211> 82
   <212> PRT
   <213> Artificial Sequence
<220>
   <223> Synthetically generated peptide
<400> 716
   Leu Glu Ile Asp Leu Arg Ser Ala Glu Gly Leu Lys Leu Asn Arg Arg
   1 5 10 15
   Pro Ile Lys Lys Lys Thr Phe Ala Val Val Lys Ile Asp Glu Lys Cys
   20 25 30
   Arg Lys Ser Asn Leu Asp Glu Ser Arg Arg Ser Asn Pro Thr Trp Asn
   35 40 45
   Tyr Lys Ser Glu Met Pro Ile Asn Gly Asn Glu Gln Phe Ile Phe Ile
   50 55 60
   Glu Val Phe Tyr Arg Thr Gly Ser Gly His Asp Lys Lys Ile Gly Glu
   65 70 75 80
   Ala Lys
<210> 717
   <211> 82
   <212> PRT
   <213> Artificial Sequence
<220>
   <223> Synthetically generated peptide
<400> 717
   Leu Glu Ile Asp Leu Arg Ser Ala Glu Gly Leu Lys Leu Asn Arg Arg
   1 5 10 15
   Pro Ile Lys Lys Lys Thr Phe Ala Val Val Lys Ile Asp Glu Lys Cys
   20 25 30
   Arg Lys Ser Asn Leu Asp Glu Ser Arg Arg Ser Asn Pro Thr Trp Asn
   35 40 45
   Tyr Lys Ser Glu Met Pro Ile Asn Gly Asn Glu Gln Phe Ile Phe Ile
   50 55 60
   Glu Val Phe Tyr Arg Thr Gly Ser Gly His Asp Lys Lys Ile Gly Glu
   65 70 75 80
   Ala Lys
<210> 718
   <211> 91
   <212> PRT
   <213> Artificial Sequence
<220>
   <223> Synthetically generated peptide
<400> 718
   Leu Glu Val Thr Leu Ile Ser Ala Gln Gly Leu Lys Pro Pro Ser Gly
    1               5                  10                  15
   Leu Arg Arg Arg Leu Leu Gln Ala Tyr Ala Val Ala Trp Val Asp Ala
               20                  25                  30
   Ala Arg Arg Leu Gln Trp Asp Arg Ala Gly Gly Val Asp Pro Glu Trp
           35                  40                  45
   His Glu Arg Leu Leu Phe Arg Val His Glu Ala Ala Asp Asp Ser Arg
       50                  55                  60
   Ala Ala Val Thr Val Glu Ile Tyr Ala Ala Pro Ala Gly Gly Trp His
   65                  70                  75                  80
   Ile Gly Gly Asp Ser Leu Val Gly Ser Ala Arg
                   85                  90
<210> 719
   <211> 88
   <212> PRT
   <213> Artificial Sequence
<220>
   <223> synthetically generated peptide
<400> 719
   Leu Glu Val Thr Val Ile Ser Ala Gln Asp Leu His Arg Arg Leu Gly
    1               5                  10                  15
   Arg Arg Val Arg Ala Ala Tyr Ala Val Ala Trp Ala Asp Ala Ala His
               20                  25                  30
   Lys Leu Arg Thr Gly Val Asp Leu Ala Gly Gly Ala Asp Pro Thr Trp
           35                  40                  45
   Asn Asp Arg Phe Leu Phe Arg Val Glu Glu Ala Phe Leu Arg Ser Asp
       50                  55                  60
   Thr Ala Ala Val Thr Val Glu Val Arg Ala Pro Arg Arg Phe Gly Gly
   65                  70                  75                  80
   Asp Ala Val Leu Gly Val Thr Arg
                   85
<210> 720
   <211> 83
   <212> PRT
   <213> Artificial Sequence
<220>
   <223> Synthetically generated peptide
<400> 720
   Leu Glu Leu Asn Ile Ile Ser Ala Gln Asp Leu Ala Pro Val Ser Arg
    1               5                  10                  15
   Lys Met Lys Thr Tyr Ala Val Ala Trp Val His Ser Lys Leu Thr Thr
               20                  25                  30
   Arg Val Asp Tyr Thr Gly Gly Gly Asn Pro Thr Trp Asn Asp Lys Phe
           35                  40                  45
   Val Phe Arg Val Ser Glu Asp Phe Leu Tyr Ala Asp Thr Ser Ala Val
       50                  55                  60
   Val Val Glu Ile Tyr Ala Leu His Trp Phe Arg Asp Val His Val Gly
   65 70 75 80
   Thr Val Arg
<210> 721
   <211> 83
   <212> PRT
   <213> Artificial Sequence
<220>
   <223> Synthetically generated peptide
<400> 721
   Leu Glu Leu Asn Ile Ile Ser Ala Gln Asp Leu Ala Pro Val Ala Arg
   1 5 10 15
   Lys Thr Lys Thr Tyr Ala Val Ala Trp Val His Ser Lys Leu Thr Thr
   20 25 30
   Arg Val Asp Tyr Asn Gly Gly Thr Asn Pro Thr Trp Asn Asp Lys Phe
   35 40 45
   Val Phe Arg Val Asn Glu Glu Phe Leu Tyr Ala Asp Thr Ser Ala Val
   50 55 60
   Val Ile Glu Ile Tyr Ala Leu His Trp Phe Arg Asp Val His Val Gly
   65 70 75 80
   Thr Val Arg
<210> 722
   <211> 85
   <212> PRT
   <213> Artificial Sequence
<220>
   <223> Synthetically generated peptide
<400> 722
   Leu Glu Leu Asn Ile Ile Ser Ala Gln Glu Leu Ala Pro Val Ala Arg
   1 5 10 15
   Cys Met Lys Thr Tyr Ala Ile Ala Trp Ile Asp Pro Glu Arg Lys Leu
   20 25 30
   Thr Thr Arg Val Asp Asn Thr Gly Gly Thr Ser Pro Thr Trp Asn Asp
   35 40 45
   Lys Phe Val Phe Arg Leu Asp Glu Glu Ala Leu Tyr Asp Ala Thr Ser
   50 55 60
   Ile Val Val Ile Glu Ile Tyr Ala Leu His Trp Phe Lys Asp Ile His
   65 70 75 80
   Val Gly Thr Val Gln
   85
<210> 723
   <211> 85
   <212> PRT
   <213> Artificial Sequence
<220>
   <223> Synthetically generated peptide
<400> 723
   Leu Glu Leu Asn Val Ile Ser Ala Gln Asp Leu Ala Glu Val Gly Arg
   1 5 10 15
   Ser Met Arg Thr Tyr Ala Val Ala Trp Val Asp Pro Asp Arg Lys Leu
   20 25 30
   Ser Thr Arg Val Asp Ser Gln Ser Gly Thr Asn Pro Ala Trp Asn Asp
   35 40 45
   Lys Phe Val Phe Arg Val Asp Glu Asp Phe Leu Tyr Asp Glu Asn Ser
   50 55 60
   Thr Ile Thr Ile Asp Ile Tyr Ala Ile His Trp Phe Lys Asp Ile His
   65 70 75 80
   Val Gly Thr Ala His
   85
<210> 724
   <211> 87
   <212> PRT
   <213> Artificial Sequence
<220>
   <223> Synthetically generated peptide
<400> 724
   Leu Glu Ile Asn Leu Ile Ser Ala Gln Asp Leu Ala Pro Val Ser Arg
   1 5 10 15
   Asn Met Lys Thr Tyr Ser Val Ala Trp Ile Asn Thr Asp Pro Met Arg
   20 25 30
   Lys Leu Thr Thr Arg Val Asp Gln Ser Asn Arg Ala Asn Pro Ile Trp
   35 40 45
   Asn Glu Lys Phe Val Phe Arg Val Asn Asp Lys Ile Leu Tyr Val Asp
   50 55 60
   Ala Ser Ala Ile Val Ile Glu Ile Tyr Ala Ala Ala Trp Ala Lys Asp
   65 70 75 80
   Ala Leu Val Gly Thr Val Asn
   85
<210> 725
   <211> 87
   <212> PRT
   <213> Artificial Sequence
<220>
   <223> Synthetically generated peptide
<400> 725
   Leu Glu Ile Asn Leu Ile Ser Ala Gln Asp Leu Ala Pro Val Ser Arg
   1 5 10 15
   Asn Met Lys Thr Tyr Ser Val Ala Trp Ile Asn Thr Asp Pro Met Arg
   20 25 30
   Lys Leu Thr Thr Arg Val Asp Gln Ser Asn Arg Ala Asn Pro Ile Trp
   35 40 45
   Asn Glu Lys Phe Val Phe Arg Val Asn Asp Lys Ile Leu Asp Val Asp
   50 55 60
   Ala Ser Ala Ile Val Ile Glu Ile Tyr Ala Ala Ala Trp Ala Lys Asp
   65 70 75 80
   Ala Leu Val Gly Thr Val Asn
   85
<210> 726
   <211> 88
   <212> PRT
   <213> Artificial Sequence
<220>
   <223> Synthetically generated peptide
<400> 726
   Leu Glu Ile Asn Leu Ile Ser Ala Gln Gly Leu Lys Glu Pro Thr Gly
   1 5 10 15
   Lys Leu Arg Arg Leu Gln Thr Tyr Ala Ser Val Trp Val Asp Ser Ser
   20 25 30
   Ser Lys Leu Arg Thr Arg Ile Asp Arg Ile Gly Ser Glu Asn Pro Ile
   35 40 45
   Trp Asn Asp Lys Phe Val Phe Gln Val Ser Pro Glu Phe Leu Ser Ser
   50 55 60
   Glu Thr Ser Gly Val Ser Ile Glu Ile Tyr Ala Val Gly Tyr Leu Arg
   65 70 75 80
   Asp His Leu Ile Gly Thr Val Arg
   85

<210> 727
   <211> 89
   <212> PRT
   <213> Artificial Sequence
<220>
   <223> Synthetically generated peptide
<400> 727
   Leu Glu Val Thr Val Val Ser Gly Lys His Leu Lys Asn Val Asn Trp
    1               5                  10                  15
   Arg Arg Gly Asp Leu Arg Ala Tyr Val Val Ala Tyr Leu Asp Pro Ser
               20                  25                  30
   Arg Arg Ala Ala Trp Asp Asp Val Gly Gly Cys Lys Pro Ala Trp Asn
           35                  40                  45
   Glu Arg Val Val Leu Pro Leu Pro Pro His Leu Ser Pro His Asp Pro
       50                  55                  60
   Ser Leu Leu Leu Ser Leu Asp Val Phe His Ser Lys Pro Ser Asp Ser
   65                  70                  75                  80
   Pro Lys Pro Leu Val Gly Ser Ala Arg
                   85
<210> 728
   <211> 87
   <212> PRT
   <213> Artificial Sequence
<220>
   <223> Synthetically generated peptide
<400> 728
   Leu Val Val Thr Val Val Ser Ala Lys His Leu Lys Asn Val Asn Trp
    1               5                  10                  15
   Arg Asn Gly Asp Leu Lys Pro Tyr Val Val Leu Tyr Leu Asp Gln Asp
               20                  25                  30
   His Pro Leu Ser Thr Arg Ser Asp Asp Ser Ser Ser Ile Lys Pro Val
           35                  40                  45
   Trp Asn Glu Arg Ile Thr Leu Pro Leu Thr Arg Ser Val His Glu Ser
       50                  55                  60
   Val Leu Asn Ile Glu Val Phe His Ser Asn Ser Ser Asp Leu Ala Lys
   65                  70                  75                  80
   Thr Leu Val Gly Ser Val Arg
                   85
<210> 729
   <211> 89
   <212> PRT
   <213> Artificial sequence
<220>
   <223> Synthetically generated peptide
<400> 729
   Val Glu Val Thr Val Ser Ser Ala Arg Asp Leu Lys Asn Val Asn Trp
    1               5                  10                  15
   Arg Asn Gly Asp Leu Lys Pro Tyr Ala Val Leu Trp Val Asp Asp Gly
               20                  25                  30
   Ala Lys Cys Ser Thr Arg Val Asp Leu Asp Asn Ala Asp Asn Pro Asn
           35                  40                  45
   Trp Asp Asp Lys Leu Thr Leu Pro Leu Pro Pro Ser Ser Arg Leu Asp
       50                  55                  60
   Asp Ala Leu Leu Tyr Leu Asp Val Val His Ala Asn Ala Ala Glu Gly
   65                  70                  75                  80
   Val Lys Pro Leu Val Gly Ser Ala Arg
                   85
<210> 730
   <211> 89
   <212> PRT
   <213> Artificial sequence
<220>
   <223> Synthetically generated peptide
<400> 730
   Val Glu Val Thr Val Ala Ser Ala Arg Asp Leu Lys Asn Val Asn Trp
    1               5                  10                  15
   Arg Asn Gly Asp Leu Lys Pro Tyr Ala Val Val Trp Ile Asp Asp Gly
               20                  25                  30
   Ala Lys Cys Ser Thr Arg Val Asp Leu Asp Asn Ala Asp Asn Pro Thr
           35                  40                  45
   Trp Asp Asp Lys Leu Thr Val Pro Leu Pro Pro Ser Thr Arg Leu Asp
       50                  55                  60
   Asp Ala Val Leu Tyr Leu Asp Val Val His Ala Asn Ala Thr Asp Gly
   65                  70                  75                  80
   Val Lys Pro Leu Val Gly Ser Ala Arg
                   85
<210> 731
   <211> 89
   <212> PRT
   <213> Artificial Sequence
<220>
   <223> synthetically generated peptide
<400> 731
   Val Glu Val Thr Val Gly Ala Ala Arg Asp Leu Lys Asn Val Asn Trp
    1               5                  10                  15
   Arg Asn Gly Asp Leu Lys Pro Tyr Ala Val Leu Trp Ile Asp Ala Gly
               20                  25                  30
   Ala Arg Cys Ser Thr Arg Val Asp Leu Asp Asn Gly Glu Asn Pro Thr
           35                  40                  45
   Trp Asp Asp Lys Val Val Val Pro Leu Pro Pro Ala Ser Arg Leu Gln
       50                  55                  60
   Asp Ala Val Leu Tyr Leu Asp Ile Val His Ala Asn Ala Pro Glu Gly
   65                  70                  75                  80
   Val Lys Pro Leu Val Gly Ser Ala Arg
                   85
<210> 732
   <211> 88
   <212> PRT
   <213> Artificial Sequence
<220>
   <223> Synthetically generated peptide
<400> 732
   Val Glu Val Thr Ile Ser Ser Ala Lys Asp Ile Lys Asn Val Asn Trp
   1 5 10 15
   Arg Asn Gly Pro Asn Lys Pro Tyr Ala Val Val Trp Ile Asp Pro Lys
   20 25 30
   Phe Lys Ser Ser Thr Arg Val Asp Glu Asp Gly Asn Thr Cys Thr Thr
   35 40 45
   Trp Asn Glu Thr Phe Val Ile Ala Leu Pro Pro Ala Asn Asp Asp Asp
   50 55 60
   Asp Lys Val Tyr Ile Asn Ile Val His Ala Gly Arg Glu Glu Asn Thr
   65 70 75 80
   Lys Pro Leu Ile Gly Ser Ala His
   85
<210> 733
   <211> 88
   <212> PRT
   <213> Artificial Sequence
<220>
   <223> Synthetically generated peptide
<400> 733
   Val Glu Val Thr Ile Ser Ser Ala Lys Asp Ile Lys Asn Val Asn Trp
   1 5 10 15
   Arg Asn Gly Pro Asn Lys Pro Tyr Ala Val Val Trp Ile Asp Pro Lys
   20 25 30
   Phe Lys Ser Ser Thr Arg Val Asp Glu Asp Gly Asn Thr Cys Thr Thr
   35 40 45
   Trp Asn Glu Thr Phe Val Ile Ala Leu Pro Pro Ala Asn Asp Asp Asp
   50 55 60
   Asp Lys Val Tyr Ile Asn Ile Val His Ala Gly Arg Glu Glu Asn Thr
   65 70 75 80
   Lys Pro Leu Ile Gly Ser Ala His
   85
<210> 734
   <211> 92
   <212> PRT
   <213> Artificial sequence
<220>
   <223> Synthetically generated peptide
<400> 734
   Leu Glu Leu Thr Leu Leu Ser Ala Ser Asp Leu Arg Gly Val Asn Leu
   1 5 10 15
   Val Ser Lys Met Glu Val Tyr Ala Val Val Tyr Leu Ala Gly Asp Pro
   20 25 30
   Arg Ala Arg Gln Arg Val Ala Thr Asp Arg Ala Gly Gly Arg Asn Pro
   35 40 45
   Ser Trp Lys Gly Lys Asp Ala Thr Val Arg Leu Ala Val Pro Ala Ser
   50 55 60
   Gly Ala Gly Ser Gly Ala Val Arg Val Leu Leu Arg Ala Glu Arg Ala
   65 70 75 80
   Gly Leu Gly Gly Asp Arg Asp Val Gly Glu Val Phe
   85 90
<210> 735
   <211> 86
   <212> PRT
   <213> Artificial Sequence
<220>
   <223> Synthetically generated peptide
<400> 735
   Leu Glu Leu Thr Leu Ile Ser Ala Lys Asp Leu Lys Asp Val Asn Leu
    1               5                  10                  15
   Leu Ser Lys Met Glu Val Tyr Ala Val Val Ser Leu Ser Gly Asp Arg
               20                  25                  30
   Arg Ser Arg Gln Arg Ile Ala Thr Asp Arg Ala Gly Gly Arg Asn Pro
           35                  40                  45
   Ala Trp Asn Ala Ala Pro Leu Arg Phe Thr Val Pro Ala Ser Gly Ala
       50                  55                  60
   Gly Ser Leu His Val Leu Leu Arg Ala Glu Arg Ala Leu Gly Asp Arg
   65                  70                  75                  80
   Asp Val Gly Glu Val His
                   85
<210> 736
   <211> 86
   <212> PRT
   <213> Artificial sequence
<220>
   <223> synthetically generated peptide
<400> 736
   Leu Glu Val Thr Leu Ile Ser Ala Arg Asn Leu Lys Lys Val Asn Leu
    1               5                  10                  15
   Ile Thr Pro Met Glu Val Tyr Ala Val Val Ser Val Ser Gly Asn Pro
               20                  25                  30
   Leu Ala Arg Gln Cys Thr Leu Pro Asp Arg His Gly Gly Arg Asn Pro
           35                  40                  45
   Thr Trp Asn Ala Thr Leu His Leu Ala Val Pro Ala Ala Ala Pro Gly
       50                  55                  60
   Ala Phe Leu His Val Leu Leu Arg Thr Glu Arg Ala Leu Gly Asp Arg
   65                  70                  75                  80
   Asp Val Gly Glu Val Phe
                   85
<210> 737
   <211> 88
   <212> PRT
   <213> Artificial Sequence
<220>
   <223> Synthetically generated peptide
<400> 737
   Leu Glu Val Thr Leu His Ser Ala Arg Asp Leu Lys Asn Val Asn Phe
   1 5 10 15
   Ile Ser Arg Met Glu Val Tyr Ala Val Ala Thr Ile Ser Gly Asp Pro
   20 25 30
   Leu Thr Arg Gln Cys Thr Pro Pro Asp Pro Tyr Gly Gly Arg His Pro
   35 40 45
   Ala Trp Asn Ala Thr Leu Arg Phe Thr Val Pro Pro Thr Ala Ala Ser
   50 55 60
   Ala Ala Gly Cys Leu His Val Leu Leu Arg Ala Glu Arg Ser Leu Gly
   65 70 75 80
   Asp Arg Asp Ile Gly Glu Val Ile
   85
<210> 738
   <211> 91
   <212> PRT
   <213> Artificial sequence
<220>
   <223> Synthetically generated peptide
<400> 738
   Leu Glu Leu Thr Leu Val Ser Ala Ser Asp Leu Lys Lys Val Thr Leu
   1 5 10 15
   Phe Ser Arg Met His Val Tyr Ala Val Ala Ser Ile Ser Gly Ser Asn
   20 25 30
   Val Pro Met Pro Met His Gly Thr His Ala Asp Arg Asn Gly Gly Ser
   35 40 45
   Asn Pro Ala Trp Asn Thr Val Leu His Phe Pro Val Pro Ala Arg Phe
   50 55 60
   Asp Thr Arg Gly Leu Ala Leu His Val Gln Leu Arg Ala Arg Arg Ser
   65 70 75 80
   Phe Gly Gly His Arg Asp Val Gly Asp Val Phe
   85 90
<210> 739
   <211> 91
   <212> PRT
   <213> Artificial Sequence
<220>
   <223> Synthetically generated peptide
<400> 739
   Leu Glu Leu Thr Leu Val Ser Ala Ser Asp Leu Lys Lys Val Thr Leu
   1               5                   10                  15
   Phe Ser Arg Met His Val Tyr Ala Val Ala Ser Ile Ser Gly Ser Asn
               20                  25                  30
   Val Pro Met Pro Met His Gly Thr His Ala Asp Arg Asn Gly Gly Ser
           35                  40                  45
   Asn Pro Ala Trp Asn Thr Val Leu His Phe Pro Val Pro Ala Arg Phe
       50                  55                  60
   Asp Thr Arg Gly Leu Ala Leu His Val Gln Leu Arg Ala Arg Arg Ser
   65                  70                  75                  80
   Phe Gly Gly His Arg Asp Val Gly Asp Val Phe
                   85                  90
<210> 740
   <211> 89
   <212> PRT
   <213> Artificial Sequence
<220>
   <223> Synthetically generated peptide
<400> 740
   Leu Glu Val Thr Leu Val Ser Ala Lys Asn Leu Lys Lys Val Thr Met
   1               5                   10                  15
   Phe Ser Lys Met Arg Val Tyr Ala Val Ala Ser Ile Ser Gly Gly Asp
               20                  25                  30
   Pro Arg Val Pro Thr Thr His Ala Asp Arg Glu Gly Gly Arg Ser Pro
           35                  40                  45
   Met Trp His Ala Pro Leu Arg Phe Pro Ile Pro Asp Ala Gly Ala Asp
       50                  55                  60
   Met Arg Ala Ile Ala Leu His Val Leu Leu Arg Ala Glu Arg Val Phe
   65                  70                  75                  80
   Gly Asp Ser Asp Val Gly Glu Val Phe
                   85
<210> 741
   <211> 92
   <212> PRT
   <213> Artificial Sequence
<220>
   <223> Synthetically generated peptide
<400> 741
   Leu Glu Leu Asn Val Tyr Ser Ala Lys Asp Leu Glu Asn Val Asn Leu
    1               5                   10                  15
   Ile Thr Lys Met Asp Val Tyr Ala Val Val Trp Ile Thr Gly Asp Asp
               20                  25                  30
   Ser Arg Lys Asn His Lys Glu Lys Thr Pro Ile Asp Arg Thr Gly Glu
           35                  40                  45
   Ser Glu Pro Thr Trp Asn His Thr Val Lys Phe Ser Val Asp Gln Arg
       50                  55                  60
   Leu Ala His Glu Gly Arg Leu Thr Leu Val Val Lys Leu Val Cys Asp
   65                  70                  75                  80
   Arg Ile Phe Gly Asp Lys Asp Leu Gly Glu Val Gln
                   85                  90
<210> 742
   <211> 94
   <212> PRT
   <213> Artificial sequence
<220>
   <223> Synthetically generated peptide
<400> 742
   Leu Glu Leu Lys Ile Val Ser Ala Ser Asp Val Asn His Ile Asp Ala
    1               5                  10                  15
   Thr Asp Lys Met Asp Val Tyr Ala Val Val Ser Ile Asn Gly Asp Thr
               20                  25                  30
   Thr Gln Gln Lys Gln Ala Ala Lys Thr Pro Ile Asp Tyr Asp Gly Gly
           35                  40                  45
   Ser Asn Pro Thr Trp Asn His Thr Val Lys Phe Ser Val Asn Glu Arg
       50                  55                  60
   Glu Ala Asn Glu Gly Leu Leu Thr Ile Thr Val Lys Leu Phe Ser Tyr
   65                  70                  75                  80
   Trp Leu Glu Gly Asp Asn Asp Leu Tyr Leu Gly Glu Val Asn
                   85                  90
<210> 743
   <211> 90
   <212> PRT
   <213> Artificial Sequence
<220>
   <223> Synthetically generated peptide
<400> 743
   Leu Glu Leu Asn Ile Asn Ser Ala Arg Asn Leu Leu Asn Val Asn Leu
    1               5                  10                  15
   Ile Thr Lys Met Asn Val Phe Thr Ala Ile Thr Ile Asn Gly Glu Asn
               20                  25                  30
   Thr Arg Lys Lys Gln Lys Ala Lys Thr Thr Val Asp Arg Tyr Gly Gly
           35                  40                  45
   Ser Asn Pro Thr Trp Asn Gln Thr Ile Lys Phe Ser Val Asp Glu Arg
       50                  55                  60
   Ser Arg Gly His Ser Ser Leu Val Met Arg Val Ile Ser Arg Arg Val
   65                  70                  75                  80
   Leu Gly Asn Lys Glu Ile Gly Arg Val Asn
                   85                  90
<210> 744
   <211> 91
   <212> PRT
   <213> Artificial Sequence
<220>
   <223> synthetically generated peptide
<400> 744
   Leu Glu Leu Asn Ile Ile Ser Ala Lys Asp Ile Lys Asn Val Asn Leu
   1               5                   10                  15
   Phe Ser Lys Met Asp Val Tyr Ala Ala Val Ser Leu Ser Gly Asp Pro
               20                  25                  30
   Leu His Pro Gln Gly Ala Thr Thr His Val His Lys Asp Ala Gly Ser
           35                  40                  45
   Asn Pro Thr Trp Asn Tyr Pro Val Lys Phe Ser Val Asn Glu Ser Leu
       50                  55                  60
   Ala Lys Glu Asn Arg Leu Ser Leu Glu Ile Lys Leu Ile Ser Asp Arg
   65                  70                  75                  80
   Thr Leu Gly Asp Thr Val Ile Gly Thr Val His
                   85                  90
<210> 745
   <211> 92
   <212> PRT
   <213> Artificial sequence
<220>
   <223> Synthetically generated peptide
<400> 745
   Leu Glu Leu Val Ile Lys Phe Ala Lys Asn Ile Glu Asp Val Asn Ala
   1               5                   10                  15
   Phe Ser Ser Met Asp Val Tyr Ala Ser Val Ala Ile Leu Lys Asp Arg
               20                  25                  30
   Lys Val Lys Asn Arg Ile Asn Thr Pro Val Ala Phe Ala Ala Tyr Thr
           35                  40                  45
   Asn Pro Lys Trp Asn Gln Met Met Lys Phe Ser Ile Asp Glu Lys Ser
       50                  55                  60
   Ala Gln Glu Gly Arg Leu Met Leu Leu Val Glu Leu Met Ser His Arg
   65                  70                  75                  80
   Pro Phe Leu Gly Asp Lys Glu Ile Gly Phe Val Arg
                   85                  90
<210> 746
   <211> 90
   <212> PRT
   <213> Artificial Sequence
<220>
   <223> Synthetically generated peptide
<400> 746
   Leu Asp Leu Thr Ile Ile Ser Ala Glu Asp Leu Lys Asp Val Gln Leu
   1               5                   10                  15
   Ile Gly Lys Gln Asp Leu Tyr Ala Val Val Ser Ile Asn Gly Asp Ala
               20                  25                  30
   Arg Thr Lys Gln Lys Thr Lys Val Asp Lys Asp Cys Gly Thr Lys Pro
           35                  40                  45
   Lys Trp Lys His Gln Met Lys Leu Thr Val Asp Asp Ala Ala Ala Arg
       50                  55                  60
   Asp Asn Arg Leu Thr Leu Val Phe Glu Ile Val Ala Asp Arg Pro Ile
   65                  70                  75                  80
   Ala Gly Asp Lys Pro Val Gly Glu Val Ser
                   85                  90
<210> 747
   <211> 90
   <212> PRT
   <213> Artificial Sequence
<220>
   <223> Synthetically generated peptide
<400> 747
   Leu Asp Leu Thr Ile Ile Ser Ala Glu Asp Leu Lys Asp Val Gln Leu
   1               5                   10                  15
   Ile Gly Lys Gln Asp Leu Tyr Ala Val Val Ser Ile Asn Gly Asp Ala
               20                  25                  30
   Arg Thr Lys Gln Lys Thr Lys Val Asp Lys Asp Cys Gly Thr Lys Pro
           35                  40                  45
   Lys Trp Lys His Gln Met Lys Leu Thr Val Asp Asp Ala Ala Ala Arg
       50                  55                  60
   Asp Asn Arg Leu Thr Leu Val Phe Glu Ile Val Ala Asp Arg Pro Ile
   65                  70                  75                  80
   Ala Gly Asp Lys Pro Val Gly Glu Val Ser
                   85                  90
<210> 748
   <211> 99
   <212> PRT
   <213> Artificial Sequence
<220>
   <223> Synthetically generated peptide
<400> 748
   Leu Glu Leu Thr Val Tyr Glu Ala Asp Asp Leu His Asn Ala Ile His
   1               5                   10                  15
   Gly Arg Ile Ile Lys Ala Ala Glu Ser Leu Lys Glu Ser Leu Gly Val
               20                  25                  30
   His Arg Leu Ala His Arg Ile Tyr Val Asp Val Asp Val Gly Ala Ala
           35                  40                  45
   Arg Val Ala Arg Thr Arg Glu Val Glu Phe His Pro Thr Asn Pro Val
       50                  55                  60
   Trp Asn Gln Ser Phe Arg Leu His Cys Ala Tyr Pro Ala Ala Pro Val
   65                  70                  75                  80
   Ala Phe Thr Val Lys Ser Gln His Leu Val Gly Ala Gly Val Leu Gly
                   85                  90                  95
   Ala Ala Arg
<210> 749
   <211> 99
   <212> PRT
   <213> Artificial Sequence
<220>
   <223> Synthetically generated peptide
<400> 749
   Leu Glu Leu Thr Val Tyr Glu Ala Asp Asp Leu His Asn Ala Ile His
   1               5                   10                  15
   Gly Arg Ile Ile Lys Ala Ala Glu Ser Leu Lys Glu Ser Leu Gly Val
               20                  25                  30
   His Arg Leu Ala His Arg Ile Tyr Val Asp Val Asp Val Gly Ala Ala
           35                  40                  45
   Arg Val Ala Arg Thr Arg Glu Val Glu Phe His Pro Thr Asn Pro Val
       50                  55                  60
   Trp Asn Gln Ser Phe Arg Leu His Cys Ala Tyr Pro Ala Ala Pro Val
   65                  70                  75                  80
   Ala Phe Thr Val Lys Ser Gln His Leu Val Gly Ala Gly Val Leu Gly
                   85                  90                  95
   Ala Ala Arg
<210> 750
   <211> 38
   <212> PRT
   <213> Artificial Sequence
<220>
   <223> Synthetically generated peptide
<400> 750
   Ser Pro Lys Trp Asn Glu Thr Phe His Ile Tyr Ser Ala His Ser Ile
   1               5                   10                  15
   Ser Asn Ile Ile Phe Thr Val Lys Gln Asp Asn Pro Ile Gly Ala Thr
               20                  25                  30
   Leu Ile Gly Arg Ala Tyr
           35
<210> 751
   <211> 60
   <212> PRT
   <213> Artificial sequence
<220>
   <223> synthetically generated peptide
<400> 751
   Tyr Ala Thr Val Asp Leu Asp Lys Ala Arg Val Gly Arg Thr Arg Met
   1               5                   10                  15
   Ile Gly Asn Gln Pro Ser Asn Pro Lys Trp Asn Glu Thr Phe Glu Ile
               20                  25                  30
   Tyr Cys Ala His Tyr Ile Ser Asn Ile Val Phe Thr Val Lys Asp Asp
           35                  40                  45
   Asn Pro Ile Gly Ala Thr Leu Ile Gly Arg Ala Tyr
       50                  55                  60
<210> 752
   <211> 100
   <212> PRT
   <213> Artificial sequence
<220>
   <223> Synthetically generated peptide
<400> 752
   Leu His Val Thr Ile Phe Glu Val Asp His Leu Lys Ala Gly Ser Val
    1               5                  10                  15
   Val Val Phe Ser Glu Ser Leu Arg Arg Thr Leu Arg Lys Pro Leu Val
               20                  25                  30
   Leu Ala Lys Gly Thr Pro Lys Ile Tyr Ala Ser Ile Asp Leu Asp Lys
           35                  40                  45
   Ala Arg Val Gly Arg Thr Arg Met Ile Glu Asn Glu Pro Asn Asn Pro
       50                  55                  60
   Lys Trp Asn Glu Ser Phe His Ile Tyr Cys Gly His Pro Ser Thr Asn
   65                  70                  75                  80
   Val Ile Phe Thr Val Lys Asp Asp Asn Pro Ile Gly Ala Thr Leu Ile
                   85                  90                  95
   Gly Arg Ala Tyr
               100
<210> 753
   <211> 100
   <212> PRT
   <213> Artificial sequence
<220>
   <223> Synthetically generated peptide
<400> 753
   Leu His Ala Thr Ile Phe Glu Val Asp Lys Leu Lys Asn Ile Gly Gly
    1               5                  10                  15
   Gly Asn Ile Leu Ser Lys Ile Arg Gln Asn Phe Glu Glu Thr Val Gly
               20                  25                  30
   Phe Gly Lys Gly Thr Thr Lys Leu Tyr Ala Thr Ile Asp Leu Glu Lys
           35                  40                  45
   Ala Arg Val Gly Arg Thr Arg Ile Ile Glu Lys Glu His Val Asn Pro
       50                  55                  60
   Gln Trp Asn Glu Ser Phe His Ile Tyr Cys Ala His Leu Ala Ser Asp
   65                  70                  75                  80
   Ile Ile Phe Thr Val Lys Asp Asp Asn Pro Ile Gly Ala Thr Leu Ile
                   85                  90                  95
   Gly Arg Ala Tyr
               100
<210> 754
   <211> 100
   <212> PRT
   <213> Artificial sequence
<220>
   <223> Synthetically generated peptide
<400> 754
   Leu His Ala Thr Ile Phe Glu Val Asp Lys Leu Lys Asn Ile Gly Gly
   1               5                   10                  15
   Gly Asn Ile Leu Ser Lys Ile Arg Gln Asn Phe Glu Glu Thr Val Gly
               20                  25                  30
   Phe Gly Lys Gly Thr Thr Lys Leu Tyr Ala Thr Ile Asp Leu Glu Lys
           35                  40                  45
   Ala Arg Val Gly Arg Thr Arg Ile Ile Glu Lys Glu His Val Asn Pro
       50                  55                  60
   Gln Trp Asn Glu Ser Phe His Ile Tyr Cys Ala His Leu Ala Ser Asp
   65                  70                  75                  80
   Ile Ile Phe Thr Val Lys Asp Asp Asn Pro Ile Gly Ala Thr Leu Ile
                   85                  90                  95
   Gly Arg Ala Tyr
               100
<210> 755
   <211> 98
   <212> PRT
   <213> Artificial sequence
<220>
   <223> Synthetically generated peptide
<400> 755
   Leu His Ala Thr Ile Tyr Glu Val Asp Glu Leu His Gly Gly Gly Gly
   1               5                   10                  15
   Gly Asn Phe Phe Ser Lys Leu Lys Gln Asn Ile Glu Glu Thr Val Gly
               20                  25                  30
   Ile Gly Lys Gly Val Thr Lys Leu Tyr Ala Thr Ile Asp Leu Glu Lys
           35                  40                  45
   Ala Arg Val Gly Arg Thr Arg Ile Ile Glu Asn Glu Thr Thr Asn Pro
       50                  55                  60
   Lys Trp Asn Glu Ser Phe His Ile Tyr Cys Gly His Leu Asn Ile Ile
   65                  70                  75                  80
   Phe Thr Val Lys Asp Asp Asn Pro Ile Gly Ala Thr Leu Ile Gly Arg
                   85                  90                  95
   Ala Tyr
<210> 756
   <211> 96
   <212> PRT
   <213> Artificial Sequence
<220>
   <223> Synthetically generated peptide
<400> 756
   Leu His Ala Thr Ile Tyr Glu Ile Asp Arg Leu His Thr Gly Gly Ser
   1               5                   10                  15
   Ser Asn Val Phe Ser Met Leu Arg Gln Asn Phe Glu Glu Ala Val Gly
               20                  25                  30
   Ile Gly Lys Gly Gln Thr Lys Leu Tyr Ala Thr Ile Asp Leu Glu Lys
           35                  40                  45
   Ala Arg Val Gly Arg Thr Arg Ile Ser Glu Pro Ser Asn Pro Arg Trp
       50                  55                  60
   Tyr Glu Ser Phe His Ile Tyr Cys Ala His Lys Asn Val Ile Phe Thr
   65                  70                  75                  80
   Val Lys Asp Asp Asn Pro Ile Gly Ala Thr Leu Ile Gly Arg Thr Tyr
                   85                  90                  95
<210> 757
   <211> 98
   <212> PRT
   <213> Artificial sequence
<220>
   <223> Synthetically generated peptide
<400> 757
   Leu His Val Thr Ile Tyr Glu Val Asp Gln Leu His Ser Gly Gly Gly
   1               5                   10                  15
   Gly Asn Phe Phe Thr Lys Leu Lys Ala Asn Ile Glu Glu Thr Val Gly
               20                  25                  30
   Phe Gly Lys Gly Thr Pro Lys Ile Tyr Ala Ser Ile Asp Leu Glu Lys
           35                  40                  45
   Ala Arg Val Gly Arg Thr Arg Met Ile Glu His Glu Pro Asn Asn Pro
       50                  55                  60
   Arg Trp Tyr Glu Ser Phe His Ile Tyr Cys Ala His Met Asn Val Ile
   65                  70                  75                  80
   Phe Thr Val Lys Asp Asp Asn Pro Ile Gly Ala Thr Leu Ile Gly Arg
                   85                  90                  95
   Ala Tyr
<210> 758
   <211> 98
   <212> PRT
   <213> Artificial Sequence
<220>
   <223> Synthetically generated peptide
<400> 758
   Leu His Val Thr Val Tyr Glu Val Asp Arg Leu His Ala Gly Gly Gly
   1               5                   10                  15
   Gly Asn Ile Phe Ser Lys Leu Arg Ala Asn Ile Glu Glu Lys Val Gly
               20                  25                  30
   Phe Gly Lys Gly Thr Pro Lys Ile Tyr Ala Ser Ile Asp Leu Glu Lys
           35                  40                  45
   Ala Arg Val Gly Arg Thr Arg Met Ile Glu His Glu Pro Thr Asn Pro
       50                  55                  60
   Arg Trp Tyr Glu Ser Phe His Ile Tyr Cys Ala His Leu Asn Ile Ile
   65                  70                  75                  80
   Phe Thr Val Lys Asp Asp Asn Pro Ile Gly Ala Thr Leu Ile Gly Arg
                   85                  90                  95
   Ala Tyr
<210> 759
   <211> 98
   <212> PRT
   <213> Artificial sequence
<220>
   <223> Synthetically generated peptide
<400> 759
   Leu His Val Thr Val Tyr Glu Val Asp Lys Leu Arg Glu Gly Gly Gly
   1               5                   10                  15
   Pro Asn Val Phe Gly Lys Leu Met Ala Asn Ile Gln Glu Thr Val Gly
               20                  25                  30
   Phe Gly Glu Gly Thr Pro Lys Ile Tyr Ala Thr Ile Asp Leu Glu Lys
           35                  40                  45
   Ser Arg Val Gly Arg Thr Arg Met Ile Glu Asn Glu Pro Gln Asn Pro
       50                  55                  60
   Arg Trp Tyr Glu Ser Phe His Ile Tyr Cys Ala His His Asn Ile Ile
   65                  70                  75                  80
   Phe Thr Val Lys Asp Asp Asn Pro Ile Gly Ala Thr Leu Leu Gly Arg
                   85                  90                  95
   Ala Tyr
<210> 760
   <211> 96
   <212> PRT
   <213> Artificial Sequence
<220>
   <223> Synthetically generated peptide
<400> 760
   Leu His Val Thr Ile Tyr Glu Val Asp Lys Leu His Ser Gly Gly Gly
   1               5                   10                  15
   Pro His Phe Phe Arg Lys Leu Val Glu Asn Ile Glu Glu Thr Val Gly
               20                  25                  30
   Phe Gly Lys Gly Val Ser Lys Leu Tyr Ala Thr Ile Asp Leu Glu Lys
           35                  40                  45
   Ala Arg Val Gly Arg Thr Arg Ile Asn Glu Gln Ser Asn Pro Arg Trp
       50                  55                  60
   Tyr Glu Ser Phe His Val Tyr Cys Ala His Gln Asn Val Ile Phe Thr
   65                  70                  75                  80
   Val Lys Asp Asp Asn Pro Ile Gly Ala Thr Leu Ile Gly Arg Ala Tyr
                   85                  90                  95
<210> 761
   <211> 97
   <212> PRT
   <213> Artificial Sequence
<220>
   <223> Synthetically generated peptide
<400> 761
   Leu His Val Thr Ile Tyr Glu Val Asp Lys Leu Lys Thr Ser Gly Gly
   1               5                   10                  15
   Asn Val Phe Thr Lys Leu Val Gln Asn Ile Glu Glu Thr Val Gly Phe
               20                  25                  30
   Gly Lys Gly Val Thr Lys Leu Tyr Ala Thr Ile Asp Leu Glu Lys Ala
           35                  40                  45
   Arg Val Gly Arg Thr Arg Ile Ile Glu Lys Asp His Ser Asn Pro Arg
       50                  55                  60
   Trp Tyr Glu Ser Phe His Ile Tyr Cys Ala His Met Asn Ile Ile Phe
   65                  70                  75                  80
   Thr Val Lys Asp Asp Asn Pro Ile Gly Ala Thr Leu Ile Gly Arg Ala
                   85                  90                  95
   Tyr
<210> 762
   <211> 101
   <212> PRT
   <213> Artificial sequence
<220>
   <223> Synthetically generated peptide
<400> 762
   Leu His Ala Thr Ile Tyr Glu Val Asp Asp Leu His Thr Gly Gly Leu
   1               5                   10                  15
   Arg Ser Gly Phe Phe Gly Lys Ile Leu Ala Asn Val Glu Glu Thr Ile
               20                  25                  30
   Gly Val Gly Lys Gly Glu Thr Gln Leu Tyr Ala Thr Ile Asp Leu Gln
           35                  40                  45
   Arg Ala Arg Val Gly Arg Thr Arg Lys Ile Lys Asp Glu Ala Lys Asn
       50                  55                  60
   Pro Lys Trp Tyr Glu Ser Phe His Ile Tyr Cys Ala His Leu Ala Ser
   65                  70                  75                  80
   Asp Ile Ile Phe Thr Val Lys Asp Asp Asn Pro Ile Gly Ala Thr Leu
                   85                  90                  95
   Ile Gly Arg Ala Tyr
               100
<210> 763
   <211> 60
   <212> PRT
   <213> Artificial Sequence
<220>
   <223> Synthetically generated peptide
<400> 763
   Tyr Ala Thr Ile Asp Leu Gln Lys Ala Arg Val Gly Arg Thr Arg Lys
   1               5                   10                  15
   Ile Lys Asn Glu Pro Lys Asn Pro Lys Trp Tyr Glu Ser Phe His Ile
               20                  25                  30
   Tyr Cys Ala His Leu Ala Ser Asp Ile Ile Phe Thr Val Lys Asp Asp
           35                  40                  45
   Asn Pro Ile Gly Ala Thr Leu Ile Gly Arg Ala Tyr
       50                  55                  60
<210> 764
   <211> 60
   <212> PRT
   <213> Artificial Sequence
<220>
   <223> Synthetically generated peptide
<400> 764
   Tyr Ala Thr Ile Asp Leu Gln Lys Ala Arg Val Gly Arg Thr Arg Lys
   1               5                   10                  15
   Ile Thr Asp Glu Pro Lys Asn Pro Lys Trp Tyr Glu Ser Phe His Ile
               20                  25                  30
   Tyr Cys Ala His Met Ala Ser Asp Ile Ile Phe Thr Val Lys Asp Asp
           35                  40                  45
   Asn Pro Ile Gly Ala Thr Leu Ile Gly Arg Ala Tyr
       50                  55                  60
<210> 765
   <211> 101
   <212> PRT
   <213> Artificial sequence
<220>
   <223> Synthetically generated peptide
<400> 765
   Leu His Ala Thr Ile Tyr Glu Val Asp His Leu His Ala Glu Gly Gly
   1               5                   10                  15
   Arg Ser Gly Phe Leu Gly Ser Ile Leu Ala Asn Val Glu Glu Thr Ile
               20                  25                  30
   Gly Val Gly Lys Gly Glu Thr Gln Leu Tyr Ala Thr Ile Asp Leu Glu
           35                  40                  45
   Lys Ala Arg Val Gly Arg Thr Arg Lys Ile Thr Lys Glu Pro Lys Asn
       50                  55                  60
   Pro Lys Trp Phe Glu Ser Phe His Ile Tyr Cys Gly His Met Ala Lys
   65                  70                  75                  80
   His Val Ile Phe Thr Val Lys Asp Ala Asn Pro Ile Gly Ala Thr Leu
                   85                  90                  95
   Ile Gly Arg Gly Tyr
               100
<210> 766
   <211> 99
   <212> PRT
   <213> Artificial Sequence
<220>
   <223> synthetically generated peptide
<400> 766
   Leu His Val Thr Ile Phe Glu Val Asp Asn Leu Gln Gly Glu Glu Glu
   1 5 10 15
   Gly Gly His Phe Phe Ser Lys Ile Lys Gln His Phe Glu Glu Thr Val
   20 25 30
   Gly Ile Gly Lys Gly Thr Pro Lys Leu Tyr Ala Thr Ile Asp Leu Glu
   35 40 45
   Lys Ala Arg Val Gly Arg Thr Arg Ile Ile Glu Asn Glu Pro Lys Asn
   50 55 60
   Pro Arg Trp Tyr Glu Ser Phe His Ile Tyr Cys Ala His Met Asn Val
   65 70 75 80
   Ile Phe Thr Ile Lys Asp Asp Asn Pro Phe Gly Ala Ser Leu Ile Gly
   85 90 95
   Arg Ala Tyr
<210> 767
   <211> 99
   <212> PRT
   <213> Artificial Sequence
<220>
   <223> Synthetically generated peptide
<400> 767
   Leu His Val Thr Ile Phe Glu Val Asp Asn Leu Gln Gly Glu Glu Glu
   1 5 10 15
   Gly Gly His Phe Phe Ser Lys Ile Lys Gln His Phe Glu Glu Thr Val
   20 25 30
   Gly Ile Gly Lys Gly Thr Pro Lys Leu Tyr Ala Thr Ile Asp Leu Glu
   35 40 45
   Lys Ala Arg Val Gly Arg Thr Arg Ile Ile Glu Asn Glu Pro Lys Asn
   50 55 60
   Pro Arg Trp Tyr Glu Ser Phe His Ile Tyr Cys Ala His Met Asn Val
   65 70 75 80
   Ile Phe Thr Ile Lys Asp Asp Asn Pro Phe Gly Ala Ser Leu Ile Gly
   85 90 95
   Arg Ala Tyr
<210> 768
   <211> 98
   <212> PRT
   <213> Artificial Sequence
<220>
   <223> Synthetically generated peptide
<400> 768
   Leu His Val Thr Ile Tyr Glu Val Asp Asn Leu Gln Lys Glu Gly Gly
   1 5 10 15
   Gly His Phe Phe Ser Lys Ile Lys Glu His Val Glu Glu Thr Ile Gly
   20 25 30
   Phe Gly Lys Gly Thr Pro Ala Ile Tyr Ala Thr Val Asp Leu Glu Lys
   35 40 45
   Ala Arg Val Gly Arg Thr Arg Lys Ile Lys Asn Glu Pro Asn Asn Pro
   50 55 60
   Arg Trp Tyr Glu Ser Phe His Ile Tyr Cys Ala His Met Asn Val Ile
   65 70 75 80
   Phe Thr Val Lys Asp Asp Asn Pro Ile Gly Ala Thr Leu Ile Gly Arg
   85 90 95
   Ala Tyr
<210> 769
   <211> 99
   <212> PRT
   <213> Artificial Sequence
<220>
   <223> Synthetically generated peptide
<400> 769
   Leu His Ala Thr Ile Tyr Glu Val Asp Lys Leu His Gly Ser Ser Gly
   1 5 10 15
   Asn Phe Leu Arg Lys Ile Thr Gly Lys Leu Glu Glu Thr Val Gly Leu
   20 25 30
   Gly Lys Gly Val Ser Lys Leu Tyr Ala Thr Val Asp Leu Glu Arg Ala
   35 40 45
   Arg Val Gly Arg Thr Arg Val Ile Glu Lys Glu Pro Ser Asn Pro Asn
   50 55 60
   Trp Ser Glu Ser Phe His Ile Tyr Cys Ala His Val Ala Ala Asn Val
   65 70 75 80
   Ile Phe Thr Val Lys Glu Ser Asn Pro Ile Gly Ala Ser Leu Ile Gly
   85 90 95
   Arg Ala Tyr
<210> 770
   <211> 103
   <212> PRT
   <213> Artificial Sequence
<220>
   <223> Synthetically generated peptide
<400> 770
   Leu His Ala Thr Ile Phe Glu Ala Ala Ser Leu Ser Asn Pro His Arg
   1               5                   10                  15
   Ala Ser Gly Ser Ala Pro Lys Phe Ile Arg Lys Phe Val Glu Gly Ile
               20                  25                  30
   Glu Asp Thr Val Gly Val Gly Lys Gly Ala Thr Lys Val Tyr Ser Thr
           35                  40                  45
   Ile Asp Leu Glu Lys Ala Arg Val Gly Arg Thr Arg Met Ile Thr Asn
       50                  55                  60
   Glu Pro Ile Asn Pro Arg Trp Tyr Glu Ser Phe His Ile Tyr Cys Ala
   65                  70                  75                  80
   His Met Asn Val Ile Phe Thr Val Lys Ile Asp Asn Pro Ile Gly Ala
                   85                  90                  95
   Thr Asn Ile Gly Arg Ala Tyr
               100
<210> 771
   <211> 105
   <212> PRT
   <213> Artificial Sequence
<220>
   <223> synthetically generated peptide
<400> 771
   Leu His Ala Thr Ile Phe Glu Ala Glu Ser Leu Ser Asn Pro His Arg
   1               5                   10                  15
   Ala Thr Gly Gly Ala Pro Lys Phe Ile Arg Lys Leu Val Glu Gly Ile
               20                  25                  30
   Glu Asp Thr Val Gly Val Gly Lys Gly Ala Thr Lys Ile Tyr Ala Thr
           35                  40                  45
   Val Asp Leu Glu Lys Ala Arg Val Gly Arg Thr Arg Met Ile Ser Asn
       50                  55                  60
   Glu Pro Val Asn Pro Arg Trp Tyr Glu Ser Phe His Ile Tyr Cys Ala
   65                  70                  75                  80
   His Met Ala Ala Asp Val Ile Phe Thr Val Lys Ile Asp Asn Ser Ile
                   85                  90                  95
   Gly Ala Ser Leu Ile Gly Arg Ala Tyr
               100                 105
<210> 772
   <211> 105
   <212> PRT
   <213> Artificial Sequence
<220>
   <223> Synthetically generated peptide
<400> 772
   Leu His Val Thr Ile Phe Glu Ala Asn Ser Ile Ser His Pro Asp Arg
   1 5 10 15
   Lys Thr Gly Gly Ala Pro Lys Phe Phe Arg Lys Leu Val Glu Asn Ile
   20 25 30
   Glu Glu Thr Val Gly Phe Gly Lys Gly Ala Ser Met Leu Tyr Ala Ser
   35 40 45
   Val Asp Leu Asp Lys Ala Arg Val Gly Arg Thr Arg Ile Ile Lys Asp
   50 55 60
   Glu Pro Val Asn Pro Lys Trp Tyr Glu Ser Phe His Ile Tyr Cys Ala
   65 70 75 80
   His Met Ala Ala Asn Val Ile Phe Thr Val Lys Asp Asp Asn Pro Ile
   85 90 95
   Gly Ala Thr Leu Ile Gly Arg Ala Tyr
   100 105
<210> 773
   <211> 105
   <212> PRT
   <213> Artificial Sequence
<220>
   <223> Synthetically generated peptide
<400> 773
   Leu His Val Thr Ile Phe Glu Ala Asn Ser Ile Ser His Pro Asp Arg
   1 5 10 15
   Lys Thr Gly Gly Ala Pro Lys Phe Phe Arg Lys Leu Val Glu Asn Ile
   20 25 30
   Glu Glu Thr Val Gly Phe Gly Lys Gly Ala Ser Met Leu Tyr Ala Ser
   35 40 45
   Val Asp Leu Asp Lys Ala Arg Val Gly Arg Thr Arg Ile Ile Lys Asp
   50 55 60
   Glu Pro Val Asn Pro Lys Trp Tyr Glu Ser Phe His Ile Tyr Cys Ala
   65 70 75 80
   His Met Ala Ala Asn Val Ile Phe Thr Val Lys Asp Asp Asn Pro Ile
   85 90 95
   Gly Ala Thr Leu Ile Gly Arg Ala Tyr
   100 105
<210> 774
   <211> 59
   <212> PRT
   <213> Artificial Sequence
<220>
   <223> Synthetically generated peptide
<400> 774
   Tyr Ala Thr Ile Asp Leu Gly Lys Ala Arg Val Gly Arg Thr Arg Leu
   1 5 10 15
   Leu Asp Glu His Lys Asn Pro Arg Trp Tyr Glu Ser Phe His Ile Tyr
   20 25 30
   Cys Ala His Met Ala Ser Asp Val Val Phe Thr Val Lys Ala Asp Asn
   35 40 45
   Pro Ile Gly Ala Glu Leu Ile Gly Arg Ala Tyr
   50 55
<210> 775
   <211> 99
   <212> PRT
   <213> Artificial Sequence
<220>
   <223> synthetically generated peptide
<400> 775
   Leu His Val Thr Ile Phe Glu Val Asp Lys Leu Arg Thr Asn Phe Gly
   1 5 10 15
   Arg Glu Ile Phe Asn Lys Val Val Gln Gly Ile Glu Gly Ala Ile Gly
   20 25 30
   Phe Asn Lys Thr Ala Ser Thr Leu Tyr Ala Thr Ile Asp Leu Gly Lys
   35 40 45
   Ala Arg Val Gly Arg Thr Arg Leu Leu Asp Glu His Lys Asn Pro Arg
   50 55 60
   Trp Tyr Glu Ser Phe His Ile Tyr Cys Ala His Met Ala Ser Asp Val
   65 70 75 80
   Val Phe Thr Val Lys Ala Asp Asn Pro Ile Gly Ala Glu Leu Ile Gly
   85 90 95
   Arg Ala Tyr
<210> 776
   <211> 99
   <212> PRT
   <213> Artificial sequence
<220>
   <223> Synthetically generated peptide
<400> 776
   Leu His Val Thr Ile Phe Glu Val Asp Arg Leu His Thr Asn Phe Gly
   1 5 10 15
   Arg Asp Phe Phe Asn Lys Val Val Gln Gly Ile Glu Gly Ala Ile Gly
   20 25 30
   Phe Asn Lys Ala Ala Ser Arg Leu Tyr Ala Thr Ile Asp Leu Gly Lys
   35 40 45
   Ala Arg Val Gly Arg Thr Arg Leu Leu Asp Asp His Lys Asn Pro Arg
   50 55 60
   Trp Tyr Glu Ser Phe His Ile Tyr Cys Ala His Met Ala Ala Asn Val
   65 70 75 80
   Ile Ile Thr Val Lys Phe Asp Asn Pro Ile Gly Ala Glu Val Ile Gly
   85 90 95
   Arg Ala Tyr
<210> 777
   <211> 59
   <212> PRT
   <213> Artificial Sequence
<220>
   <223> Synthetically generated peptide
<400> 777
   Tyr Ala Thr Val Asp Ile Asp Lys Ala Arg Val Ala Arg Thr Arg Thr
   1 5 10 15
   Val Glu Pro Thr Gly Thr Pro Arg Trp Lys Glu Ser Phe His Ile Tyr
   20 25 30
   Cys Ala His Tyr Ala Gly Asp Val Ile Phe Thr Val Lys Ala Glu Asn
   35 40 45
   Pro Val Gly Ala Thr Leu Ile Gly Arg Ala Tyr
   50 55
<210> 778
   <211> 105
   <212> PRT
   <213> Artificial Sequence
<220>
   <223> Synthetically generated peptide
<400> 778
   Met His Val Thr Ile Phe Glu Ala Glu Ser Leu Ser Asn Pro Ser Arg
   1 5 10 15
   Pro Ser Ser Gln Ala Pro Gln Phe Leu Arg Lys Leu Val Glu Gly Ile
   20 25 30
   Glu Asp Thr Val Gly Val Gly Lys Gly Thr Ser Lys Val Tyr Ala Thr
   35 40 45
   Ile Gly Leu Asp Lys Ala Arg Val Gly Arg Thr Arg Thr Leu Ala Asp
   50 55 60
   Asp Thr Ala Ala Pro Arg Trp Tyr Glu Ser Phe His Val Tyr Cys Ala
   65 70 75 80
   His Leu Ala Thr His Val Ala Phe Thr Leu Lys Ala Lys Asn Pro Ile
   85 90 95
   Gly Ala Ser Leu Leu Gly Val Gly Tyr
   100 105
<210> 779
   <211> 89
   <212> PRT
   <213> Artificial sequence
<220>
   <223> Synthetically generated peptide
<400> 779
   Leu Glu Ala Thr Ile Ala Asp His Leu Ser Asn Pro Thr Arg Ala Thr
   1 5 10 15
   Gly Ala Ala Pro Gly Ile Phe Arg Lys Phe Val Glu Gly Phe Glu Asp
   20 25 30
   Ser Leu Gly Leu Gly Lys Gly Ala Thr Arg Leu Tyr Ala Thr Ile Asp
   35 40 45
   Leu Gly Arg Ala Arg Val Gly Arg Thr Arg Val Val Asp Asp Glu Pro
   50 55 60
   Val Asn Pro Arg Trp Tyr Glu Val Phe His Ile Tyr Cys Ala His Phe
   65 70 75 80
   Ala Ala Asp Val Val Phe Ser Val Lys
   85
<210> 780
   <211> 103
   <212> PRT
   <213> Artificial Sequence
<220>
   <223> Synthetically generated peptide
<400> 780
   Leu Glu Ala Thr Ile Ala Asp His Leu Ser Asn Pro Thr Arg Ala Thr
   1               5                   10                  15
   Gly Ala Ala Pro Gly Ile Phe Arg Lys Phe Val Glu Gly Phe Glu Asp
               20                  25                  30
   Ser Leu Gly Leu Gly Lys Gly Ala Thr Arg Leu Tyr Ala Thr Ile Asp
           35                  40                  45
   Leu Gly Arg Ala Arg Val Gly Arg Thr Arg Val Val Asp Asp Glu Pro
       50                  55                  60
   Val Asn Pro Arg Trp Tyr Glu Val Phe His Ile Tyr Cys Ala His Phe
   65                  70                  75                  80
   Ala Ala Asp Val Val Phe Ser Val Lys Ala Ala Gln Pro Ile Gly Ala
                   85                  90                  95
   Thr Leu Ile Gly Arg Ala Tyr
               100
<210> 781
   <211> 107
   <212> PRT
   <213> Artificial Sequence
<220>
   <223> synthetically generated peptide
<400> 781
   Leu Asp Ala Thr Ile Phe Glu Ala Thr Asn Leu Thr Asn Pro Thr Arg
   1               5                   10                  15
   Leu Thr Gly Asn Ala Pro Glu Gly Phe Arg Lys Trp Trp Glu Gly Leu
               20                  25                  30
   Glu Asn Gly Leu Glu Lys Thr Thr Gly Leu Gly Pro Gly Gly Thr Arg
           35                  40                  45
   Leu Tyr Ala Thr Val Asp Leu Gly Arg Ala Arg Leu Gly Arg Thr Arg
       50                  55                  60
   Val Ile Asp Asp Glu Pro Val Ser Pro Arg Trp Asp Glu Arg Phe His
   65                  70                  75                  80
   Phe Tyr Cys Ala His Phe Ala Glu Asn Val Val Phe Ser Val Lys Val
                   85                  90                  95
   Ser Val Asp Ala Lys Leu Ile Gly Arg Ala Tyr
               100                 105
<210> 782
   <211> 107
   <212> PRT
   <213> Artificial sequence
<220>
   <223> Synthetically generated peptide
<400> 782
   Leu Asp Ala Thr Ile Phe Glu Ala Thr Asn Leu Thr Asn Pro Thr Arg
   1 5 10 15
   Leu Thr Gly Asn Ala Pro Glu Gly Phe Arg Lys Trp Trp Glu Gly Leu
   20 25 30
   Glu Asn Gly Leu Glu Lys Thr Thr Gly Leu Gly Pro Gly Gly Thr Arg
   35 40 45
   Leu Tyr Ala Thr Val Asp Leu Gly Arg Ala Arg Leu Gly Arg Thr Arg
   50 55 60
   Val Ile Asp Asp Glu Pro Val Ser Pro Arg Trp Asp Glu Arg Phe His
   65 70 75 80
   Phe Tyr Cys Ala His Phe Ala Glu Asn Val Val Phe Ser Val Lys Val
   85 90 95
   Ser Val Asp Ala Lys Leu Ile Gly Arg Ala Tyr
   100 105
<210> 783
   <211> 105
   <212> PRT
   <213> Artificial Sequence
<220>
   <223> Synthetically generated peptide
<400> 783
   Leu Asp Ala Thr Ile Phe Glu Ala Thr Asn Leu Thr Asn Pro Thr Arg
   1 5 10 15
   Leu Thr Gly Ser Ala Pro Glu Gly Ile Arg Lys Trp Trp Glu Gly Val
   20 25 30
   Glu Lys Thr Thr Gly Val Gly Gln Gly Gly Thr Arg Leu Tyr Ala Thr
   35 40 45
   Val Asp Leu Gly Lys Ala Arg Leu Gly Arg Thr Arg Val Ile Asp Asp
   50 55 60
   Glu Pro Val Asn Pro Arg Trp Asp Glu Arg Phe His Leu Tyr Cys Ala
   65 70 75 80
   His Phe Ala Asp Asn Val Val Phe Ser Val Lys Val Ser Leu Pro Ile
   85 90 95
   Asp Ala Ala Leu Ile Gly Arg Ala Tyr
   100 105
<210> 784
   <211> 105
   <212> PRT
   <213> Artificial Sequence
<220>
   <223> Synthetically generated peptide
<400> 784
   Leu Asp Ala Thr Ile Phe Glu Ala Thr Asn Leu Thr Asn Pro Thr Arg
   1 5 10 15
   Leu Thr Gly Ser Ala Pro Glu Gly Ile Arg Lys Trp Trp Glu Gly Val
   20 25 30
   Glu Lys Thr Thr Gly Val Gly Gln Gly Gly Thr Arg Leu Tyr Ala Thr
   35 40 45
   Val Asp Leu Gly Lys Ala Arg Leu Gly Arg Thr Arg Val Ile Asp Asp
   50 55 60
   Glu Pro Val Asn Pro Arg Trp Asp Glu Arg Phe His Leu Tyr Cys Ala
   65 70 75 80
   His Phe Ala Asp Asn Val Val Phe Ser Val Lys Val Ser Leu Pro Ile
   85 90 95
   Asp Ala Ala Leu Ile Gly Arg Ala Tyr
   100 105

<210> 785
   <211> 115
   <212> PRT
   <213> Artificial sequence
<220>
   <223> Synthetically generated peptide
<400> 785
   Leu Asp Leu Thr Ile Val Glu Ala Arg Arg Leu Pro Asn Met Asp Phe
   1 5 10 15
   Met Val Asn His Leu Arg Ser Cys Leu Thr Cys Glu Pro Cys Lys Ser
   20 25 30
   Pro Ala Gln Thr Ala Ala Lys Glu Gly Asp Ser Lys Ile Arg Gly His
   35 40 45
   Arg Lys Ile Ile Thr Ser Asp Pro Tyr Val Thr Val Cys Leu Pro Gln
   50 55 60
   Ala Thr Val Ala Arg Thr Arg Val Leu Lys Asn Ser Gln Asn Pro Lys
   65 70 75 80
   Trp Asn Glu His Phe Ile Ile Pro Leu Ala His Pro Val Thr Glu Leu
   85 90 95
   Asp Ile Asn Val Lys Asp Asn Asp Leu Phe Gly Ala Asp Ala Ile Gly
   100 105 110
   Thr Ala Lys
   115
<210> 786
   <211> 115
   <212> PRT
   <213> Artificial Sequence
<220>
   <223> Synthetically generated peptide
<400> 786
   Leu Asp Leu Thr Ile Val Glu Ala Arg Arg Leu Pro Asn Met Asp Phe
   1 5 10 15
   Met Val Asn His Leu Arg Ser Cys Leu Thr Cys Glu Pro Cys Lys Ser
   20 25 30
   Pro Ala Gln Thr Ala Ala Lys Glu Gly Asp Ser Lys Ile Arg Gly His
   35 40 45
   Arg Lys Ile Ile Thr Ser Asp Pro Tyr Val Thr Val Cys Leu Pro Gln
   50 55 60
   Ala Thr Val Ala Arg Thr Arg Val Leu Lys Asn Ser Gln Asn Pro Lys
   65 70 75 80
   Trp Asn Glu His Phe Ile Ile Pro Leu Ala His Pro Val Thr Glu Leu
   85 90 95
   Asp Ile Asn Val Lys Asp Asn Asp Leu Phe Gly Ala Asp Ala Ile Gly
   100 105 110
   Thr Ala Lys
   115
<210> 787
   <211> 118
   <212> PRT
   <213> Artificial Sequence
<220>
   <223> synthetically generated peptide
<400> 787
   Leu Asp Leu Thr Ile Ile Glu Ala Arg Lys Leu Pro Asn Met Asp Ile
   1 5 10 15
   Val Ser Asn His Leu Arg Lys Cys Leu Thr Cys Glu Thr Cys Lys Ala
   20 25 30
   Pro Ser Gln Ala Ala Ala Ala Gln Glu Pro Gly Glu Val Gly Lys Val
   35 40 45
   His His His His Lys Ile Met Thr Ser Asp Pro Tyr Val Thr Ile Thr
   50 55 60
   Val Pro Gln Ser Thr Leu Ala Arg Thr Pro Val Leu Lys Ser Ala Asp
   65 70 75 80
   Asn Pro Glu Trp Asn Glu Arg Phe Ile Ile Pro Met Ala His Pro Leu
   85 90 95
   Thr Glu Leu Glu Ile Asn Val Lys Asp Asp Asp Leu Leu Gly Ala Glu
   100 105 110
   Val Ile Gly Thr Thr Lys
   115
<210> 788
   <211> 123
   <212> PRT
   <213> Artificial Sequence
<220>
   <223> Synthetically generated peptide
<400> 788
   Leu Asp Leu Lys Ile Val Lys Ala Arg Arg Leu Pro Asn Met Asp Met
   1 5 10 15
   Phe Ser Glu His Leu Arg Arg Leu Phe Thr Ala Cys Asn Ala Cys Ala
   20 25 30
   Arg Pro Thr Asp Thr Asp Asp Val Asp Pro Arg Asp Lys Gly Glu Phe
   35 40 45
   Gly Asp Lys Asn Ile Arg Ser His Arg Lys Val Ile Thr Ser Asp Pro
   50 55 60
   Tyr Val Thr Val Val Val Pro Gln Ala Thr Leu Ala Arg Thr Arg Val
   65 70 75 80
   Leu Lys Asn Ser Gln Glu Pro Leu Trp Asp Glu Lys Phe Asn Ile Ser
   85 90 95
   Ile Ala His Pro Phe Ala Tyr Leu Glu Phe Gln Val Lys Asp Asp Asp
   100 105 110
   Val Phe Gly Ala Gln Ile Ile Gly Thr Ala Lys
   115 120
<210> 789
   <211> 123
   <212> PRT
   <213> Artificial Sequence
<220>
   <223> synthetically generated peptide
<400> 789
   Leu Asp Leu Lys Ile Val Lys Ala Arg Arg Leu Pro Asn Met Asp Met
   1 5 10 15
   Phe Ser Glu His Leu Arg Arg Leu Phe Thr Ala Cys Asn Ala Cys Ala
   20 25 30
   Arg Pro Thr Asp Thr Asp Asp Val Asp Pro Arg Asp Lys Gly Glu Phe
   35 40 45
   Gly Asp Lys Asn Ile Arg Ser His Arg Lys Val Ile Thr Ser Asp Pro
   50 55 60
   Tyr Val Thr Val Val Val Pro Gln Ala Thr Leu Ala Arg Thr Arg Val
   65 70 75 80
   Leu Lys Asn Ser Gln Glu Pro Leu Trp Asp Glu Lys Phe Asn Ile Ser
   85 90 95
   Ile Ala His Pro Phe Ala Tyr Leu Glu Phe Gln Val Lys Asp Asp Asp
   100 105 110
   Val Phe Gly Ala Gln Ile Ile Gly Thr Ala Lys
   115 120
<210> 790
   <211> 123
   <212> PRT
   <213> Artificial sequence
<220>
   <223> Synthetically generated peptide
<400> 790
   Leu Asp Leu Lys Ile Val Lys Ala Arg Arg Leu Pro Asn Met Asp Met
   1 5 10 15
   Phe Ser Glu His Leu Arg Arg Leu Phe Thr Ala Cys Asn Ala Cys Ala
   20 25 30
   Arg Pro Thr Asp Thr Asp Asp Val Asp Pro Arg Asp Lys Gly Glu Phe
   35 40 45
   Gly Asp Lys Asn Ile Arg Ser His Arg Lys Val Ile Thr Ser Asp Pro
   50 55 60
   Tyr Val Thr Val Val Val Pro Gln Ala Thr Leu Ala Arg Thr Arg Val
   65 70 75 80
   Leu Lys Asn Ser Gln Glu Pro Leu Trp Asp Glu Lys Phe Asn Ile Ser
   85 90 95
   Ile Ala His Pro Phe Ala Tyr Leu Glu Phe Gln Val Lys Asp Asp Asp
   100 105 110
   Val Phe Gly Ala Gln Ile Ile Gly Thr Ala Lys
   115 120
<210> 791
   <211> 113
   <212> PRT
   <213> Artificial sequence
<220>
   <223> Synthetically generated peptide
<400> 791
   Leu Asp Leu Trp Val Val Glu Ala Arg Leu Leu Pro Asn Met Asp Met
   1 5 10 15
   Phe Ser Glu His Val Arg Arg Cys Phe Ala Ala Cys Lys Pro Pro Thr
   20 25 30
   Ser Cys Ala Thr Ala Arg Gln Pro Arg His Arg His His Arg Arg Lys
   35 40 45
   Ile Ile Thr Ser Asp Pro Tyr Val Thr Leu Ser Val Ala Gly Ala Val
   50 55 60
   Val Ala Arg Thr Arg Val Ile Pro Asn Asp Gln Asp Pro Val Trp Asp
   65 70 75 80
   Glu Arg Phe Ala Val Pro Leu Ala His Tyr Ala Ala Ala Leu Glu Phe
   85 90 95
   His Val Lys Asp Asn Asp Thr Phe Gly Ala Gln Leu Ile Gly Thr Val
   100 105 110
   Thr
<210> 792
   <211> 114
   <212> PRT
   <213> Artificial sequence
<220>
   <223> Synthetically generated peptide
<400> 792
   Leu Asp Ile Trp Ile Thr Glu Ala Lys Cys Leu Pro Asn Met Asp Ile
   1 5 10 15
   Met Ser Met Arg Arg Phe Phe Thr Gly Tyr Gly Ala Cys Gly Ser Ser
   20 25 30
   Cys Ala Gly Asp Asn Ala Arg Arg Gly Gly Val Gly Val Arg Pro Lys
   35 40 45
   Lys Ile Ile Thr Ser Asp Pro Tyr Val Ser Val Cys Leu Ala Gly Ala
   50 55 60
   Thr Val Ala Gln Thr Arg Val Ile Pro Asn Ser Glu Asn Pro Arg Trp
   65 70 75 80
   Glu Glu Arg Phe Arg Val Glu Val Ala His Ala Val Ser Arg Leu Glu
   85 90 95
   Phe His Val Lys Asp Asn Asp Val Phe Gly Ala Gln Leu Ile Gly Val
   100 105 110
   Ala Ser
<210> 793
   <211> 116
   <212> PRT
   <213> Artificial sequence
<220>
   <223> Synthetically generated peptide
<400> 793
   Leu Asp Ile Trp Ile Thr Glu Ala Lys Cys Leu Pro Asn Met Asp Ile
   1 5 10 15
   Met Ser Met Arg Arg Phe Phe Thr Gly Tyr Gly Ala Cys Gly Ser Ser
   20 25 30
   Cys Gly Gly Thr Gly Asp Asn Ala Arg Arg Ala Gly Gly Gly Val Arg
   35 40 45
   Pro Lys Lys Ile Ile Thr Ser Asp Pro Tyr Val Ser Val Cys Leu Ala
   50 55 60
   Gly Ala Thr Val Ala Gln Thr Arg Val Ile Pro Asn Ser Glu Asn Pro
   65 70 75 80
   Arg Trp Glu Glu Arg Phe Arg Val Glu Val Ala His Ala Val Ser Arg
   85 90 95
   Leu Glu Phe His Val Lys Asp Asn Asp Val Phe Gly Ala Gln Leu Ile
   100 105 110
   Gly Val Ala Ser
   115
<210> 794
   <211> 111
   <212> PRT
   <213> Artificial Sequence
<220>
   <223> Synthetically generated peptide
<400> 794
   Leu Asp Ile Gln Ile Val Glu Ala Lys Cys Leu Pro Asn Met Asp Leu
   1 5 10 15
   Met Thr Glu Arg Met Arg Lys Cys Phe Thr Gly Tyr Gly Ala Cys Ser
   20 25 30
   Thr Glu Cys Gly Lys Ser Asp Pro His Thr Asp Val Arg Lys Ile Ile
   35 40 45
   Thr Ser Asp Pro Tyr Val Ser Val Cys Leu Ser Gly Ala Thr Val Ala
   50 55 60
   Gln Thr Arg Val Ile Ala Asn Ser Glu Asn Pro Lys Trp Asp Glu His
   65                  70                  75                  80
   Phe Tyr Val Gln Val Ala His Ser Val Ser Arg Val Glu Phe His Val
                   85                  90                  95
   Lys Asp Asn Asp Val Phe Gly Ala Glu Leu Ile Gly Val Ala Ser
               100                 105                 110
<210> 795
   <211> 112
   <212> PRT
   <213> Artificial Sequence
<220>
   <223> Synthetically generated peptide
<400> 795
   Leu Asp Leu Phe Ile Ile Glu Ala Lys Ser Leu Pro Asn Leu Asp Leu
    1               5                  10                  15
   Ser Thr Glu Ala Ile Arg Lys Cys Leu Thr Met Gly Asn Ser Cys Thr
               20                  25                  30
   Pro Pro Phe Val Lys Gly Leu Lys Thr His Ser Gly Lys Asp Lys Ile
           35                  40                  45
   Ile Thr Ser Asp Pro Tyr Val Ser Ile Cys Leu Ala Gly Ala Thr Ile
       50                  55                  60
   Ala Gln Thr Arg Val Ile Pro Asn Cys Glu Asn Pro Leu Trp Asp Glu
   65                  70                  75                  80
   His Phe Leu Val Pro Val Ala His Pro Ala His Lys Ile Glu Phe Leu
                   85                  90                  95
   Val Lys Asp Asn Asp Ile Leu Gly Ala Glu Leu Ile Gly Val Val Glu
               100                 105                 110
<210> 796
   <211> 85
   <212> PRT
   <213> Artificial sequence
<220>
   <223> synthetically generated peptide
<400> 796
   Leu Met Val Glu Leu Leu His Gly Arg Arg Ile Arg Lys Val Asp Gly
    1               5                  10                  15
   Glu Lys Ser Ser Lys Phe Thr Ser Asp Pro Tyr Val Thr Val Ser Ile
               20                  25                  30
   Ser Gly Ala Val Ile Gly Arg Thr Phe Val Ile Ser Asn Ser Glu Asn
           35                  40                  45
   Pro Val Trp Met Gln His Phe Asp Val Pro Val Ala His Ser Ala Ala
       50                  55                  60
   Glu Val His Phe Val Val Lys Asp Asn Asp Pro Ile Gly Ser Lys Ile
   65                  70                  75                  80
   Ile Gly Val Val Gly
                   85
<210> 797
   <211> 108
   <212> PRT
   <213> Artificial Sequence
<220>
   <223> synthetically generated peptide
<400> 797
   Leu Asp Ile Trp Val Lys Glu Ala Lys His Leu Pro Asn Met Ile Cys
    1               5                  10                  15
   Tyr Arg Asn Lys Leu Val Gly Gly Ile Ser Phe Ser Glu Leu Gly Arg
                   20                  25                  30
   Arg Ile Arg Lys Val Asp Gly Glu Lys Ser Ser Lys Phe Thr Ser Asp
           35                  40                  45
   Pro Tyr Val Thr Val Ser Ile Ser Gly Ala Val Ile Gly Arg Thr Phe
       50                  55                  60
   Val Ile Ser Asn Ser Glu Asn Pro Val Trp Met Gln His Phe Asp Val
   65                  70                  75                  80
   Pro Val Ala His Ser Ala Ala Glu Val His Phe Val Val Lys Asp Asn
                   85                  90                  95
   Asp Pro Ile Gly Ser Lys Ile Ile Gly Val Val Gly
               100                 105
<210> 798
   <211> 104
   <212> PRT
   <213> Artificial Sequence
<220>
   <223> Synthetically generated peptide
<400> 798
   Leu Asp Ile Trp Val Lys Glu Ala Lys His Leu Pro Asn Met Asp Gly
    1               5                  10                  15
   Phe His Asn Arg Leu Gly Gly Met Leu Ser Gly Leu Gly Arg Lys Lys
               20                  25                  30
   Val Glu Gly Glu Lys Ser Ser Lys Ile Thr Ser Asp Pro Tyr Val Thr
           35                  40                  45
   Val Ser Ile Ser Gly Ala Val Ile Gly Arg Thr Phe Val Ile Ser Asn
       50                  55                  60
   Ser Glu Asn Pro Val Trp Met Gln His Phe Asp Val Pro Val Ala His
   65                  70                  75                  80
   Ser Ala Ala Glu Val His Phe Val Val Lys Asp Ser Asp Ile Ile Gly
                   85                  90                  95
   Ser Gln Ile Met Gly Ala Val Gly
               100
<210> 799
   <211> 107
   <212> PRT
   <213> Artificial sequence
<220>
   <223> Synthetically generated peptide
<400> 799
   Leu Asp Ile Trp Val Lys Glu Ala Lys His Leu Pro Asn Met Asp Gly
   1               5                   10                  15
   Phe His Asn Thr Leu Val Gly Gly Met Phe Phe Gly Leu Gly Arg Arg
               20                  25                  30
   Asn His Lys Val Asp Gly Glu Asn Ser Ser Lys Ile Thr Ser Asp Pro
           35                  40                  45
   Tyr Val Thr Val Ser Ile Ser Gly Ala Val Ile Gly Arg Thr Phe Val
       50                  55                  60
   Ile Ser Asn Ser Glu Asn Pro Val Trp Met Gln His Phe Asp Val Pro
   65                  70                  75                  80
   Val Ala His Ser Ala Ala Lys Val His Phe Val Val Lys Asp Ser Asp
                   85                  90                  95
   Ile Ile Gly Ser Gln Ile Ile Gly Ala Val Glu
               100                 105
<210> 800
   <211> 104
   <212> PRT
   <213> Artificial Sequence
<220>
   <223> Synthetically generated peptide
<400> 800
   Leu Asp Ile Trp Val Arg Glu Ala Lys Asn Leu Pro Asn Met Asp Leu
   1               5                   10                  15
   Phe His Lys Lys Leu Asp Asn Leu Leu Gly Gly Leu Ala Lys Leu Gly
               20                  25                  30
   Ser Lys Lys Glu Gly Ser Pro Lys Ile Thr Ser Asp Pro Tyr Val Thr
           35                  40                  45
   Val Ser Val Ser Asn Ala Val Val Ala Arg Thr Tyr Val Ile Asn Asn
       50                  55                  60
   Ser Glu Asn Pro Ile Trp Met Gln His Phe Tyr Val Pro Val Ala His
   65                  70                  75                  80
   Tyr Ala Ser Glu Val His Phe Val Val Lys Asp Asn Asp Val Val Gly
                   85                  90                  95
   Ser Gln Ile Ile Gly Ala Val Gly
               100
<210> 801
   <211> 103
   <212> PRT
   <213> Artificial sequence
<220>
   <223> Synthetically generated peptide
<400> 801
   Leu Asp Ile Trp Val Ser Cys Ala Asn Asn Leu Pro Asn Leu Asp Leu
   1 5 10 15
   Phe His Lys Thr Leu Gly Val Val Phe Gly Gly Met Thr Asn Met Ile
   20 25 30
   Glu Gly Gln Leu Ser Lys Lys Ile Thr Ser Asp Pro Tyr Val Ser Ile
   35 40 45
   Ser Val Ala Gly Ala Val Ile Gly Arg Thr Tyr Val Ile Ser Asn Ser
   50 55 60
   Glu Asn Pro Val Trp Gln Gln His Phe Tyr Val Pro Val Ala His His
   65 70 75 80
   Ala Ala Glu Val His Phe Val Val Lys Asp Ser Asp Ala Val Gly Ser
   85 90 95
   Gln Leu Ile Gly Ile Val Thr
   100
<210> 802
   <211> 103
   <212> PRT
   <213> Artificial sequence
<220>
   <223> Synthetically generated peptide
<400> 802
   Leu Asp Ile Trp Ile Tyr His Ala Lys Asn Leu Pro Asn Met Asp Met
   1 5 10 15
   Phe His Lys Thr Leu Gly Asp Met Phe Gly Arg Leu Pro Gly Lys Ile
   20 25 30
   Glu Gly Gln Leu Thr Ser Lys Ile Thr Ser Asp Pro Tyr Val Ser Val
   35 40 45
   Ser Val Ala Gly Ala Val Ile Gly Arg Thr Tyr Val Met Ser Asn Ser
   50 55 60
   Glu Asn Pro Val Trp Met Gln His Phe Tyr Val Pro Val Ala His His
   65 70 75 80
   Ala Ala Glu Val His Phe Val Val Lys Asp Ser Asp Val Val Gly Ser
   85 90 95
   Gln Leu Ile Gly Leu Val Thr
   100
<210> 803
   <211> 95
   <212> PRT
   <213> Artificial Sequence
<220>
   <223> Synthetically generated peptide
<400> 803
   Leu Glu Ile Trp Val Tyr Glu Ala Lys Asn Leu Pro Asn Met Asp Met
   1 5 10 15
   Phe His Lys Thr Ile Gly Asp Met Phe Gly Gln Met Ser Asn Lys Ile
   20 25 30
   Thr Ser Asp Pro Tyr Val Ser Ile Asn Ile Ala Asp Ala Thr Ile Gly
   35 40 45
   Arg Thr Tyr Val Ile Asn Asn Asn Glu Asn Pro Val Trp Met Gln His
   50 55 60
   Phe Asn Val Pro Val Ala His Tyr Ala Ala Glu Val Gln Phe Leu Val
   65 70 75 80
   Lys Asp Asp Asp Ile Val Gly Ser Gln Leu Met Gly Thr Val Ala
   85 90 95
<210> 804
   <211> 107
   <212> PRT
   <213> Artificial Sequence
<220>
   <223> Synthetically generated peptide
<220>
   <221> VARIANT
   <222> 86
   <223> Xaa = any amino acid
<400> 804
   Leu Asp Ile Trp Val Leu Glu Ala Asn Asn Leu Pro Asn Met Asp Met
   1 5 10 15
   Phe His Arg Thr Leu Gly Asp Met Phe Ala Asn Phe Ser Ser Asn Ile
   20 25 30
   Ser Lys Lys Val Gly Gly Arg Ser Asp Glu Lys Ile Thr Ser Asp Pro
   35 40 45
   Tyr Val Thr Ile Ala Val Ala Gly Ala Val Ile Gly Arg Thr Phe Val
   50 55 60
   Ile Ser Asn Asn Glu Asn Pro Val Trp Met Gln His Phe Asn Val Pro
   65 70 75 80
   Val Ala His His Ala Xaa Glu Val Gln Phe Val Val Lys Asp Ser Asp
   85 90 95
   Ile Leu Gly Ser Asp Ile Ile Gly Val Val Ala
   100 105
<210> 805
   <211> 107
   <212> PRT
   <213> Artificial Sequence
<220>
   <223> Synthetically generated peptide
<400> 805
   Leu Asp Ile Trp Val Leu Glu Ala Asn Asn Leu Pro Asn Met Asp Met
   1 5 10 15
   Phe His Arg Thr Leu Gly Asp Met Phe Ala Asn Phe Ser Ser Asn Ile
   20 25 30
   Ser Lys Lys Val Gly Gly Arg Ser Asp Glu Lys Ile Thr Ser Asp Pro
   35 40 45
   Tyr Val Thr Ile Ala Val Ala Gly Ala Val Ile Gly Arg Thr Phe Val
   50 55 60
   Ile Ser Asn Asn Glu Asn Pro Val Trp Met Gln His Phe Asn Val Pro
   65 70 75 80
   Val Ala His His Ala Ala Glu Val Gln Phe Val Val Lys Asp Ser Asp
   85 90 95
   Ile Leu Gly Ser Asp Ile Ile Gly Val Val Ala
   100 105
<210> 806
   <211> 107
   <212> PRT
   <213> Artificial Sequence
<220>
   <223> Synthetically generated peptide
<400> 806
   Leu Asp Ile Trp Val Leu Glu Ala Lys Asn Leu Pro Asn Met Asp Met
   1 5 10 15
   Phe His Lys Thr Leu Gly Asp Met Phe Gly Asn Phe Ser Ser Asn Ile
   20 25 30
   Ser Lys Lys Ile Gly Gly Arg Ser Glu Gly Lys Asn Thr Ser Asp Pro
   35 40 45
   Tyr Val Thr Ile Ala Val Ser Gly Ala Val Ile Gly Arg Thr Phe Val
   50 55 60
   Ile Asn Asn Asp Glu Asn Pro Val Trp Arg Gln His Phe Tyr Val Pro
   65 70 75 80
   Val Ala His His Ala Ala Glu Val Gln Phe Val Val Lys Asp Ile Asp
   85 90 95
   Ile Leu Gly Ser Glu Ile Ile Gly Val Val Thr
   100 105
<210> 807
   <211> 105
   <212> PRT
   <213> Artificial Sequence
<220>
   <223> Synthetically generated peptide
<400> 807
   Leu Asp Val Trp Val Tyr Asp Ala Arg Asn Leu Pro Asn Lys Asp Leu
   1               5                   10                  15
   Phe Ser Lys Arg Val Gly Asp Leu Leu Gly Pro Arg Leu Ile Gly Ala
               20                  25                  30
   Val Gly Ser Lys Met Ser Ser Ala Asn Met Thr Ser Asp Pro Tyr Val
           35                  40                  45
   Thr Ile Gln Val Ser Tyr Ala Thr Val Ala Arg Thr Tyr Val Val Pro
       50                  55                  60
   Asn Asn Glu Asn Pro Val Trp Thr Gln Asn Phe Leu Val Pro Val Gly
   65                  70                  75                  80
   His Asp Ala Ala Glu Val Glu Phe Val Val Lys Asp Asn Asp Val Phe
                   85                  90                  95
   Gly Ala Gln Leu Ile Gly Thr Val Ser
               100                 105
<210> 808
   <211> 105
   <212> PRT
   <213> Artificial Sequence
<220>
   <223> synthetically generated peptide
<400> 808
   Leu Asp Val Trp Val Tyr Asp Ala Arg Asn Leu Pro Asn Lys Asp Leu
   1               5                   10                  15
   Phe Ser Lys Arg Val Gly Asp Leu Leu Gly Pro Arg Leu Ile Gly Ala
               20                  25                  30
   Val Gly Ser Lys Met Ser Ser Ala Asn Met Thr Ser Asp Pro Tyr Val
           35                  40                  45
   Thr Ile Gln Val Ser Tyr Ala Thr Val Ala Arg Thr Tyr Val Val Pro
       50                  55                  60
   Asn Asn Glu Asn Pro Val Trp Thr Gln Asn Phe Leu Val Pro Val Gly
   65                  70                  75                  80
   His Asp Ala Ala Glu Val Glu Phe Val Val Lys Asp Asn Asp Val Phe
                   85                  90                  95
   Gly Ala Gln Leu Ile Gly Thr Val Ser
               100                 105
<210> 809
   <211> 101
   <212> PRT
   <213> Artificial Sequence
<220>
   <223> Synthetically generated peptide
<400> 809
   Leu Asp Ile Trp Ile His Glu Ala Arg Asn Leu Pro Asn Met Asp Ile
   1 5 10 15
   Val Ser Lys Thr Val Val Asp Ile Leu Gly Thr Lys Lys Lys Lys Lys
   20 25 30
   Ala Ala Asn Gly Ala Met Thr Ser Asp Pro Tyr Val Thr Val Gln Leu
   35 40 45
   Ala Ser Ala Thr Val Ala Arg Thr Tyr Val Val Asn Asp Asp Glu Asn
   50 55 60
   Pro Val Trp Ala Gln His Phe Leu Ile Pro Val Ala His Glu Ala Pro
   65 70 75 80
   Ala Val His Phe Leu Val Lys Asp Ser Asp Val Phe Gly Ala Glu Leu
   85 90 95
   Ile Gly Glu Val Val
   100
<210> 810
   <211> 101
   <212> PRT
   <213> Artificial Sequence
<220>
   <223> Synthetically generated peptide
<400> 810
   Leu Asp Ile Trp Ile His Glu Ala Arg Asn Leu Pro Asn Met Asp Ile
   1 5 10 15
   Val Ser Lys Thr Val Val Asp Ile Leu Gly Thr Lys Lys Lys Lys Lys
   20 25 30
   Ala Ala Asn Gly Ala Met Thr Ser Asp Pro Tyr Val Thr Val Gln Leu
   35 40 45
   Ala Ser Ala Thr Val Ala Arg Thr Tyr Val Val Asn Asp Asp Glu Asn
   50 55 60
   Pro Val Trp Ala Gln His Phe Leu Ile Pro Val Ala His Glu Ala Pro
   65 70 75 80
   Ala Val His Phe Leu Val Lys Asp Ser Asp Val Phe Gly Ala Glu Leu
   85 90 95
   Ile Gly Glu Val Val
   100
<210> 811
   <211> 122
   <212> PRT
   <213> Artificial sequence
<220>
   <223> Synthetically generated peptide
<400> 811
   Leu Asp Leu Thr Ile His Glu Arg Leu Pro Asn Met Asp Phe Leu Ser
   1               5                   10                  15
   Thr Leu Leu Arg Arg Leu Cys Leu Cys Leu Arg Pro Pro Ala Arg Arg
               20                  25                  30
   Pro Ser Pro Gly Gln Ser Arg Gly Ser Val Pro Ala Asp Glu Asp Gly
           35                  40                  45
   Arg Arg Gln Pro His Gly His His Leu Leu Pro Thr Ser Asp Pro Tyr
       50                  55                  60
   Ala Ala Val Val Val Ala Gly Asn Thr Leu Ala Arg Thr His Val Val
   65                  70                  75                  80
   Arg Asp Ser Glu Asp Pro Glu Trp Ser Thr His Val Leu Leu His Leu
                   85                  90                  95
   Ala His His Ala Thr Gly Val Ala Phe His Val Lys Asp Ala Asp Pro
               100                 105                 110
   Phe Gly Ser Asp Leu Ile Gly Val Ala Ile
           115                 120
<210> 812
   <211> 64
   <212> PRT
   <213> Artificial sequence
<220>
   <223> Synthetically generated peptide
<400> 812
   Tyr Val Asn Ile Gln Phe Gly Asp Gln Ile Phe Thr Ser Lys Ile Thr
   1               5                   10                  15
   Gln Gly Lys Gly Lys Lys Val Trp Trp Asn Glu Lys Phe Arg Phe Pro
               20                  25                  30
   Leu Ser Ser Asp Glu Cys Lys Glu Leu Ala Lys Val Thr Leu Lys Ile
           35                  40                  45
   Met Glu Arg Asp Lys Phe Ser Glu Asp Ser Leu Val Gly Glu Thr Lys
       50                  55                  60
<210> 813
   <211> 64
   <212> PRT
   <213> Artificial Sequence
<220>
   <223> Synthetically generated peptide
<400> 813
   Leu Asp Val Thr Leu Lys Ser Ala Ser Asp Leu Arg Glu Asp Met Ser
   1               5                   10                  15
   Val Lys Leu Asp Ala Tyr Cys Val Val Ser Cys Ala Ser Thr Ala His
               20                  25                  30
   Arg Ser Asn Thr Val Thr Asp Ala Gly Lys Thr Met Asn Trp Glu Gln
           35                  40                  45
   Thr Phe His Phe Asp Lys Val Ala Ser Thr Ser Val Leu Lys Leu Glu
       50                  55                  60
<210> 814
   <211> 92
   <212> PRT
   <213> Artificial Sequence
<220>
   <223> Synthetically generated peptide
<400> 814
   Leu Glu Ile Asn Val Thr Ser Ala Lys Gly Leu Lys Lys Val Ser Lys
   1               5                   10                  15
   Met Asp Val Phe Val Ala Val Lys Leu Ser Gly Asp Pro Lys Cys Ser
               20                  25                  30
   Asp His Arg Glu Gln Arg Thr Gln Ala Ala Arg Asp Gly Gly Thr Ser
           35                  40                  45
   Pro Lys Trp Ser Asn Asp Val Met Lys Phe Ile Leu Asp Gln Asn Leu
       50                  55                  60
   Ala Glu Ala Asn Arg Leu Val Ile Thr Phe Lys Ile Lys Cys Glu Gln
   65                  70                  75                  80
   Arg Gly Gly Val Asp Lys Asp Ile Gly Glu Val His
                   85                  90
<210> 815
   <211> 90
   <212> PRT
   <213> Artificial sequence
<220>
   <223> Synthetically generated peptide
<400> 815
   Val Glu Ile Asn Val Leu Ser Ala Gln Asp Leu Asn Ser Ile Asn Leu
   1               5                   10                  15
   Leu Phe Arg Pro Thr Val Tyr Val Ser Val Ser Val Thr Arg Gly Ser
               20                  25                  30
   Arg Asp Lys Gln Val Thr Pro Ala Ala Ala Trp Asp Lys Lys Phe Leu
           35                  40                  45
   Arg Trp Asn Tyr Arg Met Lys Phe Tyr Ile Glu Asp Asp Lys Val Arg
       50                  55                  60
   Arg Asn Glu Ser Val Phe Val Phe Gln Ile Lys Cys Lys Arg Phe Phe
   65                  70                  75                  80
   Gly Ser Asp Gln Val Val Gly Lys Leu Phe
                   85                  90
<210> 816
   <211> 82
   <212> PRT
   <213> Artificial sequence
<220>
   <223> Synthetically generated peptide
<400> 816
   Leu Tyr Val Tyr Ile Leu Gln Ala Lys Asp Leu Pro Ala Lys Glu Thr
   1               5                   10                  15
   Phe Ala Lys Leu His Val Gly Arg His Lys Ser Lys Thr Arg Val Ala
               20                  25                  30
   Arg Asp Thr Ser Ser Pro Ile Trp Asn Glu Glu Phe Val Phe Arg Ile
           35                  40                  45
   Ser Asp Val Asp Glu Gly Asp Asp Val Val Val Ser Ile Leu His His
       50                  55                  60
   Glu Gln Gln Asp His Gln Ser Ile Val Ser Thr Gly Leu Ile Gly Lys
   65                  70                  75                  80
   Val Arg
<210> 817
   <211> 84
   <212> PRT
   <213> Artificial Sequence
<220>
   <223> Synthetically generated peptide
<400> 817
   Leu Tyr Ile Arg Val Ala Lys Ala Lys Arg Ala Lys Asn Asp Gly Ser
   1               5                   10                  15
   Asn Pro Val Tyr Ala Lys Leu Val Ile Gly Thr Asn Gly Val Lys Thr
               20                  25                  30
   Arg Ser Gln Thr Gly Lys Asp Trp Asp Gln Val Phe Ala Phe Glu Lys
           35                  40                  45
   Glu Ser Leu Asn Ser Thr Ser Leu Glu Val Ser Val Trp Ser Glu Glu
       50                  55                  60
   Lys Ile Glu Lys Glu Asp Lys Thr Thr Thr Thr Thr Glu Ser Cys Leu
   65                  70                  75                  80
   Gly Thr Val Ser
<210> 818
   <211> 102
   <212> PRT
   <213> Artificial Sequence
<220>
   <223> Synthetically generated peptide
<400> 818
   Met Thr Val Gln Ile Ala Lys Gly Leu His Ile Ile Asp Asp Gly Asn
   1               5                   10                  15
   Ser His Ser Phe Phe Cys Thr Leu Arg Leu Val Val Asp Ser Gln Gly
               20                  25                  30
   Ala Glu Pro Gln Lys Leu Phe Pro Gln Ser Ala Arg Thr Lys Cys Val
           35                  40                  45
   Lys Pro Ser Thr Thr Ile Val Asn Asp Leu Met Glu Cys Thr Ser Lys
       50                  55                  60
   Trp Asn Glu Leu Phe Ile Phe Glu Ile Pro Arg Lys Gly Val Ala Arg
   65                  70                  75                  80
   Leu Glu Val Glu Val Thr Asn Leu Ala Ala Lys Ala Gly Lys Gly Glu
                   85                  90                  95
   Val Val Gly Ser Leu Ser
               100
<210> 819
   <211> 99
   <212> PRT
   <213> Artificial Sequence
<220>
   <223> Synthetically generated peptide
<400> 819
   Ile Asp Leu Lys Ile Ile Ser Cys Lys Asp Ile Asn Ala Phe Asn Phe
    1               5                  10                  15
   Phe Gln Lys Leu Thr Leu Tyr Ala Gln Val Ser Ile Ser Thr Thr Asn
               20                  25                  30
   Pro Lys Thr Lys Leu Thr Lys Gln Gln Gln Arg Thr Pro Thr Asp Thr
           35                  40                  45
   Asp Asp Asp Gly Thr Asn Pro Glu Trp Asn His Gln Thr Arg Phe Asn
       50                  55                  60
   Leu Ser Phe Leu Ser Ser His Pro Asp Pro Ser Glu Phe Phe Leu Ser
   65                  70                  75                  80
   Phe Glu Phe Arg His Asp Gly Leu Ile Leu Gly Asn Lys Phe Leu Gly
                   85                  90                  95
   Glu Cys Arg
<210> 820
   <211> 84
   <212> PRT
   <213> Artificial Sequence
<220>
   <223> Synthetically generated peptide
<400> 820
   Val Thr Ile Arg Ser Ile Ser Cys Arg Gly Val Lys Ala Phe Val Pro
    1               5                  10                  15
   Phe Gln Lys Pro Pro Leu Tyr Ala Ala Val Ser Leu Ala Gly Arg Arg
               20                  25                  30
   Glu Lys Thr Ser Gly Asp Pro Asp Gly Gly Glu Asn Pro Asp Trp Asp
           35                  40                  45
   Ala Ala Val Phe Ala Phe Asp Leu Pro Ala Ala Ala Asp Gly Met Leu
       50                  55                  60
   Gln Phe Glu Val Lys Ala Gln Val Pro Leu Leu Gly Ser Lys Leu Val
   65                  70                  75                  80
   Gly Lys Val Ser
<210> 821
   <211> 91
   <212> PRT
   <213> Artificial sequence
<220>
   <223> Synthetically generated peptide
<400> 821
   Phe Glu Leu Arg Ile Ile Gln Ala Arg Asn Ile Glu Ser Val Lys Ser
   1 5 10 15
   Thr Lys Asn Leu Phe Ala Arg Leu Tyr Leu Pro Thr Gly Asn Asn Lys
   20 25 30
   Arg Ile Gln Leu Asn Ser Lys Ser Val Ser Thr Lys Ser Val Pro Phe
   35 40 45
   Trp Asp Glu Ser Phe Asn Leu Asp Cys Ser Cys Pro Gln Glu Phe Leu
   50 55 60
   Glu Asn Leu Asn Gln Gln Ser Leu Glu Val Glu Leu Arg Gln Arg Lys
   65 70 75 80
   Ile Trp Gly Ser Gln Leu Ile Gly Lys Phe Glu
   85 90
<210> 822
   <211> 102
   <212> PRT
   <213> Oryza sativa
<400> 822
   Met Ser Ser Asp Gly Gly Pro Val Leu Gly Gly Val Glu Pro Val Gly
   1 5 10 15
   Asn Glu Asn Asp Leu His Leu Val Asp Leu Ala Arg Phe Ala Val Thr
   20 25 30
   Glu His Asn Lys Lys Ala Asn Ser Leu Leu Glu Phe Glu Lys Leu Val
   35 40 45
   Ser Val Lys Gln Gln Val Val Ala Gly Thr Leu Tyr Tyr Phe Thr Ile
   50 55 60
   Glu Val Lys Glu Gly Asp Ala Lys Lys Leu Tyr Glu Ala Lys Val Trp
   65 70 75 80
   Glu Lys Pro Trp Met Asp Phe Lys Glu Leu Gln Glu Phe Lys Pro Val
   85 90 95
   Asp Ala Ser Ala Asn Ala
   100
<210> 823
   <211> 134
   <212> PRT
   <213> Zea mays
<400> 823
   Met Arg Lys His Arg Ile Val Ser Leu Val Ala Ala Leu Leu Ile Leu
   1               5                   10                  15
   Leu Ala Leu Ala Val Ser Ser Thr Arg Asn Ala Gln Glu Asp Ser Met
               20                  25                  30
   Ala Asp Asn Thr Gly Thr Leu Ala Gly Gly Ile Lys Asp Val Pro Gly
           35                  40                  45
   Asn Glu Asn Asp Leu His Leu Gln Glu Leu Ala Arg Phe Ala Val Asp
       50                  55                  60
   Glu His Asn Lys Lys Ala Asn Ala Leu Leu Gly Phe Glu Lys Leu Val
   65                  70                  75                  80
   Lys Ala Lys Thr Gln Val Val Ala Gly Thr Met Tyr Tyr Leu Thr Ile
                   85                  90                  95
   Glu Val Lys Asp Gly Glu Val Lys Lys Leu Tyr Glu Ala Lys Val Trp
               100                 105                 110
   Glu Lys Pro Trp Glu Asn Phe Lys Glu Leu Gln Glu Phe Lys Pro Val
           115                 120                 125
   Asp Glu Gly Ala Ser Ala
       130
<210> 824
   <211> 125
   <212> PRT
   <213> Triticum aestivum
<400> 824
   Leu Leu Ala Ile Val Val Pro Phe Thr Gln Thr Arg Thr Gln Ser Ala
   1               5                   10                  15
   Arg Asp Lys Ala Ala Met Ala Glu Asp Ala Gly Pro Leu Val Gly Gly
               20                  25                  30
   Ile Lys Asp Ser Pro Met Gly Gln Glu Asn Asp Leu Asp Val Ile Ala
           35                  40                  45
   Leu Ala Arg Phe Ala Val Ser Glu His Asn Asn Lys Ala Asn Ala Leu
       50                  55                  60
   Leu Glu Phe Glu Asn Val Val Lys Leu Lys Lys Gln Thr Val Ala Gly
   65                  70                  75                  80
   Thr Met His Tyr Ile Thr Ile Arg Val Thr Glu Gly Gly Ala Lys Lys
                   85                  90                  95
   Leu Tyr Glu Ala Lys Val Trp Glu Lys Pro Trp Glu Asn Phe Lys Gln
               100                 105                 110
   Leu Gln Glu Phe Lys Pro Val Glu Asp Ala Ala Ile Ala
           115                 120                 125
<210> 825
   <211> 140
   <212> PRT
   <213> Hordeum vulgare subsp. vulgare
<400> 825
   Met Trp Lys Tyr Arg Val Leu Gly Ser Val Ala Ala Leu Leu Leu Leu
   1               5                   10                  15
   Leu Ala Val Val Val Pro Phe Thr Gln Thr Trp Thr Gln Ser Ala Arg
               20                  25                  30
   Asp Lys Ala Ala Met Ala Glu Asp Ala Gly Pro Leu Met Gly Gly Ile
           35                  40                  45
   Glu Asp Ser Pro Met Gly Gln Glu Asn Asp Leu Asp Val Ile Ala Leu
       50                  55                  60
   Ala Arg Phe Ala Val Ser Glu His Asn Lys Lys Ala Asn Ala Leu Leu
   65 70 75 80
   Glu Phe Glu Asn Val Val Lys Leu Lys Lys Gln Thr Val Ala Gly Thr
   85 90 95
   Met Tyr Tyr Ile Thr Ile Arg Val Thr Glu Gly Gly Thr Lys Lys Leu
   100 105 110
   Tyr Glu Ala Lys Val Trp Glu Lys Leu Trp Glu Asn Phe Lys Gln Leu
   115 120 125
   Glu Glu Phe Lys Pro Val Gln Asp Ala Ala Ile Ala
   130 135 140
<210> 826
   <211> 135
   <212> PRT
   <213> Coix lacryma-jobi
<400> 826
   Met Arg Lys Gln Arg Ile Val Pro Leu Val Ala Ala Leu Leu Val Leu
   1 5 10 15
   Leu Ala Leu Ala Val Ser Ser Thr Arg Asn Ala Arg Glu Glu Glu Ser
   20 25 30
   Met Ala Asp Asp Ala Gly Met Leu Ala Gly Gly Ile Lys Asp Val Pro
   35 40 45
   Ala Asn Glu Asn Asp Leu His Leu Gln Glu Leu Ala Arg Phe Ala Val
   50 55 60
   Asp Glu His Asn Lys Lys Ala Asn Ala Leu Leu Gly Tyr Glu Lys Leu
   65 70 75 80
   Val Lys Ala Lys Thr Gln Val Val Ala Gly Thr Met Tyr Tyr Leu Thr
   85 90 95
   Ile Glu Val Lys Asp Gly Glu Val Lys Lys Leu Tyr Glu Ala Lys Val
   100 105 110
   Trp Glu Lys Pro Trp Glu Asn Phe Lys Glu Leu Leu Glu Phe Lys Pro
   115 120 125
   Val Glu Glu Asp Ala Ser Ala
   130 135
<210> 827
   <211> 135
   <212> PRT
   <213> Zea mays
<400> 827
   Met Arg Lys His Arg Ile Val Ser Leu Val Ala Ala Leu Leu Val Leu
   1 5 10 15
   Leu Ala Leu Ala Ala Val Ser Ser Thr Arg Ser Thr Gln Lys Glu Ser
   20 25 30
   Val Ala Asp Asn Ala Gly Met Leu Ala Gly Gly Ile Lys Asp Val Pro
   35 40 45
   Ala Asn Glu Asn Asp Leu Gln Leu Gln Glu Leu Ala Arg Phe Ala Val
   50 55 60
   Asn Glu His Asn Gln Lys Ala Asn Ala Leu Leu Gly Phe Glu Lys Leu
   65 70 75 80
   Val Lys Ala Lys Thr Gln Val Val Ala Gly Thr Met Tyr Tyr Leu Thr
   85 90 95
   Ile Glu Val Lys Asp Gly Glu Val Lys Lys Leu Tyr Glu Ala Lys Val
   100 105 110
   Trp Glu Lys Pro Trp Glu Asn Phe Lys Gln Leu Gln Glu Phe Lys Pro
   115 120 125
   Val Glu Glu Gly Ala Ser Ala
   130 135
<210> 828
   <211> 130
   <212> PRT
   <213> Sorghum bicolor
<400> 828
   Ala Arg Val Val Pro Leu Val Ala Ala Leu Leu Val Leu Leu Ala Leu
   1 5 10 15
   Ala Val Ser Ser Thr Arg Asn Arg Asn Ala Gln Glu Gly Glu Glu Ser
   20 25 30
   Met Ala Leu Asp Gly Gly Ile Lys Asp Val Pro Ala Asn Glu Asn Asp
   35 40 45
   Leu His Leu Gln Glu Leu Ala Arg Phe Ala Val Asp Glu His Asn Lys
   50 55 60
   Lys Ala Asn Ala Leu Leu Gly Tyr Glu Lys Leu Val Lys Ala Lys Thr
   65 70 75 80
   Gln Val Val Ala Gly Thr Met Tyr Tyr Leu Thr Val Glu Val Lys Asp
   85 90 95
   Gly Glu Val Lys Lys Leu Tyr Glu Ala Lys Val Trp Glu Lys Pro Trp
   100 105 110
   Glu Asn Phe Lys Glu Leu Gln Glu Phe Lys Pro Val Glu Glu Gly Ala
   115 120 125
   Ser Ala
   130
<210> 829
   <211> 97
   <212> PRT
   <213> Arachis hypogaea
<400> 829
   Ala Ala Val Gly Ala Pro Arg Glu Val Ala Gly Asn Glu Asn Ser Leu
    1               5                  10                  15
   Glu Ile Asp Ser Leu Ala Arg Phe Ala Val Asp Glu His Asn Lys Lys
               20                  25                  30
   Gln Asn Gly Leu Leu Glu Phe Lys Arg Val Ile Ser Ala Lys Gln Gln
           35                  40                  45
   Val Val Ala Gly Thr Leu His His Ile Thr Leu Glu Ala Ala Ser Gly
       50                  55                  60
   Asp Ser Lys Asn Val Tyr Glu Ala Lys Val Trp Glu Lys Pro Trp Met
   65                  70                  75                  80
   Asn Phe Lys Glu Val Gln Glu Phe Lys Leu Ala Gly Asp Gly Ser Asn
                   85                  90                  95
   Ala
<210> 830
   <211> 101
   <212> PRT
   <213> Castanea sativa
<400> 830
   Ala Ala Leu Val Gly Gly Val Ser Asp Val Lys Gly His Glu Asn Ser
    1               5                  10                  15
   Leu Gln Ile Asp Asp Leu Ala Arg Phe Ala Val Asp Asp His Asn Lys
               20                  25                  30
   Lys Ala Asn Thr Leu Leu Gln Phe Lys Lys Val Val Asn Ala Lys Gln
           35                  40                  45
   Gln Val Val Ser Gly Thr Ile Tyr Ile Leu Thr Leu Glu Val Glu Asp
       50                  55                  60
   Gly Gly Lys Lys Lys Val Tyr Glu Ala Lys Ile Trp Glu Lys Pro Trp
   65                  70                  75                  80
   Leu Asn Phe Lys Glu Val Gln Glu Phe Lys Leu Ile Gly Asp Ala Pro
                   85                  90                  95
   Thr His His Ser Ala
               100
<210> 831
   <211> 105
   <212> PRT
   <213> Saccharum officinarum
<400> 831
   Ala Glu Ala His Asn Gly Arg Arg Val Gly Met Val Gly Asp Val Arg
    1               5                  10                  15
   Asp Ala Pro Ala Gly His Glu Asn Asp Leu Glu Ala Ile Glu Leu Ala
               20                  25                  30
   Arg Phe Ala Val Ala Glu His Asn Ser Lys Thr Asn Ala Met Leu Glu
           35                  40                  45
   Phe Glu Arg Leu Val Lys Val Arg His Gln Val Val Ala Gly Thr Met
       50                  55                  60
   His His Phe Thr Val Gln Val Lys Glu Ala Gly Gly Gly Lys Lys Leu
   65                  70                  75                  80
   Tyr Glu Ala Lys Val Trp Glu Lys Val Trp Glu Asn Phe Lys Gln Leu
                   85                  90                  95
   Gln Ser Phe Gln Pro Val Gly Asp Ala
               100                 105
<210> 832
   <211> 95
   <212> PRT
   <213> Vigna unguiculata
<400> 832
   Ala Ala Gly Gly Asn Arg Asp Val Ala Gly Asn Gln Asn Ser Leu Glu
   1               5                   10                  15
   Ile Asp Ser Leu Ala Arg Phe Ala Val Glu Glu His Asn Lys Lys Gln
               20                  25                  30
   Asn Ala Leu Leu Glu Phe Gly Arg Val Val Ser Ala Gln Gln Gln Val
           35                  40                  45
   Val Ser Gly Thr Leu Tyr Thr Ile Thr Leu Glu Ala Lys Asp Gly Gly
       50                  55                  60
   Gln Lys Lys Val Tyr Glu Ala Lys Val Trp Glu Lys Pro Trp Leu Asn
   65                  70                  75                  80
   Phe Lys Glu Leu Gln Glu Phe Lys His Val Gly Asp Ala Pro Ala
                   85                  90                  95
<210> 833
   <211> 156
   <212> PRT
   <213> Oryza sativa
<400> 833
   Met Ala Gly Ser Gly Val Leu Glu Val His Phe Val Asp Ala Lys Gly
   1               5                   10                  15
   Leu Thr Gly Asn Asp Phe Leu Gly Lys Ile Asp Pro Tyr Val Val Val
               20                  25                  30
   Gln Tyr Arg Ser Gln Glu Arg Lys Ser Ser Val Ala Arg Asp Gln Gly
           35                  40                  45
   Lys Asn Pro Ser Trp Asn Glu Val Phe Lys Phe Gln Ile Asn Ser Thr
       50                  55                  60
   Ala Ala Thr Gly Gln His Lys Leu Phe Leu Arg Leu Met Asp His Asp
   65                  70                  75                  80
   Thr Phe Ser Arg Asp Asp Phe Leu Gly Glu Ala Thr Ile Asn Val Thr
                   85                  90                  95
   Asp Leu Ile Ser Leu Gly Met Glu His Gly Thr Trp Glu Met Ser Glu
               100                 105                 110
   Ser Lys His Arg Val Val Leu Ala Asp Lys Thr Tyr His Gly Glu Ile
           115                 120                 125
   Arg Val Ser Leu Thr Phe Thr Ala Ser Ala Lys Ala Gln Asp His Ala
       130                 135                 140
   Glu Gln Val Gly Gly Trp Ala His Ser Phe Arg Gln
   145                 150                 155
<210> 834
   <211> 144
   <212> PRT
   <213> Oryza sativa
<400> 834
   Met Val Gln Gly Thr Leu Glu Val Leu Leu Val Gly Ala Lys Gly Leu
   1 5 10 15
   Glu Asn Thr Asp Tyr Leu Cys Asn Met Asp Pro Tyr Ala Val Leu Lys
   20 25 30
   Cys Arg Ser Gln Glu Gln Lys Ser Ser Val Ala Ser Gly Lys Gly Ser
   35 40 45
   Asp Pro Glu Trp Asn Glu Thr Phe Met Phe Ser Val Thr His Asn Ala
   50 55 60
   Thr Glu Leu Ile Ile Lys Leu Met Asp Ser Asp Ser Gly Thr Asp Asp
   65 70 75 80
   Asp Phe Val Gly Glu Ala Thr Ile Ser Leu Glu Ala Ile Tyr Thr Glu
   85 90 95
   Gly Ser Ile Pro Pro Thr Val Tyr Asn Val Val Lys Glu Glu Glu Tyr
   100 105 110
   Arg Gly Glu Ile Lys Val Gly Leu Thr Phe Thr Pro Glu Asp Asp Arg
   115 120 125
   Asp Arg Gly Leu Ser Glu Glu Asp Ile Gly Gly Trp Lys Gln Ser Ser
   130 135 140
<210> 835
   <211> 138
   <212> PRT
   <213> cucurbita maxima
<400> 835
   Met Gly Met Gly Met Met Glu Val His Leu Ile Ser Gly Lys Gly Leu
   1 5 10 15
   Gln Ala His Asp Pro Leu Asn Lys Pro Ile Asp Pro Tyr Ala Glu Ile
   20 25 30
   Asn Phe Lys Gly Gln Glu Arg Met Ser Lys Val Ala Lys Asn Ala Gly
   35 40 45
   Pro Asp Pro Ile Trp Asn Glu Lys Phe Lys Phe Leu Val Glu Tyr Pro
   50 55 60
   Gly Ser Gly Gly Asp Phe His Ile Leu Phe Lys Val Met Asp His Asp
   65 70 75 80
   Ala Ile Asp Gly Asp Asp Tyr Ile Gly Asp Val Lys Ile Asp Val Gln
   85 90 95
   Asn Leu Leu Ala Glu Gly Val Arg Lys Gly Trp Ser Glu Leu Pro Pro
   100 105 110
   Arg Met Tyr Gln Val Leu Ala His Lys Ile Tyr Phe Lys Gly Glu Ile
   115 120 125
   Glu Val Gly Val Phe Phe Gln Arg Gln Gly
   130 135
<210> 836
   <211> 150
   <212> PRT
   <213> Cucurbita maxima
<400> 836
   Met Gly Met Gly Met Met Glu Val His Leu Ile Ser Gly Lys Gly Leu
   1 5 10 15
   Gln Ala His Asp Pro Leu Asn Lys Pro Ile Asp Pro Tyr Ala Glu Ile
   20 25 30
   Asn Phe Lys Gly Gln Glu Arg Met Ser Lys Val Ala Lys Asn Ala Gly
   35 40 45
   Pro Asn Pro Leu Trp Asp Glu Lys Phe Lys Phe Leu Ala Glu Tyr Pro
   50 55 60
   Gly Ser Gly Gly Asp Phe His Ile Leu Phe Lys Val Met Asp His Asp
   65 70 75 80
   Ala Ile Asp Gly Asp Asp Tyr Ile Gly Asp Val Lys Ile Asp Val Lys
                   85                  90                  95
   Asn Leu Leu Ala Glu Gly Val Arg Lys Gly Lys Ser Glu Met Pro Pro
               100                 105                 110
   Arg Met Tyr His Val Leu Ala His Lys Ile His Phe Lys Gly Glu Ile
           115                 120                 125
   Glu Val Gly Val Ser Phe Lys Leu Gln Gly Gly Gly Gly Cys Gly Gly
       130                 135                 140
   Cys Tyr Pro Trp Glu Asn
   145                 150
<210> 837
   <211> 147
   <212> PRT
   <213> Arabidopsis thaliana
<400> 837
   Met Pro His Gly Thr Leu Glu Val Val Leu Val Ser Ala Lys Gly Leu
   1               5                   10                  15
   Glu Asp Ala Asp Phe Leu Asn Asn Met Asp Pro Tyr Val Gln Leu Thr
               20                  25                  30
   Cys Arg Thr Gln Asp Gln Lys Ser Asn Val Ala Glu Gly Met Gly Thr
           35                  40                  45
   Thr Pro Glu Trp Asn Glu Thr Phe Ile Phe Thr Val Ser Glu Gly Thr
       50                  55                  60
   Thr Glu Leu Lys Ala Lys Ile Phe Asp Lys Asp Val Gly Thr Glu Asp
   65                  70                  75                  80
   Asp Ala Val Gly Glu Ala Thr Ile Pro Leu Glu Pro Val Phe Val Glu
                   85                  90                  95
   Gly Ser Ile Pro Pro Thr Ala Tyr Asn Val Val Lys Asp Glu Glu Tyr
               100                 105                 110
   Lys Gly Glu Ile Trp Val Ala Leu Ser Phe Lys Pro Ser Glu Asn Arg
           115                 120                 125
   Ser Arg Gly Met Asp Glu Glu Ser Tyr Gly Gly Trp Lys Asn Ser Glu
       130                 135                 140
   Ala Ser Tyr
   145